# EUROPEAN PATENT APPLICATION

(11) **EP 4 063 864 A2**
(43) Date of publication of application: **28.09.2022**
(21) Application number: 22163971.9
(22) Date of filing: 24.03.2022
(51) Int. Cl.: G01N 33/564, C07K 14/00, C07K 16/00, G01N 33/68

(54) **METHOD AND MEANS FOR DETECTING AN AUTOANTIBODY**

(30) Priority: 25.03.2021 EP 21165011
(71) Applicant: Euroimmun Medizinische Labordiagnostika AG, 23560 Lübeck (DE)
(72) Inventor: ZENG, Zitao, 23560 Lübeck (DE); KOMOROWSKI, Lars, 23909 Ratzeburg (DE); MISKE, Ramona, 23564 Lübeck (DE); SCHARF, Madeleine, 23923 Selmsdorf (DE); DENNO, Yvonne, 23558 Lübeck (DE); RADZIMSKI, Christiane, 23858 Reinfeld (DE); ROCHOW, Nadine, 23923 Selmsdorf (DE); BRAKOPP, Stefanie, 19243 Wittenburg OT Lehsen (DE)

(57) **Abstract**

The present invention relates to a method for detecting in a sample an autoantibody binding to a polypeptide having the sequence SEQ ID NO1, SEQ ID NO11 or SEQ ID NO24, a carrier comprising said polypeptide, an autoantibody binding said polypeptide, a use of the autoantibody for the diagnosis of a disease, a method for isolating an autoantibody binding to said polypeptide, a kit comprising said polypeptide, a use of said polypeptide, said carrier or said autoantibody for the manufacture of a kit or medical device, a method comprising the step contacting a medical or diagnostic device comprising said polypeptide with a buffered solution comprising an antibody binding specifically to said polypeptide, a use of a sample comprising said autoantibody as a positive control and a diluted sample comprising said autoantibody.

SEQUENCE LISTING

<110>  EUROIMMUN Medizinische Labordiagnostika AG

<120>  Method and means for detecting an autoantibody

<130>  20PP048EP2

<160>  93

<170>  PatentIn version 3.5

<210>  1
<211>  856
<212>  PRT
<213>  Homo sapiens

<400>  1

Met Lys Pro Arg Pro Ala Gly Phe Val Asp Asn Lys Leu Lys Gln Arg
1               5                   10                  15

Val Ile Gln Tyr Leu Thr Ser Asn Lys Cys Gly Lys Tyr Val Asp Ile
            20                  25                  30

Gly Val Leu Ala Ser Asp Leu Gln Arg Val Tyr Ser Ile Asp Tyr Gly
        35                  40                  45

Arg Arg Lys Arg Asn Ala Phe Arg Ile Gln Val Glu Lys Val Phe Ser
    50                  55                  60

Ile Ile Ser Ser Glu Lys Glu Leu Lys Asn Leu Thr Glu Leu Glu Asp
65                  70                  75                  80

Glu His Leu Ala Lys Arg Ala Arg Gln Gly Glu Glu Asp Asn Glu Tyr
                85                  90                  95

Thr Glu Ser Tyr Ser Asp Asp Asp Ser Ser Met Glu Asp Tyr Pro Asp
            100                 105                 110

Pro Gln Ser Ala Asn His Met Asn Ser Ser Leu Leu Ser Leu Tyr Arg
        115                 120                 125

Lys Gly Asn Pro Asp Ser Val Ser Asn Thr Pro Glu Met Glu Gln Arg
    130                 135                 140

Glu Thr Thr Ser Ser Thr Pro Arg Ile Ser Ser Lys Thr Gly Ser Ile
145                 150                 155                 160

Pro Leu Lys Thr Pro Ala Lys Asp Ser Glu Gly Gly Trp Phe Ile Asp
                165                 170                 175

Lys Thr Pro Ser Val Lys Lys Asp Ser Phe Phe Leu Asp Leu Ser Cys
            180                 185                 190

Glu Lys Ser Asn Pro Lys Lys Pro Ile Thr Glu Ile Gln Asp Ser Lys
        195                 200                 205

Asp Ser Ser Leu Leu Glu Ser Asp Met Lys Arg Lys Gly Lys Leu Lys
    210                 215                 220

Asn Lys Gly Ser Lys Arg Lys Lys Glu Asp Leu Gln Glu Val Asp Gly
225                 230                 235                 240

Glu Ile Glu Ala Val Leu Gln Lys Lys Ala Lys Ala Arg Gly Leu Glu
                245                 250                 255

Phe Gln Ile Ser Asn Val Lys Phe Glu Asp Val Gly Gly Asn Asp Met
            260                 265                 270

Thr Leu Lys Glu Val Cys Lys Met Leu Ile His Met Arg His Pro Glu
        275                 280                 285

Val Tyr His His Leu Gly Val Val Pro Pro Arg Gly Val Leu Leu His
    290                 295                 300

Gly Pro Pro Gly Cys Gly Lys Thr Leu Leu Ala His Ala Ile Ala Gly
305                 310                 315                 320

Glu Leu Asp Leu Pro Ile Leu Lys Val Ala Ala Pro Glu Ile Val Ser
                325                 330                 335

Gly Val Ser Gly Glu Ser Glu Gln Lys Leu Arg Glu Leu Phe Glu Gln
            340                 345                 350

Ala Val Ser Asn Ala Pro Cys Ile Ile Phe Ile Asp Glu Ile Asp Ala
        355                 360                 365

Ile Thr Pro Lys Arg Glu Val Ala Ser Lys Asp Met Glu Arg Arg Ile
    370                 375                 380

Val Ala Gln Leu Leu Thr Cys Met Asp Asp Leu Asn Asn Val Ala Ala
385                 390                 395                 400

Thr Ala Arg Val Leu Val Ile Gly Ala Thr Asn Arg Pro Asp Ser Leu
                405                 410                 415

Asp Pro Ala Leu Arg Arg Ala Gly Arg Phe Asp Arg Glu Ile Cys Leu
            420                 425                 430

Gly Ile Pro Asp Glu Ala Ser Arg Glu Arg Ile Leu Gln Thr Leu Cys
        435                 440                 445

Arg Lys Leu Arg Leu Pro Gln Ala Phe Asp Phe Cys His Leu Ala His
    450                 455                 460

Leu Thr Pro Gly Phe Val Gly Ala Asp Leu Met Ala Leu Cys Arg Glu
465                 470                 475                 480

Ala Ala Met Cys Ala Val Asn Arg Val Leu Met Lys Leu Gln Glu Gln
                485                 490                 495

Gln Lys Lys Asn Pro Glu Met Glu Asp Leu Pro Ser Lys Gly Val Gln
            500                 505                 510

Glu Glu Arg Leu Gly Thr Glu Pro Thr Ser Glu Thr Gln Asp Glu Leu
        515                 520                 525

Gln Arg Leu Leu Gly Leu Leu Arg Asp Gln Asp Pro Leu Ser Glu Glu
    530                 535                 540

Gln Met Gln Gly Leu Cys Ile Glu Leu Asn Asp Phe Ile Val Ala Leu
545                 550                 555                 560

Ser Ser Val Gln Pro Ser Ala Lys Arg Glu Gly Phe Val Thr Val Pro
                565                 570                 575

Asn Val Thr Trp Ala Asp Ile Gly Ala Leu Glu Asp Ile Arg Glu Glu
            580                 585                 590

Leu Thr Met Ala Ile Leu Ala Pro Val Arg Asn Pro Asp Gln Phe Lys
        595                 600                 605

Ala Leu Gly Leu Val Thr Pro Ala Gly Val Leu Leu Ala Gly Pro Pro
    610                 615                 620

Gly Cys Gly Lys Thr Leu Leu Ala Lys Ala Val Ala Asn Glu Ser Gly
625                 630                 635                 640

Leu Asn Phe Ile Ser Val Lys Gly Pro Glu Leu Leu Asn Met Tyr Val
                645                 650                 655

Gly Glu Ser Glu Arg Ala Val Arg Gln Val Phe Gln Arg Ala Lys Asn
            660                 665                 670

Ser Ala Pro Cys Val Ile Phe Phe Asp Glu Val Asp Ala Leu Cys Pro
        675                 680                 685

Arg Arg Ser Asp Arg Glu Thr Gly Ala Ser Val Arg Val Val Asn Gln
    690                 695                 700

Leu Leu Thr Glu Met Asp Gly Leu Glu Ala Arg Gln Gln Val Phe Ile
705                 710                 715                 720

Met Ala Ala Thr Asn Arg Pro Asp Ile Ile Asp Pro Ala Ile Leu Arg
                725                 730                 735

Pro Gly Arg Leu Asp Lys Thr Leu Phe Val Gly Leu Pro Pro Pro Ala
            740                 745                 750

Asp Arg Leu Ala Ile Leu Lys Thr Ile Thr Lys Asn Gly Thr Lys Pro
        755                 760                 765

Pro Leu Asp Ala Asp Val Asn Leu Glu Ala Ile Ala Gly Asp Leu Arg
    770                 775                 780

Cys Asp Cys Tyr Thr Gly Ala Asp Leu Ser Ala Leu Val Arg Glu Ala
785                 790                 795                 800

Ser Ile Cys Ala Leu Arg Gln Glu Met Ala Arg Gln Lys Ser Gly Asn
                805                 810                 815

Glu Lys Gly Glu Leu Lys Val Ser His Lys His Phe Glu Glu Ala Phe
            820                 825                 830

Lys Lys Val Arg Ser Ser Ile Ser Lys Lys Asp Gln Ile Met Tyr Glu
        835                 840                 845

Arg Leu Gln Glu Ser Leu Ser Arg
    850                 855

<210>  2
<211>  659
<212>  PRT
<213>  Homo sapiens

<400>  2

Met Glu Asp Tyr Pro Asp Pro Gln Asp Ser Lys Asp Ser Ser Leu Leu
1               5                   10                  15

Glu Ser Asp Met Lys Arg Lys Gly Lys Leu Lys Asn Lys Gly Ser Lys
            20                  25                  30

Arg Lys Lys Glu Asp Leu Gln Glu Val Asp Gly Glu Ile Glu Ala Val
        35                  40                  45

Leu Gln Lys Lys Ala Lys Ala Arg Gly Leu Glu Phe Gln Ile Ser Asn
    50                  55                  60

Val Lys Phe Glu Asp Val Gly Gly Asn Asp Met Thr Leu Lys Glu Val
65                  70                  75                  80

Cys Lys Met Leu Ile His Met Arg His Pro Glu Val Tyr His His Leu
                85                  90                  95

Gly Val Val Pro Pro Arg Gly Val Leu Leu His Gly Pro Pro Gly Cys
            100                 105                 110

Gly Lys Thr Leu Leu Ala His Ala Ile Ala Gly Glu Leu Asp Leu Pro
        115                 120                 125

Ile Leu Lys Val Ala Ala Pro Glu Ile Val Ser Gly Val Ser Gly Glu
    130                 135                 140

Ser Glu Gln Lys Leu Arg Glu Leu Phe Glu Gln Ala Val Ser Asn Ala
145                 150                 155                 160

Pro Cys Ile Ile Phe Ile Asp Glu Ile Asp Ala Ile Thr Pro Lys Arg
                165                 170                 175

Glu Val Ala Ser Lys Asp Met Glu Arg Arg Ile Val Ala Gln Leu Leu
            180                 185                 190

Thr Cys Met Asp Asp Leu Asn Asn Val Ala Ala Thr Ala Arg Val Leu
        195                 200                 205

Val Ile Gly Ala Thr Asn Arg Pro Asp Ser Leu Asp Pro Ala Leu Arg
    210                 215                 220

Arg Ala Gly Arg Phe Asp Arg Glu Ile Cys Leu Gly Ile Pro Asp Glu
225                 230                 235                 240

Ala Ser Arg Glu Arg Ile Leu Gln Thr Leu Cys Arg Lys Leu Arg Leu
                245                 250                 255

Pro Gln Ala Phe Asp Phe Cys His Leu Ala His Leu Thr Pro Gly Phe
            260                 265                 270

Val Gly Ala Asp Leu Met Ala Leu Cys Arg Glu Ala Ala Met Cys Ala
        275                 280                 285

Val Asn Arg Val Leu Met Lys Leu Gln Glu Gln Gln Lys Lys Asn Pro

290                 295                 300

Glu Met Glu Asp Leu Pro Ser Lys Gly Val Gln Glu Glu Arg Leu Gly
305                 310                 315                 320

Thr Glu Pro Thr Ser Glu Thr Gln Asp Glu Leu Gln Arg Leu Leu Gly
                325                 330                 335

Leu Leu Arg Asp Gln Asp Pro Leu Ser Glu Glu Gln Met Gln Gly Leu
            340                 345                 350

Cys Ile Glu Leu Asn Asp Phe Ile Val Ala Leu Ser Ser Val Gln Pro
        355                 360                 365

Ser Ala Lys Arg Glu Gly Phe Val Thr Val Pro Asn Val Thr Trp Ala
    370                 375                 380

Asp Ile Gly Ala Leu Glu Asp Ile Arg Glu Glu Leu Thr Met Ala Ile
385                 390                 395                 400

Leu Ala Pro Val Arg Asn Pro Asp Gln Phe Lys Ala Leu Gly Leu Val
                405                 410                 415

Thr Pro Ala Gly Val Leu Leu Ala Gly Pro Pro Gly Cys Gly Lys Thr
            420                 425                 430

Leu Leu Ala Lys Ala Val Ala Asn Glu Ser Gly Leu Asn Phe Ile Ser
        435                 440                 445

Val Lys Gly Pro Glu Leu Leu Asn Met Tyr Val Gly Glu Ser Glu Arg
    450                 455                 460

Ala Val Arg Gln Val Phe Gln Arg Ala Lys Asn Ser Ala Pro Cys Val
465                 470                 475                 480

Ile Phe Phe Asp Glu Val Asp Ala Leu Cys Pro Arg Arg Ser Asp Arg
                485                 490                 495

Glu Thr Gly Ala Ser Val Arg Val Val Asn Gln Leu Leu Thr Glu Met
            500                 505                 510

Asp Gly Leu Glu Ala Arg Gln Gln Val Phe Ile Met Ala Ala Thr Asn
        515                 520                 525

Arg Pro Asp Ile Ile Asp Pro Ala Ile Leu Arg Pro Gly Arg Leu Asp
    530                 535                 540

Lys Thr Leu Phe Val Gly Leu Pro Pro Pro Ala Asp Arg Leu Ala Ile
545                 550                 555                 560

Leu Lys Thr Ile Thr Lys Asn Gly Thr Lys Pro Pro Leu Asp Ala Asp
                565                 570                 575

Val Asn Leu Glu Ala Ile Ala Gly Asp Leu Arg Cys Asp Cys Tyr Thr
            580                 585                 590

Gly Ala Asp Leu Ser Ala Leu Val Arg Glu Ala Ser Ile Cys Ala Leu
        595                 600                 605

Arg Gln Glu Met Ala Arg Gln Lys Ser Gly Asn Glu Lys Gly Glu Leu
    610                 615                 620

Lys Val Ser His Lys His Phe Glu Glu Ala Phe Lys Lys Val Arg Ser
625                 630                 635                 640

Ser Ile Ser Lys Lys Asp Gln Ile Met Tyr Glu Arg Leu Gln Glu Ser
                645                 650                 655

Leu Ser Arg

<210>  3
<211>  638
<212>  DNA
<213>  Artificial Sequence

<220>
<223>  NVL gene synthesis

<400>  3
agcggtctca catgaagcca cgtcctgcag gatttgtgga caacaaactc aagcaacgcg      60

ttattcagta tctgaccagt aacaagtgcg gcaaatacgt agacataggg gttttggcgt     120

ctgatctgca gagggtgtac tctatcgact atggtcggag gaaacggaat gcctttcgaa     180

ttcaggtcga aaaggtgttc tccatcatca gctcagagaa agagctgaag aatctcactg     240

aacttgagga cgaacatctc gccaaaagag ccagacaagg cgaagaggac aatgagtaca     300

cagagtccta cagtgatgat gactcatcaa tggaggacta tcctgatccc cagtcagcta     360

accacatgaa ctcttctctg cttagcctgt ataggaaggg gaatcccgat agtgtgagca     420

acacaccgga aatggagcag agagaaacca ccagttccac tccacgcata tcctccaaaa     480

ccggtagcat tcccttgaaa actcctgcta aagactctga aggaggctgg tttatcgaca     540

aaacgcctag cgtcaagaag gatagcttct tcctggatct gagctgtgag aagtccaatc     600

ccaagaagcc aatcacagag attcaggatg agaccgaa                             638

<210> 4
<211> 29
<212> DNA
<213> Artificial Sequence

<220>
<223> sense NVL-IF1

<400> 4
ataggtctcc aggattcaaa agattcttc 29


<210> 5
<211> 34
<212> DNA
<213> Artificial Sequence

<220>
<223> asense NVL-IF1

<400> 5
tatggtctca tcgacccggc tgagggactc ctgc 34


<210> 6
<211> 1980
<212> DNA
<213> Artificial Sequence

<220>
<223> NVL-PCR-fragment

<400> 6
ataggtctcc aggattcaaa agattcttct cttttggaga gtgatatgaa acggaaaggc 60

aagctaaaga ataaaggaag caaaaggaag aaagaagatc ttcaggaagt agatggagaa 120

attgaagctg tcctacaaaa gaaagctaaa gccagggggt tagaattcca gatctccaac 180

gtgaagtttg aagatgtggg aggcaatgat atgacattaa aagaggtctg caagatgctc 240

atacacatgc gtcacccgga ggtgtaccac cacctgggcg tcgtgccccc tcgtggagtt 300

ctccttcatg gaccaccagg ctgtgggaag acattacttg cacatgcaat tgctggggaa 360

cttgacctgc caattttgaa agtggctgct ccagagattg tgtctggagt atccggagag 420

tctgagcaga agctgagaga actatttgag caagctgtgt caaatgcacc atgtatcatt 480

ttcattgatg aaattgatgc tattaccccc aaaagagaag tggcttcaaa agatatggaa 540

cgaagaattg tagcccaact cctaacctgc atggatgatc tgaataatgt ggctgctaca 600

gcccgggtcc tagttattgg agctactaat cgaccagact cgttagaccc tgctttgaga 660

cgtgcgggaa ggttcgaccg agaaatatgc ctaggtatcc cagatgaagc atccagggaa 720

agaatacttc aaacattgtg cagaaaactg aggcttcctc aagcttttga tttctgtcac 780

ttagcacacc taactccagg ctttgttggt gctgatctca tggcactgtg ccgagaggca 840

gcaatgtgtg cagtcaatag agtcttaatg aagctacagg aacagcagaa gaaaaatcct 900

gaaatggaag atttgccatc taaaggagtc caggaggaaa ggctgggaac tgagcccact 960

tctgaaacac aggatgaatt acaaaggctg ctggggttgc taagagacca agatcccctc 1020

tcagaggagc agatgcaagg actgtgcatt gaactgaatg atttcattgt tgctctatcc 1080

tcagtccaac cctctgccaa aagggaaggc tttgtcactg tccctaatgt gacatgggca 1140

gatattggtg ccctggaaga cattagagag gagctcacca tggcaatatt ggcaccagta 1200

cgcaacccag accagttcaa agctcttgga ttggtgactc cagctggggt cctccttgct 1260

ggtcctcctg gctgtgggaa gactctgctg gcgaaggctg ttgcaaatga gtccggacta 1320

aattttatat ctgtcaaggg ccccgaatta ctaaacatgt atgttggtga gagtgaacgt 1380

gctgtgcgac aagtttttca acgagccaag aactcagcac cctgtgtgat attctttgat 1440

gaagtggatg ctttatgtcc tcgaagatca gaccgagaga caggggcaag tgtccgagtg 1500

gtgaatcagc tacttacaga gatggatggt ctggaagcac gccagcaggt ttttattatg 1560

gcagccacta acaggccaga tataattgac cctgcaatcc tgcgcccggg ccgcctggac 1620

aaaacactgt ttgtgggttt accgccccct gcagatcgcc ttgccatctt aaaaactatc 1680

acaaaaaatg gtaccaaacc accactggat gcagatgtaa atttggaagc aattgctggt 1740

gaccttcgct gtgattgcta tacgggcgca gatctctctg ctttggtacg agaagcttct 1800

atctgtgccc tgagacagga aatggcaaga cagaagagtg gaaatgaaaa aggtgaactc 1860

aaggttagtc ataagcattt tgaagaagct ttcaagaaag taagatcatc tatatcaaaa 1920

aaggatcaaa tcatgtatga acgtttgcag gagtccctca gccgggtcga tgagaccata 1980

<210> 7
<211> 8182
<212> DNA
<213> Artificial Sequence

<220>
<223> pTriEx-1-NVL[human]-IF1-H8

<400> 7
ggagtcgctg cgacgctgcc ttcgccccgt gccccgctcc gccgccgcct cgcgccgccc 60

gccccggctc tgactgaccg cgttactccc acaggtgagc gggcgggacg gcccttctcc 120

ttcgggctgt aattagcgct tggtttaatg acggcttgtt tcttttctgt ggctgcgtga 180

aagccttgag gggctccggg agggcccttt gtgcgggggg agcggctcgg ggctgtccgc 240

ggggggacgg ctgccttcgg gggggacggg gcagggcggg gttcggcttc tggcgtgtga 300

ccggcggctc tagagcctct gctaaccatg ttcatgcctt cttctttttc ctacagctcc 360

tgggcaacgt gctggttatt gtgctgtctc atcattttgg caaagaattg gatcggaccg 420

aaattaatac gactcactat aggggaattg tgagcggata acaattcccc ggagttaatc 480

cgggaccttt aattcaaccc aacacaatat attatagtta aataagaatt attatcaaat 540

catttgtata ttaattaaaa tactatactg taaattacat tttatttaca atcaaaggag 600

atataccatg aagccacgtc ctgcaggatt tgtggacaac aaactcaagc aacgcgttat 660

tcagtatctg accagtaaca agtgcggcaa atacgtagac ataggggttt tggcgtctga 720

tctgcagagg gtgtactcta tcgactatgg tcggaggaaa cggaatgcct ttcgaattca 780

ggtcgaaaag gtgttctcca tcatcagctc agagaaagag ctgaagaatc tcactgaact 840

tgaggacgaa catctcgcca aaagagccag acaaggcgaa gaggacaatg agtacacaga 900

gtcctacagt gatgatgact catcaatgga ggactatcct gatccccagt cagctaacca 960

catgaactct tctctgctta gcctgtatag gaaggggaat cccgatagtg tgagcaacac 1020

accggaaatg gagcagagag aaaccaccag ttccactcca cgcatatcct ccaaaaccgg 1080

tagcattccc ttgaaaactc ctgctaaaga ctctgaagga ggctggttta tcgacaaaac 1140

gcctagcgtc aagaaggata gcttcttcct ggatctgagc tgtgagaagt ccaatcccaa 1200

gaagccaatc acagagattc aggattcaaa agattcttct cttttggaga gtgatatgaa 1260

acggaaaggc aagctaaaga ataaaggaag caaaaggaag aaagaagatc ttcaggaagt 1320

agatggagaa attgaagctg tcctacaaaa gaaagctaaa gccagggggt tagaattcca 1380

gatctccaac gtgaagtttg aagatgtggg aggcaatgat atgacattaa aagaggtctg 1440

caagatgctc atacacatgc gtcacccgga ggtgtaccac cacctgggcg tcgtgccccc 1500

tcgtggagtt ctccttcatg gaccaccagg ctgtgggaag acattacttg cacatgcaat 1560

tgctggggaa cttgacctgc caattttgaa agtggctgct ccagagattg tgtctggagt 1620

atccggagag tctgagcaga agctgagaga actatttgag caagctgtgt caaatgcacc 1680

atgtatcatt ttcattgatg aaattgatgc tattaccccc aaaagagaag tggcttcaaa 1740

agatatggaa cgaagaattg tagcccaact cctaacctgc atggatgatc tgaataatgt 1800

ggctgctaca gcccgggtcc tagttattgg agctactaat cgaccagact cgttagaccc 1860

tgctttgaga cgtgcgggaa ggttcgaccg agaaatatgc ctaggtatcc cagatgaagc 1920

atccagggaa agaatacttc aaacattgtg cagaaaactg aggcttcctc aagcttttga 1980

tttctgtcac ttagcacacc taactccagg ctttgttggt gctgatctca tggcactgtg 2040

ccgagaggca gcaatgtgtg cagtcaatag agtcttaatg aagctacagg aacagcagaa 2100

gaaaaatcct gaaatggaag atttgccatc taaaggagtc caggaggaaa ggctgggaac 2160

tgagcccact tctgaaacac aggatgaatt acaaaggctg ctggggttgc taagagacca 2220

agatcccctc tcagaggagc agatgcaagg actgtgcatt gaactgaatg atttcattgt 2280

tgctctatcc tcagtccaac cctctgccaa aagggaaggc tttgtcactg tccctaatgt 2340

gacatgggca gatattggtg ccctggaaga cattagagag gagctcacca tggcaatatt 2400

ggcaccagta cgcaacccag accagttcaa agctcttgga ttggtgactc cagctggggt 2460

cctccttgct ggtcctcctg gctgtgggaa gactctgctg gcgaaggctg ttgcaaatga 2520

gtccggacta aattttatat ctgtcaaggg ccccgaatta ctaaacatgt atgttggtga 2580

gagtgaacgt gctgtgcgac aagtttttca acgagccaag aactcagcac cctgtgtgat 2640

attctttgat gaagtggatg ctttatgtcc tcgaagatca gaccgagaga caggggcaag 2700

tgtccgagtg gtgaatcagc tacttacaga gatggatggt ctggaagcac gccagcaggt 2760

ttttattatg gcagccacta acaggccaga tataattgac cctgcaatcc tgcgcccggg 2820

ccgcctggac aaaacactgt ttgtgggttt accgccccct gcagatcgcc ttgccatctt 2880

aaaaactatc acaaaaaatg gtaccaaacc accactggat gcagatgtaa atttggaagc 2940

aattgctggt gaccttcgct gtgattgcta tacgggcgca gatctctctg ctttggtacg 3000

agaagcttct atctgtgccc tgagacagga aatggcaaga cagaagagtg gaaatgaaaa 3060

aggtgaactc aaggttagtc ataagcattt tgaagaagct ttcaagaaag taagatcatc 3120

tatatcaaaa aaggatcaaa tcatgtatga acgtttgcag gagtccctca gccgggtcga 3180

gcaccaccat caccatcacc atcactaagt gattaacctc aggtgcaggc tgcctatcag 3240

aaggtggtgg ctggtgtggc caatgccctg gctcacaaat accactgaga tcgatctttt 3300

tccctctgcc aaaaattatg gggacatcat gaagcccctt gagcatctga cttctggcta 3360

ataaaggaaa tttattttca ttgcaatagt gtgttggaat tttttgtgtc tctcactcgg 3420

aaggacatat gggagggcaa atcatttaaa acatcagaat gagtatttgg tttagagttt 3480

ggcaacatat gcccatatgt aactagcata accccttggg gcctctaaac gggtcttgag 3540

gggttttttg ctgaaagcat gcggaggaaa ttctccttga agtttccctg gtgttcaaag 3600

taaaggagtt tgcaccagac gcacctctgt tcactggtcc ggcgtattaa aacacgatac 3660

attgttatta gtacatttat taagcgctag attctgtgcg ttgttgattt acagacaatt 3720

gttgtacgta ttttaataat tcattaaatt tataatcttt agggtggtat gttagagcga 3780

aaatcaaatg attttcagcg tctttatatc tgaatttaaa tattaaatcc tcaatagatt 3840

tgtaaaatag gtttcgatta gtttcaaaca agggttgttt ttccgaaccg atggctggac 3900

tatctaatgg attttcgctc aacgccacaa aacttgccaa atcttgtagc agcaatctag 3960

ctttgtcgat attcgtttgt gttttgtttt gtaataaagg ttcgacgtcg ttcaaaatat 4020

tatgcgcttt tgtatttctt tcatcactgt cgttagtgta caattgactc gacgtaaaca 4080

cgttaaatag agcttggaca tatttaacat cgggcgtgtt agctttatta ggccgattat 4140

cgtcgtcgtc ccaaccctcg tcgttagaag ttgcttccga agacgatttt gccatagcca 4200

cacgacgcct attaattgtg tcggctaaca cgtccgcgat caaatttgta gttgagcttt 4260

ttggaattat ttctgattgc gggcgttttt gggcgggttt caatctaact gtgcccgatt 4320

ttaattcaga caacacgtta gaaagcgatg gtgcaggcgg tggtaacatt tcagacggca 4380

aatctactaa tggcggcggt ggtggagctg atgataaatc taccatcggt ggaggcgcag 4440

gcggggctgg cggcggaggc ggaggcggag gtggtggcgg tgatgcagac ggcggtttag 4500

gctcaaatgt ctctttaggc aacacagtcg gcacctcaac tattgtactg gtttcgggcg 4560

ccgtttttgg tttgaccggt ctgagacgag tgcgattttt ttcgtttcta atagcttcca 4620

acaattgttg tctgtcgtct aaaggtgcag cgggttgagg ttccgtcggc attggtggag 4680

cgggcggcaa ttcagacatc gatggtggtg gtggtggtgg aggcgctgga atgttaggca 4740

cgggagaagg tggtggcggc ggtgccgccg gtataatttg ttctggttta gtttgttcgc 4800

gcacgattgt gggcaccggc gcaggcgccg ctggctgcac aacggaaggt cgtctgcttc 4860

gaggcagcgc ttggggtggt ggcaattcaa tattataatt ggaatacaaa tcgtaaaaat 4920

ctgctataag cattgtaatt tcgctatcgt ttaccgtgcc gatatttaac aaccgctcaa 4980

tgtaagcaat tgtattgtaa agagattgtc tcaagctcgg aacgctgcgc tcggtcgttc 5040

ggctgcggcg agcggtatca gctcactcaa aggcggtaat acggttatcc acagaatcag 5100

gggataacgc aggaaagaac atgtgagcaa aaggccagca aaaggccagg aaccgtaaaa 5160

aggccgcgtt gctggcgttt ttccataggc tccgcccccc tgacgagcat cacaaaaatc 5220

gacgctcaag tcagaggtgg cgaaacccga caggactata aagataccag gcgtttcccc 5280

ctggaagctc cctcgtgcgc tctcctgttc cgaccctgcc gcttaccgga tacctgtccg 5340

cctttctccc ttcgggaagc gtggcgcttt ctcaatgctc acgctgtagg tatctcagtt 5400

cggtgtaggt cgttcgctcc aagctgggct gtgtgcacga accccccgtt cagcccgacc 5460

gctgcgcctt atccggtaac tatcgtcttg agtccaaccc ggtaagacac gacttatcgc 5520

cactggcagc agccactggt aacaggatta gcagagcgag gtatgtaggc ggtgctacag 5580

agttcttgaa gtggtggcct aactacggct acactagaag gacagtattt ggtatctgcg 5640

ctctgctgaa gccagttacc ttcggaaaaa gagttggtag ctcttgatcc ggcaaacaaa 5700

ccaccgctgg tagcggtggt ttttttgttt gcaagcagca gattacgcgc agaaaaaaag 5760

gatctcaaga agatcctttg ttaccaatgc ttaatcagtg aggcacctat ctcagcgatc 5820

tgtctatttc gttcatccat agttgcctga ctccccgtcg tgtagataac tacgatacgg 5880

gagggcttac catctggccc cagtgctgca atgataccgc gagacccacg ctcaccggct 5940

ccagatttat cagcaataaa ccagccagcc ggaagggccg agcgcagaag tggtcctgca 6000

actttatccg cctccatcca gtctattaat tgttgccggg aagctagagt aagtagttcg 6060

ccagttaata gtttgcgcaa cgttgttgcc attgctacag gcatcgtggt gtcacgctcg 6120

tcgtttggta tggcttcatt cagctccggt tcccaacgat caaggcgagt tacatgatcc 6180

cccatgttgt gcaaaaaagc ggttagctcc ttcggtcctc cgatcgttgt cagaagtaag 6240

ttggccgcag tgttatcact catggttatg gcagcactgc ataattctct tactgtcatg 6300

ccatccgtaa gatgcttttc tgtgactggt gagtactcaa ccaagtcatt ctgagaatag 6360

tgtatgcggc gaccgagttg ctcttgcccg gcgtcaatac gggataatac cgcgccacat 6420

agcagaactt taaaagtgct catcattgga aaacgttctt cggggcgaaa actctcaagg 6480

atcttaccgc tgttgagatc cagttcgatg taacccactc gtgcacccaa ctgatcttca 6540

gcatctttta ctttcaccag cgtttctggg tgagcaaaaa caggaaggca aaatgccgca 6600

aaaaagggaa taagggcgac acggaaatgt tgaatactca tactcttcct ttttcaatat 6660

tattgaagca tttatcaggg ttattgtctc atgtccgcgc gtttcctgca tcttttaatc 6720

aaatcccaag atgtgtataa accaccaaac tgccaaaaaa tgaaaactgt cgacaagctc 6780

tgtccgtttg ctggcaactg caagggtctc aatcctattt gtaattattg aataataaaa 6840

caattataaa tgtcaaattt gttttttatt aacgatacaa accaaacgca acaagaacat 6900

ttgtagtatt atctataatt gaaaacgcgt agttataatc gctgaggtaa tatttaaaat 6960

cattttcaaa tgattcacag ttaatttgcg acaatataat tttattttca cataaactag 7020

acgccttgtc gtcttcttct tcgtattcct tctctttttc attttctctt tcataaaaat 7080

taacatagtt attatcgtat ccatatatgt atctatcgta tagagtaaat tttttgttgt 7140

cataaatata tatgtctttt ttaatggggt gtatagtacc gctgcgcata gtttttctgt 7200

aatttacaac agtgctattt tctggtagtt cttcggagtg tgttgcttta attattaaat 7260

ttatataatc aatgaatttg ggatcgtcgg ttttgtacaa tatgttgccg gcatagtacg 7320

cagcttcttc tagttcaatt acaccatttt ttagcagcac cggattaaca taactttcca 7380

aaatgttgta cgaaccgtta aacaaaaaca gttcacctcc cttttctata ctattgtctg 7440

cgagcagttg tttgttgtta aaaataacag ccattgtaat gagacgcaca aactaatatc 7500

acaaactgga aatgtctatc aatatatagt tgctctagtt attaatagta atcaattacg 7560

gggtcattag ttcatagccc atatatggag ttccgcgtta cataacttac ggtaaatggc 7620

ccgcctggct gaccgcccaa cgacccccgc ccattgacgt caataatgac gtatgttccc 7680

atagtaacgc caatagggac tttccattga cgtcaatggg tggactattt acggtaaact 7740

gcccacttgg cagtacatca agtgtatcat atgccaagta cgccccctat tgacgtcaat 7800

gacggtaaat ggcccgcctg gcattatgcc cagtacatga ccttatggga ctttcctact 7860

tggcagtaca tctacgtatt agtcatcgct attaccatgc atggtcgagg tgagccccac 7920

gttctgcttc actctcccca tctccccccc ctccccaccc ccaattttgt atttatttat 7980

tttttaatta ttttgtgcag cgatgggggc gggggggggg ggggggcgcg cgccaggcgg 8040

ggcggggcgg ggcgaggggc ggggcggggc gaggcggaga ggtgcggcgg cagccaatca 8100

gagcggcgcg ctccgaaagt ttccttttat ggcgaggcgg cggcggcggc ggccctataa 8160

aaagcgaagc gcgcggcggg cg 8182

<210> 8
<211> 866
<212> PRT
<213> Artificial Sequence

<220>
<223> NVL[human]-IF1-H8

<400> 8

Met Lys Pro Arg Pro Ala Gly Phe Val Asp Asn Lys Leu Lys Gln Arg
1               5                   10                  15

Val Ile Gln Tyr Leu Thr Ser Asn Lys Cys Gly Lys Tyr Val Asp Ile
            20                  25                  30

Gly Val Leu Ala Ser Asp Leu Gln Arg Val Tyr Ser Ile Asp Tyr Gly
        35                  40                  45

Arg Arg Lys Arg Asn Ala Phe Arg Ile Gln Val Glu Lys Val Phe Ser
    50                  55                  60

Ile Ile Ser Ser Glu Lys Glu Leu Lys Asn Leu Thr Glu Leu Glu Asp
65                  70                  75                  80

Glu His Leu Ala Lys Arg Ala Arg Gln Gly Glu Glu Asp Asn Glu Tyr
                85                  90                  95

Thr Glu Ser Tyr Ser Asp Asp Asp Ser Ser Met Glu Asp Tyr Pro Asp
            100                 105                 110

Pro Gln Ser Ala Asn His Met Asn Ser Ser Leu Leu Ser Leu Tyr Arg
        115                 120                 125

Lys Gly Asn Pro Asp Ser Val Ser Asn Thr Pro Glu Met Glu Gln Arg
    130                 135                 140

Glu Thr Thr Ser Ser Thr Pro Arg Ile Ser Ser Lys Thr Gly Ser Ile
145                 150                 155                 160

Pro Leu Lys Thr Pro Ala Lys Asp Ser Glu Gly Gly Trp Phe Ile Asp
                165                 170                 175

Lys Thr Pro Ser Val Lys Lys Asp Ser Phe Phe Leu Asp Leu Ser Cys
            180                 185                 190

Glu Lys Ser Asn Pro Lys Lys Pro Ile Thr Glu Ile Gln Asp Ser Lys
        195                 200                 205

Asp Ser Ser Leu Leu Glu Ser Asp Met Lys Arg Lys Gly Lys Leu Lys
    210                 215                 220

Asn Lys Gly Ser Lys Arg Lys Lys Glu Asp Leu Gln Glu Val Asp Gly
225                 230                 235                 240

Glu Ile Glu Ala Val Leu Gln Lys Lys Ala Lys Ala Arg Gly Leu Glu
                245                 250                 255

Phe Gln Ile Ser Asn Val Lys Phe Glu Asp Val Gly Gly Asn Asp Met
            260                 265                 270

Thr Leu Lys Glu Val Cys Lys Met Leu Ile His Met Arg His Pro Glu
        275                 280                 285

Val Tyr His His Leu Gly Val Val Pro Pro Arg Gly Val Leu Leu His
    290                 295                 300

Gly Pro Pro Gly Cys Gly Lys Thr Leu Leu Ala His Ala Ile Ala Gly
305                 310                 315                 320

Glu Leu Asp Leu Pro Ile Leu Lys Val Ala Ala Pro Glu Ile Val Ser
                325                 330                 335

Gly Val Ser Gly Glu Ser Glu Gln Lys Leu Arg Glu Leu Phe Glu Gln
            340                 345                 350

Ala Val Ser Asn Ala Pro Cys Ile Ile Phe Ile Asp Glu Ile Asp Ala
        355                 360                 365

Ile Thr Pro Lys Arg Glu Val Ala Ser Lys Asp Met Glu Arg Arg Ile
    370                 375                 380

Val Ala Gln Leu Leu Thr Cys Met Asp Asp Leu Asn Asn Val Ala Ala
385                 390                 395                 400

Thr Ala Arg Val Leu Val Ile Gly Ala Thr Asn Arg Pro Asp Ser Leu
                405                 410                 415

Asp Pro Ala Leu Arg Arg Ala Gly Arg Phe Asp Arg Glu Ile Cys Leu
            420                 425                 430

Gly Ile Pro Asp Glu Ala Ser Arg Glu Arg Ile Leu Gln Thr Leu Cys

435                 440                 445

Arg Lys Leu Arg Leu Pro Gln Ala Phe Asp Phe Cys His Leu Ala His
    450                 455                 460

Leu Thr Pro Gly Phe Val Gly Ala Asp Leu Met Ala Leu Cys Arg Glu
465                 470                 475                 480

Ala Ala Met Cys Ala Val Asn Arg Val Leu Met Lys Leu Gln Glu Gln
                485                 490                 495

Gln Lys Lys Asn Pro Glu Met Glu Asp Leu Pro Ser Lys Gly Val Gln
            500                 505                 510

Glu Glu Arg Leu Gly Thr Glu Pro Thr Ser Glu Thr Gln Asp Glu Leu
        515                 520                 525

Gln Arg Leu Leu Gly Leu Leu Arg Asp Gln Asp Pro Leu Ser Glu Glu
    530                 535                 540

Gln Met Gln Gly Leu Cys Ile Glu Leu Asn Asp Phe Ile Val Ala Leu
545                 550                 555                 560

Ser Ser Val Gln Pro Ser Ala Lys Arg Glu Gly Phe Val Thr Val Pro
                565                 570                 575

Asn Val Thr Trp Ala Asp Ile Gly Ala Leu Glu Asp Ile Arg Glu Glu
            580                 585                 590

Leu Thr Met Ala Ile Leu Ala Pro Val Arg Asn Pro Asp Gln Phe Lys
        595                 600                 605

Ala Leu Gly Leu Val Thr Pro Ala Gly Val Leu Leu Ala Gly Pro Pro
    610                 615                 620

Gly Cys Gly Lys Thr Leu Leu Ala Lys Ala Val Ala Asn Glu Ser Gly
625                 630                 635                 640

Leu Asn Phe Ile Ser Val Lys Gly Pro Glu Leu Leu Asn Met Tyr Val
                645                 650                 655

Gly Glu Ser Glu Arg Ala Val Arg Gln Val Phe Gln Arg Ala Lys Asn
            660                 665                 670

Ser Ala Pro Cys Val Ile Phe Phe Asp Glu Val Asp Ala Leu Cys Pro
        675                 680                 685

Arg Arg Ser Asp Arg Glu Thr Gly Ala Ser Val Arg Val Val Asn Gln
    690                 695                 700

Leu Leu Thr Glu Met Asp Gly Leu Glu Ala Arg Gln Gln Val Phe Ile
705                 710                 715                 720

Met Ala Ala Thr Asn Arg Pro Asp Ile Ile Asp Pro Ala Ile Leu Arg
                725                 730                 735

Pro Gly Arg Leu Asp Lys Thr Leu Phe Val Gly Leu Pro Pro Pro Ala
            740                 745                 750

Asp Arg Leu Ala Ile Leu Lys Thr Ile Thr Lys Asn Gly Thr Lys Pro
        755                 760                 765

Pro Leu Asp Ala Asp Val Asn Leu Glu Ala Ile Ala Gly Asp Leu Arg
    770                 775                 780

Cys Asp Cys Tyr Thr Gly Ala Asp Leu Ser Ala Leu Val Arg Glu Ala
785                 790                 795                 800

Ser Ile Cys Ala Leu Arg Gln Glu Met Ala Arg Gln Lys Ser Gly Asn
                805                 810                 815

Glu Lys Gly Glu Leu Lys Val Ser His Lys His Phe Glu Glu Ala Phe
            820                 825                 830

Lys Lys Val Arg Ser Ser Ile Ser Lys Lys Asp Gln Ile Met Tyr Glu
        835                 840                 845

Arg Leu Gln Glu Ser Leu Ser Arg Val Glu His His His His His His
    850                 855                 860

His His
865

<210>  9
<211>  7800
<212>  DNA
<213>  Artificial Sequence

<220>
<223>  pET24d-NVL[human]-IF1-H6

<400>  9
ggggaattgt gagcggataa caattcccct ctagaaataa ttttgtttaa ctttaagaag      60

gagatatacc atgaagccac gtcctgcagg atttgtggac aacaaactca agcaacgcgt     120

tattcagtat ctgaccagta acaagtgcgg caaatacgta gacatagggg ttttggcgtc     180

tgatctgcag agggtgtact ctatcgacta tggtcggagg aaacggaatg cctttcgaat 240

tcaggtcgaa aaggtgttct ccatcatcag ctcagagaaa gagctgaaga atctcactga 300

acttgaggac gaacatctcg ccaaaagagc cagacaaggc gaagaggaca atgagtacac 360

agagtcctac agtgatgatg actcatcaat ggaggactat cctgatcccc agtcagctaa 420

ccacatgaac tcttctctgc ttagcctgta taggaagggg aatcccgata gtgtgagcaa 480

cacaccggaa atggagcaga gagaaaccac cagttccact ccacgcatat cctccaaaac 540

cggtagcatt cccttgaaaa ctcctgctaa agactctgaa ggaggctggt ttatcgacaa 600

aacgcctagc gtcaagaagg atagcttctt cctggatctg agctgtgaga agtccaatcc 660

caagaagcca atcacagaga ttcaggattc aaaagattct tctcttttgg agagtgatat 720

gaaacggaaa ggcaagctaa agaataaagg aagcaaaagg aagaaagaag atcttcagga 780

agtagatgga gaaattgaag ctgtcctaca aaagaaagct aaagccaggg ggttagaatt 840

ccagatctcc aacgtgaagt ttgaagatgt gggaggcaat gatatgacat taaaagaggt 900

ctgcaagatg ctcatacaca tgcgtcaccc ggaggtgtac caccacctgg gcgtcgtgcc 960

ccctcgtgga gttctccttc atggaccacc aggctgtggg aagacattac ttgcacatgc 1020

aattgctggg gaacttgacc tgccaatttt gaaagtggct gctccagaga ttgtgtctgg 1080

agtatccgga gagtctgagc agaagctgag agaactattt gagcaagctg tgtcaaatgc 1140

accatgtatc attttcattg atgaaattga tgctattacc cccaaaagag aagtggcttc 1200

aaaagatatg gaacgaagaa ttgtagccca actcctaacc tgcatggatg atctgaataa 1260

tgtggctgct acagcccggg tcctagttat tggagctact aatcgaccag actcgttaga 1320

ccctgctttg agacgtgcgg gaaggttcga ccgagaaata tgcctaggta tcccagatga 1380

agcatccagg gaaagaatac ttcaaacatt gtgcagaaaa ctgaggcttc ctcaagcttt 1440

tgatttctgt cacttagcac acctaactcc aggctttgtt ggtgctgatc tcatggcact 1500

gtgccgagag gcagcaatgt gtgcagtcaa tagagtctta atgaagctac aggaacagca 1560

gaagaaaaat cctgaaatgg aagatttgcc atctaaagga gtccaggagg aaaggctggg 1620

aactgagccc acttctgaaa cacaggatga attacaaagg ctgctggggt tgctaagaga 1680

ccaagatccc ctctcagagg agcagatgca aggactgtgc attgaactga atgatttcat 1740

tgttgctcta tcctcagtcc aaccctctgc caaaagggaa ggctttgtca ctgtccctaa 1800

tgtgacatgg gcagatattg gtgccctgga agacattaga gaggagctca ccatggcaat 1860

attggcacca gtacgcaacc cagaccagtt caaagctctt ggattggtga ctccagctgg 1920

ggtcctcctt gctggtcctc ctggctgtgg gaagactctg ctggcgaagg ctgttgcaaa 1980

tgagtccgga ctaaatttta tatctgtcaa gggccccgaa ttactaaaca tgtatgttgg 2040

tgagagtgaa cgtgctgtgc gacaagtttt tcaacgagcc aagaactcag caccctgtgt 2100

gatattcttt gatgaagtgg atgctttatg tcctcgaaga tcagaccgag agacaggggc 2160

aagtgtccga gtggtgaatc agctacttac agagatggat ggtctggaag cacgccagca 2220

ggtttttatt atggcagcca ctaacaggcc agatataatt gaccctgcaa tcctgcgccc 2280

gggccgcctg gacaaaacac tgtttgtggg tttaccgccc cctgcagatc gccttgccat 2340

cttaaaaact atcacaaaaa atggtaccaa accaccactg gatgcagatg taaatttgga 2400

agcaattgct ggtgaccttc gctgtgattg ctatacgggc gcagatctct ctgctttggt 2460

acgagaagct tctatctgtg ccctgagaca ggaaatggca agacagaaga gtggaaatga 2520

aaaaggtgaa ctcaaggtta gtcataagca ttttgaagaa gctttcaaga aagtaagatc 2580

atctatatca aaaaaggatc aaatcatgta tgaacgtttg caggagtccc tcagccgggt 2640

cgagcaccac caccaccacc actgagatcc ggctgctaac aaagcccgaa aggaagctga 2700

gttggctgct gccaccgctg agcaataact agcataaccc cttggggcct ctaaacgggt 2760

cttgaggggt tttttgctga aaggaggaac tatatccgga ttggcgaatg ggacgcgccc 2820

tgtagcggcg cattaagcgc ggcgggtgtg gtggttacgc gcagcgtgac cgctacactt 2880

gccagcgccc tagcgcccgc tcctttcgct ttcttccctt cctttctcgc cacgttcgcc 2940

ggctttcccc gtcaagctct aaatcggggg ctccctttag ggttccgatt tagtgcttta 3000

cggcacctcg accccaaaaa acttgattag ggtgatggtt cacgtagtgg gccatcgccc 3060

tgatagacgg tttttcgccc tttgacgttg gagtccacgt tctttaatag tggactcttg 3120

ttccaaactg gaacaacact caaccctatc tcggtctatt cttttgattt ataagggatt 3180

ttgccgattt cggcctattg gttaaaaaat gagctgattt aacaaaaatt taacgcgaat 3240

tttaacaaaa tattaacgct tacaatttag gtggcacttt tcggggaaat gtgcgcggaa 3300

cccctatttg tttatttttc taaatacatt caaatatgta tccgctcatg aattaattct 3360

tagaaaaact catcgagcat caaatgaaac tgcaatttat tcatatcagg attatcaata 3420

ccatattttt gaaaaagccg tttctgtaat gaaggagaaa actcaccgag gcagttccat 3480

aggatggcaa gatcctggta tcggtctgcg attccgactc gtccaacatc aatacaacct 3540

attaatttcc cctcgtcaaa aataaggtta tcaagtgaga aatcaccatg agtgacgact 3600

gaatccggtg agaatggcaa aagtttatgc atttctttcc agacttgttc aacaggccag 3660

ccattacgct cgtcatcaaa atcactcgca tcaaccaaac cgttattcat tcgtgattgc 3720

gcctgagcga gacgaaatac gcgatcgctg ttaaaaggac aattacaaac aggaatcgaa 3780

tgcaaccggc gcaggaacac tgccagcgca tcaacaatat tttcacctga atcaggatat 3840

tcttctaata cctggaatgc tgttttcccg gggatcgcag tggtgagtaa ccatgcatca 3900

tcaggagtac ggataaaatg cttgatggtc ggaagaggca taaattccgt cagccagttt 3960

agtctgacca tctcatctgt aacatcattg gcaacgctac ctttgccatg tttcagaaac 4020

aactctggcg catcgggctt cccatacaat cgatagattg tcgcacctga ttgcccgaca 4080

ttatcgcgag cccatttata cccatataaa tcagcatcca tgttggaatt taatcgcggc 4140

ctagagcaag acgtttcccg ttgaatatgg ctcataacac cccttgtatt actgtttatg 4200

taagcagaca gttttattgt tcatgaccaa aatcccttaa cgtgagtttt cgttccactg 4260

agcgtcagac cccgtagaaa agatcaaagg atcttcttga gatccttttt ttctgcgcgt 4320

aatctgctgc ttgcaaacaa aaaaaccacc gctaccagcg gtggtttgtt tgccggatca 4380

agagctacca actctttttc cgaaggtaac tggcttcagc agagcgcaga taccaaatac 4440

tgtccttcta gtgtagccgt agttaggcca ccacttcaag aactctgtag caccgcctac 4500

atacctcgct ctgctaatcc tgttaccagt ggctgctgcc agtggcgata agtcgtgtct 4560

taccgggttg gactcaagac gatagttacc ggataaggcg cagcggtcgg gctgaacggg 4620

gggttcgtgc acacagccca gcttggagcg aacgacctac accgaactga gatacctaca 4680

gcgtgagcta tgagaaagcg ccacgcttcc cgaagggaga aaggcggaca ggtatccggt 4740

aagcggcagg gtcggaacag gagagcgcac gagggagctt ccagggggaa acgcctggta 4800

tctttatagt cctgtcgggt ttcgccacct ctgacttgag cgtcgatttt tgtgatgctc 4860

gtcagggggg cggagcctat ggaaaaacgc cagcaacgcg gcctttttac ggttcctggc 4920

cttttgctgg ccttttgctc acatgttctt tcctgcgtta tcccctgatt ctgtggataa 4980

ccgtattacc gcctttgagt gagctgatac cgctcgccgc agccgaacga ccgagcgcag 5040

cgagtcagtg agcgaggaag cggaagagcg cctgatgcgg tattttctcc ttacgcatct 5100

gtgcggtatt tcacaccgca tatatggtgc actctcagta caatctgctc tgatgccgca 5160

tagttaagcc agtatacact ccgctatcgc tacgtgactg ggtcatggct gcgccccgac 5220

acccgccaac acccgctgac gcgccctgac gggcttgtct gctcccggca tccgcttaca 5280

gacaagctgt gaccgtctcc gggagctgca tgtgtcagag gttttcaccg tcatcaccga 5340

aacgcgcgag gcagctgcgg taaagctcat cagcgtggtc gtgaagcgat tcacagatgt 5400

ctgcctgttc atccgcgtcc agctcgttga gtttctccag aagcgttaat gtctggcttc 5460

tgataaagcg ggccatgtta agggcggttt tttcctgttt ggtcactgat gcctccgtgt 5520

aagggggatt tctgttcatg ggggtaatga taccgatgaa acgagagagg atgctcacga 5580

tacgggttac tgatgatgaa catgcccggt tactggaacg ttgtgagggt aaacaactgg 5640

cggtatggat gcggcgggac cagagaaaaa tcactcaggg tcaatgccag cgcttcgtta 5700

atacagatgt aggtgttcca cagggtagcc agcagcatcc tgcgatgcag atccggaaca 5760

taatggtgca gggcgctgac ttccgcgttt ccagacttta cgaaacacgg aaaccgaaga 5820

ccattcatgt tgttgctcag gtcgcagacg ttttgcagca gcagtcgctt cacgttcgct 5880

cgcgtatcgg tgattcattc tgctaaccag taaggcaacc ccgccagcct agccgggtcc 5940

tcaacgacag gagcacgatc atgcgcaccc gtggggccgc catgccggcg ataatggcct 6000

gcttctcgcc gaaacgtttg gtggcgggac cagtgacgaa ggcttgagcg agggcgtgca 6060

agattccgaa taccgcaagc gacaggccga tcatcgtcgc gctccagcga aagcggtcct 6120

cgccgaaaat gacccagagc gctgccggca cctgtcctac gagttgcatg ataaagaaga 6180

cagtcataag tgcggcgacg atagtcatgc cccgcgccca ccggaaggag ctgactgggt 6240

tgaaggctct caagggcatc ggtcgagatc ccggtgccta atgagtgagc taacttacat 6300

taattgcgtt gcgctcactg cccgctttcc agtcgggaaa cctgtcgtgc cagctgcatt 6360

aatgaatcgg ccaacgcgcg gggagaggcg gtttgcgtat tgggcgccag ggtggttttt 6420

cttttcacca gtgagacggg caacagctga ttgcccttca ccgcctggcc ctgagagagt 6480

tgcagcaagc ggtccacgct ggtttgcccc agcaggcgaa aatcctgttt gatggtggtt 6540

aacggcggga tataacatga gctgtcttcg gtatcgtcgt atcccactac cgagatatcc 6600

gcaccaacgc gcagcccgga ctcggtaatg gcgcgcattg cgcccagcgc catctgatcg 6660

ttggcaacca gcatcgcagt gggaacgatg ccctcattca gcatttgcat ggtttgttga 6720

aaaccggaca tggcactcca gtcgccttcc cgttccgcta tcggctgaat ttgattgcga 6780

gtgagatatt tatgccagcc agccagacgc agacgcgccg agacagaact taatgggccc 6840

gctaacagcg cgatttgctg gtgacccaat gcgaccagat gctccacgcc cagtcgcgta 6900

ccgtcttcat gggagaaaat aatactgttg atgggtgtct ggtcagagac atcaagaaat 6960

aacgccggaa cattagtgca ggcagcttcc acagcaatgg catcctggtc atccagcgga 7020

tagttaatga tcagcccact gacgcgttgc gcgagaagat tgtgcaccgc cgctttacag 7080

gcttcgacgc cgcttcgttc taccatcgac accaccacgc tggcacccag ttgatcggcg 7140

cgagatttaa tcgccgcgac aatttgcgac ggcgcgtgca gggccagact ggaggtggca 7200

acgccaatca gcaacgactg tttgcccgcc agttgttgtg ccacgcggtt gggaatgtaa 7260

ttcagctccg ccatcgccgc ttccactttt tcccgcgttt tcgcagaaac gtggctggcc 7320

tggttcacca cgcgggaaac ggtctgataa gagacaccgg catactctgc gacatcgtat 7380

aacgttactg gtttcacatt caccaccctg aattgactct cttccgggcg ctatcatgcc 7440

ataccgcgaa aggttttgcg ccattcgatg gtgtccggga tctcgacgct ctcccttatg 7500

cgactcctgc attaggaagc agcccagtag taggttgagg ccgttgagca ccgccgccgc 7560

aaggaatggt gcatgcaagg agatggcgcc caacagtccc ccggccacgg ggcctgccac 7620

catacccacg ccgaaacaag cgctcatgag cccgaagtgg cgagcccgat cttccccatc 7680

ggtgatgtcg gcgatatagg cgccagcaac cgcacctgtg gcgccggtga tgccggccac 7740

gatgcgtccg gcgtagagga tcgagatctc gatcccgcga aattaatacg actcactata      7800

<210>  10
<211>  864
<212>  PRT
<213>  Artificial Sequence

<220>
<223>  NVL[human]-IF1-H6

<400>  10

Met Lys Pro Arg Pro Ala Gly Phe Val Asp Asn Lys Leu Lys Gln Arg
1               5                   10                  15

Val Ile Gln Tyr Leu Thr Ser Asn Lys Cys Gly Lys Tyr Val Asp Ile
            20                  25                  30

Gly Val Leu Ala Ser Asp Leu Gln Arg Val Tyr Ser Ile Asp Tyr Gly
        35                  40                  45

Arg Arg Lys Arg Asn Ala Phe Arg Ile Gln Val Glu Lys Val Phe Ser
    50                  55                  60

Ile Ile Ser Ser Glu Lys Glu Leu Lys Asn Leu Thr Glu Leu Glu Asp
65                  70                  75                  80

Glu His Leu Ala Lys Arg Ala Arg Gln Gly Glu Glu Asp Asn Glu Tyr
                85                  90                  95

Thr Glu Ser Tyr Ser Asp Asp Asp Ser Ser Met Glu Asp Tyr Pro Asp
            100                 105                 110

Pro Gln Ser Ala Asn His Met Asn Ser Ser Leu Leu Ser Leu Tyr Arg
        115                 120                 125

Lys Gly Asn Pro Asp Ser Val Ser Asn Thr Pro Glu Met Glu Gln Arg
    130                 135                 140

Glu Thr Thr Ser Ser Thr Pro Arg Ile Ser Ser Lys Thr Gly Ser Ile
145                 150                 155                 160

Pro Leu Lys Thr Pro Ala Lys Asp Ser Glu Gly Gly Trp Phe Ile Asp
                165                 170                 175

Lys Thr Pro Ser Val Lys Lys Asp Ser Phe Phe Leu Asp Leu Ser Cys
            180                 185                 190

Glu Lys Ser Asn Pro Lys Lys Pro Ile Thr Glu Ile Gln Asp Ser Lys
        195                 200                 205

Asp Ser Ser Leu Leu Glu Ser Asp Met Lys Arg Lys Gly Lys Leu Lys
    210                 215                 220

Asn Lys Gly Ser Lys Arg Lys Lys Glu Asp Leu Gln Glu Val Asp Gly
225                 230                 235                 240

Glu Ile Glu Ala Val Leu Gln Lys Lys Ala Lys Ala Arg Gly Leu Glu
                245                 250                 255

Phe Gln Ile Ser Asn Val Lys Phe Glu Asp Val Gly Gly Asn Asp Met
            260                 265                 270

Thr Leu Lys Glu Val Cys Lys Met Leu Ile His Met Arg His Pro Glu
        275                 280                 285

Val Tyr His His Leu Gly Val Val Pro Pro Arg Gly Val Leu Leu His
    290                 295                 300

Gly Pro Pro Gly Cys Gly Lys Thr Leu Leu Ala His Ala Ile Ala Gly
305                 310                 315                 320

Glu Leu Asp Leu Pro Ile Leu Lys Val Ala Ala Pro Glu Ile Val Ser
                325                 330                 335

Gly Val Ser Gly Glu Ser Glu Gln Lys Leu Arg Glu Leu Phe Glu Gln
            340                 345                 350

Ala Val Ser Asn Ala Pro Cys Ile Ile Phe Ile Asp Glu Ile Asp Ala
        355                 360                 365

Ile Thr Pro Lys Arg Glu Val Ala Ser Lys Asp Met Glu Arg Arg Ile
    370                 375                 380

Val Ala Gln Leu Leu Thr Cys Met Asp Asp Leu Asn Asn Val Ala Ala
385                 390                 395                 400

Thr Ala Arg Val Leu Val Ile Gly Ala Thr Asn Arg Pro Asp Ser Leu
                405                 410                 415

Asp Pro Ala Leu Arg Arg Ala Gly Arg Phe Asp Arg Glu Ile Cys Leu
            420                 425                 430

Gly Ile Pro Asp Glu Ala Ser Arg Glu Arg Ile Leu Gln Thr Leu Cys
        435                 440                 445

Arg Lys Leu Arg Leu Pro Gln Ala Phe Asp Phe Cys His Leu Ala His

450                 455                 460

Leu Thr Pro Gly Phe Val Gly Ala Asp Leu Met Ala Leu Cys Arg Glu
465                 470                 475                 480

Ala Ala Met Cys Ala Val Asn Arg Val Leu Met Lys Leu Gln Glu Gln
                485                 490                 495

Gln Lys Lys Asn Pro Glu Met Glu Asp Leu Pro Ser Lys Gly Val Gln
            500                 505                 510

Glu Glu Arg Leu Gly Thr Glu Pro Thr Ser Glu Thr Gln Asp Glu Leu
        515                 520                 525

Gln Arg Leu Leu Gly Leu Leu Arg Asp Gln Asp Pro Leu Ser Glu Glu
    530                 535                 540

Gln Met Gln Gly Leu Cys Ile Glu Leu Asn Asp Phe Ile Val Ala Leu
545                 550                 555                 560

Ser Ser Val Gln Pro Ser Ala Lys Arg Glu Gly Phe Val Thr Val Pro
                565                 570                 575

Asn Val Thr Trp Ala Asp Ile Gly Ala Leu Glu Asp Ile Arg Glu Glu
            580                 585                 590

Leu Thr Met Ala Ile Leu Ala Pro Val Arg Asn Pro Asp Gln Phe Lys
        595                 600                 605

Ala Leu Gly Leu Val Thr Pro Ala Gly Val Leu Leu Ala Gly Pro Pro
    610                 615                 620

Gly Cys Gly Lys Thr Leu Leu Ala Lys Ala Val Ala Asn Glu Ser Gly
625                 630                 635                 640

Leu Asn Phe Ile Ser Val Lys Gly Pro Glu Leu Leu Asn Met Tyr Val
                645                 650                 655

Gly Glu Ser Glu Arg Ala Val Arg Gln Val Phe Gln Arg Ala Lys Asn
            660                 665                 670

Ser Ala Pro Cys Val Ile Phe Phe Asp Glu Val Asp Ala Leu Cys Pro
        675                 680                 685

Arg Arg Ser Asp Arg Glu Thr Gly Ala Ser Val Arg Val Val Asn Gln
    690                 695                 700

Leu Leu Thr Glu Met Asp Gly Leu Glu Ala Arg Gln Gln Val Phe Ile
705                 710                 715                 720

Met Ala Ala Thr Asn Arg Pro Asp Ile Ile Asp Pro Ala Ile Leu Arg
                725                 730                 735

Pro Gly Arg Leu Asp Lys Thr Leu Phe Val Gly Leu Pro Pro Pro Ala
            740                 745                 750

Asp Arg Leu Ala Ile Leu Lys Thr Ile Thr Lys Asn Gly Thr Lys Pro
        755                 760                 765

Pro Leu Asp Ala Asp Val Asn Leu Glu Ala Ile Ala Gly Asp Leu Arg
    770                 775                 780

Cys Asp Cys Tyr Thr Gly Ala Asp Leu Ser Ala Leu Val Arg Glu Ala
785                 790                 795                 800

Ser Ile Cys Ala Leu Arg Gln Glu Met Ala Arg Gln Lys Ser Gly Asn
                805                 810                 815

Glu Lys Gly Glu Leu Lys Val Ser His Lys His Phe Glu Glu Ala Phe
            820                 825                 830

Lys Lys Val Arg Ser Ser Ile Ser Lys Lys Asp Gln Ile Met Tyr Glu
        835                 840                 845

Arg Leu Gln Glu Ser Leu Ser Arg Val Glu His His His His His His
    850                 855                 860

<210>  11
<211>  341
<212>  PRT
<213>  Homo sapiens

<400>  11

Met Pro Lys Arg Lys Val Thr Phe Gln Gly Val Gly Asp Glu Glu Asp
1               5                   10                  15

Glu Asp Glu Ile Ile Val Pro Lys Lys Lys Leu Val Asp Pro Val Ala
            20                  25                  30

Gly Ser Gly Gly Pro Gly Ser Arg Phe Lys Gly Lys His Ser Leu Asp
        35                  40                  45

Ser Asp Glu Glu Glu Asp Asp Asp Asp Gly Gly Ser Ser Lys Tyr Asp
    50                  55                  60

Ile Leu Ala Ser Glu Asp Val Glu Gly Gln Glu Ala Ala Thr Leu Pro
65                  70                  75                  80

Ser Glu Gly Gly Val Arg Ile Thr Pro Phe Asn Leu Gln Glu Glu Met
                85                  90                  95

Glu Glu Gly His Phe Asp Ala Asp Gly Asn Tyr Phe Leu Asn Arg Asp
            100                 105                 110

Ala Gln Ile Arg Asp Ser Trp Leu Asp Asn Ile Asp Trp Val Lys Ile
        115                 120                 125

Arg Glu Arg Pro Pro Gly Gln Arg Gln Ala Ser Asp Ser Glu Glu Glu
    130                 135                 140

Asp Ser Leu Gly Gln Thr Ser Met Ser Ala Gln Ala Leu Leu Glu Gly
145                 150                 155                 160

Leu Leu Glu Leu Leu Leu Pro Arg Glu Thr Val Ala Gly Ala Leu Arg
                165                 170                 175

Arg Leu Gly Ala Arg Gly Gly Gly Lys Gly Arg Lys Gly Pro Gly Gln
            180                 185                 190

Pro Ser Ser Pro Gln Arg Leu Asp Arg Leu Ser Gly Leu Ala Asp Gln
        195                 200                 205

Met Val Ala Arg Gly Asn Leu Gly Val Tyr Gln Glu Thr Arg Glu Arg
    210                 215                 220

Leu Ala Met Arg Leu Lys Gly Leu Gly Cys Gln Thr Leu Gly Pro His
225                 230                 235                 240

Asn Pro Thr Pro Pro Pro Ser Leu Asp Met Phe Ala Glu Glu Leu Ala
                245                 250                 255

Glu Glu Glu Leu Glu Thr Pro Thr Pro Thr Gln Arg Gly Glu Ala Glu
            260                 265                 270

Ser Arg Gly Asp Gly Leu Val Asp Val Met Trp Glu Tyr Lys Trp Glu
        275                 280                 285

Asn Thr Gly Asp Ala Glu Leu Tyr Gly Pro Phe Thr Ser Ala Gln Met
    290                 295                 300

Gln Thr Trp Val Ser Glu Gly Tyr Phe Pro Asp Gly Val Tyr Cys Arg
305                 310                 315                 320

Lys Leu Asp Pro Pro Gly Gly Gln Phe Tyr Asn Ser Lys Arg Ile Asp
                325                 330                 335

Phe Asp Leu Tyr Thr
            340

<210>  12
<211>  1026
<212>  DNA
<213>  Artificial Sequence

<220>
<223>  CD2BP2, Acc. BC001947

<400>  12
atgccaaaga ggaaagtgac cttccaaggc gtgggagatg aggaggatga ggatgaaatc      60

attgtcccca agaagaagct ggtggaccct gtggctgggt cagggggtcc tgggagccgc      120

tttaaaggca aacactcttt ggatagcgat gaggaggagg atgatgatga tggggggtcc      180

agcaaatatg acatcttggc ctcagaggat gtagaaggtc aggaggcagc cacactcccc      240

agcgaggggg gtgttcggat cacacccttt aacctgcagg aggagatgga ggaaggccac      300

tttgatgccg atggcaacta cttcctgaac cgggatgctc agatccgaga cagctggctg      360

gacaacattg actgggtgaa gatccgggag cggccacctg gccagcgcca ggcctcagac      420

tcggaggagg aggacagctt gggccagacc tcaatgagtg cccaagccct cttggaggga      480

cttttggagc tcctattgcc tagagagaca gtggctgggg cactgaggcg tctgggggcc      540

cgaggaggag gcaaagggag aaaggggcct gggcaaccca gttcccctca gcgcctggac      600

cggctctccg ggttggccga ccagatggtg gcccggggca accttggtgt gtaccaggaa      660

acaagggaac ggttggctat gcgtctgaag ggtttggggt gtcagaccct aggaccccac      720

aatcccacac ccccaccctc cctggacatg ttcgctgagg agttggcgga ggaggaactg      780

gagaccccaa cccctaccca gagaggagaa gcagagtcgc ggggagatgg tctggtggat      840

gtgatgtggg aatataagtg ggagaacacg ggggatgccg agctgtatgg gcccttcacc      900

agcgcccaga tgcagacctg ggtgagtgaa ggctacttcc cggacggtgt ttattgccgg      960

aagctggacc cccctggtgg tcagttctac aactccaaac gcattgactt tgacctctac     1020

acctga                                                                1026

<210>  13
<211>  543
<212>  DNA
<213>  Artificial Sequence

<220>
<223>  CD2BP2, gene synthesis fragment 1, aa1-173

<400>  13
agcgaagacg gcatgccaaa gaggaaagtg accttccaag gcgtgggaga tgaggaggat      60

gaggatgaaa tcattgtccc caagaagaag ctggtggacc ctgtggctgg gtcagggggt     120

cctgggagcc gctttaaagg caaacactct ttggatagcg atgaggagga ggatgatgat     180

gatggggggt ccagcaaata tgacatcttg gcctcagagg atgtagaagg tcaggaggca     240

gccacactcc ccagcgaggg gggtgttcgg atcacaccct ttaacctgca ggaggagatg     300

gaggaaggcc actttgatgc cgatggcaac tacttcctga accgggatgc tcagatccga     360

gacagctggc tggacaacat tgactgggtg aagatccggg agcggccacc tggccagcgc     420

caggcctcag actcggagga ggaggacagc ttgggccaga cctcaatgag tgcccaagcc     480

ctcttggagg gacttttgga gctcctattg cctagagaga cagtggctgg ggccgtcttc     540

acc                                                                   543

<210>  14
<211>  536
<212>  DNA
<213>  Artificial Sequence

<220>
<223>  CD2BP2, gene synthesis fragment 2, aa174-341

<400>  14
agcgaagacc cggggcactg aggcgtctgg gggcccgagg aggaggcaaa gggagaaagg      60

ggcctgggca acccagttcc cctcagcgcc tggaccggct ctccgggttg gccgaccaga     120

tggtggcccg gggcaacctt ggtgtgtacc aggaaacaag ggaacggttg gctatgcgtc     180

tgaagggttt ggggtgtcag accctaggac cccacaatcc cacaccccca ccctccctgg     240

acatgttcgc tgaggagttg gcggaggagg aactggagac cccaacccct acccagagag     300

gagaagcaga gtcgcgggga gatggtctgg tggatgtgat gtgggaatat aagtgggaga     360

acacggggga tgccgagctg tatgggccct tcaccagcgc ccagatgcag acctgggtga     420

gtgaaggcta cttcccggac ggtgtttatt gccggaagct ggacccccct ggtggtcagt     480

tctacaactc caaacgcatt gactttgacc tctacacctg atcgaccgtc ttctca         536

<210>  15
<211>  517
<212>  DNA
<213>  Artificial Sequence

<220>
<223>  CD2BP2, gene synthesis fragment 1, BbsI

<400>  15
catgccaaag aggaaagtga ccttccaagg cgtgggagat gaggaggatg aggatgaaat      60

cattgtcccc aagaagaagc tggtggaccc tgtggctggg tcagggggtc ctgggagccg     120

ctttaaaggc aaacactctt tggatagcga tgaggaggag gatgatgatg atggggggtc 180

cagcaaatat gacatcttgg cctcagagga tgtagaaggt caggaggcag ccacactccc 240

cagcgagggg ggtgttcgga tcacaccctt taacctgcag gaggagatgg aggaaggcca 300

ctttgatgcc gatggcaact acttcctgaa ccgggatgct cagatccgag acagctggct 360

ggacaacatt gactgggtga agatccggga gcggccacct ggccagcgcc aggcctcaga 420

ctcggaggag gaggacagct tgggccagac ctcaatgagt gcccaagccc tcttggaggg 480

acttttggag ctcctattgc ctagagagac agtggct 517

<210> 16
<211> 510
<212> DNA
<213> Artificial Sequence

<220>
<223> CD2BP2, gene synthesis fragment 2, BbsI

<400> 16
ggggcactga ggcgtctggg ggcccgagga ggaggcaaag ggagaaaggg gcctgggcaa 60

cccagttccc ctcagcgcct ggaccggctc tccgggttgg ccgaccagat ggtggcccgg 120

ggcaaccttg gtgtgtacca ggaaacaagg gaacggttgg ctatgcgtct gaagggtttg 180

gggtgtcaga ccctaggacc ccacaatccc acacccccac cctccctgga catgttcgct 240

gaggagttgg cggaggagga actggagacc ccaaccccta cccagagagg agaagcagag 300

tcgcggggag atggtctggt ggatgtgatg tgggaatata agtgggagaa cacgggggat 360

gccgagctgt atgggccctt caccagcgcc cagatgcaga cctgggtgag tgaaggctac 420

ttcccggacg gtgtttattg ccggaagctg gacccccctg gtggtcagtt ctacaactcc 480

aaacgcattg actttgacct ctacacctga 510

<210> 17
<211> 39
<212> DNA
<213> Artificial Sequence

<220>
<223> sense CD2BP2-fragment-2

<400> 17
atagaagacc cggggcactg aggcgtctgg gggcccgag 39

<210> 18
<211> 39
<212> DNA
<213> Artificial Sequence

<220>
<223> asense CD2BP2-fragment-2

<400>  18
tatgaagacg gtcgagggtg tagaggtcaa agtcaatgc                              39


<210>  19
<211>  534
<212>  DNA
<213>  Artificial Sequence

<220>
<223>  CD2BP2-PCR-fragment

<400>  19
atagaagacc cggggcactg aggcgtctgg gggcccgagg aggaggcaaa gggagaaagg       60

ggcctgggca acccagttcc cctcagcgcc tggaccggct ctccgggttg gccgaccaga      120

tggtggcccg gggcaacctt ggtgtgtacc aggaaacaag ggaacggttg gctatgcgtc      180

tgaagggttt ggggtgtcag accctaggac cccacaatcc cacaccccca ccctccctgg      240

acatgttcgc tgaggagttg gcggaggagg aactggagac cccaacccct acccagagag      300

gagaagcaga gtcgcgggga gatggtctgg tggatgtgat gtgggaatat aagtgggaga      360

acacggggga tgccgagctg tatgggccct tcaccagcgc ccagatgcag acctgggtga      420

gtgaaggcta cttcccggac ggtgtttatt gccggaagct ggacccccct ggtggtcagt      480

tctacaactc caaacgcatt gactttgacc tctacaccct cgaccgtctt cata            534


<210>  20
<211>  6637
<212>  DNA
<213>  Artificial Sequence

<220>
<223>  pTriEx-1-CD2BP2[human]

<400>  20
ggagtcgctg cgacgctgcc ttcgccccgt gccccgctcc gccgccgcct cgcgccgccc       60

gccccggctc tgactgaccg cgttactccc acaggtgagc gggcgggacg gcccttctcc      120

ttcgggctgt aattagcgct tggtttaatg acggcttgtt tcttttctgt ggctgcgtga       180

aagccttgag gggctccggg agggcccttt gtgcgggggg agcggctcgg ggctgtccgc      240

ggggggacgg ctgccttcgg gggggacggg gcagggcggg gttcggcttc tggcgtgtga       300

ccggcggctc tagagcctct gctaaccatg ttcatgcctt cttctttttc ctacagctcc      360

tgggcaacgt gctggttatt gtgctgtctc atcattttgg caaagaattg gatcggaccg      420

aaattaatac gactcactat aggggaattg tgagcggata acaattcccc ggagttaatc      480

cgggaccttt aattcaaccc aacacaatat attatagtta aataagaatt attatcaaat      540

catttgtata ttaattaaaa tactatactg taaattacat tttatttaca atcaaaggag      600

atataccatg ccaaagagga aagtgacctt ccaaggcgtg ggagatgagg aggatgagga      660

tgaaatcatt gtccccaaga agaagctggt ggaccctgtg gctgggtcag ggggtcctgg 720

gagccgcttt aaaggcaaac actctttgga tagcgatgag gaggaggatg atgatgatgg 780

ggggtccagc aaatatgaca tcttggcctc agaggatgta gaaggtcagg aggcagccac 840

actccccagc gaggggggtg ttcggatcac accctttaac ctgcaggagg agatggagga 900

aggccacttt gatgccgatg gcaactactt cctgaaccgg gatgctcaga tccgagacag 960

ctggctggac aacattgact gggtgaagat ccgggagcgg ccacctggcc agcgccaggc 1020

ctcagactcg gaggaggagg acagcttggg ccagacctca atgagtgccc aagccctctt 1080

ggagggactt ttggagctcc tattgcctag agagacagtg gctggggcac tgaggcgtct 1140

gggggcccga ggaggaggca aagggagaaa ggggcctggg caacccagtt cccctcagcg 1200

cctggaccgg ctctccgggt tggccgacca gatggtggcc cggggcaacc ttggtgtgta 1260

ccaggaaaca agggaacggt tggctatgcg tctgaagggt ttggggtgtc agaccctagg 1320

accccacaat cccacacccc caccctccct ggacatgttc gctgaggagt tggcggagga 1380

ggaactggag accccaaccc ctacccagag aggagaagca gagtcgcggg gagatggtct 1440

ggtggatgtg atgtgggaat ataagtggga gaacacgggg gatgccgagc tgtatgggcc 1500

cttcaccagc gcccagatgc agacctgggt gagtgaaggc tacttcccgg acggtgttta 1560

ttgccggaag ctggaccccc ctggtggtca gttctacaac tccaaacgca ttgactttga 1620

cctctacacc ctcgagcacc accatcacca tcaccatcac taagtgatta acctcaggtg 1680

caggctgcct atcagaaggt ggtggctggt gtggccaatg ccctggctca caaataccac 1740

tgagatcgat ctttttccct ctgccaaaaa ttatggggac atcatgaagc cccttgagca 1800

tctgacttct ggctaataaa ggaaatttat tttcattgca atagtgtgtt ggaatttttt 1860

gtgtctctca ctcggaagga catatgggag ggcaaatcat ttaaaacatc agaatgagta 1920

tttggtttag agtttggcaa catatgccca tatgtaacta gcataacccc ttggggcctc 1980

taaacgggtc ttgaggggtt ttttgctgaa agcatgcgga ggaaattctc cttgaagttt 2040

ccctggtgtt caaagtaaag gagtttgcac cagacgcacc tctgttcact ggtccggcgt 2100

attaaaacac gatacattgt tattagtaca tttattaagc gctagattct gtgcgttgtt 2160

gatttacaga caattgttgt acgtatttta ataattcatt aaatttataa tctttagggt 2220

ggtatgttag agcgaaaatc aaatgatttt cagcgtcttt atatctgaat ttaaatatta 2280

aatcctcaat agatttgtaa aataggtttc gattagtttc aaacaagggt tgtttttccg 2340

aaccgatggc tggactatct aatggatttt cgctcaacgc cacaaaactt gccaaatctt 2400

gtagcagcaa tctagctttg tcgatattcg tttgtgtttt gttttgtaat aaaggttcga 2460

cgtcgttcaa aatattatgc gcttttgtat ttctttcatc actgtcgtta gtgtacaatt 2520

gactcgacgt aaacacgtta aatagagctt ggacatattt aacatcgggc gtgttagctt 2580

tattaggccg attatcgtcg tcgtcccaac cctcgtcgtt agaagttgct tccgaagacg 2640

attttgccat agccacacga cgcctattaa ttgtgtcggc taacacgtcc gcgatcaaat 2700

ttgtagttga gctttttgga attatttctg attgcgggcg tttttgggcg ggtttcaatc 2760

taactgtgcc cgattttaat tcagacaaca cgttagaaag cgatggtgca ggcggtggta 2820

acatttcaga cggcaaatct actaatggcg gcggtggtgg agctgatgat aaatctacca 2880

tcggtggagg cgcaggcggg gctggcggcg gaggcggagg cggaggtggt ggcggtgatg 2940

cagacggcgg tttaggctca aatgtctctt taggcaacac agtcggcacc tcaactattg 3000

tactggtttc gggcgccgtt tttggtttga ccggtctgag acgagtgcga tttttttcgt 3060

ttctaatagc ttccaacaat tgttgtctgt cgtctaaagg tgcagcgggt tgaggttccg 3120

tcggcattgg tggagcgggc ggcaattcag acatcgatgg tggtggtggt ggtggaggcg 3180

ctggaatgtt aggcacggga gaaggtggtg gcggcggtgc cgccggtata atttgttctg 3240

gtttagtttg ttcgcgcacg attgtgggca ccggcgcagg cgccgctggc tgcacaacgg 3300

aaggtcgtct gcttcgaggc agcgcttggg gtggtggcaa ttcaatatta taattggaat 3360

acaaatcgta aaaatctgct ataagcattg taatttcgct atcgtttacc gtgccgatat 3420

ttaacaaccg ctcaatgtaa gcaattgtat tgtaaagaga ttgtctcaag ctcggaacgc 3480

tgcgctcggt cgttcggctg cggcgagcgg tatcagctca ctcaaaggcg gtaatacggt 3540

tatccacaga atcaggggat aacgcaggaa agaacatgtg agcaaaaggc cagcaaaagg 3600

ccaggaaccg taaaaaggcc gcgttgctgg cgtttttcca taggctccgc ccccctgacg 3660

agcatcacaa aaatcgacgc tcaagtcaga ggtggcgaaa cccgacagga ctataaagat 3720

accaggcgtt tccccctgga agctccctcg tgcgctctcc tgttccgacc ctgccgctta 3780

ccggatacct gtccgccttt ctcccttcgg gaagcgtggc gctttctcaa tgctcacgct 3840

gtaggtatct cagttcggtg taggtcgttc gctccaagct gggctgtgtg cacgaacccc 3900

ccgttcagcc cgaccgctgc gccttatccg gtaactatcg tcttgagtcc aacccggtaa 3960

gacacgactt atcgccactg gcagcagcca ctggtaacag gattagcaga gcgaggtatg 4020

taggcggtgc tacagagttc ttgaagtggt ggcctaacta cggctacact agaaggacag 4080

tatttggtat ctgcgctctg ctgaagccag ttaccttcgg aaaaagagtt ggtagctctt 4140

gatccggcaa acaaaccacc gctggtagcg gtggtttttt tgtttgcaag cagcagatta 4200

cgcgcagaaa aaaaggatct caagaagatc ctttgttacc aatgcttaat cagtgaggca 4260

cctatctcag cgatctgtct atttcgttca tccatagttg cctgactccc cgtcgtgtag 4320

ataactacga tacgggaggg cttaccatct ggccccagtg ctgcaatgat accgcgagac 4380

ccacgctcac cggctccaga tttatcagca ataaaccagc cagccggaag ggccgagcgc 4440

agaagtggtc ctgcaacttt atccgcctcc atccagtcta ttaattgttg ccgggaagct 4500

agagtaagta gttcgccagt taatagtttg cgcaacgttg ttgccattgc tacaggcatc 4560

gtggtgtcac gctcgtcgtt tggtatggct tcattcagct ccggttccca acgatcaagg 4620

cgagttacat gatcccccat gttgtgcaaa aaagcggtta gctccttcgg tcctccgatc 4680

gttgtcagaa gtaagttggc cgcagtgtta tcactcatgg ttatggcagc actgcataat 4740

tctcttactg tcatgccatc cgtaagatgc ttttctgtga ctggtgagta ctcaaccaag 4800

tcattctgag aatagtgtat gcggcgaccg agttgctctt gcccggcgtc aatacgggat 4860

aataccgcgc cacatagcag aactttaaaa gtgctcatca ttggaaaacg ttcttcgggg 4920

cgaaaactct caaggatctt accgctgttg agatccagtt cgatgtaacc cactcgtgca 4980

cccaactgat cttcagcatc ttttactttc accagcgttt ctgggtgagc aaaaacagga 5040

aggcaaaatg ccgcaaaaaa gggaataagg gcgacacgga aatgttgaat actcatactc 5100

ttcctttttc aatattattg aagcatttat cagggttatt gtctcatgtc cgcgcgtttc 5160

ctgcatcttt taatcaaatc ccaagatgtg tataaaccac caaactgcca aaaaatgaaa 5220

actgtcgaca agctctgtcc gtttgctggc aactgcaagg gtctcaatcc tatttgtaat 5280

tattgaataa taaaacaatt ataaatgtca aatttgtttt ttattaacga tacaaaccaa 5340

acgcaacaag aacatttgta gtattatcta taattgaaaa cgcgtagtta taatcgctga 5400

ggtaatattt aaaatcattt tcaaatgatt cacagttaat ttgcgacaat ataattttat 5460

tttcacataa actagacgcc ttgtcgtctt cttcttcgta ttccttctct tttcattttt 5520

tctcttcata aaaattaaca tagttattat cgtatccata tatgtatcta tcgtatagag 5580

taaatttttt gttgtcataa atatatatgt cttttttaat ggggtgtata gtaccgctgc 5640

gcatagtttt tctgtaattt acaacagtgc tattttctgg tagttcttcg gagtgtgttg 5700

ctttaattat taaatttata taatcaatga atttgggatc gtcggttttg tacaatatgt 5760

tgccggcata gtacgcagct tcttctagtt caattacacc attttttagc agcaccggat 5820

taacataact ttccaaaatg ttgtacgaac cgttaaacaa aaacagttca cctccctttt 5880

ctatactatt gtctgcgagc agttgtttgt tgttaaaaat aacagccatt gtaatgagac 5940

gcacaaacta atatcacaaa ctggaaatgt ctatcaatat atagttgctc tagttattaa 6000

tagtaatcaa ttacggggtc attagttcat agcccatata tggagttccg cgttacataa 6060

cttacggtaa atggcccgcc tggctgaccg cccaacgacc cccgcccatt gacgtcaata 6120

atgacgtatg ttcccatagt aacgccaata gggactttcc attgacgtca atgggtggac 6180

tatttacggt aaactgccca cttggcagta catcaagtgt atcatatgcc aagtacgccc 6240

cctattgacg tcaatgacgg taaatggccc gcctggcatt atgcccagta catgacctta 6300

tgggactttc ctacttggca gtacatctac gtattagtca tcgctattac catgcatggt 6360

cgaggtgagc cccacgttct gcttcactct ccccatctcc cccccctccc cacccccaat 6420

tttgtattta tttatttttt aattattttg tgcagcgatg ggggcggggg gggggggggg 6480

gcgcgcgcca ggcggggcgg ggcggggcga ggggcggggc ggggcgaggc ggagaggtgc 6540

ggcggcagcc aatcagagcg gcgcgctccg aaagtttcct tttatggcga ggcggcggcg 6600

gcggcggccc tataaaaagc gaagcgcgcg gcgggcg 6637

<210> 21
<211> 351
<212> PRT
<213> Artificial Sequence

<220>
<223> CD2BP2[human]-H8

<400> 21

Met Pro Lys Arg Lys Val Thr Phe Gln Gly Val Gly Asp Glu Glu Asp
1               5                   10                  15

Glu Asp Glu Ile Ile Val Pro Lys Lys Lys Leu Val Asp Pro Val Ala
            20                  25                  30

Gly Ser Gly Gly Pro Gly Ser Arg Phe Lys Gly Lys His Ser Leu Asp
        35                  40                  45

Ser Asp Glu Glu Glu Asp Asp Asp Asp Gly Gly Ser Ser Lys Tyr Asp
    50                  55                  60

Ile Leu Ala Ser Glu Asp Val Glu Gly Gln Glu Ala Ala Thr Leu Pro
65                  70                  75                  80

Ser Glu Gly Gly Val Arg Ile Thr Pro Phe Asn Leu Gln Glu Glu Met
                85                  90                  95

Glu Glu Gly His Phe Asp Ala Asp Gly Asn Tyr Phe Leu Asn Arg Asp
            100                 105                 110

Ala Gln Ile Arg Asp Ser Trp Leu Asp Asn Ile Asp Trp Val Lys Ile
        115                 120                 125

Arg Glu Arg Pro Pro Gly Gln Arg Gln Ala Ser Asp Ser Glu Glu Glu
    130                 135                 140

Asp Ser Leu Gly Gln Thr Ser Met Ser Ala Gln Ala Leu Leu Glu Gly
145                 150                 155                 160

Leu Leu Glu Leu Leu Leu Pro Arg Glu Thr Val Ala Gly Ala Leu Arg

                165                 170                 175

Arg Leu Gly Ala Arg Gly Gly Gly Lys Gly Arg Lys Gly Pro Gly Gln
            180                 185                 190

Pro Ser Ser Pro Gln Arg Leu Asp Arg Leu Ser Gly Leu Ala Asp Gln
        195                 200                 205

Met Val Ala Arg Gly Asn Leu Gly Val Tyr Gln Glu Thr Arg Glu Arg
    210                 215                 220

Leu Ala Met Arg Leu Lys Gly Leu Gly Cys Gln Thr Leu Gly Pro His
225                 230                 235                 240

Asn Pro Thr Pro Pro Pro Ser Leu Asp Met Phe Ala Glu Glu Leu Ala
                245                 250                 255

Glu Glu Glu Leu Glu Thr Pro Thr Pro Thr Gln Arg Gly Glu Ala Glu
            260                 265                 270

Ser Arg Gly Asp Gly Leu Val Asp Val Met Trp Glu Tyr Lys Trp Glu
        275                 280                 285

Asn Thr Gly Asp Ala Glu Leu Tyr Gly Pro Phe Thr Ser Ala Gln Met
    290                 295                 300

Gln Thr Trp Val Ser Glu Gly Tyr Phe Pro Asp Gly Val Tyr Cys Arg
305                 310                 315                 320

Lys Leu Asp Pro Pro Gly Gly Gln Phe Tyr Asn Ser Lys Arg Ile Asp
                325                 330                 335

Phe Asp Leu Tyr Thr Leu Glu His His His His His His His His
            340                 345                 350

<210> 22
<211> 6273
<212> DNA
<213> Artificial Sequence

<220>
<223> pET24d-N-CD2BP2[human]

<400> 22
ggggaattgt gagcggataa caattcccct ctagaaataa ttttgtttaa ctttaagaag 60

gagatatacc atgagccatc atcatcatca tcatcatcat tccatgccaa agaggaaagt 120

gaccttccaa ggcgtgggag atgaggagga tgaggatgaa atcattgtcc ccaagaagaa 180

gctggtggac cctgtggctg ggtcaggggg tcctgggagc cgctttaaag gcaaacactc 240

tttggatagc gatgaggagg aggatgatga tgatgggggg tccagcaaat atgacatctt 300

ggcctcagag gatgtagaag gtcaggaggc agccacactc cccagcgagg ggggtgttcg 360

gatcacaccc tttaacctgc aggaggagat ggaggaaggc cactttgatg ccgatggcaa 420

ctacttcctg aaccgggatg ctcagatccg agacagctgg ctggacaaca ttgactgggt 480

gaagatccgg gagcggccac ctggccagcg ccaggcctca gactcggagg aggaggacag 540

cttgggccag acctcaatga gtgcccaagc cctcttggag ggacttttgg agctcctatt 600

gcctagagag acagtggctg gggcactgag gcgtctgggg gcccgaggag gaggcaaagg 660

gagaaagggg cctgggcaac ccagttcccc tcagcgcctg gaccggctct ccgggttggc 720

cgaccagatg gtggcccggg gcaaccttgg tgtgtaccag gaaacaaggg aacggttggc 780

tatgcgtctg aagggtttgg ggtgtcagac cctaggaccc cacaatccca cacccccacc 840

ctccctggac atgttcgctg aggagttggc ggaggaggaa ctggagaccc caacccctac 900

ccagagagga gaagcagagt cgcggggaga tggtctggtg gatgtgatgt gggaatataa 960

gtgggagaac acgggggatg ccgagctgta tgggcccttc accagcgccc agatgcagac 1020

ctgggtgagt gaaggctact tcccggacgg tgtttattgc cggaagctgg acccccctgg 1080

tggtcagttc tacaactcca aacgcattga ctttgacctc tacacctgat cgagtgagat 1140

ccggctgcta acaaagcccg aaaggaagct gagttggctg ctgccaccgc tgagcaataa 1200

ctagcataac cccttggggc ctctaaacgg gtcttgaggg gttttttgct gaaaggagga 1260

actatatccg gattggcgaa tgggacgcgc cctgtagcgg cgcattaagc gcggcgggtg 1320

tggtggttac gcgcagcgtg accgctacac ttgccagcgc cctagcgccc gctcctttcg 1380

ctttcttccc ttcctttctc gccacgttcg ccggctttcc ccgtcaagct ctaaatcggg 1440

ggctcccttt agggttccga tttagtgctt tacggcacct cgaccccaaa aaacttgatt 1500

agggtgatgg ttcacgtagt gggccatcgc cctgatagac ggtttttcgc cctttgacgt 1560

tggagtccac gttctttaat agtggactct tgttccaaac tggaacaaca ctcaacccta 1620

tctcggtcta ttcttttgat ttataaggga ttttgccgat ttcggcctat tggttaaaaa 1680

atgagctgat ttaacaaaaa tttaacgcga attttaacaa aatattaacg tttacaattt 1740

caggtggcac ttttcgggga aatgtgcgcg gaacccctat ttgtttattt ttctaaatac 1800

attcaaatat gtatccgctc atgaattaat tcttagaaaa actcatcgag catcaaatga 1860

aactgcaatt tattcatatc aggattatca ataccatatt tttgaaaaag ccgtttctgt 1920

aatgaaggag aaaactcacc gaggcagttc cataggatgg caagatcctg gtatcggtct 1980

gcgattccga ctcgtccaac atcaatacaa cctattaatt tcccctcgtc aaaaataagg 2040

ttatcaagtg agaaatcacc atgagtgacg actgaatccg gtgagaatgg caaaagttta 2100

tgcatttctt tccagacttg ttcaacaggc cagccattac gctcgtcatc aaaatcactc 2160

gcatcaacca aaccgttatt cattcgtgat tgcgcctgag cgagacgaaa tacgcgatcg 2220

ctgttaaaag gacaattaca aacaggaatc gaatgcaacc ggcgcaggaa cactgccagc 2280

gcatcaacaa tattttcacc tgaatcagga tattcttcta atacctggaa tgctgttttc 2340

ccggggatcg cagtggtgag taaccatgca tcatcaggag tacggataaa atgcttgatg 2400

gtcggaagag gcataaattc cgtcagccag tttagtctga ccatctcatc tgtaacatca 2460

ttggcaacgc tacctttgcc atgtttcaga aacaactctg gcgcatcggg cttcccatac 2520

aatcgataga ttgtcgcacc tgattgcccg acattatcgc gagcccattt atacccatat 2580

aaatcagcat ccatgttgga atttaatcgc ggcctagagc aagacgtttc ccgttgaata 2640

tggctcataa caccccttgt attactgttt atgtaagcag acagttttat tgttcatgac 2700

caaaatccct taacgtgagt tttcgttcca ctgagcgtca gaccccgtag aaaagatcaa 2760

aggatcttct tgagatcctt tttttctgcg cgtaatctgc tgcttgcaaa caaaaaaacc 2820

accgctacca gcggtggttt gtttgccgga tcaagagcta ccaactcttt ttccgaaggt 2880

aactggcttc agcagagcgc agataccaaa tactgtcctt ctagtgtagc cgtagttagg 2940

ccaccacttc aagaactctg tagcaccgcc tacatacctc gctctgctaa tcctgttacc 3000

agtggctgct gccagtggcg ataagtcgtg tcttaccggg ttggactcaa gacgatagtt 3060

accggataag gcgcagcggt cgggctgaac ggggggttcg tgcacacagc ccagcttgga 3120

gcgaacgacc tacaccgaac tgagatacct acagcgtgag ctatgagaaa gcgccacgct 3180

tcccgaaggg agaaaggcgg acaggtatcc ggtaagcggc agggtcggaa caggagagcg 3240

cacgagggag cttccagggg gaaacgcctg gtatctttat agtcctgtcg ggtttcgcca 3300

cctctgactt gagcgtcgat ttttgtgatg ctcgtcaggg gggcggagcc tatggaaaaa 3360

cgccagcaac gcggcctttt tacggttcct ggccttttgc tggccttttg ctcacatgtt 3420

ctttcctgcg ttatcccctg attctgtgga taaccgtatt accgcctttg agtgagctga 3480

taccgctcgc cgcagccgaa cgaccgagcg cagcgagtca gtgagcgagg aagcggaaga 3540

gcgcctgatg cggtattttc tccttacgca tctgtgcggt atttcacacc gcatatatgg 3600

tgcactctca gtacaatctg ctctgatgcc gcatagttaa gccagtatac actccgctat 3660

cgctacgtga ctgggtcatg gctgcgcccc gacacccgcc aacacccgct gacgcgccct 3720

gacgggcttg tctgctcccg gcatccgctt acagacaagc tgtgaccgtc tccgggagct 3780

gcatgtgtca gaggttttca ccgtcatcac cgaaacgcgc gaggcagctg cggtaaagct 3840

catcagcgtg gtcgtgaagc gattcacaga tgtctgcctg ttcatccgcg tccagctcgt 3900

tgagtttctc cagaagcgtt aatgtctggc ttctgataaa gcgggccatg ttaagggcgg 3960

ttttttcctg tttggtcact gatgcctccg tgtaaggggg atttctgttc atgggggtaa 4020

tgataccgat gaaacgagag aggatgctca cgatacgggt tactgatgat gaacatgccc 4080

ggttactgga acgttgtgag ggtaaacaac tggcggtatg gatgcggcgg gaccagagaa 4140

aaatcactca gggtcaatgc cagcgcttcg ttaatacaga tgtaggtgtt ccacagggta 4200

gccagcagca tcctgcgatg cagatccgga acataatggt gcagggcgct gacttccgcg 4260

tttccagact ttacgaaaca cggaaaccga agaccattca tgttgttgct caggtcgcag 4320

acgttttgca gcagcagtcg cttcacgttc gctcgcgtat cggtgattca ttctgctaac 4380

cagtaaggca accccgccag cctagccggg tcctcaacga caggagcacg atcatgcgca 4440

cccgtggggc cgccatgccg gcgataatgg cctgcttctc gccgaaacgt ttggtggcgg 4500

gaccagtgac gaaggcttga gcgagggcgt gcaagattcc gaataccgca agcgacaggc 4560

cgatcatcgt cgcgctccag cgaaagcggt cctcgccgaa aatgacccag agcgctgccg 4620

gcacctgtcc tacgagttgc atgataaaga agacagtcat aagtgcggcg acgatagtca 4680

tgccccgcgc ccaccggaag gagctgactg ggttgaaggc tctcaagggc atcggtcgag 4740

atcccggtgc ctaatgagtg agctaactta cattaattgc gttgcgctca ctgcccgctt 4800

tccagtcggg aaacctgtcg tgccagctgc attaatgaat cggccaacgc gcggggagag 4860

gcggtttgcg tattgggcgc cagggtggtt tttcttttca ccagtgagac gggcaacagc 4920

tgattgccct tcaccgcctg gccctgagag agttgcagca agcggtccac gctggtttgc 4980

cccagcaggc gaaaatcctg tttgatggtg gttaacggcg ggatataaca tgagctgtct 5040

tcggtatcgt cgtatcccac taccgagata tccgcaccaa cgcgcagccc ggactcggta 5100

atggcgcgca ttgcgcccag cgccatctga tcgttggcaa ccagcatcgc agtgggaacg 5160

atgccctcat tcagcatttg catggtttgt tgaaaaccgg acatggcact ccagtcgcct 5220

tcccgttccg ctatcggctg aatttgattg cgagtgagat atttatgcca gccagccaga 5280

cgcagacgcg ccgagacaga acttaatggg cccgctaaca gcgcgatttg ctggtgaccc 5340

aatgcgacca gatgctccac gcccagtcgc gtaccgtctt catgggagaa aataatactg 5400

ttgatgggtg tctggtcaga gacatcaaga aataacgccg gaacattagt gcaggcagct 5460

tccacagcaa tggcatcctg gtcatccagc ggatagttaa tgatcagccc actgacgcgt 5520

tgcgcgagaa gattgtgcac cgccgcttta caggcttcga cgccgcttcg ttctaccatc 5580

gacaccacca cgctggcacc cagttgatcg gcgcgagatt taatcgccgc gacaatttgc 5640

gacggcgcgt gcagggccag actggaggtg gcaacgccaa tcagcaacga ctgtttgccc 5700

gccagttgtt gtgccacgcg gttgggaatg taattcagct ccgccatcgc cgcttccact 5760

ttttcccgcg ttttcgcaga aacgtggctg gcctggttca ccacgcggga aacggtctga 5820

taagagacac cggcatactc tgcgacatcg tataacgtta ctggtttcac attcaccacc 5880

ctgaattgac tctcttccgg gcgctatcat gccataccgc gaaaggtttt gcgccattcg 5940

atggtgtccg ggatctcgac gctctccctt atgcgactcc tgcattagga agcagcccag 6000

tagtaggttg aggccgttga gcaccgccgc cgcaaggaat ggtgcatgca aggagatggc 6060

gcccaacagt cccccggcca cggggcctgc caccataccc acgccgaaac aagcgctcat 6120

gagcccgaag tggcgagccc gatcttcccc atcggtgatg tcggcgatat aggcgccagc 6180

aaccgcacct gtggcgccgg tgatgccggc cacgatgcgt ccggcgtaga ggatcgagat 6240

ctcgatcccg cgaaattaat acgactcact ata 6273

<210> 23
<211> 352
<212> PRT
<213> Artificial Sequence

<220>
<223> H8-CD2BP2[human]

<400> 23

Met Ser His His His His His His His His Ser Met Pro Lys Arg Lys
1               5                   10                  15

Val Thr Phe Gln Gly Val Gly Asp Glu Glu Asp Glu Asp Glu Ile Ile
            20                  25                  30

Val Pro Lys Lys Lys Leu Val Asp Pro Val Ala Gly Ser Gly Gly Pro
        35                  40                  45

Gly Ser Arg Phe Lys Gly Lys His Ser Leu Asp Ser Asp Glu Glu Glu
    50                  55                  60

Asp Asp Asp Asp Gly Gly Ser Ser Lys Tyr Asp Ile Leu Ala Ser Glu
65                  70                  75                  80

Asp Val Glu Gly Gln Glu Ala Ala Thr Leu Pro Ser Glu Gly Gly Val
                85                  90                  95

Arg Ile Thr Pro Phe Asn Leu Gln Glu Glu Met Glu Glu Gly His Phe
            100                 105                 110

Asp Ala Asp Gly Asn Tyr Phe Leu Asn Arg Asp Ala Gln Ile Arg Asp
        115                 120                 125

Ser Trp Leu Asp Asn Ile Asp Trp Val Lys Ile Arg Glu Arg Pro Pro
    130                 135                 140

Gly Gln Arg Gln Ala Ser Asp Ser Glu Glu Glu Asp Ser Leu Gly Gln
145                 150                 155                 160

Thr Ser Met Ser Ala Gln Ala Leu Leu Glu Gly Leu Leu Glu Leu Leu
                165                 170                 175

Leu Pro Arg Glu Thr Val Ala Gly Ala Leu Arg Arg Leu Gly Ala Arg
            180                 185                 190

Gly Gly Gly Lys Gly Arg Lys Gly Pro Gly Gln Pro Ser Ser Pro Gln
        195                 200                 205

Arg Leu Asp Arg Leu Ser Gly Leu Ala Asp Gln Met Val Ala Arg Gly
    210                 215                 220

Asn Leu Gly Val Tyr Gln Glu Thr Arg Glu Arg Leu Ala Met Arg Leu
225                 230                 235                 240

Lys Gly Leu Gly Cys Gln Thr Leu Gly Pro His Asn Pro Thr Pro Pro
                245                 250                 255

Pro Ser Leu Asp Met Phe Ala Glu Glu Leu Ala Glu Glu Glu Leu Glu
            260                 265                 270

Thr Pro Thr Pro Thr Gln Arg Gly Glu Ala Glu Ser Arg Gly Asp Gly
        275                 280                 285

Leu Val Asp Val Met Trp Glu Tyr Lys Trp Glu Asn Thr Gly Asp Ala
    290                 295                 300

Glu Leu Tyr Gly Pro Phe Thr Ser Ala Gln Met Gln Thr Trp Val Ser
305                 310                 315                 320

Glu Gly Tyr Phe Pro Asp Gly Val Tyr Cys Arg Lys Leu Asp Pro Pro
                325                 330                 335

Gly Gly Gln Phe Tyr Asn Ser Lys Arg Ile Asp Phe Asp Leu Tyr Thr
            340                 345                 350

<210>  24
<211>  1668
<212>  PRT
<213>  Homo sapiens

<400>  24
   Met Ser Ala Arg Ala Ala Ala Ala Lys Ser Thr Ala Met Glu Glu Thr
   1               5                   10                  15
   Ala Ile Trp Glu Gln His Thr Val Thr Leu His Arg Ala Pro Gly Phe
               20                  25                  30
   Gly Phe Gly Ile Ala Ile Ser Gly Gly Arg Asp Asn Pro His Phe Gln
           35                  40                  45
   Ser Gly Glu Thr Ser Ile Val Ile Ser Asp Val Leu Lys Gly Gly Pro
       50                  55                  60
   Ala Glu Gly Gln Leu Gln Glu Asn Asp Arg Val Ala Met Val Asn Gly
   65                  70                  75                  80
   Val Ser Met Asp Asn Val Glu His Ala Phe Ala Val Gln Gln Leu Arg
                   85                  90                  95
   Lys Ser Gly Lys Asn Ala Lys Ile Thr Ile Arg Arg Lys Lys Lys Val
               100                 105                 110
   Gln Ile Pro Val Ser Arg Pro Asp Pro Glu Pro Val Ser Asp Asn Glu
           115                 120                 125
   Glu Asp Ser Tyr Asp Glu Glu Ile His Asp Pro Arg Ser Gly Arg Ser
       130                 135                 140
   Gly Val Val Asn Arg Arg Ser Glu Lys Ile Trp Pro Arg Asp Arg Ser
   145                 150                 155                 160
   Ala Ser Arg Glu Arg Ser Leu Ser Pro Arg Ser Asp Arg Arg Ser Val
                   165                 170                 175
   Ala Ser Ser Gln Pro Ala Lys Pro Thr Lys Val Thr Leu Val Lys Ser
               180                 185                 190
   Arg Lys Asn Glu Glu Tyr Gly Leu Arg Leu Ala Ser His Ile Phe Val
           195                 200                 205
   Lys Glu Ile Ser Gln Asp Ser Leu Ala Ala Arg Asp Gly Asn Ile Gln
       210                 215                 220
   Glu Gly Asp Val Val Leu Lys Ile Asn Gly Thr Val Thr Glu Asn Met
   225                 230                 235                 240
   Ser Leu Thr Asp Ala Lys Thr Leu Ile Glu Arg Ser Lys Gly Lys Leu
                   245                 250                 255
   Lys Met Val Val Gln Arg Asp Glu Arg Ala Thr Leu Leu Asn Val Pro
               260                 265                 270
   Asp Leu Ser Asp Ser Ile His Ser Ala Asn Ala Ser Glu Arg Asp Asp
   275 280 285
   Ile Ser Glu Ile Gln Ser Leu Ala Ser Asp His Ser Gly Arg Ser His
   290 295 300
   Asp Arg Pro Pro Arg Arg Ser Arg Ser Arg Ser Pro Asp Gln Arg Ser
   305 310 315 320
   Glu Pro Ser Asp His Ser Arg His Ser Pro Gln Gln Pro Ser Asn Gly
   325 330 335
   Ser Leu Arg Ser Arg Asp Glu Glu Arg Ile Ser Lys Pro Gly Ala Val
   340 345 350
   Ser Thr Pro Val Lys His Ala Asp Asp His Thr Pro Lys Thr Val Glu
   355 360 365
   Glu Val Thr Val Glu Arg Asn Glu Lys Gln Thr Pro Ser Leu Pro Glu
   370 375 380
   Pro Lys Pro Val Tyr Ala Gln Val Gly Gln Pro Asp Val Asp Leu Pro
   385 390 395 400
   Val Ser Pro Ser Asp Gly Val Leu Pro Asn Ser Thr His Glu Asp Gly
   405 410 415
   Ile Leu Arg Pro Ser Met Lys Leu Val Lys Phe Arg Lys Gly Asp Ser
   420 425 430
   Val Gly Leu Arg Leu Ala Gly Gly Asn Asp Val Gly Ile Phe Val Ala
   435 440 445
   Gly Val Leu Glu Asp Ser Pro Ala Ala Lys Glu Gly Leu Glu Glu Gly
   450 455 460
   Asp Gln Ile Leu Arg Val Asn Asn Val Asp Phe Thr Asn Ile Ile Arg
   465 470 475 480
   Glu Glu Ala Val Leu Phe Leu Leu Asp Leu Pro Lys Gly Glu Glu Val
   485 490 495
   Thr Ile Leu Ala Gln Lys Lys Lys Asp Val Tyr Arg Arg Ile Val Glu
   500 505 510
   Ser Asp Val Gly Asp Ser Phe Tyr Ile Arg Thr His Phe Glu Tyr Glu
   515 520 525
   Lys Glu Ser Pro Tyr Gly Leu Ser Phe Asn Lys Gly Glu Val Phe Arg
       530                 535                 540
   Val Val Asp Thr Leu Tyr Asn Gly Lys Leu Gly Ser Trp Leu Ala Ile
   545                 550                 555                 560
   Arg Ile Gly Lys Asn His Lys Glu Val Glu Arg Gly Ile Ile Pro Asn
                   565                 570                 575
   Lys Asn Arg Ala Glu Gln Leu Ala Ser Val Gln Tyr Thr Leu Pro Lys
               580                 585                 590
   Thr Ala Gly Gly Asp Arg Ala Asp Phe Trp Arg Phe Arg Gly Leu Arg
           595                 600                 605
   Ser Ser Lys Arg Asn Leu Arg Lys Ser Arg Glu Asp Leu Ser Ala Gln
       610                 615                 620
   Pro Val Gln Thr Lys Phe Pro Ala Tyr Glu Arg Val Val Leu Arg Glu
   625                 630                 635                 640
   Ala Gly Phe Leu Arg Pro Val Thr Ile Phe Gly Pro Ile Ala Asp Val
                   645                 650                 655
   Ala Arg Glu Lys Leu Ala Arg Glu Glu Pro Asp Ile Tyr Gln Ile Ala
               660                 665                 670
   Lys Ser Glu Pro Arg Asp Ala Gly Thr Asp Gln Arg Ser Ser Gly Ile
           675                 680                 685
   Ile Arg Leu His Thr Ile Lys Gln Ile Ile Asp Gln Asp Lys His Ala
       690                 695                 700
   Leu Leu Asp Val Thr Pro Asn Ala Val Asp Arg Leu Asn Tyr Ala Gln
   705                 710                 715                 720
   Trp Tyr Pro Ile Val Val Phe Leu Asn Pro Asp Ser Lys Gln Gly Val
                   725                 730                 735
   Lys Thr Met Arg Met Arg Leu Cys Pro Glu Ser Arg Lys Ser Ala Arg
               740                 745                 750
   Lys Leu Tyr Glu Arg Ser His Lys Leu Arg Lys Asn Asn His His Leu
           755                 760                 765
   Phe Thr Thr Thr Ile Asn Leu Asn Ser Met Asn Asp Gly Trp Tyr Gly
       770                 775                 780
   Ala Leu Lys Glu Ala Ile Gln Gln Gln Gln Asn Gln Leu Val Trp Val
   785                 790                 795                 800
   Ser Glu Gly Lys Ala Asp Gly Ala Thr Ser Asp Asp Leu Asp Leu His
                   805                 810                 815
   Asp Asp Arg Leu Ser Tyr Leu Ser Ala Pro Gly Ser Glu Tyr Ser Met
               820                 825                 830
   Tyr Ser Thr Asp Ser Arg His Thr Ser Asp Tyr Glu Asp Thr Asp Thr
           835                 840                 845
   Glu Gly Gly Ala Tyr Thr Asp Gln Glu Leu Asp Glu Thr Leu Asn Asp
       850                 855                 860
   Glu Val Gly Thr Pro Pro Glu Ser Ala Ile Thr Arg Ser Ser Glu Pro
   865                 870                 875                 880
   Val Arg Glu Asp Ser Ser Gly Met His His Glu Asn Gln Thr Tyr Pro
                   885                 890                 895
   Pro Tyr Ser Pro Gln Ala Gln Pro Gln Pro Ile His Arg Ile Asp Ser
               900                 905                 910
   Pro Gly Phe Lys Pro Ala Ser Gln Gln Val Tyr Arg Lys Asp Pro Tyr
           915                 920                 925
   Pro Glu Glu Met Met Arg Gln Asn His Val Leu Lys Gln Pro Ala Val
       930                 935                 940
   Ser His Pro Gly His Arg Pro Asp Lys Glu Pro Asn Leu Thr Tyr Glu
   945                 950                 955                 960
   Pro Gln Leu Pro Tyr Val Glu Lys Gln Ala Ser Arg Asp Leu Glu Gln
                   965                 970                 975
   Pro Thr Tyr Arg Tyr Glu Ser Ser Ser Tyr Thr Asp Gln Phe Ser Arg
               980                 985                 990
   Asn Tyr Glu His Arg Leu Arg Tyr  Glu Asp Arg Val Pro  Met Tyr Glu
           995                 1000                 1005
   Glu Gln  Trp Ser Tyr Tyr Asp  Asp Lys Gln Pro Tyr  Pro Ser Arg
       1010                 1015                 1020
   Pro Pro  Phe Asp Asn Gln His  Ser Gln Asp Leu Asp  Ser Arg Gln
       1025                 1030                 1035
   His Pro Glu Glu Ser Ser Glu Arg Gly Tyr Phe Pro Arg Phe Glu
   1040 1045 1050
   Glu Pro Ala Pro Leu Ser Tyr Asp Ser Arg Pro Arg Tyr Glu Gln
   1055 1060 1065
   Ala Pro Arg Ala Ser Ala Leu Arg His Glu Glu Gln Pro Ala Pro
   1070 1075 1080
   Gly Tyr Asp Thr His Gly Arg Leu Arg Pro Glu Ala Gln Pro His
   1085 1090 1095
   Pro Ser Ala Gly Pro Lys Pro Ala Glu Ser Lys Gln Tyr Phe Glu
   1100 1105 1110
   Gln Tyr Ser Arg Ser Tyr Glu Gln Val Pro Pro Gln Gly Phe Thr
   1115 1120 1125
   Ser Arg Ala Gly His Phe Glu Pro Leu His Gly Ala Ala Ala Val
   1130 1135 1140
   Pro Pro Leu Ile Pro Ser Ser Gln His Lys Pro Glu Ala Leu Pro
   1145 1150 1155
   Ser Asn Thr Lys Pro Leu Pro Pro Pro Pro Thr Gln Thr Glu Glu
   1160 1165 1170
   Glu Glu Asp Pro Ala Met Lys Pro Gln Ser Val Leu Thr Arg Val
   1175 1180 1185
   Lys Met Phe Glu Asn Lys Arg Ser Ala Ser Leu Glu Thr Lys Lys
   1190 1195 1200
   Asp Val Asn Asp Thr Gly Ser Phe Lys Pro Pro Glu Val Ala Ser
   1205 1210 1215
   Lys Pro Ser Gly Ala Pro Ile Ile Gly Pro Lys Pro Thr Ser Gln
   1220 1225 1230
   Asn Gln Phe Ser Glu His Asp Lys Thr Leu Tyr Arg Ile Pro Glu
   1235 1240 1245
   Pro Gln Lys Pro Gln Leu Lys Pro Pro Glu Asp Ile Val Arg Ser
   1250 1255 1260
   Asn His Tyr Asp Pro Glu Glu Asp Glu Glu Tyr Tyr Arg Lys Gln
   1265                1270                1275
   Leu Ser Tyr Phe Asp Arg Arg Ser Phe Glu Asn Lys Pro Pro Ala
       1280                1285                1290
   His Ile Ala Ala Ser His Leu Ser Glu Pro Ala Lys Pro Ala His
       1295                1300                1305
   Ser Gln Asn Gln Ser Asn Phe Ser Ser Tyr Ser Ser Lys Gly Lys
       1310                1315                1320
   Pro Pro Glu Ala Asp Gly Val Asp Arg Ser Phe Gly Glu Lys Arg
       1325                1330                1335
   Tyr Glu Pro Ile Gln Ala Thr Pro Pro Pro Pro Pro Leu Pro Ser
       1340                1345                1350
   Gln Tyr Ala Gln Pro Ser Gln Pro Val Thr Ser Ala Ser Leu His
       1355                1360                1365
   Ile His Ser Lys Gly Ala His Gly Glu Gly Asn Ser Val Ser Leu
       1370                1375                1380
   Asp Phe Gln Asn Ser Leu Val Ser Lys Pro Asp Pro Pro Pro Ser
       1385                1390                1395
   Gln Asn Lys Pro Ala Thr Phe Arg Pro Pro Asn Arg Glu Asp Thr
       1400                1405                1410
   Ala Gln Ala Ala Phe Tyr Pro Gln Lys Ser Phe Pro Asp Lys Ala
       1415                1420                1425
   Pro Val Asn Gly Thr Glu Gln Thr Gln Lys Thr Val Thr Pro Ala
       1430                1435                1440
   Tyr Asn Arg Phe Thr Pro Lys Pro Tyr Thr Ser Ser Ala Arg Pro
       1445                1450                1455
   Phe Glu Arg Lys Phe Glu Ser Pro Lys Phe Asn His Asn Leu Leu
       1460                1465                1470
   Pro Ser Glu Thr Ala His Lys Pro Asp Leu Ser Ser Lys Thr Pro
       1475                1480                1485
   Thr Ser Pro Lys Thr Leu Val Lys Ser His Ser Leu Ala Gln Pro
       1490                1495                1500
   Pro Glu Phe Asp Ser Gly Val Glu Thr Phe Ser Ile His Ala Glu
       1505                1510                1515
   Lys Pro Lys Tyr Gln Ile Asn Asn Ile Ser Thr Val Pro Lys Ala
       1520                1525                1530
   Ile Pro Val Ser Pro Ser Ala Val Glu Glu Asp Glu Asp Glu Asp
       1535                1540                1545
   Gly His Thr Val Val Ala Thr Ala Arg Gly Ile Phe Asn Ser Asn
       1550                1555                1560
   Gly Gly Val Leu Ser Ser Ile Glu Thr Gly Val Ser Ile Ile Ile
       1565                1570                1575
   Pro Gln Gly Ala Ile Pro Glu Gly Val Glu Gln Glu Ile Tyr Phe
       1580                1585                1590
   Lys Val Cys Arg Asp Asn Ser Ile Leu Pro Pro Leu Asp Lys Glu
       1595                1600                1605
   Lys Gly Glu Thr Leu Leu Ser Pro Leu Val Met Cys Gly Pro His
       1610                1615                1620
   Gly Leu Lys Phe Leu Lys Pro Val Glu Leu Arg Leu Pro His Cys
       1625                1630                1635
   Asp Pro Lys Thr Trp Gln Asn Lys Cys Leu Pro Gly Asp Pro Asn
       1640                1645                1650
   Tyr Leu Val Gly Ala Asn Cys Val Ser Val Leu Ile Asp His Phe
       1655                1660                1665

<210>  25
<211>  33
<212>  DNA
<213>  Artificial Sequence

<220>
<223>  sense TJP1-IF2-ZF1

<400>  25
ataggtctct catgtccgcc agagctgcgg ctg                                    33

<210>  26
<211>  38
<212>  DNA
<213>  Artificial Sequence

<220>
<223>  asense TJP1-IF2-ZF1

<400>  26
ataggtctct acacctgttg agaggctggc ttaaatcc                              38


<210>  27
<211>  35
<212>  DNA
<213>  Artificial Sequence

<220>
<223>  sense TJP1-IF2-ZF2

<400>  27
atacgtctca gtgtatagaa aggatccata tcccg                                 35


<210>  28
<211>  41
<212>  DNA
<213>  Artificial Sequence

<220>
<223>  asense TJP1-IF2-ZF2

<400>  28
atacgtctcc tcgagaaagt ggtcaataag gacagaaaca c                          41


<210>  29
<211>  2788
<212>  DNA
<213>  Artificial Sequence

<220>
<223>  TJP1-PCR-fragment-1

<400>  29
ataggtctct catgtccgcc agagctgcgg ctgccaagag cacagcaatg gaggaaacag      60

ctatatggga acaacataca gtgacgcttc acagggctcc tggatttgga tttggaattg     120

caatatctgg tggacgagat aatcctcatt ttcagagtgg ggaaacgtca atagtgattt     180

cagatgtgct gaaaggagga ccagctgaag gacagctaca ggaaaatgac cgagttgcaa     240

tggttaacgg agtttcaatg gataatgttg aacatgcttt tgctgttcag caactaagga     300

aaagtgggaa aaatgcaaaa attacaatta gaaggaagaa gaaagttcaa ataccagtaa     360

gtcgtcctga tcctgaacca gtatctgata atgaagaaga tagttatgat gaggaaatac     420

atgatccaag aagtggccgg agtggtgtgg ttaacagaag gagtgagaag atttggccga     480

gggatagaag tgcaagtaga gagaggagct tgtccccgcg gtcagacagg cggtcagtgg     540

cttccagcca gcctgctaaa cctactaaag tcacactggt gaaatcccgg aaaaatgaag     600

aatatggtct tcgattggca agccatatat ttgttaagga aatttcacaa gatagtttgg     660

cagcaagaga tggcaatatt caagaaggtg atgttgtatt gaagataaat ggtactgtga     720

cagaaaatat gtcattgaca gatgcaaaga cattgataga aaggtctaaa ggcaaattaa     780

aaatggtagt tcaaagagat gaacgggcta cgctattgaa tgtccctgat ctttctgaca 840

gcatccactc tgctaatgcc tctgagagag acgacatttc agaaattcag tcactggcat 900

cagatcattc tggtcgatca cacgataggc ctccccgccg cagccggtca cgatctcctg 960

accagcggtc agagccttct gatcattcca ggcactcgcc gcagcagcca agcaatggca 1020

gtctccggag tagagatgaa gagagaattt ctaaacctgg ggctgtctca actcctgtaa 1080

agcatgctga tgatcacaca cctaaaacag tggaagaagt tacagttgaa agaaatgaga 1140

aacaaacacc ttctcttcca gaaccaaagc ctgtgtatgc ccaagttggg caaccagatg 1200

tggatttacc tgtcagtcca tctgatggtg tcctacctaa ttcaactcat gaagatggga 1260

ttcttcggcc cagcatgaaa ttggtaaaat tcagaaaagg agatagtgtg ggtttgcggc 1320

tggctggtgg aaatgatgtt ggaatatttg tagctggcgt tctagaagat agccctgcag 1380

ccaaggaagg cttagaggaa ggtgatcaaa ttctcagggt aaacaacgta gattttacaa 1440

atatcataag agaagaagcc gtccttttcc tgcttgacct ccctaaagga gaagaagtga 1500

ccatattggc tcagaagaag aaggatgttt atcgtcgcat tgtagaatca gatgtaggag 1560

attctttcta tattagaacc cattttgaat atgaaaagga atctccctat ggacttagtt 1620

ttaacaaagg agaggtgttc cgtgttgtgg ataccttgta caatggaaaa ctgggctctt 1680

ggcttgctat tcgaattggt aaaaatcata aggaggtaga acgaggcatc atccctaata 1740

agaacagagc tgagcagcta gccagtgtac agtatacact tccaaaaaca gcaggcggag 1800

accgtgctga cttctggaga ttcagaggtc ttcgcagctc caagagaaat cttcgaaaaa 1860

gcagagagga tttgtccgct cagcctgttc aaacaaagtt tccagcttat gaaagagtgg 1920

ttcttcgaga agctggattt ctgaggcctg taaccatttt tggaccaata gctgatgttg 1980

ccagagaaaa gctggcaaga gaagaaccag atatttatca aattgcaaag agtgaaccac 2040

gagacgctgg aactgaccaa cgtagctctg gcattattcg cctgcataca ataaagcaaa 2100

tcatagatca agacaaacat gctttattag atgtaacacc aaatgcagtt gatcgtctta 2160

actatgccca gtggtatcca attgttgtat ttcttaaccc tgattctaag caaggagtaa 2220

aaacaatgag aatgaggtta tgtccagaat ctcggaaaag tgccaggaag ttatacgagc 2280

gatctcataa acttcgtaaa aataatcacc atctttttac aactacaatt aacttaaatt 2340

caatgaatga tggttggtat ggtgcgctga aagaagcaat tcaacaacag caaaaccagc 2400

tggtatgggt ttccgaggga aaggcggatg gtgctacaag tgatgacctt gatttgcatg 2460

atgatcgtct gtcctacctg tcagctccag gtagtgaata ctcaatgtat agcacggaca 2520

gtagacacac ttctgactat gaagacacag acacagaagg cggggcctac actgatcaag 2580

aactagatga aactcttaat gatgaggttg ggactccacc ggagtctgcc attacacggt 2640

cctctgagcc tgtaagagag gactcctctg gaatgcatca tgaaaaccaa acatatcctc 2700

cttactcacc acaagcgcag ccacaaccaa ttcatagaat agactcccct ggatttaagc 2760

cagcctctca acaggtgtag agacctat 2788

<210> 30
<211> 2266
<212> DNA
<213> Artificial Sequence

<220>
<223> TJP1-PCR-fragment-2

<400> 30
atacgtctca gtgtatagaa aggatccata tcccgaggaa atgatgaggc agaaccatgt 60

tttgaaacag ccagccgtta gtcacccagg gcacaggcca gacaaagagc ctaatctgac 120

ctatgaaccc caactcccat acgtagagaa acaagccagc agagacctcg agcagcccac 180

atacagatac gagtcctcaa gctatacgga ccagttttct cgaaactatg aacatcgtct 240

gcgatacgaa gatcgcgtcc ccatgtatga agaacagtgg tcatattatg atgacaaaca 300

gccctaccca tctcggccac cttttgataa tcagcactct caagaccttg actccagaca 360

gcatcccgaa gagtcctcag aacgagggta ctttccacgt tttgaagagc cagcccctct 420

gtcttacgac agcagaccac gttacgaaca ggcacctaga gcatccgccc tgcggcacga 480

agagcagcca gctcctgggt atgacacaca tggtagactc agaccggaag cccagcccca 540

cccttcagca gggcccaagc ctgcagagtc caagcagtat tttgagcaat attcacgcag 600

ttacgagcaa gtaccacccc aaggatttac ctctagagca ggtcattttg agcctctcca 660

tggtgctgca gctgtccctc cgctgatacc ttcatctcag cataagccag aagctctgcc 720

ttcaaacacc aaaccactgc ctccaccccc aactcaaacc gaagaagagg aagatccagc 780

aatgaagcca cagtctgtac tcaccagagt taagatgttt gaaaacaaaa gatctgcatc 840

cttagagacc aagaaggatg taaatgacac tggcagtttt aagcctccag aagtagcatc 900

taaaccttca ggtgctccca tcattggtcc caaacccact tctcagaatc aattcagtga 960

acatgacaaa actctgtaca ggatcccaga acctcaaaaa cctcaactga agccacctga 1020

agatattgtt cggtccaatc attatgaccc tgaagaagat gaagaatatt atcgaaaaca 1080

gctgtcatac tttgaccgaa gaagttttga gaataagcct cctgcacaca ttgctgccag 1140

ccatctctcc gagcctgcaa agccagctca ttctcagaat caatcaaatt tttctagtta 1200

ttcttcaaag ggaaagcctc ctgaagctga tggtgtggat agatcatttg gcgagaaacg 1260

ctatgaaccc atccaggcca ctccccctcc tcctccattg ccctcgcagt atgcccagcc 1320

atctcagcct gtcaccagcg cgtctctcca catacattct aagggagcac atggtgaagg 1380

taattcagtg tcattggatt ttcagaattc cttagtgtcc aaaccagacc cacctccatc 1440

tcagaataag ccagcaactt tcagaccacc aaaccgagaa gatactgctc aggcagcttt 1500

ctatccccag aaaagttttc cagataaagc cccagttaat ggaactgaac agactcagaa 1560

aacagtcact ccagcataca atcgattcac accaaaacca tatacaagtt ctgcccgacc 1620

atttgaacgc aagtttgaaa gtcctaaatt caatcacaat cttctgccaa gtgaaactgc 1680

acataaacct gacttgtctt caaaaactcc cacttctcca aaaactcttg tgaaatcgca 1740

cagtttggca cagcctcctg agtttgacag tggagttgaa actttctcta tccatgcaga 1800

gaagcctaaa tatcaaataa ataatatcag cacagtgcct aaagctattc ctgtgagtcc 1860

ttcagctgtg gaagaggatg aagatgaaga tggtcatact gtggtggcca cagcccgagg 1920

catatttaac agcaatgggg gcgtgctgag ttccatagaa actggtgtta gtataattat 1980

ccctcaagga gccattcccg aaggagttga gcaggaaatc tatttcaagg tctgccggga 2040

caacagcatc cttccacctt tagataaaga gaaaggtgaa acactgctga gtcctttggt 2100

gatgtgtggt ccccatggcc tcaagttcct gaagcctgtg gagctgcgct taccacactg 2160

tgatcctaaa acctggcaaa acaagtgtct tcccggagat ccaaattatc tcgttggagc 2220

aaactgtgtt tctgtcctta ttgaccactt tctcgaggag acgtat 2266

<210> 31
<211> 10618
<212> DNA
<213> Artificial Sequence

<220>
<223> pTriEx-1-TJP1[human]-IF2

<400> 31
ggggaattgt gagcggataa caattccccg gagttaatcc gggaccttta attcaaccca 60

acacaatata ttatagttaa ataagaatta ttatcaaatc atttgtatat taattaaaat 120

actatactgt aaattacatt ttatttacaa tcaaaggaga tataccatgt ccgccagagc 180

tgcggctgcc aagagcacag caatggagga aacagctata tgggaacaac atacagtgac 240

gcttcacagg gctcctggat ttggatttgg aattgcaata tctggtggac gagataatcc 300

tcattttcag agtggggaaa cgtcaatagt gatttcagat gtgctgaaag gaggaccagc 360

tgaaggacag ctacaggaaa atgaccgagt tgcaatggtt aacggagttt caatggataa 420

tgttgaacat gcttttgctg ttcagcaact aaggaaaagt gggaaaaatg caaaaattac 480

aattagaagg aagaagaaag ttcaaatacc agtaagtcgt cctgatcctg aaccagtatc 540

tgataatgaa gaagatagtt atgatgagga aatacatgat ccaagaagtg gccggagtgg 600

tgtggttaac agaaggagtg agaagatttg gccgagggat agaagtgcaa gtagagagag 660

gagcttgtcc ccgcggtcag acaggcggtc agtggcttcc agccagcctg ctaaacctac 720

taaagtcaca ctggtgaaat cccggaaaaa tgaagaatat ggtcttcgat tggcaagcca 780

tatatttgtt aaggaaattt cacaagatag tttggcagca agagatggca atattcaaga 840

aggtgatgtt gtattgaaga taaatggtac tgtgacagaa aatatgtcat tgacagatgc 900

aaagacattg atagaaaggt ctaaaggcaa attaaaaatg gtagttcaaa gagatgaacg 960

ggctacgcta ttgaatgtcc ctgatctttc tgacagcatc cactctgcta atgcctctga 1020

gagagacgac atttcagaaa ttcagtcact ggcatcagat cattctggtc gatcacacga 1080

taggcctccc cgccgcagcc ggtcacgatc tcctgaccag cggtcagagc cttctgatca 1140

ttccaggcac tcgccgcagc agccaagcaa tggcagtctc cggagtagag atgaagagag 1200

aatttctaaa cctggggctg tctcaactcc tgtaaagcat gctgatgatc acacacctaa 1260

aacagtggaa gaagttacag ttgaaagaaa tgagaaacaa acaccttctc ttccagaacc 1320

aaagcctgtg tatgcccaag ttgggcaacc agatgtggat ttacctgtca gtccatctga 1380

tggtgtccta cctaattcaa ctcatgaaga tgggattctt cggcccagca tgaaattggt 1440

aaaattcaga aaaggagata gtgtgggttt gcggctggct ggtggaaatg atgttggaat 1500

atttgtagct ggcgttctag aagatagccc tgcagccaag gaaggcttag aggaaggtga 1560

tcaaattctc agggtaaaca acgtagattt tacaaatatc ataagagaag aagccgtcct 1620

tttcctgctt gacctcccta aaggagaaga agtgaccata ttggctcaga agaagaagga 1680

tgtttatcgt cgcattgtag aatcagatgt aggagattct ttctatatta gaacccattt 1740

tgaatatgaa aaggaatctc cctatggact tagttttaac aaaggagagg tgttccgtgt 1800

tgtggatacc ttgtacaatg gaaaactggg ctcttggctt gctattcgaa ttggtaaaaa 1860

tcataaggag gtagaacgag gcatcatccc taataagaac agagctgagc agctagccag 1920

tgtacagtat acacttccaa aaacagcagg cggagaccgt gctgacttct ggagattcag 1980

aggtcttcgc agctccaaga gaaatcttcg aaaaagcaga gaggatttgt ccgctcagcc 2040

tgttcaaaca aagtttccag cttatgaaag agtggttctt cgagaagctg gatttctgag 2100

gcctgtaacc atttttggac caatagctga tgttgccaga gaaaagctgg caagagaaga 2160

accagatatt tatcaaattg caaagagtga accacgagac gctggaactg accaacgtag 2220

ctctggcatt attcgcctgc atacaataaa gcaaatcata gatcaagaca aacatgcttt 2280

attagatgta acaccaaatg cagttgatcg tcttaactat gcccagtggt atccaattgt 2340

tgtatttctt aaccctgatt ctaagcaagg agtaaaaaca atgagaatga ggttatgtcc 2400

agaatctcgg aaaagtgcca ggaagttata cgagcgatct cataaacttc gtaaaaataa 2460

tcaccatctt tttacaacta caattaactt aaattcaatg aatgatggtt ggtatggtgc 2520

gctgaaagaa gcaattcaac aacagcaaaa ccagctggta tgggtttccg agggaaaggc 2580

ggatggtgct acaagtgatg accttgattt gcatgatgat cgtctgtcct acctgtcagc 2640

tccaggtagt gaatactcaa tgtatagcac ggacagtaga cacacttctg actatgaaga 2700

cacagacaca gaaggcgggg cctacactga tcaagaacta gatgaaactc ttaatgatga 2760

ggttgggact ccaccggagt ctgccattac acggtcctct gagcctgtaa gagaggactc 2820

ctctggaatg catcatgaaa accaaacata tcctccttac tcaccacaag cgcagccaca 2880

accaattcat agaatagact cccctggatt taagccagcc tctcaacagg tgtatagaaa 2940

ggatccatat cccgaggaaa tgatgaggca gaaccatgtt ttgaaacagc cagccgttag 3000

tcacccaggg cacaggccag acaaagagcc taatctgacc tatgaacccc aactcccata 3060

cgtagagaaa caagccagca gagacctcga gcagcccaca tacagatacg agtcctcaag 3120

ctatacggac cagttttctc gaaactatga acatcgtctg cgatacgaag atcgcgtccc 3180

catgtatgaa gaacagtggt catattatga tgacaaacag ccctacccat ctcggccacc 3240

ttttgataat cagcactctc aagaccttga ctccagacag catcccgaag agtcctcaga 3300

acgagggtac tttccacgtt ttgaagagcc agcccctctg tcttacgaca gcagaccacg 3360

ttacgaacag gcacctagag catccgccct gcggcacgaa gagcagccag ctcctgggta 3420

tgacacacat ggtagactca gaccggaagc ccagccccac ccttcagcag ggcccaagcc 3480

tgcagagtcc aagcagtatt ttgagcaata ttcacgcagt tacgagcaag taccacccca 3540

aggatttacc tctagagcag gtcattttga gcctctccat ggtgctgcag ctgtccctcc 3600

gctgatacct tcatctcagc ataagccaga agctctgcct tcaaacacca aaccactgcc 3660

tccaccccca actcaaaccg aagaagagga agatccagca atgaagccac agtctgtact 3720

caccagagtt aagatgtttg aaaacaaaag atctgcatcc ttagagacca agaaggatgt 3780

aaatgacact ggcagtttta agcctccaga agtagcatct aaaccttcag gtgctcccat 3840

cattggtccc aaacccactt ctcagaatca attcagtgaa catgacaaaa ctctgtacag 3900

gatcccagaa cctcaaaaac ctcaactgaa gccacctgaa gatattgttc ggtccaatca 3960

ttatgaccct gaagaagatg aagaatatta tcgaaaacag ctgtcatact ttgaccgaag 4020

aagttttgag aataagcctc ctgcacacat tgctgccagc catctctccg agcctgcaaa 4080

gccagctcat tctcagaatc aatcaaattt ttctagttat tcttcaaagg gaaagcctcc 4140

tgaagctgat ggtgtggata gatcatttgg cgagaaacgc tatgaaccca tccaggccac 4200

tccccctcct cctccattgc cctcgcagta tgcccagcca tctcagcctg tcaccagcgc 4260

gtctctccac atacattcta agggagcaca tggtgaaggt aattcagtgt cattggattt 4320

tcagaattcc ttagtgtcca aaccagaccc acctccatct cagaataagc cagcaacttt 4380

cagaccacca aaccgagaag atactgctca ggcagctttc tatccccaga aaagttttcc 4440

agataaagcc ccagttaatg gaactgaaca gactcagaaa acagtcactc cagcatacaa 4500

tcgattcaca ccaaaaccat atacaagttc tgcccgacca tttgaacgca agtttgaaag 4560

tcctaaattc aatcacaatc ttctgccaag tgaaactgca cataaacctg acttgtcttc 4620

aaaaactccc acttctccaa aaactcttgt gaaatcgcac agtttggcac agcctcctga 4680

gtttgacagt ggagttgaaa ctttctctat ccatgcagag aagcctaaat atcaaataaa 4740

taatatcagc acagtgccta aagctattcc tgtgagtcct tcagctgtgg aagaggatga 4800

agatgaagat ggtcatactg tggtggccac agcccgaggc atatttaaca gcaatggggg 4860

cgtgctgagt tccatagaaa ctggtgttag tataattatc cctcaaggag ccattcccga 4920

aggagttgag caggaaatct atttcaaggt ctgccgggac aacagcatcc ttccaccttt 4980

agataaagag aaaggtgaaa cactgctgag tcctttggtg atgtgtggtc cccatggcct 5040

caagttcctg aagcctgtgg agctgcgctt accacactgt gatcctaaaa cctggcaaaa 5100

caagtgtctt cccggagatc caaattatct cgttggagca aactgtgttt ctgtccttat 5160

tgaccacttt ctcgagcacc accatcacca tcaccatcac taagtgatta acctcaggtg 5220

caggctgcct atcagaaggt ggtggctggt gtggccaatg ccctggctca caaataccac 5280

tgagatcgat ctttttccct ctgccaaaaa ttatggggac atcatgaagc cccttgagca 5340

tctgacttct ggctaataaa ggaaatttat tttcattgca atagtgtgtt ggaatttttt 5400

gtgtctctca ctcggaagga catatgggag ggcaaatcat ttaaaacatc agaatgagta 5460

tttggtttag agtttggcaa catatgccca tatgtaacta gcataacccc ttggggcctc 5520

taaacgggtc ttgaggggtt ttttgctgaa agcatgcgga ggaaattctc cttgaagttt 5580

ccctggtgtt caaagtaaag gagtttgcac cagacgcacc tctgttcact ggtccggcgt 5640

attaaaacac gatacattgt tattagtaca tttattaagc gctagattct gtgcgttgtt 5700

gatttacaga caattgttgt acgtatttta ataattcatt aaatttataa tctttagggt 5760

ggtatgttag agcgaaaatc aaatgatttt cagcgtcttt atatctgaat ttaaatatta 5820

aatcctcaat agatttgtaa aataggtttc gattagtttc aaacaagggt tgtttttccg 5880

aaccgatggc tggactatct aatggatttt cgctcaacgc cacaaaactt gccaaatctt 5940

gtagcagcaa tctagctttg tcgatattcg tttgtgtttt gttttgtaat aaaggttcga 6000

cgtcgttcaa aatattatgc gcttttgtat ttctttcatc actgtcgtta gtgtacaatt 6060

gactcgacgt aaacacgtta aatagagctt ggacatattt aacatcgggc gtgttagctt 6120

tattaggccg attatcgtcg tcgtcccaac cctcgtcgtt agaagttgct tccgaagacg 6180

attttgccat agccacacga cgcctattaa ttgtgtcggc taacacgtcc gcgatcaaat 6240

ttgtagttga gctttttgga attatttctg attgcggcgt ttttgggcgg gtttcaatc 6300

taactgtgcc cgattttaat tcagacaaca cgttagaaag cgatggtgca ggcggtggta 6360

acatttcaga cggcaaatct actaatggcg gcggtggtgg agctgatgat aaatctacca 6420

tcggtggagg cgcaggcggg gctggcggcg gaggcggagg cggaggtggt ggcggtgatg 6480

cagacggcgg tttaggctca aatgtctctt taggcaacac agtcggcacc tcaactattg 6540

tactggtttc gggcgccgtt tttggtttga ccggtctgag acgagtgcga tttttttcgt 6600

ttctaatagc ttccaacaat tgttgtctgt cgtctaaagg tgcagcgggt tgaggttccg 6660

tcggcattgg tggagcgggc ggcaattcag acatcgatgg tggtggtggt ggtggaggcg 6720

ctggaatgtt aggcacggga gaaggtggtg gcggcggtgc cgccggtata atttgttctg 6780

gtttagtttg ttcgcgcacg attgtgggca ccggcgcagg cgccgctggc tgcacaacgg 6840

aaggtcgtct gcttcgaggc agcgcttggg gtggtggcaa ttcaatatta taattggaat 6900

acaaatcgta aaaatctgct ataagcattg taatttcgct atcgtttacc gtgccgatat 6960

ttaacaaccg ctcaatgtaa gcaattgtat tgtaaagaga ttgtctcaag ctcggaacgc 7020

tgcgctcggt cgttcggctg cggcgagcgg tatcagctca ctcaaaggcg gtaatacggt 7080

tatccacaga atcaggggat aacgcaggaa agaacatgtg agcaaaaggc cagcaaaagg 7140

ccaggaaccg taaaaaggcc gcgttgctgg cgtttttcca taggctccgc ccccctgacg 7200

agcatcacaa aaatcgacgc tcaagtcaga ggtggcgaaa cccgacagga ctataaagat 7260

accaggcgtt tccccctgga agctccctcg tgcgctctcc tgttccgacc ctgccgctta 7320

ccggatacct gtccgccttt ctcccttcgg gaagcgtggc gctttctcaa tgctcacgct 7380

gtaggtatct cagttcggtg taggtcgttc gctccaagct gggctgtgtg cacgaacccc 7440

ccgttcagcc cgaccgctgc gccttatccg gtaactatcg tcttgagtcc aacccggtaa 7500

gacacgactt atcgccactg gcagcagcca ctggtaacag gattagcaga gcgaggtatg 7560

taggcggtgc tacagagttc ttgaagtggt ggcctaacta cggctacact agaaggacag 7620

tatttggtat ctgcgctctg ctgaagccag ttaccttcgg aaaaagagtt ggtagctctt 7680

gatccggcaa acaaaccacc gctggtagcg gtggtttttt tgtttgcaag cagcagatta 7740

cgcgcagaaa aaaaggatct caagaagatc ctttgttacc aatgcttaat cagtgaggca 7800

cctatctcag cgatctgtct atttcgttca tccatagttg cctgactccc cgtcgtgtag 7860

ataactacga tacgggaggg cttaccatct ggccccagtg ctgcaatgat accgcgagac 7920

ccacgctcac cggctccaga tttatcagca ataaaccagc cagccggaag ggccgagcgc 7980

agaagtggtc ctgcaacttt atccgcctcc atccagtcta ttaattgttg ccgggaagct 8040

agagtaagta gttcgccagt taatagtttg cgcaacgttg ttgccattgc tacaggcatc 8100

gtggtgtcac gctcgtcgtt tggtatggct tcattcagct ccggttccca acgatcaagg 8160

cgagttacat gatcccccat gttgtgcaaa aaagcggtta gctccttcgg tcctccgatc 8220

gttgtcagaa gtaagttggc cgcagtgtta tcactcatgg ttatggcagc actgcataat 8280

tctcttactg tcatgccatc cgtaagatgc ttttctgtga ctggtgagta ctcaaccaag 8340

tcattctgag aatagtgtat gcggcgaccg agttgctctt gcccggcgtc aatacgggat 8400

aataccgcgc cacatagcag aactttaaaa gtgctcatca ttggaaaacg ttcttcgggg 8460

cgaaaactct caaggatctt accgctgttg agatccagtt cgatgtaacc cactcgtgca 8520

cccaactgat cttcagcatc ttttactttc accagcgttt ctgggtgagc aaaaacagga 8580

aggcaaaatg ccgcaaaaaa gggaataagg gcgacacgga aatgttgaat actcatactc 8640

ttcctttttc aatattattg aagcatttat cagggttatt gtctcatgtc cgcgcgtttc 8700

ctgcatcttt taatcaaatc ccaagatgtg tataaaccac caaactgcca aaaaatgaaa 8760

actgtcgaca agctctgtcc gtttgctggc aactgcaagg gtctcaatcc tatttgtaat 8820

tattgaataa taaaacaatt ataaatgtca aatttgtttt ttattaacga tacaaaccaa 8880

acgcaacaag aacatttgta gtattatcta taattgaaaa cgcgtagtta taatcgctga 8940

ggtaatattt aaaatcattt tcaaatgatt cacagttaat ttgcgacaat ataattttat 9000

tttcacataa actagacgcc ttgtcgtctt cttcttcgta ttccttctct tttttcattt 9060

tctcttcata aaaattaaca tagttattat cgtatccata tatgtatcta tcgtatagag 9120

taaatttttt gttgtcataa atatatatgt cttttttaat ggggtgtata gtaccgctgc 9180

gcatagtttt tctgtaattt acaacagtgc tattttctgg tagttcttcg gagtgtgttg 9240

ctttaattat taaatttata taatcaatga atttgggatc gtcggttttg tacaatatgt 9300

tgccggcata gtacgcagct tcttctagtt caattacacc attttttagc agcaccggat 9360

taacataact ttccaaaatg ttgtacgaac cgttaaacaa aaacagttca cctccctttt 9420

ctatactatt gtctgcgagc agttgtttgt tgttaaaaat aacagccatt gtaatgagac 9480

gcacaaacta atatcacaaa ctggaaatgt ctatcaatat atagttgctc tagttattaa 9540

tagtaatcaa ttacggggtc attagttcat agcccatata tggagttccg cgttacataa 9600

cttacggtaa atggcccgcc tggctgaccg cccaacgacc cccgcccatt gacgtcaata 9660

atgacgtatg ttcccatagt aacgccaata gggactttcc attgacgtca atgggtggac 9720

tatttacggt aaactgccca cttggcagta catcaagtgt atcatatgcc aagtacgccc 9780

cctattgacg tcaatgacgg taaatggccc gcctggcatt atgcccagta catgacctta 9840

tgggactttc ctacttggca gtacatctac gtattagtca tcgctattac catgcatggt 9900

cgaggtgagc cccacgttct gcttcactct ccccatctcc cccccctccc cacccccaat 9960

tttgtattta tttatttttt aattattttg tgcagcgatg ggggcggggg gggggggggg 10020

gcgcgcgcca ggcggggcgg ggcggggcga ggggcggggc ggggcgaggc ggagaggtgc 10080

ggcggcagcc aatcagagcg gcgcgctccg aaagtttcct tttatggcga ggcggcggcg 10140

gcggcggccc tataaaaagc gaagcgcgcg gcgggcggga gtcgctgcga cgctgccttc 10200

gccccgtgcc ccgctccgcc gccgcctcgc gccgcccgcc ccggctctga ctgaccgcgt 10260

tactcccaca ggtgagcggg cgggacggcc cttctccttc gggctgtaat tagcgcttgg 10320

tttaatgacg gcttgtttct tttctgtggc tgcgtgaaag ccttgagggg ctccgggagg 10380

gccctttgtg cggggggagc ggctcggggc tgtccgcggg gggacggctg ccttcggggg 10440

ggacggggca gggcggggtt cggcttctgg cgtgtgaccg gcggctctag agcctctgct 10500

aaccatgttc atgccttctt ctttttccta cagctcctgg gcaacgtgct ggttattgtg 10560

ctgtctcatc attttggcaa agaattggat cggaccgaaa ttaatacgac tcactata 10618

<210> 32
<211> 1678
<212> PRT
<213> Artificial Sequence

<220>
<223> TJP1[human]-IF2-H8

<400> 32
   Met Ser Ala Arg Ala Ala Ala Ala Lys Ser Thr Ala Met Glu Glu Thr
   1 5 10 15
   Ala Ile Trp Glu Gln His Thr Val Thr Leu His Arg Ala Pro Gly Phe
   20 25 30
   Gly Phe Gly Ile Ala Ile Ser Gly Gly Arg Asp Asn Pro His Phe Gln
   35 40 45
   Ser Gly Glu Thr Ser Ile Val Ile Ser Asp Val Leu Lys Gly Gly Pro
   50 55 60
   Ala Glu Gly Gln Leu Gln Glu Asn Asp Arg Val Ala Met Val Asn Gly
   65 70 75 80
   Val Ser Met Asp Asn Val Glu His Ala Phe Ala Val Gln Gln Leu Arg
   85 90 95
   Lys Ser Gly Lys Asn Ala Lys Ile Thr Ile Arg Arg Lys Lys Lys Val
   100 105 110
   Gln Ile Pro Val Ser Arg Pro Asp Pro Glu Pro Val Ser Asp Asn Glu
   115 120 125
   Glu Asp Ser Tyr Asp Glu Glu Ile His Asp Pro Arg Ser Gly Arg Ser
   130 135 140
   Gly Val Val Asn Arg Arg Ser Glu Lys Ile Trp Pro Arg Asp Arg Ser
   145                 150                 155                 160
   Ala Ser Arg Glu Arg Ser Leu Ser Pro Arg Ser Asp Arg Arg Ser Val
                   165                 170                 175
   Ala Ser Ser Gln Pro Ala Lys Pro Thr Lys Val Thr Leu Val Lys Ser
               180                 185                 190
   Arg Lys Asn Glu Glu Tyr Gly Leu Arg Leu Ala Ser His Ile Phe Val
           195                 200                 205
   Lys Glu Ile Ser Gln Asp Ser Leu Ala Ala Arg Asp Gly Asn Ile Gln
       210                 215                 220
   Glu Gly Asp Val Val Leu Lys Ile Asn Gly Thr Val Thr Glu Asn Met
   225                 230                 235                 240
   Ser Leu Thr Asp Ala Lys Thr Leu Ile Glu Arg Ser Lys Gly Lys Leu
                   245                 250                 255
   Lys Met Val Val Gln Arg Asp Glu Arg Ala Thr Leu Leu Asn Val Pro
               260                 265                 270
   Asp Leu Ser Asp Ser Ile His Ser Ala Asn Ala Ser Glu Arg Asp Asp
           275                 280                 285
   Ile Ser Glu Ile Gln Ser Leu Ala Ser Asp His Ser Gly Arg Ser His
       290                 295                 300
   Asp Arg Pro Pro Arg Arg Ser Arg Ser Arg Ser Pro Asp Gln Arg Ser
   305                 310                 315                 320
   Glu Pro Ser Asp His Ser Arg His Ser Pro Gln Gln Pro Ser Asn Gly
                   325                 330                 335
   Ser Leu Arg Ser Arg Asp Glu Glu Arg Ile Ser Lys Pro Gly Ala Val
               340                 345                 350
   Ser Thr Pro Val Lys His Ala Asp Asp His Thr Pro Lys Thr Val Glu
           355                 360                 365
   Glu Val Thr Val Glu Arg Asn Glu Lys Gln Thr Pro Ser Leu Pro Glu
       370                 375                 380
   Pro Lys Pro Val Tyr Ala Gln Val Gly Gln Pro Asp Val Asp Leu Pro
   385                 390                 395                 400
   Val Ser Pro Ser Asp Gly Val Leu Pro Asn Ser Thr His Glu Asp Gly
   405 410 415
   Ile Leu Arg Pro Ser Met Lys Leu Val Lys Phe Arg Lys Gly Asp Ser
   420 425 430
   Val Gly Leu Arg Leu Ala Gly Gly Asn Asp Val Gly Ile Phe Val Ala
   435 440 445
   Gly Val Leu Glu Asp Ser Pro Ala Ala Lys Glu Gly Leu Glu Glu Gly
   450 455 460
   Asp Gln Ile Leu Arg Val Asn Asn Val Asp Phe Thr Asn Ile Ile Arg
   465 470 475 480
   Glu Glu Ala Val Leu Phe Leu Leu Asp Leu Pro Lys Gly Glu Glu Val
   485 490 495
   Thr Ile Leu Ala Gln Lys Lys Lys Asp Val Tyr Arg Arg Ile Val Glu
   500 505 510
   Ser Asp Val Gly Asp Ser Phe Tyr Ile Arg Thr His Phe Glu Tyr Glu
   515 520 525
   Lys Glu Ser Pro Tyr Gly Leu Ser Phe Asn Lys Gly Glu Val Phe Arg
   530 535 540
   Val Val Asp Thr Leu Tyr Asn Gly Lys Leu Gly Ser Trp Leu Ala Ile
   545 550 555 560
   Arg Ile Gly Lys Asn His Lys Glu Val Glu Arg Gly Ile Ile Pro Asn
   565 570 575
   Lys Asn Arg Ala Glu Gln Leu Ala Ser Val Gln Tyr Thr Leu Pro Lys
   580 585 590
   Thr Ala Gly Gly Asp Arg Ala Asp Phe Trp Arg Phe Arg Gly Leu Arg
   595 600 605
   Ser Ser Lys Arg Asn Leu Arg Lys Ser Arg Glu Asp Leu Ser Ala Gln
   610 615 620
   Pro Val Gln Thr Lys Phe Pro Ala Tyr Glu Arg Val Val Leu Arg Glu
   625 630 635 640
   Ala Gly Phe Leu Arg Pro Val Thr Ile Phe Gly Pro Ile Ala Asp Val
   645 650 655
   Ala Arg Glu Lys Leu Ala Arg Glu Glu Pro Asp Ile Tyr Gln Ile Ala
                   660                 665                 670
   Lys Ser Glu Pro Arg Asp Ala Gly Thr Asp Gln Arg Ser Ser Gly Ile
               675                 680                 685
   Ile Arg Leu His Thr Ile Lys Gln Ile Ile Asp Gln Asp Lys His Ala
           690                 695                 700
   Leu Leu Asp Val Thr Pro Asn Ala Val Asp Arg Leu Asn Tyr Ala Gln
   705                 710                 715                 720
   Trp Tyr Pro Ile Val Val Phe Leu Asn Pro Asp Ser Lys Gln Gly Val
                   725                 730                 735
   Lys Thr Met Arg Met Arg Leu Cys Pro Glu Ser Arg Lys Ser Ala Arg
                   740                 745                 750
   Lys Leu Tyr Glu Arg Ser His Lys Leu Arg Lys Asn Asn His His Leu
               755                 760                 765
   Phe Thr Thr Thr Ile Asn Leu Asn Ser Met Asn Asp Gly Trp Tyr Gly
           770                 775                 780
   Ala Leu Lys Glu Ala Ile Gln Gln Gln Gln Asn Gln Leu Val Trp Val
   785                 790                 795                 800
   Ser Glu Gly Lys Ala Asp Gly Ala Thr Ser Asp Asp Leu Asp Leu His
                   805                 810                 815
   Asp Asp Arg Leu Ser Tyr Leu Ser Ala Pro Gly Ser Glu Tyr Ser Met
                   820                 825                 830
   Tyr Ser Thr Asp Ser Arg His Thr Ser Asp Tyr Glu Asp Thr Asp Thr
               835                 840                 845
   Glu Gly Gly Ala Tyr Thr Asp Gln Glu Leu Asp Glu Thr Leu Asn Asp
           850                 855                 860
   Glu Val Gly Thr Pro Pro Glu Ser Ala Ile Thr Arg Ser Ser Glu Pro
   865                 870                 875                 880
   Val Arg Glu Asp Ser Ser Gly Met His His Glu Asn Gln Thr Tyr Pro
                   885                 890                 895
   Pro Tyr Ser Pro Gln Ala Gln Pro Gln Pro Ile His Arg Ile Asp Ser
                   900                 905                 910
   Pro Gly Phe Lys Pro Ala Ser Gln Gln Val Tyr Arg Lys Asp Pro Tyr
           915                 920                 925
   Pro Glu Glu Met Met Arg Gln Asn His Val Leu Lys Gln Pro Ala Val
       930                 935                 940
   Ser His Pro Gly His Arg Pro Asp Lys Glu Pro Asn Leu Thr Tyr Glu
   945                 950                 955                 960
   Pro Gln Leu Pro Tyr Val Glu Lys Gln Ala Ser Arg Asp Leu Glu Gln
                   965                 970                 975
   Pro Thr Tyr Arg Tyr Glu Ser Ser Ser Tyr Thr Asp Gln Phe Ser Arg
               980                 985                 990
   Asn Tyr Glu His Arg Leu Arg Tyr  Glu Asp Arg Val Pro  Met Tyr Glu
           995                 1000                 1005
   Glu Gln  Trp Ser Tyr Tyr Asp  Asp Lys Gln Pro Tyr  Pro Ser Arg
       1010                 1015                 1020
   Pro Pro  Phe Asp Asn Gln His  Ser Gln Asp Leu Asp  Ser Arg Gln
       1025                 1030                 1035
   His Pro  Glu Glu Ser Ser Glu  Arg Gly Tyr Phe Pro  Arg Phe Glu
       1040                 1045                 1050
   Glu Pro  Ala Pro Leu Ser Tyr  Asp Ser Arg Pro Arg  Tyr Glu Gln
       1055                 1060                 1065
   Ala Pro  Arg Ala Ser Ala Leu  Arg His Glu Glu Gln  Pro Ala Pro
       1070                 1075                 1080
   Gly Tyr  Asp Thr His Gly Arg  Leu Arg Pro Glu Ala  Gln Pro His
       1085                 1090                 1095
   Pro Ser  Ala Gly Pro Lys Pro  Ala Glu Ser Lys Gln  Tyr Phe Glu
       1100                 1105                 1110
   Gln Tyr  Ser Arg Ser Tyr Glu  Gln Val Pro Pro Gln  Gly Phe Thr
       1115                 1120                 1125
   Ser Arg  Ala Gly His Phe Glu  Pro Leu His Gly Ala  Ala Ala Val
       1130                 1135                 1140
   Pro Pro  Leu Ile Pro Ser Ser  Gln His Lys Pro Glu  Ala Leu Pro
   1145                1150                1155
   Ser Asn  Thr Lys Pro Leu Pro  Pro Pro Pro Thr Gln  Thr Glu Glu
       1160                1165                1170
   Glu Glu  Asp Pro Ala Met Lys  Pro Gln Ser Val Leu  Thr Arg Val
       1175                1180                1185
   Lys Met  Phe Glu Asn Lys Arg  Ser Ala Ser Leu Glu  Thr Lys Lys
       1190                1195                1200
   Asp Val  Asn Asp Thr Gly Ser  Phe Lys Pro Pro Glu  Val Ala Ser
       1205                1210                1215
   Lys Pro  Ser Gly Ala Pro Ile  Ile Gly Pro Lys Pro  Thr Ser Gln
       1220                1225                1230
   Asn Gln  Phe Ser Glu His Asp  Lys Thr Leu Tyr Arg  Ile Pro Glu
       1235                1240                1245
   Pro Gln  Lys Pro Gln Leu Lys  Pro Pro Glu Asp Ile  Val Arg Ser
       1250                1255                1260
   Asn His  Tyr Asp Pro Glu Glu  Asp Glu Glu Tyr Tyr  Arg Lys Gln
       1265                1270                1275
   Leu Ser  Tyr Phe Asp Arg Arg  Ser Phe Glu Asn Lys  Pro Pro Ala
       1280                1285                1290
   His Ile  Ala Ala Ser His Leu  Ser Glu Pro Ala Lys  Pro Ala His
       1295                1300                1305
   Ser Gln  Asn Gln Ser Asn Phe  Ser Ser Tyr Ser Ser  Lys Gly Lys
       1310                1315                1320
   Pro Pro  Glu Ala Asp Gly Val  Asp Arg Ser Phe Gly  Glu Lys Arg
       1325                1330                1335
   Tyr Glu  Pro Ile Gln Ala Thr  Pro Pro Pro Pro Pro  Leu Pro Ser
       1340                1345                1350
   Gln Tyr  Ala Gln Pro Ser Gln  Pro Val Thr Ser Ala  Ser Leu His
       1355                1360                1365
   Ile His  Ser Lys Gly Ala His  Gly Glu Gly Asn Ser  Val Ser Leu
       1370                1375                1380
   Asp Phe Gln Asn Ser Leu Val Ser Lys Pro Asp Pro Pro Pro Ser
   1385 1390 1395
   Gln Asn Lys Pro Ala Thr Phe Arg Pro Pro Asn Arg Glu Asp Thr
   1400 1405 1410
   Ala Gln Ala Ala Phe Tyr Pro Gln Lys Ser Phe Pro Asp Lys Ala
   1415 1420 1425
   Pro Val Asn Gly Thr Glu Gln Thr Gln Lys Thr Val Thr Pro Ala
   1430 1435 1440
   Tyr Asn Arg Phe Thr Pro Lys Pro Tyr Thr Ser Ser Ala Arg Pro
   1445 1450 1455
   Phe Glu Arg Lys Phe Glu Ser Pro Lys Phe Asn His Asn Leu Leu
   1460 1465 1470
   Pro Ser Glu Thr Ala His Lys Pro Asp Leu Ser Ser Lys Thr Pro
   1475 1480 1485
   Thr Ser Pro Lys Thr Leu Val Lys Ser His Ser Leu Ala Gln Pro
   1490 1495 1500
   Pro Glu Phe Asp Ser Gly Val Glu Thr Phe Ser Ile His Ala Glu
   1505 1510 1515
   Lys Pro Lys Tyr Gln Ile Asn Asn Ile Ser Thr Val Pro Lys Ala
   1520 1525 1530
   Ile Pro Val Ser Pro Ser Ala Val Glu Glu Asp Glu Asp Glu Asp
   1535 1540 1545
   Gly His Thr Val Val Ala Thr Ala Arg Gly Ile Phe Asn Ser Asn
   1550 1555 1560
   Gly Gly Val Leu Ser Ser Ile Glu Thr Gly Val Ser Ile Ile Ile
   1565 1570 1575
   Pro Gln Gly Ala Ile Pro Glu Gly Val Glu Gln Glu Ile Tyr Phe
   1580 1585 1590
   Lys Val Cys Arg Asp Asn Ser Ile Leu Pro Pro Leu Asp Lys Glu
   1595 1600 1605
   Lys Gly Glu Thr Leu Leu Ser Pro Leu Val Met Cys Gly Pro His
   1610 1615 1620
   Gly Leu Lys Phe Leu Lys Pro Val Glu Leu Arg Leu Pro His Cys
       1625                1630                1635
   Asp Pro Lys Thr Trp Gln Asn Lys Cys Leu Pro Gly Asp Pro Asn
       1640                1645                1650
   Tyr Leu Val Gly Ala Asn Cys Val Ser Val Leu Ile Asp His Phe
       1655                1660                1665
   Leu Glu His His His His His His His His
       1670                1675

<210> 33
<211> 31
<212> DNA
<213> Artificial Sequence

<220>
<223> sense NVL-F1

<400> 33
ataggtctca catgaagcca cgtcctgcag g                                31

<210> 34
<211> 42
<212> DNA
<213> Artificial Sequence

<220>
<223> asense NVL-F1

<400> 34
tatggtctca tcgagtcatt agttagctga ctggggatca gg                    42

<210> 35
<211> 383
<212> DNA
<213> Artificial Sequence

<220>
<223> NVL-PCR-fragment 1-117

<400> 35
ataggtctca catgaagcca cgtcctgcag gatttgtgga caacaaactc aagcaacgcg    60

ttattcagta tctgaccagt aacaagtgcg gcaaatacgt agacataggg gttttggcgt   120

ctgatctgca gagggtgtac tctatcgact atggtcggag gaaacggaat gcctttcgaa   180

ttcaggtcga aaaggtgttc tccatcatca gctcagagaa agagctgaag aatctcactg   240

aacttgagga cgaacatctc gccaaaagag ccagacaagg cgaagaggac aatgagtaca   300

cagagtccta cagtgatgat gactcatcaa tggaggacta tcctgatccc cagtcagcta   360

actaatgact cgatgagacc ata                                           383

<210>  36
<211>  37
<212>  DNA
<213>  Artificial Sequence

<220>
<223>  sense NVL-F2

<400>  36
ataggtctca catgtcaatg gaggactatc ctgatcc                                37


<210>  37
<211>  42
<212>  DNA
<213>  Artificial Sequence

<220>
<223>  asense NVL-F2

<400>  37
tatggtctca tcgagtcatt agtggtggta cacctccggg tg                          42


<210>  38
<211>  596
<212>  DNA
<213>  Artificial Sequence

<220>
<223>  NVL-PCR-fragment 106-292

<400>  38
ataggtctca catgtcaatg gaggactatc ctgatcccca gtcagctaac cacatgaact      60

cttctctgct tagcctgtat aggaagggga atcccgatag tgtgagcaac acaccggaaa     120

tggagcagag agaaaccacc agttccactc cacgcatatc ctccaaaacc ggtagcattc     180

ccttgaaaac tcctgctaaa gactctgaag gaggctggtt tatcgacaaa acgcctagcg     240

tcaagaagga tagcttcttc ctggatctga gctgtgagaa gtccaatccc aagaagccaa     300

tcacagagat tcaggattca aaagattctt ctcttttgga gagtgatatg aaacggaaag     360

gcaagctaaa gaataaagga agcaaaagga agaaagaaga tcttcaggaa gtagatggag     420

aaattgaagc tgtcctacaa aagaaagcta aagccagggg gttagaattc cagatctcca     480

acgtgaagtt tgaagatgtg ggaggcaatg atatgacatt aaaagaggtc tgcaagatgc     540

tcatacacat gcgtcacccg gaggtgtacc accactaatg actcgatgag accata         596


<210>  39
<211>  33
<212>  DNA
<213>  Artificial Sequence

<220>
<223>  sense NVL-F3

<400>  39
ataggtctca catgctcata cacatgcgtc acc                                    33


<210>  40
<211>  43
<212>  DNA
<213>  Artificial Sequence

<220>
<223>  asense NVL-F3

<400>  40
tatggtctca tcgagtcatt atagagcaac aatgaaatca ttc                         43


<210>  41
<211>  875
<212>  DNA
<213>  Artificial Sequence

<220>
<223>  NVL-PCR-fragment 281-560

<400>  41
ataggtctca catgctcata cacatgcgtc acccggaggt gtaccaccac ctgggcgtcg      60

tgccccctcg tggagttctc cttcatggac caccaggctg tgggaagaca ttacttgcac     120

atgcaattgc tggggaactt gacctgccaa ttttgaaagt ggctgctcca gagattgtgt     180

ctggagtatc cggagagtct gagcagaagc tgagagaact atttgagcaa gctgtgtcaa     240

atgcaccatg tatcattttc attgatgaaa ttgatgctat tacccccaaa agagaagtgg     300

cttcaaaaga tatggaacga agaattgtag cccaactcct aacctgcatg gatgatctga     360

ataatgtggc tgctacagcc cgggtcctag ttattggagc tactaatcga ccagactcgt     420

tagaccctgc tttgagacgt gcgggaaggt tcgaccgaga aatatgccta ggtatcccag     480

atgaagcatc cagggaaaga atacttcaaa cattgtgcag aaaactgagg cttcctcaag     540

cttttgattt ctgtcactta gcacacctaa ctccaggctt tgttggtgct gatctcatgg     600

cactgtgccg agaggcagca atgtgtgcag tcaatagagt cttaatgaag ctacaggaac     660

agcagaagaa aaatcctgaa atggaagatt tgccatctaa aggagtccag gaggaaaggc     720

tgggaactga gcccacttct gaaacacagg atgaattaca aaggctgctg gggttgctaa     780

gagaccaaga tcccctctca gaggagcaga tgcaaggact gtgcattgaa ctgaatgatt     840

tcattgttgc tctataatga ctcgatgaga ccata                                875


<210>  42
<211>  33
<212>  DNA
<213>  Artificial Sequence

<220>

<223>  sense NVL-F4

<400>  42
ataggtctca catgctgtgc attgaactga atg                                    33


<210>  43
<211>  40
<212>  DNA
<213>  Artificial Sequence

<220>
<223>  asense NVL-F4

<400>  43
tatggtctca tcgagtcatt accggctgag ggactcctgc                             40


<210>  44
<211>  959
<212>  DNA
<213>  Artificial Sequence

<220>
<223>  NVL-PCR-fragment 549-856

<400>  44
ataggtctca catgctgtgc attgaactga atgatttcat tgttgctcta tcctcagtcc       60

aaccctctgc caaaagggaa ggctttgtca ctgtccctaa tgtgacatgg gcagatattg      120

gtgccctgga agacattaga gaggagctca ccatggcaat attggcacca gtacgcaacc      180

cagaccagtt caaagctctt ggattggtga ctccagctgg ggtcctcctt gctggtcctc      240

ctggctgtgg gaagactctg ctggcgaagg ctgttgcaaa tgagtccgga ctaaatttta      300

tatctgtcaa gggccccgaa ttactaaaca tgtatgttgg tgagagtgaa cgtgctgtgc      360

gacaagtttt tcaacgagcc aagaactcag caccctgtgt gatattcttt gatgaagtgg      420

atgctttatg tcctcgaaga tcagaccgag agacaggggc aagtgtccga gtggtgaatc      480

agctacttac agagatggat ggtctggaag cacgccagca ggtttttatt atggcagcca      540

ctaacaggcc agatataatt gaccctgcaa tcctgcgccc gggccgcctg gacaaaacac      600

tgtttgtggg tttaccgccc cctgcagatc gccttgccat cttaaaaact atcacaaaaa      660

atggtaccaa accaccactg gatgcagatg taaatttgga agcaattgct ggtgaccttc      720

gctgtgattg ctatacgggc gcagatctct ctgctttggt acgagaagct tctatctgtg      780

ccctgagaca ggaaatggca agacagaaga gtggaaatga aaaaggtgaa ctcaaggtta      840

gtcataagca ttttgaagaa gctttcaaga aagtaagatc atctatatca aaaaaggatc      900

aaatcatgta tgaacgtttg caggagtccc tcagccggta atgactcgat gagaccata       959


<210>  45
<211>  5605
<212>  DNA

<213>  Artificial Sequence

<220>
<223>  pET24d-N-NVL[human](aa1-117)

<400>  45
ggggaattgt gagcggataa caattcccct ctagaaataa ttttgtttaa ctttaagaag      60

gagatatacc atgagccatc atcatcatca tcatcatcat tccatgaagc cacgtcctgc     120

aggatttgtg gacaacaaac tcaagcaacg cgttattcag tatctgacca gtaacaagtg     180

cggcaaatac gtagacatag gggttttggc gtctgatctg cagagggtgt actctatcga     240

ctatggtcgg aggaaacgga atgcctttcg aattcaggtc gaaaaggtgt tctccatcat     300

cagctcagag aaagagctga agaatctcac tgaacttgag gacgaacatc tcgccaaaag     360

agccagacaa ggcgaagagg acaatgagta cacagagtcc tacagtgatg atgactcatc     420

aatggaggac tatcctgatc cccagtcagc taactaatga ctcgagtgag atccggctgc     480

taacaaagcc cgaaaggaag ctgagttggc tgctgccacc gctgagcaat aactagcata     540

accccttggg gcctctaaac gggtcttgag gggttttttg ctgaaaggag gaactatatc     600

cggattggcg aatgggacgc gccctgtagc ggcgcattaa gcgcggcggg tgtggtggtt     660

acgcgcagcg tgaccgctac acttgccagc gccctagcgc ccgctccttt cgctttcttc     720

ccttcctttc tcgccacgtt cgccggcttt ccccgtcaag ctctaaatcg ggggctccct     780

ttagggttcc gatttagtgc tttacggcac ctcgacccca aaaaacttga ttagggtgat     840

ggttcacgta gtgggccatc gccctgatag acggtttttc gccctttgac gttggagtcc     900

acgttcttta atagtggact cttgttccaa actggaacaa cactcaaccc tatctcggtc     960

tattcttttg atttataagg gattttgccg atttcggcct attggttaaa aaatgagctg    1020

atttaacaaa aatttaacgc gaattttaac aaaatattaa cgtttacaat ttcaggtggc    1080

acttttcggg gaaatgtgcg cggaacccct atttgtttat ttttctaaat acattcaaat    1140

atgtatccgc tcatgaatta attcttagaa aaactcatcg agcatcaaat gaaactgcaa    1200

tttattcata tcaggattat caataccata tttttgaaaa agccgtttct gtaatgaagg    1260

agaaaactca ccgaggcagt tccataggat ggcaagatcc tggtatcggt ctgcgattcc    1320

gactcgtcca acatcaatac aacctattaa tttcccctcg tcaaaaataa ggttatcaag    1380

tgagaaatca ccatgagtga cgactgaatc cggtgagaat ggcaaaagtt tatgcatttc    1440

tttccagact tgttcaacag gccagccatt acgctcgtca tcaaaatcac tcgcatcaac    1500

caaaccgtta ttcattcgtg attgcgcctg agcgagacga aatacgcgat cgctgttaaa    1560

aggacaatta caaacaggaa tcgaatgcaa ccggcgcagg aacactgcca gcgcatcaac    1620

aatattttca cctgaatcag gatattcttc taatacctgg aatgctgttt tcccggggat    1680

cgcagtggtg agtaaccatg catcatcagg agtacggata aaatgcttga tggtcggaag    1740

aggcataaat tccgtcagcc agtttagtct gaccatctca tctgtaacat cattggcaac 1800

gctacctttg ccatgtttca gaaacaactc tggcgcatcg ggcttcccat acaatcgata 1860

gattgtcgca cctgattgcc cgacattatc gcgagcccat ttatacccat ataaatcagc 1920

atccatgttg gaatttaatc gcggcctaga gcaagacgtt tcccgttgaa tatggctcat 1980

aacacccctt gtattactgt ttatgtaagc agacagtttt attgttcatg accaaaatcc 2040

cttaacgtga gttttcgttc cactgagcgt cagaccccgt agaaaagatc aaaggatctt 2100

cttgagatcc tttttttctg cgcgtaatct gctgcttgca aacaaaaaaa ccaccgctac 2160

cagcggtggt ttgtttgccg gatcaagagc taccaactct ttttccgaag gtaactggct 2220

tcagcagagc gcagatacca aatactgtcc ttctagtgta gccgtagtta ggccaccact 2280

tcaagaactc tgtagcaccg cctacatacc tcgctctgct aatcctgtta ccagtggctg 2340

ctgccagtgg cgataagtcg tgtcttaccg ggttggactc aagacgatag ttaccggata 2400

aggcgcagcg gtcgggctga acggggggtt cgtgcacaca gcccagcttg gagcgaacga 2460

cctacaccga actgagatac ctacagcgtg agctatgaga aagcgccacg cttcccgaag 2520

ggagaaaggc ggacaggtat ccggtaagcg gcagggtcgg aacaggagag cgcacgaggg 2580

agcttccagg gggaaacgcc tggtatcttt atagtcctgt cgggtttcgc cacctctgac 2640

ttgagcgtcg atttttgtga tgctcgtcag gggggcggag cctatggaaa aacgccagca 2700

acgcggcctt tttacggttc ctggcctttt gctggccttt tgctcacatg ttctttcctg 2760

cgttatcccc tgattctgtg gataaccgta ttaccgcctt tgagtgagct gataccgctc 2820

gccgcagccg aacgaccgag cgcagcgagt cagtgagcga ggaagcggaa gagcgcctga 2880

tgcggtattt tctccttacg catctgtgcg gtatttcaca ccgcatatat ggtgcactct 2940

cagtacaatc tgctctgatg ccgcatagtt aagccagtat acactccgct atcgctacgt 3000

gactgggtca tggctgcgcc ccgacacccg ccaacacccg ctgacgcgcc ctgacgggct 3060

tgtctgctcc cggcatccgc ttacagacaa gctgtgaccg tctccgggag ctgcatgtgt 3120

cagaggtttt caccgtcatc accgaaacgc gcgaggcagc tgcggtaaag ctcatcagcg 3180

tggtcgtgaa gcgattcaca gatgtctgcc tgttcatccg cgtccagctc gttgagtttc 3240

tccagaagcg ttaatgtctg gcttctgata aagcgggcca tgttaagggc ggttttttcc 3300

tgtttggtca ctgatgcctc cgtgtaaggg ggatttctgt tcatgggggt aatgataccg 3360

atgaaacgag agaggatgct cacgatacgg gttactgatg atgaacatgc ccggttactg 3420

gaacgttgtg agggtaaaca actggcggta tggatgcggc gggaccagag aaaaatcact 3480

cagggtcaat gccagcgctt cgttaataca gatgtaggtg ttccacaggg tagccagcag 3540

catcctgcga tgcagatccg gaacataatg gtgcagggcg ctgacttccg cgtttccaga 3600

ctttacgaaa cacggaaacc gaagaccatt catgttgttg ctcaggtcgc agacgttttg 3660

cagcagcagt cgcttcacgt tcgctcgcgt atcggtgatt cattctgcta accagtaagg 3720

caaccccgcc agcctagccg ggtcctcaac gacaggagca cgatcatgcg cacccgtggg 3780

gccgccatgc cggcgataat ggcctgcttc tcgccgaaac gtttggtggc gggaccagtg 3840

acgaaggctt gagcgagggc gtgcaagatt ccgaataccg caagcgacag gccgatcatc 3900

gtcgcgctcc agcgaaagcg gtcctcgccg aaaatgaccc agagcgctgc cggcacctgt 3960

cctacgagtt gcatgataaa gaagacagtc ataagtgcgg cgacgatagt catgccccgc 4020

gcccaccgga aggagctgac tgggttgaag gctctcaagg gcatcggtcg agatcccggt 4080

gcctaatgag tgagctaact tacattaatt gcgttgcgct cactgcccgc tttccagtcg 4140

ggaaacctgt cgtgccagct gcattaatga atcggccaac gcgcggggag aggcggtttg 4200

cgtattgggc gccagggtgg tttttctttt caccagtgag acgggcaaca gctgattgcc 4260

cttcaccgcc tggccctgag agagttgcag caagcggtcc acgctggttt gccccagcag 4320

gcgaaaatcc tgtttgatgg tggttaacgg cgggatataa catgagctgt cttcggtatc 4380

gtcgtatccc actaccgaga tatccgcacc aacgcgcagc ccggactcgg taatggcgcg 4440

cattgcgccc agcgccatct gatcgttggc aaccagcatc gcagtgggaa cgatgccctc 4500

attcagcatt tgcatggttt gttgaaaacc ggacatggca ctccagtcgc cttcccgttc 4560

cgctatcggc tgaatttgat tgcgagtgag atatttatgc cagccagcca gacgcagacg 4620

cgccgagaca gaacttaatg ggcccgctaa cagcgcgatt tgctggtgac ccaatgcgac 4680

cagatgctcc acgcccagtc gcgtaccgtc ttcatgggag aaaataatac tgttgatggg 4740

tgtctggtca gagacatcaa gaaataacgc cggaacatta gtgcaggcag cttccacagc 4800

aatggcatcc tggtcatcca gcggatagtt aatgatcagc ccactgacgc gttgcgcgag 4860

aagattgtgc accgccgctt tacaggcttc gacgccgctt cgttctacca tcgacaccac 4920

cacgctggca cccagttgat cggcgcgaga tttaatcgcc gcgacaattt gcgacggcgc 4980

gtgcagggcc agactggagg tggcaacgcc aatcagcaac gactgtttgc ccgccagttg 5040

ttgtgccacg cggttgggaa tgtaattcag ctccgccatc gccgcttcca ctttttcccg 5100

cgttttcgca gaaacgtggc tggcctggtt caccacgcgg gaaacggtct gataagagac 5160

accggcatac tctgcgacat cgtataacgt tactggtttc acattcacca ccctgaattg 5220

actctcttcc gggcgctatc atgccatacc gcgaaaggtt ttgcgccatt cgatggtgtc 5280

cgggatctcg acgctctccc ttatgcgact cctgcattag gaagcagccc agtagtaggt 5340

tgaggccgtt gagcaccgcc gccgcaagga atggtgcatg caaggagatg gcgcccaaca 5400

gtcccccggc cacggggcct gccaccatac ccacgccgaa acaagcgctc atgagcccga 5460

agtggcgagc ccgatcttcc ccatcggtga tgtcggcgat ataggcgcca gcaaccgcac 5520

ctgtggcgcc ggtgatgccg gccacgatgc gtccggcgta gaggatcgag atctcgatcc 5580

cgcgaaatta atacgactca ctata 5605

<210> 46
<211> 5818
<212> DNA
<213> Artificial Sequence

<220>
<223> pET24d-N-NVL[human](aa106-296)

<400> 46
ggggaattgt gagcggataa caattcccct ctagaaataa ttttgtttaa ctttaagaag 60

gagatatacc atgagccatc atcatcatca tcatcatcat tccatgtcaa tggaggacta 120

tcctgatccc cagtcagcta accacatgaa ctcttctctg cttagcctgt ataggaaggg 180

gaatcccgat agtgtgagca acacaccgga aatggagcag agagaaacca ccagttccac 240

tccacgcata tcctccaaaa ccggtagcat tcccttgaaa actcctgcta aagactctga 300

aggaggctgg tttatcgaca aaacgcctag cgtcaagaag gatagcttct tcctggatct 360

gagctgtgag aagtccaatc ccaagaagcc aatcacagag attcaggatt caaaagattc 420

ttctcttttg gagagtgata tgaaacggaa aggcaagcta aagaataaag gaagcaaaag 480

gaagaaagaa gatcttcagg aagtagatgg agaaattgaa gctgtcctac aaaagaaagc 540

taaagccagg gggttagaat tccagatctc caacgtgaag tttgaagatg tgggaggcaa 600

tgatatgaca ttaaaagagg tctgcaagat gctcatacac atgcgtcacc cggaggtgta 660

ccaccactaa tgactcgagt gagatccggc tgctaacaaa gcccgaaagg aagctgagtt 720

ggctgctgcc accgctgagc aataactagc ataacccctt ggggcctcta aacgggtctt 780

gaggggtttt ttgctgaaag gaggaactat atccggattg gcgaatggga cgcgccctgt 840

agcggcgcat taagcgcggc gggtgtggtg gttacgcgca gcgtgaccgc tacacttgcc 900

agcgccctag cgcccgctcc tttcgctttc ttcccttcct ttctcgccac gttcgccggc 960

tttccccgtc aagctctaaa tcgggggctc cctttagggt tccgatttag tgctttacgg 1020

cacctcgacc ccaaaaaact tgattagggt gatggttcac gtagtgggcc atcgccctga 1080

tagacggttt ttcgcccttt gacgttggag tccacgttct ttaatagtgg actcttgttc 1140

caaactggaa caacactcaa ccctatctcg gtctattctt ttgatttata agggattttg 1200

ccgatttcgg cctattggtt aaaaaatgag ctgatttaac aaaaatttaa cgcgaatttt 1260

aacaaaatat taacgtttac aatttcaggt ggcacttttc ggggaaatgt gcgcggaacc 1320

cctatttgtt tatttttcta aatacattca aatatgtatc cgctcatgaa ttaattctta 1380

gaaaaactca tcgagcatca aatgaaactg caatttattc atatcaggat tatcaatacc 1440

atatttttga aaaagccgtt tctgtaatga aggagaaaac tcaccgaggc agttccatag 1500

gatggcaaga tcctggtatc ggtctgcgat tccgactcgt ccaacatcaa tacaacctat 1560

taatttcccc tcgtcaaaaa taaggttatc aagtgagaaa tcaccatgag tgacgactga 1620

atccggtgag aatggcaaaa gtttatgcat ttctttccag acttgttcaa caggccagcc 1680

attacgctcg tcatcaaaat cactcgcatc aaccaaaccg ttattcattc gtgattgcgc 1740

ctgagcgaga cgaaatacgc gatcgctgtt aaaaggacaa ttacaaacag gaatcgaatg 1800

caaccggcgc aggaacactg ccagcgcatc aacaatattt tcacctgaat caggatattc 1860

ttctaatacc tggaatgctg ttttcccggg gatcgcagtg gtgagtaacc atgcatcatc 1920

aggagtacgg ataaaatgct tgatggtcgg aagaggcata aattccgtca gccagtttag 1980

tctgaccatc tcatctgtaa catcattggc aacgctacct ttgccatgtt tcagaaacaa 2040

ctctggcgca tcgggcttcc catacaatcg atagattgtc gcacctgatt gcccgacatt 2100

atcgcgagcc catttatacc catataaatc agcatccatg ttggaattta atcgcggcct 2160

agagcaagac gtttcccgtt gaatatggct cataacaccc cttgtattac tgtttatgta 2220

agcagacagt tttattgttc atgaccaaaa tcccttaacg tgagttttcg ttccactgag 2280

cgtcagaccc cgtagaaaag atcaaaggat cttcttgaga tccttttttt ctgcgcgtaa 2340

tctgctgctt gcaaacaaaa aaaccaccgc taccagcggt ggtttgtttg ccggatcaag 2400

agctaccaac tctttttccg aaggtaactg gcttcagcag agcgcagata ccaaatactg 2460

tccttctagt gtagccgtag ttaggccacc acttcaagaa ctctgtagca ccgcctacat 2520

acctcgctct gctaatcctg ttaccagtgg ctgctgccag tggcgataag tcgtgtctta 2580

ccgggttgga ctcaagacga tagttaccgg ataaggcgca gcggtcgggc tgaacggggg 2640

gttcgtgcac acagcccagc ttggagcgaa cgacctacac cgaactgaga tacctacagc 2700

gtgagctatg agaaagcgcc acgcttcccg aagggagaaa ggcggacagg tatccggtaa 2760

gcggcagggt cggaacagga gagcgcacga gggagcttcc agggggaaac gcctggtatc 2820

tttatagtcc tgtcgggttt cgccacctct gacttgagcg tcgatttttg tgatgctcgt 2880

caggggggcg gagcctatgg aaaaacgcca gcaacgcggc ctttttacgg ttcctggcct 2940

tttgctggcc ttttgctcac atgttctttc ctgcgttatc ccctgattct gtggataacc 3000

gtattaccgc ctttgagtga gctgataccg ctcgccgcag ccgaacgacc gagcgcagcg 3060

agtcagtgag cgaggaagcg gaagagcgcc tgatgcggta ttttctcctt acgcatctgt 3120

gcggtatttc acaccgcata tatggtgcac tctcagtaca atctgctctg atgccgcata 3180

gttaagccag tatacactcc gctatcgcta cgtgactggg tcatggctgc gccccgacac 3240

ccgccaacac ccgctgacgc gccctgacgg gcttgtctgc tcccggcatc cgcttacaga 3300

caagctgtga ccgtctccgg gagctgcatg tgtcagaggt tttcaccgtc atcaccgaaa 3360

cgcgcgaggc agctgcggta aagctcatca gcgtggtcgt gaagcgattc acagatgtct 3420

gcctgttcat ccgcgtccag ctcgttgagt ttctccagaa gcgttaatgt ctggcttctg 3480

ataaagcggg ccatgttaag ggcggttttt tcctgtttgg tcactgatgc ctccgtgtaa 3540

gggggatttc tgttcatggg ggtaatgata ccgatgaaac gagagaggat gctcacgata 3600

cgggttactg atgatgaaca tgcccggtta ctggaacgtt gtgagggtaa acaactggcg 3660

gtatggatgc ggcgggacca gagaaaaatc actcagggtc aatgccagcg cttcgttaat 3720

acagatgtag gtgttccaca gggtagccag cagcatcctg cgatgcagat ccggaacata 3780

atggtgcagg gcgctgactt ccgcgtttcc agactttacg aaacacggaa accgaagacc 3840

attcatgttg ttgctcaggt cgcagacgtt ttgcagcagc agtcgcttca cgttcgctcg 3900

cgtatcggtg attcattctg ctaaccagta aggcaacccc gccagcctag ccgggtcctc 3960

aacgacagga gcacgatcat gcgcacccgt ggggccgcca tgccggcgat aatggcctgc 4020

ttctcgccga aacgtttggt ggcgggacca gtgacgaagg cttgagcgag ggcgtgcaag 4080

attccgaata ccgcaagcga caggccgatc atcgtcgcgc tccagcgaaa gcggtcctcg 4140

ccgaaaatga cccagagcgc tgccggcacc tgtcctacga gttgcatgat aaagaagaca 4200

gtcataagtg cggcgacgat agtcatgccc cgcgcccacc ggaaggagct gactgggttg 4260

aaggctctca agggcatcgg tcgagatccc ggtgcctaat gagtgagcta acttacatta 4320

attgcgttgc gctcactgcc cgctttccag tcgggaaacc tgtcgtgcca gctgcattaa 4380

tgaatcggcc aacgcgcggg gagaggcggt ttgcgtattg ggcgccaggg tggtttttct 4440

tttcaccagt gagacgggca acagctgatt gcccttcacc gcctggccct gagagagttg 4500

cagcaagcgg tccacgctgg tttgccccag caggcgaaaa tcctgtttga tggtggttaa 4560

cggcgggata taacatgagc tgtcttcggt atcgtcgtat cccactaccg agatatccgc 4620

accaacgcgc agcccggact cggtaatggc gcgcattgcg cccagcgcca tctgatcgtt 4680

ggcaaccagc atcgcagtgg gaacgatgcc ctcattcagc atttgcatgg tttgttgaaa 4740

accggacatg gcactccagt cgccttcccg ttccgctatc ggctgaattt gattgcgagt 4800

gagatattta tgccagccag ccagacgcag acgcgccgag acagaactta atgggcccgc 4860

taacagcgcg atttgctggt gacccaatgc gaccagatgc tccacgccca gtcgcgtacc 4920

gtcttcatgg gagaaaataa tactgttgat gggtgtctgg tcagagacat caagaaataa 4980

cgccggaaca ttagtgcagg cagcttccac agcaatggca tcctggtcat ccagcggata 5040

gttaatgatc agcccactga cgcgttgcgc gagaagattg tgcaccgccg ctttacaggc 5100

ttcgacgccg cttcgttcta ccatcgacac caccacgctg gcacccagtt gatcggcgcg 5160

agatttaatc gccgcgacaa tttgcgacgg cgcgtgcagg gccagactgg aggtggcaac 5220

gccaatcagc aacgactgtt tgcccgccag ttgttgtgcc acgcggttgg gaatgtaatt 5280

cagctccgcc atcgccgctt ccactttttc ccgcgttttc gcagaaacgt ggctggcctg 5340

gttcaccacg cgggaaacgg tctgataaga gacaccggca tactctgcga catcgtataa 5400

cgttactggt ttcacattca ccaccctgaa ttgactctct tccgggcgct atcatgccat 5460

accgcgaaag gttttgcgcc attcgatggt gtccgggatc tcgacgctct cccttatgcg 5520

actcctgcat taggaagcag cccagtagta ggttgaggcc gttgagcacc gccgccgcaa 5580

ggaatggtgc atgcaaggag atggcgccca acagtccccc ggccacgggg cctgccacca 5640

tacccacgcc gaaacaagcg ctcatgagcc cgaagtggcg agcccgatct tccccatcgg 5700

tgatgtcggc gatataggcg ccagcaaccg cacctgtggc gccggtgatg ccggccacga 5760

tgcgtccggc gtagaggatc gagatctcga tcccgcgaaa ttaatacgac tcactata 5818

<210> 47
<211> 6097
<212> DNA
<213> Artificial Sequence

<220>
<223> pET24d-N-NVL[human](aa281-560)

<400> 47
ggggaattgt gagcggataa caattcccct ctagaaataa ttttgtttaa ctttaagaag 60

gagatatacc atgagccatc atcatcatca tcatcatcat tccatgctca tacacatgcg 120

tcacccggag gtgtaccacc acctgggcgt cgtgccccct cgtggagttc tccttcatgg 180

accaccaggc tgtgggaaga cattacttgc acatgcaatt gctggggaac ttgacctgcc 240

aattttgaaa gtggctgctc cagagattgt gtctggagta tccggagagt ctgagcagaa 300

gctgagagaa ctatttgagc aagctgtgtc aaatgcacca tgtatcattt tcattgatga 360

aattgatgct attaccccca aaagagaagt ggcttcaaaa gatatggaac gaagaattgt 420

agcccaactc ctaacctgca tggatgatct gaataatgtg gctgctacag cccgggtcct 480

agttattgga gctactaatc gaccagactc gttagaccct gctttgagac gtgcgggaag 540

gttcgaccga gaaatatgcc taggtatccc agatgaagca tccagggaaa gaatacttca 600

aacattgtgc agaaaactga ggcttcctca agcttttgat ttctgtcact tagcacacct 660

aactccaggc tttgttggtg ctgatctcat ggcactgtgc cgagaggcag caatgtgtgc 720

agtcaataga gtcttaatga agctacagga acagcagaag aaaaatcctg aaatggaaga 780

tttgccatct aaaggagtcc aggaggaaag gctgggaact gagcccactt ctgaaacaca 840

ggatgaatta caaaggctgc tggggttgct aagagaccaa gatcccctct cagaggagca 900

gatgcaagga ctgtgcattg aactgaatga tttcattgtt gctctataat gactcgagtg 960

agatccggct gctaacaaag cccgaaagga agctgagttg gctgctgcca ccgctgagca 1020

ataactagca taaccccttg gggcctctaa acgggtcttg aggggttttt tgctgaaagg 1080

aggaactata tccggattgg cgaatgggac gcgccctgta gcggcgcatt aagcgcggcg 1140

ggtgtggtgg ttacgcgcag cgtgaccgct acacttgcca gcgccctagc gcccgctcct 1200

ttcgctttct tcccttcctt tctcgccacg ttcgccggct ttccccgtca agctctaaat 1260

cgggggctcc ctttagggtt ccgatttagt gctttacggc acctcgaccc caaaaaactt 1320

gattagggtg atggttcacg tagtgggcca tcgccctgat agacggtttt tcgccctttg 1380

acgttggagt ccacgttctt taatagtgga ctcttgttcc aaactggaac aacactcaac 1440

cctatctcgg tctattcttt tgatttataa gggattttgc cgatttcggc ctattggtta 1500

aaaaatgagc tgatttaaca aaaatttaac gcgaatttta acaaaatatt aacgtttaca 1560

atttcaggtg gcacttttcg gggaaatgtg cgcggaaccc ctatttgttt atttttctaa 1620

atacattcaa atatgtatcc gctcatgaat taattcttag aaaaactcat cgagcatcaa 1680

atgaaactgc aatttattca tatcaggatt atcaatacca tatttttgaa aaagccgttt 1740

ctgtaatgaa ggagaaaact caccgaggca gttccatagg atggcaagat cctggtatcg 1800

gtctgcgatt ccgactcgtc caacatcaat acaacctatt aatttcccct cgtcaaaaat 1860

aaggttatca agtgagaaat caccatgagt gacgactgaa tccggtgaga atggcaaaag 1920

tttatgcatt tctttccaga cttgttcaac aggccagcca ttacgctcgt catcaaaatc 1980

actcgcatca accaaaccgt tattcattcg tgattgcgcc tgagcgagac gaaatacgcg 2040

atcgctgtta aaaggacaat tacaaacagg aatcgaatgc aaccggcgca ggaacactgc 2100

cagcgcatca acaatatttt cacctgaatc aggatattct tctaatacct ggaatgctgt 2160

tttcccgggg atcgcagtgg tgagtaacca tgcatcatca ggagtacgga taaaatgctt 2220

gatggtcgga agaggcataa attccgtcag ccagtttagt ctgaccatct catctgtaac 2280

atcattggca acgctacctt tgccatgttt cagaaacaac tctggcgcat cgggcttccc 2340

atacaatcga tagattgtcg cacctgattg cccgacatta tcgcgagccc atttataccc 2400

atataaatca gcatccatgt tggaatttaa tcgcggccta gagcaagacg tttcccgttg 2460

aatatggctc ataacacccc ttgtattact gtttatgtaa gcagacagtt ttattgttca 2520

tgaccaaaat cccttaacgt gagttttcgt tccactgagc gtcagacccc gtagaaaaga 2580

tcaaaggatc ttcttgagat cctttttttc tgcgcgtaat ctgctgcttg caaacaaaaa 2640

aaccaccgct accagcggtg gtttgtttgc cggatcaaga gctaccaact ctttttccga 2700

aggtaactgg cttcagcaga gcgcagatac caaatactgt ccttctagtg tagccgtagt 2760

taggccacca cttcaagaac tctgtagcac cgcctacata cctcgctctg ctaatcctgt 2820

taccagtggc tgctgccagt ggcgataagt cgtgtcttac cgggttggac tcaagacgat 2880

agttaccgga taaggcgcag cggtcgggct gaacgggggg ttcgtgcaca cagcccagct 2940

tggagcgaac gacctacacc gaactgagat acctacagcg tgagctatga gaaagcgcca 3000

cgcttcccga agggagaaag gcggacaggt atccggtaag cggcagggtc ggaacaggag 3060

agcgcacgag ggagcttcca gggggaaacg cctggtatct ttatagtcct gtcgggtttc 3120

gccacctctg acttgagcgt cgatttttgt gatgctcgtc aggggggcgg agcctatgga 3180

aaaacgccag caacgcggcc tttttacggt tcctggcctt ttgctggcct tttgctcaca 3240

tgttctttcc tgcgttatcc cctgattctg tggataaccg tattaccgcc tttgagtgag 3300

ctgataccgc tcgccgcagc cgaacgaccg agcgcagcga gtcagtgagc gaggaagcgg 3360

aagagcgcct gatgcggtat tttctcctta cgcatctgtg cggtatttca caccgcatat 3420

atggtgcact ctcagtacaa tctgctctga tgccgcatag ttaagccagt atacactccg 3480

ctatcgctac gtgactgggt catggctgcg ccccgacacc cgccaacacc cgctgacgcg 3540

ccctgacggg cttgtctgct cccggcatcc gcttacagac aagctgtgac cgtctccggg 3600

agctgcatgt gtcagaggtt ttcaccgtca tcaccgaaac gcgcgaggca gctgcggtaa 3660

agctcatcag cgtggtcgtg aagcgattca cagatgtctg cctgttcatc cgcgtccagc 3720

tcgttgagtt tctccagaag cgttaatgtc tggcttctga taaagcgggc catgttaagg 3780

gcggtttttt cctgtttggt cactgatgcc tccgtgtaag ggggatttct gttcatgggg 3840

gtaatgatac cgatgaaacg agagaggatg ctcacgatac gggttactga tgatgaacat 3900

gcccggttac tggaacgttg tgagggtaaa caactggcgg tatggatgcg gcgggaccag 3960

agaaaaatca ctcagggtca atgccagcgc ttcgttaata cagatgtagg tgttccacag 4020

ggtagccagc agcatcctgc gatgcagatc cggaacataa tggtgcaggg cgctgacttc 4080

cgcgtttcca gactttacga aacacggaaa ccgaagacca ttcatgttgt tgctcaggtc 4140

gcagacgttt tgcagcagca gtcgcttcac gttcgctcgc gtatcggtga ttcattctgc 4200

taaccagtaa ggcaaccccg ccagcctagc cgggtcctca acgacaggag cacgatcatg 4260

cgcacccgtg gggccgccat gccggcgata atggcctgct tctcgccgaa acgtttggtg 4320

gcgggaccag tgacgaaggc ttgagcgagg gcgtgcaaga ttccgaatac cgcaagcgac 4380

aggccgatca tcgtcgcgct ccagcgaaag cggtcctcgc cgaaaatgac ccagagcgct 4440

gccggcacct gtcctacgag ttgcatgata aagaagacag tcataagtgc ggcgacgata 4500

gtcatgcccc gcgcccaccg gaaggagctg actgggttga aggctctcaa gggcatcggt 4560

cgagatcccg gtgcctaatg agtgagctaa cttacattaa ttgcgttgcg ctcactgccc 4620

gctttccagt cgggaaacct gtcgtgccag ctgcattaat gaatcggcca acgcgcgggg 4680

agaggcggtt tgcgtattgg gcgccagggt ggtttttctt ttcaccagtg agacgggcaa 4740

cagctgattg cccttcaccg cctggccctg agagagttgc agcaagcggt ccacgctggt 4800

ttgccccagc aggcgaaaat cctgtttgat ggtggttaac ggcgggatat aacatgagct 4860

gtcttcggta tcgtcgtatc ccactaccga gatatccgca ccaacgcgca gcccggactc 4920

ggtaatggcg cgcattgcgc ccagcgccat ctgatcgttg gcaaccagca tcgcagtggg 4980

aacgatgccc tcattcagca tttgcatggt ttgttgaaaa ccggacatgg cactccagtc 5040

gccttcccgt tccgctatcg gctgaatttg attgcgagtg agatatttat gccagccagc 5100

cagacgcaga cgcgccgaga cagaacttaa tgggcccgct aacagcgcga tttgctggtg 5160

acccaatgcg accagatgct ccacgcccag tcgcgtaccg tcttcatggg agaaaataat 5220

actgttgatg ggtgtctggt cagagacatc aagaaataac gccggaacat tagtgcaggc 5280

agcttccaca gcaatggcat cctggtcatc cagcggatag ttaatgatca gcccactgac 5340

gcgttgcgcg agaagattgt gcaccgccgc tttacaggct tcgacgccgc ttcgttctac 5400

catcgacacc accacgctgg cacccagttg atcggcgcga gatttaatcg ccgcgacaat 5460

ttgcgacggc gcgtgcaggg ccagactgga ggtggcaacg ccaatcagca acgactgttt 5520

gcccgccagt tgttgtgcca cgcggttggg aatgtaattc agctccgcca tcgccgcttc 5580

cactttttcc cgcgttttcg cagaaacgtg gctggcctgg ttcaccacgc gggaaacggt 5640

ctgataagag acaccggcat actctgcgac atcgtataac gttactggtt tcacattcac 5700

caccctgaat tgactctctt ccgggcgcta tcatgccata ccgcgaaagg ttttgcgcca 5760

ttcgatggtg tccgggatct cgacgctctc ccttatgcga ctcctgcatt aggaagcagc 5820

ccagtagtag gttgaggccg ttgagcaccg ccgccgcaag gaatggtgca tgcaaggaga 5880

tggcgcccaa cagtcccccg gccacggggc ctgccaccat acccacgccg aaacaagcgc 5940

tcatgagccc gaagtggcga gcccgatctt ccccatcggt gatgtcggcg atataggcgc 6000

cagcaaccgc acctgtggcg ccggtgatgc cggccacgat gcgtccggcg tagaggatcg 6060

agatctcgat cccgcgaaat taatacgact cactata 6097

<210> 48
<211> 6181
<212> DNA
<213> Artificial Sequence

<220>
<223> pET24d-N-NVL[human](aa549-856)

<400> 48
ggggaattgt gagcggataa caattcccct ctagaaataa ttttgtttaa ctttaagaag 60

gagatatacc atgagccatc atcatcatca tcatcatcat tccatgctgt gcattgaact 120

gaatgatttc attgttgctc tatcctcagt ccaaccctct gccaaaaggg aaggctttgt 180

cactgtccct aatgtgacat gggcagatat tggtgccctg gaagacatta gagaggagct 240

caccatggca atattggcac cagtacgcaa cccagaccag ttcaaagctc ttggattggt 300

gactccagct ggggtcctcc ttgctggtcc tcctggctgt gggaagactc tgctggcgaa 360

ggctgttgca aatgagtccg gactaaattt tatatctgtc aagggccccg aattactaaa 420

catgtatgtt ggtgagagtg aacgtgctgt gcgacaagtt tttcaacgag ccaagaactc 480

agcaccctgt gtgatattct ttgatgaagt ggatgcttta tgtcctcgaa gatcagaccg 540

agagacaggg gcaagtgtcc gagtggtgaa tcagctactt acagagatgg atggtctgga 600

agcacgccag caggttttta ttatggcagc cactaacagg ccagatataa ttgaccctgc 660

aatcctgcgc ccgggccgcc tggacaaaac actgtttgtg ggtttaccgc cccctgcaga 720

tcgccttgcc atcttaaaaa ctatcacaaa aaatggtacc aaaccaccac tggatgcaga 780

tgtaaatttg gaagcaattg ctggtgacct tcgctgtgat tgctatacgg gcgcagatct 840

ctctgctttg gtacgagaag cttctatctg tgccctgaga caggaaatgg caagacagaa 900

gagtggaaat gaaaaaggtg aactcaaggt tagtcataag cattttgaag aagctttcaa 960

gaaagtaaga tcatctatat caaaaaagga tcaaatcatg tatgaacgtt tgcaggagtc 1020

cctcagccgg taatgactcg agtgagatcc ggctgctaac aaagcccgaa aggaagctga 1080

gttggctgct gccaccgctg agcaataact agcataaccc cttggggcct ctaaacgggt 1140

cttgaggggt tttttgctga aaggaggaac tatatccgga ttggcgaatg ggacgcgccc 1200

tgtagcggcg cattaagcgc ggcgggtgtg gtggttacgc gcagcgtgac cgctacactt 1260

gccagcgccc tagcgcccgc tcctttcgct ttcttccctt cctttctcgc cacgttcgcc 1320

ggctttcccc gtcaagctct aaatcggggg ctccctttag ggttccgatt tagtgcttta 1380

cggcacctcg accccaaaaa acttgattag ggtgatggtt cacgtagtgg gccatcgccc 1440

tgatagacgg tttttcgccc tttgacgttg gagtccacgt tctttaatag tggactcttg 1500

ttccaaactg gaacaacact caaccctatc tcggtctatt cttttgattt ataagggatt 1560

ttgccgattt cggcctattg gttaaaaaat gagctgattt aacaaaaatt taacgcgaat 1620

tttaacaaaa tattaacgtt tacaatttca ggtggcactt ttcggggaaa tgtgcgcgga 1680

acccctattt gtttattttt ctaaatacat tcaaatatgt atccgctcat gaattaattc 1740

ttagaaaaac tcatcgagca tcaaatgaaa ctgcaattta ttcatatcag gattatcaat 1800

accatatttt tgaaaaagcc gtttctgtaa tgaaggagaa aactcaccga ggcagttcca 1860

taggatggca agatcctggt atcggtctgc gattccgact cgtccaacat caatacaacc 1920

tattaatttc ccctcgtcaa aaataaggtt atcaagtgag aaatcaccat gagtgacgac 1980

tgaatccggt gagaatggca aaagtttatg catttctttc cagacttgtt caacaggcca 2040

gccattacgc tcgtcatcaa aatcactcgc atcaaccaaa ccgttattca ttcgtgattg 2100

cgcctgagcg agacgaaata cgcgatcgct gttaaaagga caattacaaa caggaatcga 2160

atgcaaccgg cgcaggaaca ctgccagcgc atcaacaata ttttcacctg aatcaggata 2220

ttcttctaat acctggaatg ctgttttccc ggggatcgca gtggtgagta accatgcatc 2280

atcaggagta cggataaaat gcttgatggt cggaagaggc ataaattccg tcagccagtt 2340

tagtctgacc atctcatctg taacatcatt ggcaacgcta cctttgccat gtttcagaaa 2400

caactctggc gcatcgggct tcccatacaa tcgatagatt gtcgcacctg attgcccgac 2460

attatcgcga gcccatttat acccatataa atcagcatcc atgttggaat ttaatcgcgg 2520

cctagagcaa gacgtttccc gttgaatatg gctcataaca ccccttgtat tactgtttat 2580

gtaagcagac agttttattg ttcatgacca aaatccctta acgtgagttt tcgttccact 2640

gagcgtcaga ccccgtagaa aagatcaaag gatcttcttg agatcctttt tttctgcgcg 2700

taatctgctg cttgcaaaca aaaaaaccac cgctaccagc ggtggtttgt ttgccggatc 2760

aagagctacc aactcttttt ccgaaggtaa ctggcttcag cagagcgcag ataccaaata 2820

ctgtccttct agtgtagccg tagttaggcc accacttcaa gaactctgta gcaccgccta 2880

catacctcgc tctgctaatc ctgttaccag tggctgctgc cagtggcgat aagtcgtgtc 2940

ttaccgggtt ggactcaaga cgatagttac cggataaggc gcagcggtcg ggctgaacgg 3000

ggggttcgtg cacacagccc agcttggagc gaacgaccta caccgaactg agatacctac 3060

agcgtgagct atgagaaagc gccacgcttc ccgaagggag aaaggcggac aggtatccgg 3120

taagcggcag ggtcggaaca ggagagcgca cgagggagct tccaggggga aacgcctggt 3180

atctttatag tcctgtcggg tttcgccacc tctgacttga gcgtcgattt ttgtgatgct 3240

cgtcaggggg gcggagccta tggaaaaacg ccagcaacgc ggccttttta cggttcctgg 3300

ccttttgctg gccttttgct cacatgttct ttcctgcgtt atcccctgat tctgtggata 3360

accgtattac cgcctttgag tgagctgata ccgctcgccg cagccgaacg accgagcgca 3420

gcgagtcagt gagcgaggaa gcggaagagc gcctgatgcg gtattttctc cttacgcatc 3480

tgtgcggtat ttcacaccgc atatatggtg cactctcagt acaatctgct ctgatgccgc 3540

atagttaagc cagtatacac tccgctatcg ctacgtgact gggtcatggc tgcgccccga 3600

cacccgccaa cacccgctga cgcgccctga cgggcttgtc tgctcccggc atccgcttac 3660

agacaagctg tgaccgtctc cgggagctgc atgtgtcaga ggttttcacc gtcatcaccg 3720

aaacgcgcga ggcagctgcg gtaaagctca tcagcgtggt cgtgaagcga ttcacagatg 3780

tctgcctgtt catccgcgtc cagctcgttg agtttctcca gaagcgttaa tgtctggctt 3840

ctgataaagc gggccatgtt aagggcggtt ttttcctgtt tggtcactga tgcctccgtg 3900

taagggggat ttctgttcat gggggtaatg ataccgatga aacgagagag gatgctcacg 3960

atacgggtta ctgatgatga acatgcccgg ttactggaac gttgtgaggg taaacaactg 4020

gcggtatgga tgcggcggga ccagagaaaa atcactcagg gtcaatgcca gcgcttcgtt 4080

aatacagatg taggtgttcc acagggtagc cagcagcatc ctgcgatgca gatccggaac 4140

ataatggtgc agggcgctga cttccgcgtt tccagacttt acgaaacacg gaaaccgaag 4200

accattcatg ttgttgctca ggtcgcagac gttttgcagc agcagtcgct tcacgttcgc 4260

tcgcgtatcg gtgattcatt ctgctaacca gtaaggcaac cccgccagcc tagccgggtc 4320

ctcaacgaca ggagcacgat catgcgcacc cgtggggccg ccatgccggc gataatggcc 4380

tgcttctcgc cgaaacgttt ggtggcggga ccagtgacga aggcttgagc gagggcgtgc 4440

aagattccga ataccgcaag cgacaggccg atcatcgtcg cgctccagcg aaagcggtcc 4500

tcgccgaaaa tgacccagag cgctgccggc acctgtccta cgagttgcat gataaagaag 4560

acagtcataa gtgcggcgac gatagtcatg ccccgcgccc accggaagga gctgactggg 4620

ttgaaggctc tcaagggcat cggtcgagat cccggtgcct aatgagtgag ctaacttaca 4680

ttaattgcgt tgcgctcact gcccgctttc cagtcgggaa acctgtcgtg ccagctgcat 4740

taatgaatcg gccaacgcgc ggggagaggc ggtttgcgta ttgggcgcca gggtggtttt 4800

tcttttcacc agtgagacgg gcaacagctg attgcccttc accgcctggc cctgagagag 4860

ttgcagcaag cggtccacgc tggtttgccc cagcaggcga aaatcctgtt tgatggtggt 4920

taacggcggg atataacatg agctgtcttc ggtatcgtcg tatcccacta ccgagatatc 4980

cgcaccaacg cgcagcccgg actcggtaat ggcgcgcatt gcgcccagcg ccatctgatc 5040

gttggcaacc agcatcgcag tgggaacgat gccctcattc agcatttgca tggtttgttg 5100

aaaaccggac atggcactcc agtcgccttc ccgttccgct atcggctgaa tttgattgcg 5160

agtgagatat ttatgccagc cagccagacg cagacgcgcc gagacagaac ttaatgggcc 5220

cgctaacagc gcgatttgct ggtgacccaa tgcgaccaga tgctccacgc ccagtcgcgt 5280

accgtcttca tgggagaaaa taatactgtt gatgggtgtc tggtcagaga catcaagaaa 5340

taacgccgga acattagtgc aggcagcttc cacagcaatg gcatcctggt catccagcgg 5400

atagttaatg atcagcccac tgacgcgttg cgcgagaaga ttgtgcaccg ccgctttaca 5460

ggcttcgacg ccgcttcgtt ctaccatcga caccaccacg ctggcaccca gttgatcggc 5520

gcgagattta atcgccgcga caatttgcga cggcgcgtgc agggccagac tggaggtggc 5580

aacgccaatc agcaacgact gtttgcccgc cagttgttgt gccacgcggt tgggaatgta 5640

attcagctcc gccatcgccg cttccacttt ttcccgcgtt ttcgcagaaa cgtggctggc 5700

ctggttcacc acgcgggaaa cggtctgata agagacaccg gcatactctg cgacatcgta 5760

taacgttact ggtttcacat tcaccaccct gaattgactc tcttccgggc gctatcatgc 5820

cataccgcga aaggttttgc gccattcgat ggtgtccggg atctcgacgc tctcccttat 5880

gcgactcctg cattaggaag cagcccagta gtaggttgag gccgttgagc accgccgccg 5940

caaggaatgg tgcatgcaag gagatggcgc ccaacagtcc cccggccacg gggcctgcca     6000

ccatacccac gccgaaacaa gcgctcatga gcccgaagtg gcgagcccga tcttccccat     6060

cggtgatgtc ggcgatatag gcgccagcaa ccgcacctgt ggcgccggtg atgccggcca     6120

cgatgcgtcc ggcgtagagg atcgagatct cgatcccgcg aaattaatac gactcactat     6180

a                                                                     6181

<210>  49
<211>  128
<212>  PRT
<213>  Artificial Sequence

<220>
<223>  H8-NVL[human](aa1-117)

<400>  49

Met Ser His His His His His His His His Ser Met Lys Pro Arg Pro
1               5                   10                  15

Ala Gly Phe Val Asp Asn Lys Leu Lys Gln Arg Val Ile Gln Tyr Leu
            20                  25                  30

Thr Ser Asn Lys Cys Gly Lys Tyr Val Asp Ile Gly Val Leu Ala Ser
        35                  40                  45

Asp Leu Gln Arg Val Tyr Ser Ile Asp Tyr Gly Arg Arg Lys Arg Asn
    50                  55                  60

Ala Phe Arg Ile Gln Val Glu Lys Val Phe Ser Ile Ile Ser Ser Glu
65                  70                  75                  80

Lys Glu Leu Lys Asn Leu Thr Glu Leu Glu Asp Glu His Leu Ala Lys
                85                  90                  95

Arg Ala Arg Gln Gly Glu Glu Asp Asn Glu Tyr Thr Glu Ser Tyr Ser
            100                 105                 110

Asp Asp Asp Ser Ser Met Glu Asp Tyr Pro Asp Pro Gln Ser Ala Asn
        115                 120                 125

<210>  50
<211>  199
<212>  PRT
<213>  Artificial Sequence

<220>
<223>  H8-NVL[human](aa106-292)

<400>  50

Met Ser His His His His His His His His Ser Met Ser Met Glu Asp
1               5                   10                  15

Tyr Pro Asp Pro Gln Ser Ala Asn His Met Asn Ser Ser Leu Leu Ser
            20                  25                  30

Leu Tyr Arg Lys Gly Asn Pro Asp Ser Val Ser Asn Thr Pro Glu Met
        35                  40                  45

Glu Gln Arg Glu Thr Thr Ser Ser Thr Pro Arg Ile Ser Ser Lys Thr
    50                  55                  60

Gly Ser Ile Pro Leu Lys Thr Pro Ala Lys Asp Ser Glu Gly Gly Trp
65                  70                  75                  80

Phe Ile Asp Lys Thr Pro Ser Val Lys Lys Asp Ser Phe Phe Leu Asp
                85                  90                  95

Leu Ser Cys Glu Lys Ser Asn Pro Lys Lys Pro Ile Thr Glu Ile Gln
            100                 105                 110

Asp Ser Lys Asp Ser Ser Leu Leu Glu Ser Asp Met Lys Arg Lys Gly
        115                 120                 125

Lys Leu Lys Asn Lys Gly Ser Lys Arg Lys Lys Glu Asp Leu Gln Glu
    130                 135                 140

Val Asp Gly Glu Ile Glu Ala Val Leu Gln Lys Lys Ala Lys Ala Arg
145                 150                 155                 160

Gly Leu Glu Phe Gln Ile Ser Asn Val Lys Phe Glu Asp Val Gly Gly
                165                 170                 175

Asn Asp Met Thr Leu Lys Glu Val Cys Lys Met Leu Ile His Met Arg
            180                 185                 190

His Pro Glu Val Tyr His His
        195

<210>  51
<211>  292
<212>  PRT
<213>  Artificial Sequence

<220>
<223>  H8-NVL[human](aa281-560)

<400>  51

Met Ser His His His His His His His His Ser Met Leu Ile His Met
1               5                   10                  15

Arg His Pro Glu Val Tyr His His Leu Gly Val Val Pro Pro Arg Gly
            20                  25                  30

Val Leu Leu His Gly Pro Pro Gly Cys Gly Lys Thr Leu Leu Ala His
        35                  40                  45

Ala Ile Ala Gly Glu Leu Asp Leu Pro Ile Leu Lys Val Ala Ala Pro
    50                  55                  60

Glu Ile Val Ser Gly Val Ser Gly Glu Ser Glu Gln Lys Leu Arg Glu
65                  70                  75                  80

Leu Phe Glu Gln Ala Val Ser Asn Ala Pro Cys Ile Ile Phe Ile Asp
                85                  90                  95

Glu Ile Asp Ala Ile Thr Pro Lys Arg Glu Val Ala Ser Lys Asp Met
            100                 105                 110

Glu Arg Arg Ile Val Ala Gln Leu Leu Thr Cys Met Asp Asp Leu Asn
        115                 120                 125

Asn Val Ala Ala Thr Ala Arg Val Leu Val Ile Gly Ala Thr Asn Arg
    130                 135                 140

Pro Asp Ser Leu Asp Pro Ala Leu Arg Arg Ala Gly Arg Phe Asp Arg
145                 150                 155                 160

Glu Ile Cys Leu Gly Ile Pro Asp Glu Ala Ser Arg Glu Arg Ile Leu
                165                 170                 175

Gln Thr Leu Cys Arg Lys Leu Arg Leu Pro Gln Ala Phe Asp Phe Cys
            180                 185                 190

His Leu Ala His Leu Thr Pro Gly Phe Val Gly Ala Asp Leu Met Ala
        195                 200                 205

Leu Cys Arg Glu Ala Ala Met Cys Ala Val Asn Arg Val Leu Met Lys
    210                 215                 220

Leu Gln Glu Gln Gln Lys Lys Asn Pro Glu Met Glu Asp Leu Pro Ser
225                 230                 235                 240

Lys Gly Val Gln Glu Glu Arg Leu Gly Thr Glu Pro Thr Ser Glu Thr
                245                 250                 255

Gln Asp Glu Leu Gln Arg Leu Leu Gly Leu Leu Arg Asp Gln Asp Pro
            260                 265                 270

Leu Ser Glu Glu Gln Met Gln Gly Leu Cys Ile Glu Leu Asn Asp Phe
        275                 280                 285

Ile Val Ala Leu
    290

<210>  52
<211>  320
<212>  PRT
<213>  Artificial Sequence

<220>
<223>  H8-NVL[human](aa549-856)

<400>  52

Met Ser His His His His His His His His Ser Met Leu Cys Ile Glu
1               5                   10                  15

Leu Asn Asp Phe Ile Val Ala Leu Ser Ser Val Gln Pro Ser Ala Lys
            20                  25                  30

Arg Glu Gly Phe Val Thr Val Pro Asn Val Thr Trp Ala Asp Ile Gly
        35                  40                  45

Ala Leu Glu Asp Ile Arg Glu Glu Leu Thr Met Ala Ile Leu Ala Pro
    50                  55                  60

Val Arg Asn Pro Asp Gln Phe Lys Ala Leu Gly Leu Val Thr Pro Ala
65                  70                  75                  80

Gly Val Leu Leu Ala Gly Pro Pro Gly Cys Gly Lys Thr Leu Leu Ala
                85                  90                  95

Lys Ala Val Ala Asn Glu Ser Gly Leu Asn Phe Ile Ser Val Lys Gly
            100                 105                 110

Pro Glu Leu Leu Asn Met Tyr Val Gly Glu Ser Glu Arg Ala Val Arg
        115                 120                 125

Gln Val Phe Gln Arg Ala Lys Asn Ser Ala Pro Cys Val Ile Phe Phe
    130                 135                 140

Asp Glu Val Asp Ala Leu Cys Pro Arg Arg Ser Asp Arg Glu Thr Gly
145                 150                 155                 160

Ala Ser Val Arg Val Val Asn Gln Leu Leu Thr Glu Met Asp Gly Leu
                165                 170                 175

Glu Ala Arg Gln Gln Val Phe Ile Met Ala Ala Thr Asn Arg Pro Asp
            180                 185                 190

Ile Ile Asp Pro Ala Ile Leu Arg Pro Gly Arg Leu Asp Lys Thr Leu
        195                 200                 205

Phe Val Gly Leu Pro Pro Pro Ala Asp Arg Leu Ala Ile Leu Lys Thr
    210                 215                 220

Ile Thr Lys Asn Gly Thr Lys Pro Pro Leu Asp Ala Asp Val Asn Leu
225                 230                 235                 240

Glu Ala Ile Ala Gly Asp Leu Arg Cys Asp Cys Tyr Thr Gly Ala Asp
                245                 250                 255

Leu Ser Ala Leu Val Arg Glu Ala Ser Ile Cys Ala Leu Arg Gln Glu
            260                 265                 270

Met Ala Arg Gln Lys Ser Gly Asn Glu Lys Gly Glu Leu Lys Val Ser
        275                 280                 285

His Lys His Phe Glu Glu Ala Phe Lys Lys Val Arg Ser Ser Ile Ser
    290                 295                 300

Lys Lys Asp Gln Ile Met Tyr Glu Arg Leu Gln Glu Ser Leu Ser Arg
305                 310                 315                 320

<210>  53
<211>  36
<212>  DNA
<213>  Artificial Sequence

<220>
<223>  sense CD2BP2-F1

<400>  53
atacgtctcg catgccaaag aggaaagtga ccttcc                                36

<210>  54
<211>  44
<212>  DNA
<213>  Artificial Sequence

<220>
<223>  asense CD2BP2-F1

<400>  54
tatcgtctcg tcgatcagat cttcacccag tcaatgttgt ccag                       44

<210>  55
<211>  412
<212>  DNA
<213>  Artificial Sequence

<220>
<223>  CD2BP2-PCR-fragment 1-128

<400>  55
atacgtctcg catgccaaag aggaaagtga ccttccaagg cgtgggagat gaggaggatg      60

aggatgaaat cattgtcccc aagaagaagc tggtggaccc tgtggctggg tcagggggtc      120

ctgggagccg ctttaaaggc aaacactctt tggatagcga tgaggaggag gatgatgatg      180

atggggggtc cagcaaatat gacatcttgg cctcagagga tgtagaaggt caggaggcag      240

ccacactccc cagcgagggg ggtgttcgga tcacaccctt taacctgcag gaggagatgg      300

aggaaggcca ctttgatgcc gatggcaact acttcctgaa ccgggatgct cagatccgag      360

acagctggct ggacaacatt gactgggtga agatctgatc gacgagacga ta      412


<210>  56
<211>  40
<212>  DNA
<213>  Artificial Sequence

<220>
<223>  sense CD2BP2-F2

<400>  56
atacgtctcg catggacagc tggctggaca acattgactg      40


<210>  57
<211>  43
<212>  DNA
<213>  Artificial Sequence

<220>
<223>  asense CD2BP2-F2

<400>  57
tatcgtctcg tcgatcacag ttcctcctcc gccaactcct cag      43


<210>  58
<211>  463
<212>  DNA
<213>  Artificial Sequence

<220>
<223>  CD2BP2-PCR-fragment 117-260

<400>  58
atacgtctcg catggacagc tggctggaca acattgactg ggtgaagatc cgggagcggc      60

cacctggcca gcgccaggcc tcagactcgg aggaggagga cagcttgggc cagacctcaa      120

tgagtgccca agccctcttg gagggacttt tggagctcct attgcctaga gagacagtgg      180

ctggggcact gaggcgtctg ggggcccgag gaggaggcaa agggagaaag gggcctgggc 240

aacccagttc ccctcagcgc ctggaccggc tctccgggtt ggccgaccag atggtggccc 300

ggggcaacct tggtgtgtac caggaaacaa gggaacggtt ggctatgcgt ctgaagggtt 360

tggggtgtca gaccctagga ccccacaatc ccacaccccc accctccctg gacatgttcg 420

ctgaggagtt ggcggaggag gaactgtgat cgacgagacg ata 463

<210> 59
<211> 41
<212> DNA
<213> Artificial Sequence

<220>
<223> sense CD2BP2-F3

<400> 59
atacgtctcg catggacatg ttcgctgagg agttggcgga g 41

<210> 60
<211> 43
<212> DNA
<213> Artificial Sequence

<220>
<223> asense CD2BP2-F3

<400> 60
tatcgtctcg tcgatcatca ggtgtagagg tcaaagtcaa tgc 43

<210> 61
<211> 313
<212> DNA
<213> Artificial Sequence

<220>
<223> CD2BP2-PCR-fragment 249-341

<400> 61
atacgtctcg catggacatg ttcgctgagg agttggcgga ggaggaactg gagaccccaa 60

cccctaccca gagaggagaa gcagagtcgc ggggagatgg tctggtggat gtgatgtggg 120

aatataagtg ggagaacacg ggggatgccg agctgtatgg gcccttcacc agcgcccaga 180

tgcagacctg ggtgagtgaa ggctacttcc cggacggtgt ttattgccgg aagctggacc 240

cccctggtgg tcagttctac aactccaaac gcattgactt tgacctctac acctgatgat 300

cgacgagacg ata 313

<210> 62
<211> 5634
<212> DNA
<213> Artificial Sequence

<220>
<223>  pET24d-N-CD2BP2[human](aa1-128)

<400>  62
ggggaattgt gagcggataa caattcccct ctagaaataa ttttgtttaa ctttaagaag      60

gagatatacc atgagccatc atcatcatca tcatcatcat tccatgccaa agaggaaagt      120

gaccttccaa ggcgtgggag atgaggagga tgaggatgaa atcattgtcc ccaagaagaa      180

gctggtggac cctgtggctg ggtcaggggg tcctgggagc cgctttaaag gcaaacactc      240

tttggatagc gatgaggagg aggatgatga tgatgggggg tccagcaaat atgacatctt      300

ggcctcagag gatgtagaag gtcaggaggc agccacactc cccagcgagg ggggtgttcg      360

gatcacaccc tttaacctgc aggaggagat ggaggaaggc cactttgatg ccgatggcaa      420

ctacttcctg aaccgggatg ctcagatccg agacagctgg ctggacaaca ttgactgggt      480

gaagatctga tcgagtgaga tccggctgct aacaaagccc gaaaggaagc tgagttggct      540

gctgccaccg ctgagcaata actagcataa ccccttgggg cctctaaacg ggtcttgagg      600

ggttttttgc tgaaaggagg aactatatcc ggattggcga atgggacgcg ccctgtagcg      660

gcgcattaag cgcggcgggt gtggtggtta cgcgcagcgt gaccgctaca cttgccagcg      720

ccctagcgcc cgctcctttc gctttcttcc cttcctttct cgccacgttc gccggctttc      780

cccgtcaagc tctaaatcgg gggctccctt tagggttccg atttagtgct ttacggcacc      840

tcgaccccaa aaaacttgat tagggtgatg gttcacgtag tgggccatcg ccctgataga      900

cggtttttcg ccctttgacg ttggagtcca cgttctttaa tagtggactc ttgttccaaa      960

ctggaacaac actcaaccct atctcggtct attcttttga tttataaggg attttgccga      1020

tttcggccta ttggttaaaa aatgagctga tttaacaaaa atttaacgcg aattttaaca      1080

aaatattaac gtttacaatt tcaggtggca cttttcgggg aaatgtgcgc ggaaccccta      1140

tttgtttatt tttctaaata cattcaaata tgtatccgct catgaattaa ttcttagaaa      1200

aactcatcga gcatcaaatg aaactgcaat ttattcatat caggattatc aataccatat      1260

ttttgaaaaa gccgtttctg taatgaagga gaaaactcac cgaggcagtt ccataggatg      1320

gcaagatcct ggtatcggtc tgcgattccg actcgtccaa catcaataca acctattaat      1380

ttcccctcgt caaaaataag gttatcaagt gagaaatcac catgagtgac gactgaatcc      1440

ggtgagaatg gcaaaagttt atgcatttct ttccagactt gttcaacagg ccagccatta      1500

cgctcgtcat caaaatcact cgcatcaacc aaaccgttat tcattcgtga ttgcgcctga      1560

gcgagacgaa atacgcgatc gctgttaaaa ggacaattac aaacaggaat cgaatgcaac      1620

cggcgcagga acactgccag cgcatcaaca atattttcac ctgaatcagg atattcttct      1680

aatacctgga atgctgtttt cccggggatc gcagtggtga gtaaccatgc atcatcagga      1740

gtacggataa aatgcttgat ggtcggaaga ggcataaatt ccgtcagcca gtttagtctg      1800

accatctcat ctgtaacatc attggcaacg ctacctttgc catgtttcag aaacaactct 1860

ggcgcatcgg gcttcccata caatcgatag attgtcgcac ctgattgccc gacattatcg 1920

cgagcccatt tatacccata taaatcagca tccatgttgg aatttaatcg cggcctagag 1980

caagacgttt cccgttgaat atggctcata acaccccttg tattactgtt tatgtaagca 2040

gacagtttta ttgttcatga ccaaaatccc ttaacgtgag ttttcgttcc actgagcgtc 2100

agaccccgta gaaaagatca aaggatcttc ttgagatcct ttttttctgc gcgtaatctg 2160

ctgcttgcaa acaaaaaaac caccgctacc agcggtggtt tgtttgccgg atcaagagct 2220

accaactctt tttccgaagg taactggctt cagcagagcg cagataccaa atactgtcct 2280

tctagtgtag ccgtagttag gccaccactt caagaactct gtagcaccgc ctacatacct 2340

cgctctgcta atcctgttac cagtggctgc tgccagtggc gataagtcgt gtcttaccgg 2400

gttggactca agacgatagt taccggataa ggcgcagcgg tcgggctgaa cggggggttc 2460

gtgcacacag cccagcttgg agcgaacgac ctacaccgaa ctgagatacc tacagcgtga 2520

gctatgagaa agcgccacgc ttcccgaagg gagaaaggcg gacaggtatc cggtaagcgg 2580

cagggtcgga acaggagagc gcacgaggga gcttccaggg ggaaacgcct ggtatcttta 2640

tagtcctgtc gggtttcgcc acctctgact tgagcgtcga tttttgtgat gctcgtcagg 2700

ggggcggagc ctatggaaaa acgccagcaa cgcggccttt ttacggttcc tggccttttg 2760

ctggcctttt gctcacatgt tctttcctgc gttatcccct gattctgtgg ataaccgtat 2820

taccgccttt gagtgagctg ataccgctcg ccgcagccga acgaccgagc gcagcgagtc 2880

agtgagcgag gaagcggaag agcgcctgat gcggtatttt ctccttacgc atctgtgcgg 2940

tatttcacac cgcatatatg gtgcactctc agtacaatct gctctgatgc cgcatagtta 3000

agccagtata cactccgcta tcgctacgtg actgggtcat ggctgcgccc cgacacccgc 3060

caacacccgc tgacgcgccc tgacgggctt gtctgctccc ggcatccgct tacagacaag 3120

ctgtgaccgt ctccgggagc tgcatgtgtc agaggttttc accgtcatca ccgaaacgcg 3180

cgaggcagct gcggtaaagc tcatcagcgt ggtcgtgaag cgattcacag atgtctgcct 3240

gttcatccgc gtccagctcg ttgagtttct ccagaagcgt taatgtctgg cttctgataa 3300

agcgggccat gttaagggcg gttttttcct gtttggtcac tgatgcctcc gtgtaagggg 3360

gatttctgtt catgggggta atgataccga tgaaacgaga gaggatgctc acgatacggg 3420

ttactgatga tgaacatgcc cggttactgg aacgttgtga gggtaaacaa ctggcggtat 3480

ggatgcggcg ggaccagaga aaaatcactc agggtcaatg ccagcgcttc gttaatacag 3540

atgtaggtgt tccacagggt agccagcagc atcctgcgat gcagatccgg aacataatgg 3600

tgcagggcgc tgacttccgc gtttccagac tttacgaaac acggaaaccg aagaccattc 3660

atgttgttgc tcaggtcgca gacgttttgc agcagcagtc gcttcacgtt cgctcgcgta 3720

tcggtgattc attctgctaa ccagtaaggc aaccccgcca gcctagccgg gtcctcaacg 3780

acaggagcac gatcatgcgc acccgtgggg ccgccatgcc ggcgataatg gcctgcttct 3840

cgccgaaacg tttggtggcg ggaccagtga cgaaggcttg agcgagggcg tgcaagattc 3900

cgaataccgc aagcgacagg ccgatcatcg tcgcgctcca gcgaaagcgg tcctcgccga 3960

aaatgaccca gagcgctgcc ggcacctgtc ctacgagttg catgataaag aagacagtca 4020

taagtgcggc gacgatagtc atgccccgcg cccaccggaa ggagctgact gggttgaagg 4080

ctctcaaggg catcggtcga gatcccggtg cctaatgagt gagctaactt acattaattg 4140

cgttgcgctc actgcccgct ttccagtcgg gaaacctgtc gtgccagctg cattaatgaa 4200

tcggccaacg cgcggggaga ggcggtttgc gtattgggcg ccagggtggt ttttcttttc 4260

accagtgaga cgggcaacag ctgattgccc ttcaccgcct ggccctgaga gagttgcagc 4320

aagcggtcca cgctggtttg ccccagcagg cgaaaatcct gtttgatggt ggttaacggc 4380

gggatataac atgagctgtc ttcggtatcg tcgtatccca ctaccgagat atccgcacca 4440

acgcgcagcc cggactcggt aatggcgcgc attgcgccca gcgccatctg atcgttggca 4500

accagcatcg cagtgggaac gatgccctca ttcagcattt gcatggtttg ttgaaaaccg 4560

gacatggcac tccagtcgcc ttcccgttcc gctatcggct gaatttgatt gcgagtgaga 4620

tatttatgcc agccagccag acgcagacgc gccgagacag aacttaatgg gcccgctaac 4680

agcgcgattt gctggtgacc caatgcgacc agatgctcca cgcccagtcg cgtaccgtct 4740

tcatgggaga aaataatact gttgatgggt gtctggtcag agacatcaag aaataacgcc 4800

ggaacattag tgcaggcagc ttccacagca atggcatcct ggtcatccag cggatagtta 4860

atgatcagcc cactgacgcg ttgcgcgaga agattgtgca ccgccgcttt acaggcttcg 4920

acgccgcttc gttctaccat cgacaccacc acgctggcac ccagttgatc ggcgcgagat 4980

ttaatcgccg cgacaatttg cgacggcgcg tgcagggcca gactggaggt ggcaacgcca 5040

atcagcaacg actgtttgcc cgccagttgt tgtgccacgc ggttgggaat gtaattcagc 5100

tccgccatcg ccgcttccac tttttcccgc gttttcgcag aaacgtggct ggcctggttc 5160

accacgcggg aaacggtctg ataagagaca ccggcatact ctgcgacatc gtataacgtt 5220

actggtttca cattcaccac cctgaattga ctctcttccg ggcgctatca tgccataccg 5280

cgaaaggttt tgcgccattc gatggtgtcc gggatctcga cgctctccct tatgcgactc 5340

ctgcattagg aagcagccca gtagtaggtt gaggccgttg agcaccgccg ccgcaaggaa 5400

tggtgcatgc aaggagatgg cgcccaacag tcccccggcc acggggcctg ccaccatacc 5460

cacgccgaaa caagcgctca tgagcccgaa gtggcgagcc cgatcttccc catcggtgat 5520

gtcggcgata taggcgccag caaccgcacc tgtggcgccg gtgatgccgg ccacgatgcg 5580

tccggcgtag aggatcgaga tctcgatccc gcgaaattaa tacgactcac tata 5634

<210> 63
<211> 5685
<212> DNA
<213> Artificial Sequence

<220>
<223> pET24d-N-CD2BP2[human](aa117-260)

<400> 63
ggggaattgt gagcggataa caattcccct ctagaaataa ttttgtttaa ctttaagaag 60

gagatatacc atgagccatc atcatcatca tcatcatcat tccatggaca gctggctgga 120

caacattgac tgggtgaaga tccgggagcg gccacctggc cagcgccagg cctcagactc 180

ggaggaggag gacagcttgg gccagacctc aatgagtgcc caagccctct tggagggact 240

tttggagctc ctattgccta gagagacagt ggctggggca ctgaggcgtc tgggggcccg 300

aggaggaggc aaagggagaa aggggcctgg gcaacccagt tcccctcagc gcctggaccg 360

gctctccggg ttggccgacc agatggtggc ccggggcaac cttggtgtgt accaggaaac 420

aagggaacgg ttggctatgc gtctgaaggg tttggggtgt cagaccctag gaccccacaa 480

tcccacaccc ccaccctccc tggacatgtt cgctgaggag ttggcggagg aggaactgtg 540

atcgagtgag atccggctgc taacaaagcc cgaaaggaag ctgagttggc tgctgccacc 600

gctgagcaat aactagcata accccttggg gcctctaaac gggtcttgag gggttttttg 660

ctgaaaggag gaactatatc cggattggcg aatgggacgc gccctgtagc ggcgcattaa 720

gcgcggcggg tgtggtggtt acgcgcagcg tgaccgctac acttgccagc gccctagcgc 780

ccgctccttt cgctttcttc ccttcctttc tcgccacgtt cgccggcttt ccccgtcaag 840

ctctaaatcg ggggctccct ttagggttcc gatttagtgc tttacggcac ctcgacccca 900

aaaaacttga ttagggtgat ggttcacgta gtgggccatc gccctgatag acggtttttc 960

gccctttgac gttggagtcc acgttcttta atagtggact cttgttccaa actggaacaa 1020

cactcaaccc tatctcggtc tattcttttg atttataagg gattttgccg atttcggcct 1080

attggttaaa aaatgagctg atttaacaaa aatttaacgc gaattttaac aaaatattaa 1140

cgtttacaat ttcaggtggc acttttcggg gaaatgtgcg cggaacccct atttgtttat 1200

ttttctaaat acattcaaat atgtatccgc tcatgaatta attcttagaa aaactcatcg 1260

agcatcaaat gaaactgcaa tttattcata tcaggattat caataccata tttttgaaaa 1320

agccgtttct gtaatgaagg agaaaactca ccgaggcagt tccataggat ggcaagatcc 1380

tggtatcggt ctgcgattcc gactcgtcca acatcaatac aacctattaa tttcccctcg 1440

tcaaaaataa ggttatcaag tgagaaatca ccatgagtga cgactgaatc cggtgagaat 1500

ggcaaaagtt tatgcatttc tttccagact tgttcaacag gccagccatt acgctcgtca 1560

tcaaaatcac tcgcatcaac caaaccgtta ttcattcgtg attgcgcctg agcgagacga 1620

aatacgcgat cgctgttaaa aggacaatta caaacaggaa tcgaatgcaa ccggcgcagg 1680

aacactgcca gcgcatcaac aatattttca cctgaatcag gatattcttc taatacctgg 1740

aatgctgttt tcccggggat cgcagtggtg agtaaccatg catcatcagg agtacggata 1800

aaatgcttga tggtcggaag aggcataaat tccgtcagcc agtttagtct gaccatctca 1860

tctgtaacat cattggcaac gctacctttg ccatgtttca gaaacaactc tggcgcatcg 1920

ggcttcccat acaatcgata gattgtcgca cctgattgcc cgacattatc gcgagcccat 1980

ttatacccat ataaatcagc atccatgttg gaatttaatc gcggcctaga gcaagacgtt 2040

tcccgttgaa tatggctcat aacacccctt gtattactgt ttatgtaagc agacagtttt 2100

attgttcatg accaaaatcc cttaacgtga gttttcgttc cactgagcgt cagaccccgt 2160

agaaaagatc aaaggatctt cttgagatcc tttttttctg cgcgtaatct gctgcttgca 2220

aacaaaaaaa ccaccgctac cagcggtggt ttgtttgccg gatcaagagc taccaactct 2280

ttttccgaag gtaactggct tcagcagagc gcagatacca aatactgtcc ttctagtgta 2340

gccgtagtta ggccaccact tcaagaactc tgtagcaccg cctacatacc tcgctctgct 2400

aatcctgtta ccagtggctg ctgccagtgg cgataagtcg tgtcttaccg ggttggactc 2460

aagacgatag ttaccggata aggcgcagcg gtcgggctga acggggggtt cgtgcacaca 2520

gcccagcttg gagcgaacga cctacaccga actgagatac ctacagcgtg agctatgaga 2580

aagcgccacg cttcccgaag ggagaaaggc ggacaggtat ccggtaagcg gcagggtcgg 2640

aacaggagag cgcacgaggg agcttccagg gggaaacgcc tggtatcttt atagtcctgt 2700

cgggtttcgc cacctctgac ttgagcgtcg atttttgtga tgctcgtcag gggggcggag 2760

cctatggaaa aacgccagca acgcggcctt tttacggttc ctggcctttt gctggccttt 2820

tgctcacatg ttctttcctg cgttatcccc tgattctgtg gataaccgta ttaccgcctt 2880

tgagtgagct gataccgctc gccgcagccg aacgaccgag cgcagcgagt cagtgagcga 2940

ggaagcggaa gagcgcctga tgcggtattt tctccttacg catctgtgcg gtatttcaca 3000

ccgcatatat ggtgcactct cagtacaatc tgctctgatg ccgcatagtt aagccagtat 3060

acactccgct atcgctacgt gactgggtca tggctgcgcc ccgacacccg ccaacacccg 3120

ctgacgcgcc ctgacgggct tgtctgctcc cggcatccgc ttacagacaa gctgtgaccg 3180

tctccgggag ctgcatgtgt cagaggtttt caccgtcatc accgaaacgc gcgaggcagc 3240

tgcggtaaag ctcatcagcg tggtcgtgaa gcgattcaca gatgtctgcc tgttcatccg 3300

cgtccagctc gttgagtttc tccagaagcg ttaatgtctg gcttctgata aagcgggcca 3360

tgttaagggc ggttttttcc tgtttggtca ctgatgcctc cgtgtaaggg ggatttctgt 3420

tcatgggggt aatgataccg atgaaacgag agaggatgct cacgatacgg gttactgatg 3480

atgaacatgc ccggttactg gaacgttgtg agggtaaaca actggcggta tggatgcggc 3540

gggaccagag aaaaatcact cagggtcaat gccagcgctt cgttaataca gatgtaggtg 3600

ttccacaggg tagccagcag catcctgcga tgcagatccg gaacataatg gtgcagggcg 3660

ctgacttccg cgtttccaga ctttacgaaa cacggaaacc gaagaccatt catgttgttg 3720

ctcaggtcgc agacgttttg cagcagcagt cgcttcacgt tcgctcgcgt atcggtgatt 3780

cattctgcta accagtaagg caaccccgcc agcctagccg ggtcctcaac gacaggagca 3840

cgatcatgcg cacccgtggg gccgccatgc cggcgataat ggcctgcttc tcgccgaaac 3900

gtttggtggc gggaccagtg acgaaggctt gagcgagggc gtgcaagatt ccgaataccg 3960

caagcgacag gccgatcatc gtcgcgctcc agcgaaagcg gtcctcgccg aaaatgaccc 4020

agagcgctgc cggcacctgt cctacgagtt gcatgataaa gaagacagtc ataagtgcgg 4080

cgacgatagt catgccccgc gcccaccgga aggagctgac tgggttgaag gctctcaagg 4140

gcatcggtcg agatcccggt gcctaatgag tgagctaact tacattaatt gcgttgcgct 4200

cactgcccgc tttccagtcg ggaaacctgt cgtgccagct gcattaatga atcggccaac 4260

gcgcggggag aggcggtttg cgtattgggc gccagggtgg tttttctttt caccagtgag 4320

acgggcaaca gctgattgcc cttcaccgcc tggccctgag agagttgcag caagcggtcc 4380

acgctggttt gccccagcag gcgaaaatcc tgtttgatgg tggttaacgg cgggatataa 4440

catgagctgt cttcggtatc gtcgtatccc actaccgaga tatccgcacc aacgcgcagc 4500

ccggactcgg taatggcgcg cattgcgccc agcgccatct gatcgttggc aaccagcatc 4560

gcagtgggaa cgatgccctc attcagcatt tgcatggttt gttgaaaacc ggacatggca 4620

ctccagtcgc cttcccgttc cgctatcggc tgaatttgat tgcgagtgag atatttatgc 4680

cagccagcca gacgcagacg cgccgagaca gaacttaatg ggcccgctaa cagcgcgatt 4740

tgctggtgac ccaatgcgac cagatgctcc acgcccagtc gcgtaccgtc ttcatgggag 4800

aaaataatac tgttgatggg tgtctggtca gagacatcaa gaaataacgc cggaacatta 4860

gtgcaggcag cttccacagc aatggcatcc tggtcatcca gcggatagtt aatgatcagc 4920

ccactgacgc gttgcgcgag aagattgtgc accgccgctt tacaggcttc gacgccgctt 4980

cgttctacca tcgacaccac cacgctggca cccagttgat cggcgcgaga tttaatcgcc 5040

gcgacaattt gcgacggcgc gtgcagggcc agactggagg tggcaacgcc aatcagcaac 5100

gactgtttgc ccgccagttg ttgtgccacg cggttgggaa tgtaattcag ctccgccatc 5160

gccgcttcca ctttttcccg cgttttcgca gaaacgtggc tggcctggtt caccacgcgg 5220

gaaacggtct gataagagac accggcatac tctgcgacat cgtataacgt tactggtttc 5280

acattcacca ccctgaattg actctcttcc gggcgctatc atgccatacc gcgaaaggtt 5340

ttgcgccatt cgatggtgtc cgggatctcg acgctctccc ttatgcgact cctgcattag 5400

gaagcagccc agtagtaggt tgaggccgtt gagcaccgcc gccgcaagga atggtgcatg 5460

caaggagatg gcgcccaaca gtcccccggc cacggggcct gccaccatac ccacgccgaa 5520

acaagcgctc atgagcccga agtggcgagc ccgatcttcc ccatcggtga tgtcggcgat 5580

ataggcgcca gcaaccgcac ctgtggcgcc ggtgatgccg gccacgatgc gtccggcgta 5640

gaggatcgag atctcgatcc cgcgaaatta atacgactca ctata 5685

<210> 64
<211> 5535
<212> DNA
<213> Artificial Sequence

<220>
<223> pET24d-N-CD2BP2[human](aa249-341)

<400> 64
ggggaattgt gagcggataa caattcccct ctagaaataa ttttgtttaa ctttaagaag 60

gagatatacc atgagccatc atcatcatca tcatcatcat tccatggaca tgttcgctga 120

ggagttggcg gaggaggaac tggagacccc aacccctacc cagagaggag aagcagagtc 180

gcggggagat ggtctggtgg atgtgatgtg ggaatataag tgggagaaca cgggggatgc 240

cgagctgtat gggcccttca ccagcgccca gatgcagacc tgggtgagtg aaggctactt 300

cccggacggt gtttattgcc ggaagctgga cccccctggt ggtcagttct acaactccaa 360

acgcattgac tttgacctct acacctgatg atcgagtgag atccggctgc taacaaagcc 420

cgaaaggaag ctgagttggc tgctgccacc gctgagcaat aactagcata accccttggg 480

gcctctaaac gggtcttgag gggttttttg ctgaaaggag gaactatatc cggattggcg 540

aatgggacgc gccctgtagc ggcgcattaa gcgcggcggg tgtggtggtt acgcgcagcg 600

tgaccgctac acttgccagc gccctagcgc ccgctccttt cgctttcttc ccttcctttc 660

tcgccacgtt cgccggcttt ccccgtcaag ctctaaatcg ggggctccct ttagggttcc 720

gatttagtgc tttacggcac ctcgacccca aaaaacttga ttagggtgat ggttcacgta 780

gtgggccatc gccctgatag acggtttttc gccctttgac gttggagtcc acgttcttta 840

atagtggact cttgttccaa actggaacaa cactcaaccc tatctcggtc tattcttttg 900

atttataagg gattttgccg atttcggcct attggttaaa aaatgagctg atttaacaaa 960

aatttaacgc gaattttaac aaaatattaa cgtttacaat ttcaggtggc acttttcggg 1020

gaaatgtgcg cggaacccct atttgtttat ttttctaaat acattcaaat atgtatccgc 1080

tcatgaatta attcttagaa aaactcatcg agcatcaaat gaaactgcaa tttattcata 1140

tcaggattat caataccata tttttgaaaa agccgtttct gtaatgaagg agaaaactca 1200

ccgaggcagt tccataggat ggcaagatcc tggtatcggt ctgcgattcc gactcgtcca 1260

acatcaatac aacctattaa tttcccctcg tcaaaaataa ggttatcaag tgagaaatca 1320

ccatgagtga cgactgaatc cggtgagaat ggcaaaagtt tatgcatttc tttccagact 1380

tgttcaacag gccagccatt acgctcgtca tcaaaatcac tcgcatcaac caaaccgtta 1440

ttcattcgtg attgcgcctg agcgagacga aatacgcgat cgctgttaaa aggacaatta 1500

caaacaggaa tcgaatgcaa ccggcgcagg aacactgcca gcgcatcaac aatattttca 1560

cctgaatcag gatattcttc taatacctgg aatgctgttt tcccggggat cgcagtggtg 1620

agtaaccatg catcatcagg agtacggata aaatgcttga tggtcggaag aggcataaat 1680

tccgtcagcc agtttagtct gaccatctca tctgtaacat cattggcaac gctacctttg 1740

ccatgtttca gaaacaactc tggcgcatcg ggcttcccat acaatcgata gattgtcgca 1800

cctgattgcc cgacattatc gcgagcccat ttatacccat ataaatcagc atccatgttg 1860

gaatttaatc gcggcctaga gcaagacgtt tcccgttgaa tatggctcat aacacccctt 1920

gtattactgt ttatgtaagc agacagtttt attgttcatg accaaaatcc cttaacgtga 1980

gttttcgttc cactgagcgt cagaccccgt agaaaagatc aaaggatctt cttgagatcc 2040

tttttttctg cgcgtaatct gctgcttgca aacaaaaaaa ccaccgctac cagcggtggt 2100

ttgtttgccg gatcaagagc taccaactct ttttccgaag gtaactggct tcagcagagc 2160

gcagatacca aatactgtcc ttctagtgta gccgtagtta ggccaccact tcaagaactc 2220

tgtagcaccg cctacatacc tcgctctgct aatcctgtta ccagtggctg ctgccagtgg 2280

cgataagtcg tgtcttaccg ggttggactc aagacgatag ttaccggata aggcgcagcg 2340

gtcgggctga acggggggtt cgtgcacaca gcccagcttg gagcgaacga cctacaccga 2400

actgagatac ctacagcgtg agctatgaga aagcgccacg cttcccgaag ggagaaaggc 2460

ggacaggtat ccggtaagcg gcagggtcgg aacaggagag cgcacgaggg agcttccagg 2520

gggaaacgcc tggtatcttt atagtcctgt cgggtttcgc cacctctgac ttgagcgtcg 2580

atttttgtga tgctcgtcag gggggcggag cctatggaaa aacgccagca acgcggcctt 2640

tttacggttc ctggcctttt gctggccttt tgctcacatg ttctttcctg cgttatcccc 2700

tgattctgtg gataaccgta ttaccgcctt tgagtgagct gataccgctc gccgcagccg 2760

aacgaccgag cgcagcgagt cagtgagcga ggaagcggaa gagcgcctga tgcggtattt 2820

tctccttacg catctgtgcg gtatttcaca ccgcatatat ggtgcactct cagtacaatc 2880

tgctctgatg ccgcatagtt aagccagtat acactccgct atcgctacgt gactgggtca 2940

tggctgcgcc ccgacacccg ccaacacccg ctgacgcgcc ctgacgggct tgtctgctcc 3000

cggcatccgc ttacagacaa gctgtgaccg tctccgggag ctgcatgtgt cagaggtttt 3060

caccgtcatc accgaaacgc gcgaggcagc tgcggtaaag ctcatcagcg tggtcgtgaa 3120

gcgattcaca gatgtctgcc tgttcatccg cgtccagctc gttgagtttc tccagaagcg 3180

ttaatgtctg gcttctgata aagcgggcca tgttaagggc ggttttttcc tgtttggtca 3240

ctgatgcctc cgtgtaaggg ggatttctgt tcatgggggt aatgataccg atgaaacgag 3300

agaggatgct cacgatacgg gttactgatg atgaacatgc ccggttactg gaacgttgtg 3360

agggtaaaca actggcggta tggatgcggc gggaccagag aaaaatcact cagggtcaat 3420

gccagcgctt cgttaataca gatgtaggtg ttccacaggg tagccagcag catcctgcga 3480

tgcagatccg gaacataatg gtgcagggcg ctgacttccg cgtttccaga ctttacgaaa 3540

cacggaaacc gaagaccatt catgttgttg ctcaggtcgc agacgttttg cagcagcagt 3600

cgcttcacgt tcgctcgcgt atcggtgatt cattctgcta accagtaagg caaccccgcc 3660

agcctagccg ggtcctcaac gacaggagca cgatcatgcg cacccgtggg gccgccatgc 3720

cggcgataat ggcctgcttc tcgccgaaac gtttggtggc gggaccagtg acgaaggctt 3780

gagcgagggc gtgcaagatt ccgaataccg caagcgacag gccgatcatc gtcgcgctcc 3840

agcgaaagcg gtcctcgccg aaaatgaccc agagcgctgc cggcacctgt cctacgagtt 3900

gcatgataaa gaagacagtc ataagtgcgg cgacgatagt catgccccgc gcccaccgga 3960

aggagctgac tgggttgaag gctctcaagg gcatcggtcg agatcccggt gcctaatgag 4020

tgagctaact tacattaatt gcgttgcgct cactgcccgc tttccagtcg ggaaacctgt 4080

cgtgccagct gcattaatga atcggccaac gcgcggggag aggcggtttg cgtattgggc 4140

gccagggtgg tttttctttt caccagtgag acgggcaaca gctgattgcc cttcaccgcc 4200

tggccctgag agagttgcag caagcggtcc acgctggttt gccccagcag gcgaaaatcc 4260

tgtttgatgg tggttaacgg cgggatataa catgagctgt cttcggtatc gtcgtatccc 4320

actaccgaga tatccgcacc aacgcgcagc ccggactcgg taatggcgcg cattgcgccc 4380

agcgccatct gatcgttggc aaccagcatc gcagtgggaa cgatgccctc attcagcatt 4440

tgcatggttt gttgaaaacc ggacatggca ctccagtcgc cttcccgttc cgctatcggc 4500

tgaatttgat tgcgagtgag atatttatgc cagccagcca gacgcagacg cgccgagaca 4560

gaacttaatg ggcccgctaa cagcgcgatt tgctggtgac ccaatgcgac cagatgctcc 4620

acgcccagtc gcgtaccgtc ttcatgggag aaaataatac tgttgatggg tgtctggtca 4680

gagacatcaa gaaataacgc cggaacatta gtgcaggcag cttccacagc aatggcatcc 4740

tggtcatcca gcggatagtt aatgatcagc ccactgacgc gttgcgcgag aagattgtgc 4800

accgccgctt tacaggcttc gacgccgctt cgttctacca tcgacaccac cacgctggca 4860

cccagttgat cggcgcgaga tttaatcgcc gcgacaattt gcgacggcgc gtgcagggcc 4920

agactggagg tggcaacgcc aatcagcaac gactgtttgc ccgccagttg ttgtgccacg 4980

cggttgggaa tgtaattcag ctccgccatc gccgcttcca ctttttcccg cgttttcgca 5040

gaaacgtggc tggcctggtt caccacgcgg gaaacggtct gataagagac accggcatac 5100

tctgcgacat cgtataacgt tactggtttc acattcacca ccctgaattg actctcttcc 5160

gggcgctatc atgccatacc gcgaaaggtt ttgcgccatt cgatggtgtc cgggatctcg 5220

acgctctccc ttatgcgact cctgcattag gaagcagccc agtagtaggt tgaggccgtt 5280

gagcaccgcc gccgcaagga atggtgcatg caaggagatg gcgcccaaca gtcccccggc 5340

cacggggcct gccaccatac ccacgccgaa acaagcgctc atgagcccga agtggcgagc 5400

ccgatcttcc ccatcggtga tgtcggcgat ataggcgcca gcaaccgcac ctgtggcgcc 5460

ggtgatgccg gccacgatgc gtccggcgta gaggatcgag atctcgatcc cgcgaaatta 5520

atacgactca ctata 5535

<210> 65
<211> 139
<212> PRT
<213> Artificial Sequence

<220>
<223> H8-CD2BP2[human](aa1-128)

<400> 65

Met Ser His His His His His His His His Ser Met Pro Lys Arg Lys
1               5                   10                  15

Val Thr Phe Gln Gly Val Gly Asp Glu Glu Asp Glu Asp Glu Ile Ile
            20                  25                  30

Val Pro Lys Lys Lys Leu Val Asp Pro Val Ala Gly Ser Gly Gly Pro
        35                  40                  45

Gly Ser Arg Phe Lys Gly Lys His Ser Leu Asp Ser Asp Glu Glu Glu
    50                  55                  60

Asp Asp Asp Asp Gly Gly Ser Ser Lys Tyr Asp Ile Leu Ala Ser Glu
65                  70                  75                  80

Asp Val Glu Gly Gln Glu Ala Ala Thr Leu Pro Ser Glu Gly Gly Val
                85                  90                  95

Arg Ile Thr Pro Phe Asn Leu Gln Glu Glu Met Glu Glu Gly His Phe
            100                 105                 110

Asp Ala Asp Gly Asn Tyr Phe Leu Asn Arg Asp Ala Gln Ile Arg Asp
        115                 120                 125

Ser Trp Leu Asp Asn Ile Asp Trp Val Lys Ile
    130                 135

<210>  66
<211>  156
<212>  PRT
<213>  Artificial Sequence

<220>
<223>  H8-CD2BP2[human](aa117-260)

<400>  66

Met Ser His His His His His His His His Ser Met Asp Ser Trp Leu
1               5                   10                  15

Asp Asn Ile Asp Trp Val Lys Ile Arg Glu Arg Pro Pro Gly Gln Arg
            20                  25                  30

Gln Ala Ser Asp Ser Glu Glu Glu Asp Ser Leu Gly Gln Thr Ser Met
        35                  40                  45

Ser Ala Gln Ala Leu Leu Glu Gly Leu Leu Glu Leu Leu Leu Pro Arg
    50                  55                  60

Glu Thr Val Ala Gly Ala Leu Arg Arg Leu Gly Ala Arg Gly Gly Gly
65                  70                  75                  80

Lys Gly Arg Lys Gly Pro Gly Gln Pro Ser Ser Pro Gln Arg Leu Asp
                85                  90                  95

Arg Leu Ser Gly Leu Ala Asp Gln Met Val Ala Arg Gly Asn Leu Gly
            100                 105                 110

Val Tyr Gln Glu Thr Arg Glu Arg Leu Ala Met Arg Leu Lys Gly Leu
        115                 120                 125

Gly Cys Gln Thr Leu Gly Pro His Asn Pro Thr Pro Pro Pro Ser Leu
    130                 135                 140

Asp Met Phe Ala Glu Glu Leu Ala Glu Glu Glu Leu
145                 150                 155

<210>  67
<211>  105
<212>  PRT
<213>  Artificial Sequence

<220>
<223>  H8-CD2BP2[human](aa249-341)

<400>  67

Met Ser His His His His His His His His Ser Met Asp Met Phe Ala
1               5                   10                  15

Glu Glu Leu Ala Glu Glu Glu Leu Glu Thr Pro Thr Pro Thr Gln Arg
            20                  25                  30

Gly Glu Ala Glu Ser Arg Gly Asp Gly Leu Val Asp Val Met Trp Glu
        35                  40                  45

Tyr Lys Trp Glu Asn Thr Gly Asp Ala Glu Leu Tyr Gly Pro Phe Thr
    50                  55                  60

Ser Ala Gln Met Gln Thr Trp Val Ser Glu Gly Tyr Phe Pro Asp Gly
65                  70                  75                  80

Val Tyr Cys Arg Lys Leu Asp Pro Pro Gly Gly Gln Phe Tyr Asn Ser
                85                  90                  95

Lys Arg Ile Asp Phe Asp Leu Tyr Thr
            100                 105

<210>  68
<211>  33
<212>  DNA
<213>  Artificial Sequence

<220>
<223>  sense TJP1-F1/2

<400>  68
ataggtctct catgtccgcc agagctgcgg ctg                                    33

<210>  69
<211>  43
<212>  DNA
<213>  Artificial Sequence

<220>
<223>  asense TJP1-F1/4

<400>  69
ataggtctcc tcgagttaag ggatgatgcc tcgttctacc tcc                         43

<210>  70
<211>  1754
<212>  DNA
<213>  Artificial Sequence

<220>
<223>  TJP1-PCR-fragment 1-575

<400>  70

ataggtctct catgtccgcc agagctgcgg ctgccaagag cacagcaatg gaggaaacag 60

ctatatggga acaacataca gtgacgcttc acagggctcc tggatttgga tttggaattg 120

caatatctgg tggacgagat aatcctcatt ttcagagtgg ggaaacgtca atagtgattt 180

cagatgtgct gaaaggagga ccagctgaag gacagctaca ggaaaatgac cgagttgcaa 240

tggttaacgg agtttcaatg gataatgttg aacatgcttt tgctgttcag caactaagga 300

aaagtgggaa aaatgcaaaa attacaatta gaaggaagaa gaaagttcaa ataccagtaa 360

gtcgtcctga tcctgaacca gtatctgata atgaagaaga tagttatgat gaggaaatac 420

atgatccaag aagtggccgg agtggtgtgg ttaacagaag gagtgagaag atttggccga 480

gggatagaag tgcaagtaga gagaggagct tgtccccgcg gtcagacagg cggtcagtgg 540

cttccagcca gcctgctaaa cctactaaag tcacactggt gaaatcccgg aaaaatgaag 600

aatatggtct tcgattggca agccatatat ttgttaagga aatttcacaa gatagtttgg 660

cagcaagaga tggcaatatt caagaaggtg atgttgtatt gaagataaat ggtactgtga 720

cagaaaatat gtcattgaca gatgcaaaga cattgataga aaggtctaaa ggcaaattaa 780

aaatggtagt tcaaagagat gaacgggcta cgctattgaa tgtccctgat ctttctgaca 840

gcatccactc tgctaatgcc tctgagagag acgacatttc agaaattcag tcactggcat 900

cagatcattc tggtcgatca cacgataggc ctccccgccg cagccggtca cgatctcctg 960

accagcggtc agagccttct gatcattcca ggcactcgcc gcagcagcca agcaatggca 1020

gtctccggag tagagatgaa gagagaattt ctaaacctgg ggctgtctca actcctgtaa 1080

agcatgctga tgatcacaca cctaaaacag tggaagaagt tacagttgaa agaaatgaga 1140

aacaaacacc ttctcttcca gaaccaaagc ctgtgtatgc ccaagttggg caaccagatg 1200

tggatttacc tgtcagtcca tctgatggtg tcctacctaa ttcaactcat gaagatggga 1260

ttcttcggcc cagcatgaaa ttggtaaaat tcagaaaagg agatagtgtg ggtttgcggc 1320

tggctggtgg aaatgatgtt ggaatatttg tagctggcgt tctagaagat agccctgcag 1380

ccaaggaagg cttagaggaa ggtgatcaaa ttctcagggt aaacaacgta gattttacaa 1440

atatcataag agaagaagcc gtccttttcc tgcttgacct ccctaaagga gaagaagtga 1500

ccatattggc tcagaagaag aaggatgttt atcgtcgcat tgtagaatca gatgtaggag 1560

attctttcta tattagaacc cattttgaat atgaaaagga atctccctat ggacttagtt 1620

ttaacaaagg agaggtgttc cgtgttgtgg ataccttgta caatggaaaa ctgggctctt 1680

ggcttgctat tcgaattggt aaaaatcata aggaggtaga acgaggcatc atcccttaac 1740

tcgaggagac ctat 1754

<210> 71
<211> 43

<212>  DNA
<213>  Artificial Sequence

<220>
<223>  asense TJP1-F2

<400>  71
ataggtctcc tcgagttacg gccaaatctt ctcactcctt ctg                43


<210>  72
<211>  497
<212>  DNA
<213>  Artificial Sequence

<220>
<223>  TJP1-PCR-fragment 1-156

<400>  72
ataggtctct catgtccgcc agagctgcgg ccgccaagag cacagcaatg gaggaaacag      60

ctatatggga acaacataca gtgacgcttc acagggctcc tggatttgga tttggaattg     120

caatatctgg tggacgagat aatcctcatt ttcagagtgg ggaaacgtca atagtgattt     180

cagatgtgct gaaaggagga ccagctgaag gacagctaca ggaaaatgac cgagttgcaa     240

tggttaacgg agtttcaatg gataatgttg aacatgcttt tgctgttcag caactaagga     300

aaagtgggaa aaatgcaaaa attacaatta gaaggaagaa gaaagttcaa ataccagtaa     360

gtcgtcctga tcctgaacca gtatctgata atgaagaaga tagttatgat gaggaaatac     420

atgatccaag aagtggccgg agtggtgtgg ttaacagaag gagtgagaag atttggccgt     480

aactcgagga gacctat                                                    497


<210>  73
<211>  39
<212>  DNA
<213>  Artificial Sequence

<220>
<223>  sense TJP1-F3

<400>  73
ataggtctca catgagtggt gtggttaaca gaaggagtg                             39


<210>  74
<211>  42
<212>  DNA
<213>  Artificial Sequence

<220>
<223>  asense TJP1-F3

<400>  74
ataggtctcc tcgagttacc tatcgtgtga tcgaccagaa tg                         42


<210>  75

<211>  521
<212>  DNA
<213>  Artificial Sequence

<220>
<223>  TJP1-PCR-fragment 144-306

<400>  75
ataggtctca catgagtggt gtggttaaca gaaggagtga gaagatttgg ccgagggata      60

gaagtgcaag tagagagagg agcttgtccc cgcggtcaga caggcggtca gtggcttcca     120

gccagcctgc taaacctact aaagtcacac tggtgaaatc ccggaaaaat gaagaatatg     180

gtcttcgatt ggcaagccat atatttgtta aggaaatttc acaagatagt ttggcagcaa     240

gagatggcaa tattcaagaa ggtgatgttg tattgaagat aaatggtact gtgacagaaa     300

atatgtcatt gacagatgca aagacattga tagaaaggtc taaaggcaaa ttaaaaatgg     360

tagttcaaag agatgaacgg gctacgctat tgaatgtccc tgatctttct gacagcatcc     420

actctgctaa tgcctctgag agagacgaca tttcagaaat tcagtcactg gcatcagatc     480

attctggtcg atcacacgat aggtaactcg aggagaccta t                         521


<210>  76
<211>  39
<212>  DNA
<213>  Artificial Sequence

<220>
<223>  sense TJP1-F4

<400>  76
ataggtctct catgtcactg gcatcagatc attctggtc                             39


<210>  77
<211>  878
<212>  DNA
<213>  Artificial Sequence

<220>
<223>  TJP1-PCR-fragment 294-575

<400>  77
ataggtctct catgtcactg gcatcagatc attctggtcg atcacacgat aggcctcccc      60

gccgcagccg gtcacgatct cctgaccagc ggtcagagcc ttctgatcat tccaggcact     120

cgccgcagca gccaagcaat ggcagtctcc ggagtagaga tgaagagaga atttctaaac     180

ctggggctgt ctcaactcct gtaaagcatg ctgatgatca cacacctaaa acagtggaag     240

aagttacagt tgaaagaaat gagaaacaaa caccttctct tccagaacca aagcctgtgt     300

atgcccaagt tgggcaacca gatgtggatt tacctgtcag tccatctgat ggtgtcctac     360

ctaattcaac tcatgaagat gggattcttc ggcccagcat gaaattggta aaattcagaa     420

aaggagatag tgtgggtttg cggctggctg gtggaaatga tgttggaata tttgtagctg     480

gcgttctaga agatagccct gcagccaagg aaggcttaga ggaaggtgat caaattctca 540

gggtaaacaa cgtagatttt acaaatatca taagagaaga agccgtcctt ttcctgcttg 600

acctccctaa aggagaagaa gtgaccatat tggctcagaa gaagaaggat gtttatcgtc 660

gcattgtaga atcagatgta ggagattctt tctatattag aacccatttt gaatatgaaa 720

aggaatctcc ctatggactt agttttaaca aaggagaggt gttccgtgtt gtggatacct 780

tgtacaatgg aaaactgggc tcttggcttg ctattcgaat tggtaaaaat cataaggagg 840

tagaacgagg catcatccct taactcgagg agacctat 878

<210> 78
<211> 6976
<212> DNA
<213> Artificial Sequence

<220>
<223> pET24d-N-TJP1[human](aa1-575)

<400> 78
ggggaattgt gagcggataa caattcccct ctagaaataa ttttgtttaa ctttaagaag 60

gagatatacc atgagccatc atcatcatca tcatcatcat tccatgtccg ccagagctgc 120

ggctgccaag agcacagcaa tggaggaaac agctatatgg gaacaacata cagtgacgct 180

tcacagggct cctggatttg gatttggaat tgcaatatct ggtggacgag ataatcctca 240

ttttcagagt ggggaaacgt caatagtgat ttcagatgtg ctgaaaggag gaccagctga 300

aggacagcta caggaaaatg accgagttgc aatggttaac ggagtttcaa tggataatgt 360

tgaacatgct tttgctgttc agcaactaag gaaaagtggg aaaaatgcaa aaattacaat 420

tagaaggaag aagaaagttc aaataccagt aagtcgtcct gatcctgaac cagtatctga 480

taatgaagaa gatagttatg atgaggaaat acatgatcca agaagtggcc ggagtggtgt 540

ggttaacaga aggagtgaga agatttggcc gagggataga agtgcaagta gagagaggag 600

cttgtccccg cggtcagaca ggcggtcagt ggcttccagc cagcctgcta aacctactaa 660

agtcacactg gtgaaatccc ggaaaaatga agaatatggt cttcgattgg caagccatat 720

atttgttaag gaaatttcac aagatagttt ggcagcaaga gatggcaata ttcaagaagg 780

tgatgttgta ttgaagataa atggtactgt gacagaaaat atgtcattga cagatgcaaa 840

gacattgata gaaaggtcta aaggcaaatt aaaaatggta gttcaaagag atgaacgggc 900

tacgctattg aatgtccctg atctttctga cagcatccac tctgctaatg cctctgagag 960

agacgacatt tcagaaattc agtcactggc atcagatcat tctggtcgat cacacgatag 1020

gcctccccgc cgcagccggt cacgatctcc tgaccagcgg tcagagcctt ctgatcattc 1080

caggcactcg ccgcagcagc caagcaatgg cagtctccgg agtagagatg aagagagaat 1140

ttctaaacct ggggctgtct caactcctgt aaagcatgct gatgatcaca cacctaaaac 1200

agtggaagaa gttacagttg aaagaaatga gaaacaaaca ccttctcttc cagaaccaaa 1260

gcctgtgtat gcccaagttg ggcaaccaga tgtggattta cctgtcagtc catctgatgg 1320

tgtcctacct aattcaactc atgaagatgg gattcttcgg cccagcatga aattggtaaa 1380

attcagaaaa ggagatagtg tgggtttgcg gctggctggt ggaaatgatg ttggaatatt 1440

tgtagctggc gttctagaag atagccctgc agccaaggaa ggcttagagg aaggtgatca 1500

aattctcagg gtaaacaacg tagattttac aaatatcata agagaagaag ccgtcctttt 1560

cctgcttgac ctccctaaag gagaagaagt gaccatattg gctcagaaga agaaggatgt 1620

ttatcgtcgc attgtagaat cagatgtagg agattctttc tatattagaa cccattttga 1680

atatgaaaag gaatctccct atggacttag ttttaacaaa ggagaggtgt tccgtgttgt 1740

ggataccttg tacaatggaa aactgggctc ttggcttgct attcgaattg gtaaaaatca 1800

taaggaggta gaacgaggca tcatccctta actcgagtga gatccggctg ctaacaaagc 1860

ccgaaaggaa gctgagttgg ctgctgccac cgctgagcaa taactagcat aaccccttgg 1920

ggcctctaaa cgggtcttga ggggtttttt gctgaaagga ggaactatat ccggattggc 1980

gaatgggacg cgccctgtag cggcgcatta agcgcggcgg gtgtggtggt tacgcgcagc 2040

gtgaccgcta cacttgccag cgccctagcg cccgctcctt tcgctttctt cccttccttt 2100

ctcgccacgt tcgccggctt tccccgtcaa gctctaaatc gggggctccc tttagggttc 2160

cgatttagtg ctttacggca cctcgacccc aaaaaacttg attagggtga tggttcacgt 2220

agtgggccat cgccctgata gacggttttt cgccctttga cgttggagtc cacgttcttt 2280

aatagtggac tcttgttcca aactggaaca acactcaacc ctatctcggt ctattctttt 2340

gatttataag ggattttgcc gatttcggcc tattggttaa aaaatgagct gatttaacaa 2400

aaatttaacg cgaattttaa caaaatatta acgtttacaa tttcaggtgg cacttttcgg 2460

ggaaatgtgc gcggaacccc tatttgttta tttttctaaa tacattcaaa tatgtatccg 2520

ctcatgaatt aattcttaga aaaactcatc gagcatcaaa tgaaactgca atttattcat 2580

atcaggatta tcaataccat atttttgaaa aagccgtttc tgtaatgaag gagaaaactc 2640

accgaggcag ttccatagga tggcaagatc ctggtatcgg tctgcgattc cgactcgtcc 2700

aacatcaata caacctatta atttcccctc gtcaaaaata aggttatcaa gtgagaaatc 2760

accatgagtg acgactgaat ccggtgagaa tggcaaaagt ttatgcattt ctttccagac 2820

ttgttcaaca ggccagccat tacgctcgtc atcaaaatca ctcgcatcaa ccaaaccgtt 2880

attcattcgt gattgcgcct gagcgagacg aaatacgcga tcgctgttaa aaggacaatt 2940

acaaacagga atcgaatgca accggcgcag gaacactgcc agcgcatcaa caatattttc 3000

acctgaatca ggatattctt ctaatacctg gaatgctgtt ttcccgggga tcgcagtggt 3060

gagtaaccat gcatcatcag gagtacggat aaaatgcttg atggtcggaa gaggcataaa 3120

ttccgtcagc cagtttagtc tgaccatctc atctgtaaca tcattggcaa cgctaccttt 3180

gccatgtttc agaaacaact ctggcgcatc gggcttccca tacaatcgat agattgtcgc 3240

acctgattgc ccgacattat cgcgagccca tttataccca tataaatcag catccatgtt 3300

ggaatttaat cgcggcctag agcaagacgt ttcccgttga atatggctca taacacccct 3360

tgtattactg tttatgtaag cagacagttt tattgttcat gaccaaaatc ccttaacgtg 3420

agttttcgtt ccactgagcg tcagaccccg tagaaaagat caaaggatct tcttgagatc 3480

ctttttttct gcgcgtaatc tgctgcttgc aaacaaaaaa accaccgcta ccagcggtgg 3540

tttgtttgcc ggatcaagag ctaccaactc tttttccgaa ggtaactggc ttcagcagag 3600

cgcagatacc aaatactgtc cttctagtgt agccgtagtt aggccaccac ttcaagaact 3660

ctgtagcacc gcctacatac ctcgctctgc taatcctgtt accagtggct gctgccagtg 3720

gcgataagtc gtgtcttacc gggttggact caagacgata gttaccggat aaggcgcagc 3780

ggtcgggctg aacggggggt tcgtgcacac agcccagctt ggagcgaacg acctacaccg 3840

aactgagata cctacagcgt gagctatgag aaagcgccac gcttcccgaa gggagaaagg 3900

cggacaggta tccggtaagc ggcagggtcg gaacaggaga gcgcacgagg gagcttccag 3960

ggggaaacgc ctggtatctt tatagtcctg tcgggtttcg ccacctctga cttgagcgtc 4020

gatttttgtg atgctcgtca ggggggcgga gcctatggaa aaacgccagc aacgcggcct 4080

ttttacggtt cctggccttt tgctggcctt ttgctcacat gttctttcct gcgttatccc 4140

ctgattctgt ggataaccgt attaccgcct ttgagtgagc tgataccgct cgccgcagcc 4200

gaacgaccga gcgcagcgag tcagtgagcg aggaagcgga agagcgcctg atgcggtatt 4260

ttctccttac gcatctgtgc ggtatttcac accgcatata tggtgcactc tcagtacaat 4320

ctgctctgat gccgcatagt taagccagta tacactccgc tatcgctacg tgactgggtc 4380

atggctgcgc cccgacaccc gccaacaccc gctgacgcgc cctgacgggc ttgtctgctc 4440

ccggcatccg cttacagaca agctgtgacc gtctccggga gctgcatgtg tcagaggttt 4500

tcaccgtcat caccgaaacg cgcgaggcag ctgcggtaaa gctcatcagc gtggtcgtga 4560

agcgattcac agatgtctgc ctgttcatcc gcgtccagct cgttgagttt ctccagaagc 4620

gttaatgtct ggcttctgat aaagcgggcc atgttaaggg cggttttttc ctgtttggtc 4680

actgatgcct ccgtgtaagg gggatttctg ttcatggggg taatgatacc gatgaaacga 4740

gagaggatgc tcacgatacg ggttactgat gatgaacatg cccggttact ggaacgttgt 4800

gagggtaaac aactggcggt atggatgcgg cgggaccaga gaaaaatcac tcagggtcaa 4860

tgccagcgct tcgttaatac agatgtaggt gttccacagg gtagccagca gcatcctgcg 4920

atgcagatcc ggaacataat ggtgcagggc gctgacttcc gcgtttccag actttacgaa 4980

acacggaaac cgaagaccat tcatgttgtt gctcaggtcg cagacgtttt gcagcagcag 5040

tcgcttcacg ttcgctcgcg tatcggtgat tcattctgct aaccagtaag gcaaccccgc 5100

cagcctagcc gggtcctcaa cgacaggagc acgatcatgc gcacccgtgg ggccgccatg 5160

ccggcgataa tggcctgctt ctcgccgaaa cgtttggtgg cgggaccagt gacgaaggct 5220

tgagcgaggg cgtgcaagat tccgaatacc gcaagcgaca ggccgatcat cgtcgcgctc 5280

cagcgaaagc ggtcctcgcc gaaaatgacc cagagcgctg ccggcacctg tcctacgagt 5340

tgcatgataa agaagacagt cataagtgcg gcgacgatag tcatgccccg cgcccaccgg 5400

aaggagctga ctgggttgaa ggctctcaag ggcatcggtc gagatcccgg tgcctaatga 5460

gtgagctaac ttacattaat tgcgttgcgc tcactgcccg ctttccagtc gggaaacctg 5520

tcgtgccagc tgcattaatg aatcggccaa cgcgcgggga gaggcggttt gcgtattggg 5580

cgccagggtg gtttttcttt tcaccagtga gacgggcaac agctgattgc ccttcaccgc 5640

ctggccctga gagagttgca gcaagcggtc cacgctggtt tgccccagca ggcgaaaatc 5700

ctgtttgatg gtggttaacg gcgggatata acatgagctg tcttcggtat cgtcgtatcc 5760

cactaccgag atatccgcac caacgcgcag cccggactcg gtaatggcgc gcattgcgcc 5820

cagcgccatc tgatcgttgg caaccagcat cgcagtggga acgatgccct cattcagcat 5880

ttgcatggtt tgttgaaaac cggacatggc actccagtcg ccttcccgtt ccgctatcgg 5940

ctgaatttga ttgcgagtga gatatttatg ccagccagcc agacgcagac gcgccgagac 6000

agaacttaat gggcccgcta acagcgcgat ttgctggtga cccaatgcga ccagatgctc 6060

cacgcccagt cgcgtaccgt cttcatggga gaaaataata ctgttgatgg gtgtctggtc 6120

agagacatca agaaataacg ccggaacatt agtgcaggca gcttccacag caatggcatc 6180

ctggtcatcc agcggatagt taatgatcag cccactgacg cgttgcgcga gaagattgtg 6240

caccgccgct ttacaggctt cgacgccgct tcgttctacc atcgacacca ccacgctggc 6300

acccagttga tcggcgcgag atttaatcgc cgcgacaatt tgcgacggcg cgtgcagggc 6360

cagactggag gtggcaacgc caatcagcaa cgactgtttg cccgccagtt gttgtgccac 6420

gcggttggga atgtaattca gctccgccat cgccgcttcc actttttccc gcgttttcgc 6480

agaaacgtgg ctggcctggt tcaccacgcg ggaaacggtc tgataagaga caccggcata 6540

ctctgcgaca tcgtataacg ttactggttt cacattcacc accctgaatt gactctcttc 6600

cgggcgctat catgccatac cgcgaaaggt tttgcgccat tcgatggtgt ccgggatctc 6660

gacgctctcc cttatgcgac tcctgcatta ggaagcagcc cagtagtagg ttgaggccgt 6720

tgagcaccgc cgccgcaagg aatggtgcat gcaaggagat ggcgcccaac agtcccccgg 6780

ccacggggcc tgccaccata cccacgccga aacaagcgct catgagcccg aagtggcgag 6840

cccgatcttc cccatcggtg atgtcggcga tataggcgcc agcaaccgca cctgtggcgc 6900

cggtgatgcc ggccacgatg cgtccggcgt agaggatcga gatctcgatc ccgcgaaatt 6960

aatacgactc actata 6976

<210> 79
<211> 5719
<212> DNA
<213> Artificial Sequence

<220>
<223> pET24d-N-TJP1[human](aa1-156)

<400> 79
ggggaattgt gagcggataa caattcccct ctagaaataa ttttgtttaa ctttaagaag 60

gagatatacc atgagccatc atcatcatca tcatcatcat tccatgtccg ccagagctgc 120

ggccgccaag agcacagcaa tggaggaaac agctatatgg gaacaacata cagtgacgct 180

tcacagggct cctggatttg gatttggaat tgcaatatct ggtggacgag ataatcctca 240

ttttcagagt ggggaaacgt caatagtgat ttcagatgtg ctgaaaggag gaccagctga 300

aggacagcta caggaaaatg accgagttgc aatggttaac ggagtttcaa tggataatgt 360

tgaacatgct tttgctgttc agcaactaag gaaaagtggg aaaaatgcaa aaattacaat 420

tagaaggaag aagaaagttc aaataccagt aagtcgtcct gatcctgaac cagtatctga 480

taatgaagaa gatagttatg atgaggaaat acatgatcca agaagtggcc ggagtggtgt 540

ggttaacaga aggagtgaga agatttggcc gtaactcgag tgagatccgg ctgctaacaa 600

agcccgaaag gaagctgagt tggctgctgc caccgctgag caataactag cataacccct 660

tggggcctct aaacgggtct tgaggggttt tttgctgaaa ggaggaacta tatccggatt 720

ggcgaatggg acgcgccctg tagcggcgca ttaagcgcgg cgggtgtggt ggttacgcgc 780

agcgtgaccg ctacacttgc cagcgcccta gcgcccgctc ctttcgcttt cttcccttcc 840

tttctcgcca cgttcgccgg ctttccccgt caagctctaa atcgggggct ccctttaggg 900

ttccgattta gtgctttacg gcacctcgac cccaaaaaac ttgattaggg tgatggttca 960

cgtagtgggc catcgccctg atagacggtt tttcgccctt tgacgttgga gtccacgttc 1020

tttaatagtg gactcttgtt ccaaactgga acaacactca accctatctc ggtctattct 1080

tttgatttat aagggatttt gccgatttcg gcctattggt taaaaaatga gctgatttaa 1140

caaaaattta acgcgaattt taacaaaata ttaacgttta caatttcagg tggcactttt 1200

cggggaaatg tgcgcggaac ccctatttgt ttatttttct aaatacattc aaatatgtat 1260

ccgctcatga attaattctt agaaaaactc atcgagcatc aaatgaaact gcaatttatt 1320

catatcagga ttatcaatac catatttttg aaaaagccgt ttctgtaatg aaggagaaaa 1380

ctcaccgagg cagttccata ggatggcaag atcctggtat cggtctgcga ttccgactcg 1440

tccaacatca atacaaccta ttaatttccc ctcgtcaaaa ataaggttat caagtgagaa 1500

atcaccatga gtgacgactg aatccggtga gaatggcaaa agtttatgca tttctttcca 1560

gacttgttca acaggccagc cattacgctc gtcatcaaaa tcactcgcat caaccaaacc 1620

gttattcatt cgtgattgcg cctgagcgag acgaaatacg cgatcgctgt taaaaggaca 1680

attacaaaca ggaatcgaat gcaaccggcg caggaacact gccagcgcat caacaatatt 1740

ttcacctgaa tcaggatatt cttctaatac ctggaatgct gttttcccgg ggatcgcagt 1800

ggtgagtaac catgcatcat caggagtacg gataaaatgc ttgatggtcg gaagaggcat 1860

aaattccgtc agccagttta gtctgaccat ctcatctgta acatcattgg caacgctacc 1920

tttgccatgt ttcagaaaca actctggcgc atcgggcttc ccatacaatc gatagattgt 1980

cgcacctgat tgcccgacat tatcgcgagc ccatttatac ccatataaat cagcatccat 2040

gttggaattt aatcgcggcc tagagcaaga cgtttcccgt tgaatatggc tcataacacc 2100

ccttgtatta ctgtttatgt aagcagacag ttttattgtt catgaccaaa atcccttaac 2160

gtgagttttc gttccactga gcgtcagacc ccgtagaaaa gatcaaagga tcttcttgag 2220

atcctttttt tctgcgcgta atctgctgct tgcaaacaaa aaaaccaccg ctaccagcgg 2280

tggtttgttt gccggatcaa gagctaccaa ctctttttcc gaaggtaact ggcttcagca 2340

gagcgcagat accaaatact gtccttctag tgtagccgta gttaggccac cacttcaaga 2400

actctgtagc accgcctaca tacctcgctc tgctaatcct gttaccagtg gctgctgcca 2460

gtggcgataa gtcgtgtctt accgggttgg actcaagacg atagttaccg gataaggcgc 2520

agcggtcggg ctgaacgggg ggttcgtgca cacagcccag cttggagcga acgacctaca 2580

ccgaactgag atacctacag cgtgagctat gagaaagcgc cacgcttccc gaagggagaa 2640

aggcggacag gtatccggta agcggcaggg tcggaacagg agagcgcacg agggagcttc 2700

cagggggaaa cgcctggtat ctttatagtc ctgtcgggtt tcgccacctc tgacttgagc 2760

gtcgattttt gtgatgctcg tcaggggggc ggagcctatg gaaaaacgcc agcaacgcgg 2820

cctttttacg gttcctggcc ttttgctggc cttttgctca catgttcttt cctgcgttat 2880

cccctgattc tgtggataac cgtattaccg cctttgagtg agctgatacc gctcgccgca 2940

gccgaacgac cgagcgcagc gagtcagtga gcgaggaagc ggaagagcgc ctgatgcggt 3000

attttctcct tacgcatctg tgcggtattt cacaccgcat atatggtgca ctctcagtac 3060

aatctgctct gatgccgcat agttaagcca gtatacactc cgctatcgct acgtgactgg 3120

gtcatggctg cgccccgaca cccgccaaca cccgctgacg cgccctgacg ggcttgtctg 3180

ctcccggcat ccgcttacag acaagctgtg accgtctccg ggagctgcat gtgtcagagg 3240

ttttcaccgt catcaccgaa acgcgcgagg cagctgcggt aaagctcatc agcgtggtcg 3300

tgaagcgatt cacagatgtc tgcctgttca tccgcgtcca gctcgttgag tttctccaga 3360

agcgttaatg tctggcttct gataaagcgg gccatgttaa gggcggtttt ttcctgtttg 3420

gtcactgatg cctccgtgta agggggattt ctgttcatgg gggtaatgat accgatgaaa 3480

cgagagagga tgctcacgat acgggttact gatgatgaac atgcccggtt actggaacgt 3540

tgtgagggta aacaactggc ggtatggatg cggcgggacc agagaaaaat cactcagggt 3600

caatgccagc gcttcgttaa tacagatgta ggtgttccac agggtagcca gcagcatcct 3660

gcgatgcaga tccggaacat aatggtgcag ggcgctgact tccgcgtttc cagactttac 3720

gaaacacgga aaccgaagac cattcatgtt gttgctcagg tcgcagacgt tttgcagcag 3780

cagtcgcttc acgttcgctc gcgtatcggt gattcattct gctaaccagt aaggcaaccc 3840

cgccagccta gccgggtcct caacgacagg agcacgatca tgcgcacccg tggggccgcc 3900

atgccggcga taatggcctg cttctcgccg aaacgtttgg tggcgggacc agtgacgaag 3960

gcttgagcga gggcgtgcaa gattccgaat accgcaagcg acaggccgat catcgtcgcg 4020

ctccagcgaa agcggtcctc gccgaaaatg acccagagcg ctgccggcac ctgtcctacg 4080

agttgcatga taaagaagac agtcataagt gcggcgacga tagtcatgcc ccgcgcccac 4140

cggaaggagc tgactgggtt gaaggctctc aagggcatcg gtcgagatcc cggtgcctaa 4200

tgagtgagct aacttacatt aattgcgttg cgctcactgc ccgctttcca gtcgggaaac 4260

ctgtcgtgcc agctgcatta atgaatcggc caacgcgcgg ggagaggcgg tttgcgtatt 4320

gggcgccagg gtggtttttc ttttcaccag tgagacgggc aacagctgat tgcccttcac 4380

cgcctggccc tgagagagtt gcagcaagcg gtccacgctg gtttgcccca gcaggcgaaa 4440

atcctgtttg atggtggtta acggcgggat ataacatgag ctgtcttcgg tatcgtcgta 4500

tcccactacc gagatatccg caccaacgcg cagcccggac tcggtaatgg cgcgcattgc 4560

gcccagcgcc atctgatcgt tggcaaccag catcgcagtg ggaacgatgc cctcattcag 4620

catttgcatg gtttgttgaa aaccggacat ggcactccag tcgccttccc gttccgctat 4680

cggctgaatt tgattgcgag tgagatattt atgccagcca gccagacgca gacgcgccga 4740

gacagaactt aatgggcccg ctaacagcgc gatttgctgg tgacccaatg cgaccagatg 4800

ctccacgccc agtcgcgtac cgtcttcatg ggagaaaata atactgttga tgggtgtctg 4860

gtcagagaca tcaagaaata acgccggaac attagtgcag gcagcttcca cagcaatggc 4920

atcctggtca tccagcggat agttaatgat cagcccactg acgcgttgcg cgagaagatt 4980

gtgcaccgcc gctttacagg cttcgacgcc gcttcgttct accatcgaca ccaccacgct 5040

ggcacccagt tgatcggcgc gagatttaat cgccgcgaca atttgcgacg gcgcgtgcag 5100

ggccagactg gaggtggcaa cgccaatcag caacgactgt ttgcccgcca gttgttgtgc 5160

cacgcggttg ggaatgtaat tcagctccgc catcgccgct tccacttttt cccgcgtttt 5220

cgcagaaacg tggctggcct ggttcaccac gcgggaaacg gtctgataag agacaccggc 5280

atactctgcg acatcgtata acgttactgg tttcacattc accaccctga attgactctc 5340

ttccgggcgc tatcatgcca taccgcgaaa ggttttgcgc cattcgatgg tgtccgggat 5400

ctcgacgctc tcccttatgc gactcctgca ttaggaagca gcccagtagt aggttgaggc 5460

cgttgagcac cgccgccgca aggaatggtg catgcaagga gatggcgccc aacagtcccc 5520

cggccacggg gcctgccacc atacccacgc cgaaacaagc gctcatgagc ccgaagtggc 5580

gagcccgatc ttccccatcg gtgatgtcgg cgatataggc gccagcaacc gcacctgtgg 5640

cgccggtgat gccggccacg atgcgtccgg cgtagaggat cgagatctcg atcccgcgaa 5700

attaatacga ctcactata 5719

<210> 80
<211> 5743
<212> DNA
<213> Artificial Sequence

<220>
<223> pET24d-N-TJP1[human](aa144-306)

<400> 80
ggggaattgt gagcggataa caattcccct ctagaaataa ttttgtttaa ctttaagaag 60

gagatatacc atgagccatc atcatcatca tcatcatcat tccatgagtg gtgtggttaa 120

cagaaggagt gagaagattt ggccgaggga tagaagtgca agtagagaga ggagcttgtc 180

cccgcggtca gacaggcggt cagtggcttc cagccagcct gctaaaccta ctaaagtcac 240

actggtgaaa tcccggaaaa atgaagaata tggtcttcga ttggcaagcc atatatttgt 300

taaggaaatt tcacaagata gtttggcagc aagagatggc aatattcaag aaggtgatgt 360

tgtattgaag ataaatggta ctgtgacaga aaatatgtca ttgacagatg caaagacatt 420

gatagaaagg tctaaaggca aattaaaaat ggtagttcaa agagatgaac gggctacgct 480

attgaatgtc cctgatcttt ctgacagcat ccactctgct aatgcctctg agagagacga 540

catttcagaa attcagtcac tggcatcaga tcattctggt cgatcacacg ataggtaact 600

cgagtgagat ccggctgcta acaaagcccg aaaggaagct gagttggctg ctgccaccgc 660

tgagcaataa ctagcataac cccttggggc ctctaaacgg gtcttgaggg gttttttgct 720

gaaaggagga actatatccg gattggcgaa tgggacgcgc cctgtagcgg cgcattaagc 780

gcggcgggtg tggtggttac gcgcagcgtg accgctacac ttgccagcgc cctagcgccc 840

gctcctttcg ctttcttccc ttcctttctc gccacgttcg ccggctttcc ccgtcaagct 900

ctaaatcggg ggctcccttt agggttccga tttagtgctt tacggcacct cgaccccaaa 960

aaacttgatt agggtgatgg ttcacgtagt gggccatcgc cctgatagac ggtttttcgc 1020

cctttgacgt tggagtccac gttctttaat agtggactct tgttccaaac tggaacaaca 1080

ctcaacccta tctcggtcta ttcttttgat ttataaggga ttttgccgat ttcggcctat 1140

tggttaaaaa atgagctgat ttaacaaaaa tttaacgcga attttaacaa aatattaacg 1200

tttacaattt caggtggcac ttttcgggga aatgtgcgcg gaacccctat ttgtttattt 1260

ttctaaatac attcaaatat gtatccgctc atgaattaat tcttagaaaa actcatcgag 1320

catcaaatga aactgcaatt tattcatatc aggattatca ataccatatt tttgaaaaag 1380

ccgtttctgt aatgaaggag aaaactcacc gaggcagttc cataggatgg caagatcctg 1440

gtatcggtct gcgattccga ctcgtccaac atcaatacaa cctattaatt tcccctcgtc 1500

aaaaataagg ttatcaagtg agaaatcacc atgagtgacg actgaatccg gtgagaatgg 1560

caaaagttta tgcatttctt tccagacttg ttcaacaggc cagccattac gctcgtcatc 1620

aaaatcactc gcatcaacca aaccgttatt cattcgtgat tgcgcctgag cgagacgaaa 1680

tacgcgatcg ctgttaaaag gacaattaca aacaggaatc gaatgcaacc ggcgcaggaa 1740

cactgccagc gcatcaacaa tattttcacc tgaatcagga tattcttcta atacctggaa 1800

tgctgttttc ccggggatcg cagtggtgag taaccatgca tcatcaggag tacggataaa 1860

atgcttgatg gtcggaagag gcataaattc cgtcagccag tttagtctga ccatctcatc 1920

tgtaacatca ttggcaacgc tacctttgcc atgtttcaga aacaactctg gcgcatcggg 1980

cttcccatac aatcgataga ttgtcgcacc tgattgcccg acattatcgc gagcccattt 2040

atacccatat aaatcagcat ccatgttgga atttaatcgc ggcctagagc aagacgtttc 2100

ccgttgaata tggctcataa caccccttgt attactgttt atgtaagcag acagttttat 2160

tgttcatgac caaaatccct taacgtgagt tttcgttcca ctgagcgtca gaccccgtag 2220

aaaagatcaa aggatcttct tgagatcctt tttttctgcg cgtaatctgc tgcttgcaaa 2280

caaaaaaacc accgctacca gcggtggttt gtttgccgga tcaagagcta ccaactcttt 2340

ttccgaaggt aactggcttc agcagagcgc agataccaaa tactgtcctt ctagtgtagc 2400

cgtagttagg ccaccacttc aagaactctg tagcaccgcc tacatacctc gctctgctaa 2460

tcctgttacc agtggctgct gccagtggcg ataagtcgtg tcttaccggg ttggactcaa 2520

gacgatagtt accggataag gcgcagcggt cgggctgaac ggggggttcg tgcacacagc 2580

ccagcttgga gcgaacgacc tacaccgaac tgagatacct acagcgtgag ctatgagaaa 2640

gcgccacgct tcccgaaggg agaaaggcgg acaggtatcc ggtaagcggc agggtcggaa 2700

caggagagcg cacgagggag cttccagggg gaaacgcctg gtatctttat agtcctgtcg 2760

ggtttcgcca cctctgactt gagcgtcgat ttttgtgatg ctcgtcaggg gggcggagcc 2820

tatggaaaaa cgccagcaac gcggcctttt tacggttcct ggccttttgc tggccttttg 2880

ctcacatgtt ctttcctgcg ttatcccctg attctgtgga taaccgtatt accgcctttg 2940

agtgagctga taccgctcgc cgcagccgaa cgaccgagcg cagcgagtca gtgagcgagg 3000

aagcggaaga gcgcctgatg cggtattttc tccttacgca tctgtgcggt atttcacacc 3060

gcatatatgg tgcactctca gtacaatctg ctctgatgcc gcatagttaa gccagtatac 3120

actccgctat cgctacgtga ctgggtcatg gctgcgcccc gacacccgcc aacacccgct 3180

gacgcgccct gacgggcttg tctgctcccg gcatccgctt acagacaagc tgtgaccgtc 3240

tccgggagct gcatgtgtca gaggttttca ccgtcatcac cgaaacgcgc gaggcagctg 3300

cggtaaagct catcagcgtg gtcgtgaagc gattcacaga tgtctgcctg ttcatccgcg 3360

tccagctcgt tgagtttctc cagaagcgtt aatgtctggc ttctgataaa gcgggccatg 3420

ttaagggcgg ttttttcctg tttggtcact gatgcctccg tgtaaggggg atttctgttc 3480

atgggggtaa tgataccgat gaaacgagag aggatgctca cgatacgggt tactgatgat 3540

gaacatgccc ggttactgga acgttgtgag ggtaaacaac tggcggtatg gatgcggcgg 3600

gaccagagaa aaatcactca gggtcaatgc cagcgcttcg ttaatacaga tgtaggtgtt 3660

ccacagggta gccagcagca tcctgcgatg cagatccgga acataatggt gcagggcgct 3720

gacttccgcg tttccagact ttacgaaaca cggaaaccga agaccattca tgttgttgct 3780

caggtcgcag acgttttgca gcagcagtcg cttcacgttc gctcgcgtat cggtgattca 3840

ttctgctaac cagtaaggca accccgccag cctagccggg tcctcaacga caggagcacg 3900

atcatgcgca cccgtggggc cgccatgccg gcgataatgg cctgcttctc gccgaaacgt 3960

ttggtggcgg gaccagtgac gaaggcttga gcgagggcgt gcaagattcc gaataccgca 4020

agcgacaggc cgatcatcgt cgcgctccag cgaaagcggt cctcgccgaa aatgacccag 4080

agcgctgccg gcacctgtcc tacgagttgc atgataaaga agacagtcat aagtgcggcg 4140

acgatagtca tgccccgcgc ccaccggaag gagctgactg ggttgaaggc tctcaagggc 4200

atcggtcgag atcccggtgc ctaatgagtg agctaactta cattaattgc gttgcgctca 4260

ctgcccgctt tccagtcggg aaacctgtcg tgccagctgc attaatgaat cggccaacgc 4320

gcggggagag gcggtttgcg tattgggcgc cagggtggtt tttcttttca ccagtgagac 4380

gggcaacagc tgattgccct tcaccgcctg gccctgagag agttgcagca agcggtccac 4440

gctggtttgc cccagcaggc gaaaatcctg tttgatggtg gttaacggcg ggatataaca 4500

tgagctgtct tcggtatcgt cgtatcccac taccgagata tccgcaccaa cgcgcagccc 4560

ggactcggta atggcgcgca ttgcgcccag cgccatctga tcgttggcaa ccagcatcgc 4620

agtgggaacg atgccctcat tcagcatttg catggtttgt tgaaaaccgg acatggcact 4680

ccagtcgcct tcccgttccg ctatcggctg aatttgattg cgagtgagat atttatgcca 4740

gccagccaga cgcagacgcg ccgagacaga acttaatggg cccgctaaca gcgcgatttg 4800

ctggtgaccc aatgcgacca gatgctccac gcccagtcgc gtaccgtctt catgggagaa 4860

aataatactg ttgatgggtg tctggtcaga gacatcaaga aataacgccg gaacattagt 4920

gcaggcagct tccacagcaa tggcatcctg gtcatccagc ggatagttaa tgatcagccc 4980

actgacgcgt tgcgcgagaa gattgtgcac cgccgcttta caggcttcga cgccgcttcg 5040

ttctaccatc gacaccacca cgctggcacc cagttgatcg gcgcgagatt taatcgccgc 5100

gacaatttgc gacggcgcgt gcagggccag actggaggtg gcaacgccaa tcagcaacga 5160

ctgtttgccc gccagttgtt gtgccacgcg gttgggaatg taattcagct ccgccatcgc 5220

cgcttccact ttttcccgcg ttttcgcaga aacgtggctg gcctggttca ccacgcggga 5280

aacggtctga taagagacac cggcatactc tgcgacatcg tataacgtta ctggtttcac 5340

attcaccacc ctgaattgac tctcttccgg gcgctatcat gccataccgc gaaaggtttt 5400

gcgccattcg atggtgtccg ggatctcgac gctctccctt atgcgactcc tgcattagga 5460

agcagcccag tagtaggttg aggccgttga gcaccgccgc cgcaaggaat ggtgcatgca 5520

aggagatggc gcccaacagt cccccggcca cggggcctgc caccataccc acgccgaaac 5580

aagcgctcat gagcccgaag tggcgagccc gatcttcccc atcggtgatg tcggcgatat 5640

aggcgccagc aaccgcacct gtggcgccgg tgatgccggc cacgatgcgt ccggcgtaga 5700

ggatcgagat ctcgatcccg cgaaattaat acgactcact ata 5743

<210> 81
<211> 6100
<212> DNA
<213> Artificial Sequence

<220>
<223> pET24d-N-TJP1[human](aa294-575)

<400> 81
ggggaattgt gagcggataa caattcccct ctagaaataa ttttgtttaa ctttaagaag 60

gagatatacc atgagccatc atcatcatca tcatcatcat tccatgtcac tggcatcaga 120

tcattctggt cgatcacacg ataggcctcc ccgccgcagc cggtcacgat ctcctgacca 180

gcggtcagag ccttctgatc attccaggca ctcgccgcag cagccaagca atggcagtct 240

ccggagtaga gatgaagaga gaatttctaa acctggggct gtctcaactc ctgtaaagca 300

tgctgatgat cacacaccta aaacagtgga agaagttaca gttgaaagaa atgagaaaca 360

aacaccttct cttccagaac caaagcctgt gtatgcccaa gttgggcaac cagatgtgga 420

tttacctgtc agtccatctg atggtgtcct acctaattca actcatgaag atgggattct 480

tcggcccagc atgaaattgg taaaattcag aaaaggagat agtgtgggtt tgcggctggc 540

tggtggaaat gatgttggaa tatttgtagc tggcgttcta gaagatagcc ctgcagccaa 600

ggaaggctta gaggaaggtg atcaaattct cagggtaaac aacgtagatt ttacaaatat 660

cataagagaa gaagccgtcc ttttcctgct tgacctccct aaaggagaag aagtgaccat 720

attggctcag aagaagaagg atgtttatcg tcgcattgta gaatcagatg taggagattc 780

tttctatatt agaacccatt ttgaatatga aaaggaatct ccctatggac ttagttttaa 840

caaaggagag gtgttccgtg ttgtggatac cttgtacaat ggaaaactgg gctcttggct 900

tgctattcga attggtaaaa atcataagga ggtagaacga ggcatcatcc cttaactcga 960

gtgagatccg gctgctaaca aagcccgaaa ggaagctgag ttggctgctg ccaccgctga 1020

gcaataacta gcataacccc ttggggcctc taaacgggtc ttgaggggtt ttttgctgaa 1080

aggaggaact atatccggat tggcgaatgg gacgcgccct gtagcggcgc attaagcgcg 1140

gcgggtgtgg tggttacgcg cagcgtgacc gctacacttg ccagcgccct agcgcccgct 1200

cctttcgctt tcttcccttc ctttctcgcc acgttcgccg gctttccccg tcaagctcta 1260

aatcgggggc tccctttagg gttccgattt agtgctttac ggcacctcga ccccaaaaaa 1320

cttgattagg gtgatggttc acgtagtggg ccatcgccct gatagacggt ttttcgccct 1380

ttgacgttgg agtccacgtt ctttaatagt ggactcttgt tccaaactgg aacaacactc 1440

aaccctatct cggtctattc ttttgattta taagggattt tgccgatttc ggcctattgg 1500

ttaaaaaatg agctgattta acaaaaattt aacgcgaatt ttaacaaaat attaacgttt 1560

acaatttcag gtggcacttt tcggggaaat gtgcgcggaa cccctatttg tttatttttc 1620

taaatacatt caaatatgta tccgctcatg aattaattct tagaaaaact catcgagcat 1680

caaatgaaac tgcaatttat tcatatcagg attatcaata ccatattttt gaaaaagccg 1740

tttctgtaat gaaggagaaa actcaccgag gcagttccat aggatggcaa gatcctggta 1800

tcggtctgcg attccgactc gtccaacatc aatacaacct attaatttcc cctcgtcaaa 1860

aataaggtta tcaagtgaga aatcaccatg agtgacgact gaatccggtg agaatggcaa 1920

aagtttatgc atttctttcc agacttgttc aacaggccag ccattacgct cgtcatcaaa 1980

atcactcgca tcaaccaaac cgttattcat tcgtgattgc gcctgagcga gacgaaatac 2040

gcgatcgctg ttaaaaggac aattacaaac aggaatcgaa tgcaaccggc gcaggaacac 2100

tgccagcgca tcaacaatat tttcacctga atcaggatat tcttctaata cctggaatgc 2160

tgttttcccg gggatcgcag tggtgagtaa ccatgcatca tcaggagtac ggataaaatg 2220

cttgatggtc ggaagaggca taaattccgt cagccagttt agtctgacca tctcatctgt 2280

aacatcattg gcaacgctac ctttgccatg tttcagaaac aactctggcg catcgggctt 2340

cccatacaat cgatagattg tcgcacctga ttgcccgaca ttatcgcgag cccatttata 2400

cccatataaa tcagcatcca tgttggaatt taatcgcggc ctagagcaag acgtttcccg 2460

ttgaatatgg ctcataacac cccttgtatt actgtttatg taagcagaca gttttattgt 2520

tcatgaccaa aatcccttaa cgtgagtttt cgttccactg agcgtcagac cccgtagaaa 2580

agatcaaagg atcttcttga gatccttttt ttctgcgcgt aatctgctgc ttgcaaacaa 2640

aaaaaccacc gctaccagcg gtggtttgtt tgccggatca agagctacca actctttttc 2700

cgaaggtaac tggcttcagc agagcgcaga taccaaatac tgtccttcta gtgtagccgt 2760

agttaggcca ccacttcaag aactctgtag caccgcctac atacctcgct ctgctaatcc 2820

tgttaccagt ggctgctgcc agtggcgata agtcgtgtct taccgggttg gactcaagac 2880

gatagttacc ggataaggcg cagcggtcgg gctgaacggg gggttcgtgc acacagccca 2940

gcttggagcg aacgacctac accgaactga gatacctaca gcgtgagcta tgagaaagcg 3000

ccacgcttcc cgaagggaga aaggcggaca ggtatccggt aagcggcagg gtcggaacag 3060

gagagcgcac gagggagctt ccagggggaa acgcctggta tctttatagt cctgtcgggt 3120

ttcgccacct ctgacttgag cgtcgatttt tgtgatgctc gtcagggggg cggagcctat 3180

ggaaaaacgc cagcaacgcg gcctttttac ggttcctggc cttttgctgg ccttttgctc 3240

acatgttctt tcctgcgtta tcccctgatt ctgtggataa ccgtattacc gcctttgagt 3300

gagctgatac cgctcgccgc agccgaacga ccgagcgcag cgagtcagtg agcgaggaag 3360

cggaagagcg cctgatgcgg tattttctcc ttacgcatct gtgcggtatt tcacaccgca 3420

tatatggtgc actctcagta caatctgctc tgatgccgca tagttaagcc agtatacact 3480

ccgctatcgc tacgtgactg ggtcatggct gcgccccgac acccgccaac acccgctgac 3540

gcgccctgac gggcttgtct gctcccggca tccgcttaca gacaagctgt gaccgtctcc 3600

gggagctgca tgtgtcagag gttttcaccg tcatcaccga aacgcgcgag gcagctgcgg 3660

taaagctcat cagcgtggtc gtgaagcgat tcacagatgt ctgcctgttc atccgcgtcc 3720

agctcgttga gtttctccag aagcgttaat gtctggcttc tgataaagcg ggccatgtta 3780

agggcggttt tttcctgttt ggtcactgat gcctccgtgt aagggggatt tctgttcatg 3840

ggggtaatga taccgatgaa acgagagagg atgctcacga tacgggttac tgatgatgaa 3900

catgcccggt tactggaacg ttgtgagggt aaacaactgg cggtatggat gcggcgggac 3960

cagagaaaaa tcactcaggg tcaatgccag cgcttcgtta atacagatgt aggtgttcca 4020

cagggtagcc agcagcatcc tgcgatgcag atccggaaca taatggtgca gggcgctgac 4080

ttccgcgttt ccagacttta cgaaacacgg aaaccgaaga ccattcatgt tgttgctcag 4140

gtcgcagacg ttttgcagca gcagtcgctt cacgttcgct cgcgtatcgg tgattcattc 4200

tgctaaccag taaggcaacc ccgccagcct agccgggtcc tcaacgacag gagcacgatc 4260

atgcgcaccc gtggggccgc catgccggcg ataatggcct gcttctcgcc gaaacgtttg 4320

gtggcgggac cagtgacgaa ggcttgagcg agggcgtgca agattccgaa taccgcaagc 4380

gacaggccga tcatcgtcgc gctccagcga aagcggtcct cgccgaaaat gacccagagc 4440

gctgccggca cctgtcctac gagttgcatg ataaagaaga cagtcataag tgcggcgacg 4500

atagtcatgc cccgcgccca ccggaaggag ctgactgggt tgaaggctct caagggcatc 4560

ggtcgagatc ccggtgccta atgagtgagc taacttacat taattgcgtt gcgctcactg 4620

cccgctttcc agtcgggaaa cctgtcgtgc cagctgcatt aatgaatcgg ccaacgcgcg 4680

gggagaggcg gtttgcgtat tgggcgccag ggtggttttt cttttcacca gtgagacggg 4740

caacagctga ttgcccttca ccgcctggcc ctgagagagt tgcagcaagc ggtccacgct 4800

ggtttgcccc agcaggcgaa aatcctgttt gatggtggtt aacggcggga tataacatga 4860

gctgtcttcg gtatcgtcgt atcccactac cgagatatcc gcaccaacgc gcagcccgga 4920

ctcggtaatg gcgcgcattg cgcccagcgc catctgatcg ttggcaacca gcatcgcagt 4980

gggaacgatg ccctcattca gcatttgcat ggtttgttga aaaccggaca tggcactcca 5040

gtcgccttcc cgttccgcta tcggctgaat ttgattgcga gtgagatatt tatgccagcc 5100

agccagacgc agacgcgccg agacagaact taatgggccc gctaacagcg cgatttgctg 5160

gtgacccaat gcgaccagat gctccacgcc cagtcgcgta ccgtcttcat gggagaaaat 5220

aatactgttg atgggtgtct ggtcagagac atcaagaaat aacgccggaa cattagtgca 5280

ggcagcttcc acagcaatgg catcctggtc atccagcgga tagttaatga tcagcccact 5340

gacgcgttgc gcgagaagat tgtgcaccgc cgctttacag gcttcgacgc cgcttcgttc 5400

taccatcgac accaccacgc tggcacccag ttgatcggcg cgagatttaa tcgccgcgac 5460

aatttgcgac ggcgcgtgca gggccagact ggaggtggca acgccaatca gcaacgactg 5520

tttgcccgcc agttgttgtg ccacgcggtt gggaatgtaa ttcagctccg ccatcgccgc 5580

ttccactttt tcccgcgttt tcgcagaaac gtggctggcc tggttcacca cgcgggaaac 5640

ggtctgataa gagacaccgg catactctgc gacatcgtat aacgttactg gtttcacatt 5700

caccaccctg aattgactct cttccgggcg ctatcatgcc ataccgcgaa aggttttgcg 5760

ccattcgatg gtgtccggga tctcgacgct ctcccttatg cgactcctgc attaggaagc 5820

agcccagtag taggttgagg ccgttgagca ccgccgccgc aaggaatggt gcatgcaagg 5880

agatggcgcc caacagtccc ccggccacgg ggcctgccac catacccacg ccgaaacaag 5940

cgctcatgag cccgaagtgg cgagcccgat cttccccatc ggtgatgtcg gcgatatagg 6000

cgccagcaac cgcacctgtg gcgccggtga tgccggccac gatgcgtccg gcgtagagga 6060

tcgagatctc gatcccgcga aattaatacg actcactata 6100

<210> 82
<211> 586
<212> PRT
<213> Artificial Sequence

<220>
<223>  H8-TJP1[human](aa1-575)

<400>  82

Met Ser His His His His His His His His Ser Met Ser Ala Arg Ala
1               5                   10                  15

Ala Ala Ala Lys Ser Thr Ala Met Glu Glu Thr Ala Ile Trp Glu Gln
            20                  25                  30

His Thr Val Thr Leu His Arg Ala Pro Gly Phe Gly Phe Gly Ile Ala
        35                  40                  45

Ile Ser Gly Gly Arg Asp Asn Pro His Phe Gln Ser Gly Glu Thr Ser
    50                  55                  60

Ile Val Ile Ser Asp Val Leu Lys Gly Gly Pro Ala Glu Gly Gln Leu
65                  70                  75                  80

Gln Glu Asn Asp Arg Val Ala Met Val Asn Gly Val Ser Met Asp Asn
                85                  90                  95

Val Glu His Ala Phe Ala Val Gln Gln Leu Arg Lys Ser Gly Lys Asn
            100                 105                 110

Ala Lys Ile Thr Ile Arg Arg Lys Lys Lys Val Gln Ile Pro Val Ser
        115                 120                 125

Arg Pro Asp Pro Glu Pro Val Ser Asp Asn Glu Glu Asp Ser Tyr Asp
    130                 135                 140

Glu Glu Ile His Asp Pro Arg Ser Gly Arg Ser Gly Val Val Asn Arg
145                 150                 155                 160

Arg Ser Glu Lys Ile Trp Pro Arg Asp Arg Ser Ala Ser Arg Glu Arg
                165                 170                 175

Ser Leu Ser Pro Arg Ser Asp Arg Arg Ser Val Ala Ser Ser Gln Pro
            180                 185                 190

Ala Lys Pro Thr Lys Val Thr Leu Val Lys Ser Arg Lys Asn Glu Glu
        195                 200                 205

Tyr Gly Leu Arg Leu Ala Ser His Ile Phe Val Lys Glu Ile Ser Gln
    210                 215                 220

Asp Ser Leu Ala Ala Arg Asp Gly Asn Ile Gln Glu Gly Asp Val Val

225                 230                 235                 240

Leu Lys Ile Asn Gly Thr Val Thr Glu Asn Met Ser Leu Thr Asp Ala
                245                 250                 255

Lys Thr Leu Ile Glu Arg Ser Lys Gly Lys Leu Lys Met Val Val Gln
            260                 265                 270

Arg Asp Glu Arg Ala Thr Leu Leu Asn Val Pro Asp Leu Ser Asp Ser
        275                 280                 285

Ile His Ser Ala Asn Ala Ser Glu Arg Asp Asp Ile Ser Glu Ile Gln
    290                 295                 300

Ser Leu Ala Ser Asp His Ser Gly Arg Ser His Asp Arg Pro Pro Arg
305                 310                 315                 320

Arg Ser Arg Ser Arg Ser Pro Asp Gln Arg Ser Glu Pro Ser Asp His
                325                 330                 335

Ser Arg His Ser Pro Gln Gln Pro Ser Asn Gly Ser Leu Arg Ser Arg
            340                 345                 350

Asp Glu Glu Arg Ile Ser Lys Pro Gly Ala Val Ser Thr Pro Val Lys
        355                 360                 365

His Ala Asp Asp His Thr Pro Lys Thr Val Glu Glu Val Thr Val Glu
    370                 375                 380

Arg Asn Glu Lys Gln Thr Pro Ser Leu Pro Glu Pro Lys Pro Val Tyr
385                 390                 395                 400

Ala Gln Val Gly Gln Pro Asp Val Asp Leu Pro Val Ser Pro Ser Asp
                405                 410                 415

Gly Val Leu Pro Asn Ser Thr His Glu Asp Gly Ile Leu Arg Pro Ser
            420                 425                 430

Met Lys Leu Val Lys Phe Arg Lys Gly Asp Ser Val Gly Leu Arg Leu
        435                 440                 445

Ala Gly Gly Asn Asp Val Gly Ile Phe Val Ala Gly Val Leu Glu Asp
    450                 455                 460

Ser Pro Ala Ala Lys Glu Gly Leu Glu Glu Gly Asp Gln Ile Leu Arg
465                 470                 475                 480

Val Asn Asn Val Asp Phe Thr Asn Ile Ile Arg Glu Glu Ala Val Leu
                485                 490                 495

Phe Leu Leu Asp Leu Pro Lys Gly Glu Glu Val Thr Ile Leu Ala Gln
            500                 505                 510

Lys Lys Lys Asp Val Tyr Arg Arg Ile Val Glu Ser Asp Val Gly Asp
        515                 520                 525

Ser Phe Tyr Ile Arg Thr His Phe Glu Tyr Glu Lys Glu Ser Pro Tyr
    530                 535                 540

Gly Leu Ser Phe Asn Lys Gly Glu Val Phe Arg Val Val Asp Thr Leu
545                 550                 555                 560

Tyr Asn Gly Lys Leu Gly Ser Trp Leu Ala Ile Arg Ile Gly Lys Asn
                565                 570                 575

His Lys Glu Val Glu Arg Gly Ile Ile Pro
            580                 585

<210>  83
<211>  167
<212>  PRT
<213>  Artificial Sequence

<220>
<223>  H8-TJP1[human](aa1-156)

<400>  83

Met Ser His His His His His His His His Ser Met Ser Ala Arg Ala
1               5                   10                  15

Ala Ala Ala Lys Ser Thr Ala Met Glu Glu Thr Ala Ile Trp Glu Gln
            20                  25                  30

His Thr Val Thr Leu His Arg Ala Pro Gly Phe Gly Phe Gly Ile Ala
        35                  40                  45

Ile Ser Gly Gly Arg Asp Asn Pro His Phe Gln Ser Gly Glu Thr Ser
    50                  55                  60

Ile Val Ile Ser Asp Val Leu Lys Gly Gly Pro Ala Glu Gly Gln Leu
65                  70                  75                  80

Gln Glu Asn Asp Arg Val Ala Met Val Asn Gly Val Ser Met Asp Asn
                85                  90                  95

Val Glu His Ala Phe Ala Val Gln Gln Leu Arg Lys Ser Gly Lys Asn

            100                 105                 110

Ala Lys Ile Thr Ile Arg Arg Lys Lys Lys Val Gln Ile Pro Val Ser
        115                 120                 125

Arg Pro Asp Pro Glu Pro Val Ser Asp Asn Glu Glu Asp Ser Tyr Asp
    130                 135                 140

Glu Glu Ile His Asp Pro Arg Ser Gly Arg Ser Gly Val Val Asn Arg
145                 150                 155                 160

Arg Ser Glu Lys Ile Trp Pro
                165

<210> 84
<211> 175
<212> PRT
<213> Artificial Sequence

<220>
<223> H8-TJP1[human](aa144-306)

<400> 84

Met Ser His His His His His His His His Ser Met Ser Gly Val Val
1               5                   10                  15

Asn Arg Arg Ser Glu Lys Ile Trp Pro Arg Asp Arg Ser Ala Ser Arg
            20                  25                  30

Glu Arg Ser Leu Ser Pro Arg Ser Asp Arg Arg Ser Val Ala Ser Ser
        35                  40                  45

Gln Pro Ala Lys Pro Thr Lys Val Thr Leu Val Lys Ser Arg Lys Asn
    50                  55                  60

Glu Glu Tyr Gly Leu Arg Leu Ala Ser His Ile Phe Val Lys Glu Ile
65                  70                  75                  80

Ser Gln Asp Ser Leu Ala Ala Arg Asp Gly Asn Ile Gln Glu Gly Asp
                85                  90                  95

Val Val Leu Lys Ile Asn Gly Thr Val Thr Glu Asn Met Ser Leu Thr
            100                 105                 110

Asp Ala Lys Thr Leu Ile Glu Arg Ser Lys Gly Lys Leu Lys Met Val
        115                 120                 125

Val Gln Arg Asp Glu Arg Ala Thr Leu Leu Asn Val Pro Asp Leu Ser
    130                 135                 140

Asp Ser Ile His Ser Ala Asn Ala Ser Glu Arg Asp Asp Ile Ser Glu
145                 150                 155                 160

Ile Gln Ser Leu Ala Ser Asp His Ser Gly Arg Ser His Asp Arg
                165                 170                 175

<210>  85
<211>  294
<212>  PRT
<213>  Artificial Sequence

<220>
<223>  H8-TJP1[human](aa294-575)

<400>  85

Met Ser His His His His His His His His Ser Met Ser Leu Ala Ser
1               5                   10                  15

Asp His Ser Gly Arg Ser His Asp Arg Pro Pro Arg Arg Ser Arg Ser
            20                  25                  30

Arg Ser Pro Asp Gln Arg Ser Glu Pro Ser Asp His Ser Arg His Ser
        35                  40                  45

Pro Gln Gln Pro Ser Asn Gly Ser Leu Arg Ser Arg Asp Glu Glu Arg
    50                  55                  60

Ile Ser Lys Pro Gly Ala Val Ser Thr Pro Val Lys His Ala Asp Asp
65                  70                  75                  80

His Thr Pro Lys Thr Val Glu Glu Val Thr Val Glu Arg Asn Glu Lys
                85                  90                  95

Gln Thr Pro Ser Leu Pro Glu Pro Lys Pro Val Tyr Ala Gln Val Gly
            100                 105                 110

Gln Pro Asp Val Asp Leu Pro Val Ser Pro Ser Asp Gly Val Leu Pro
        115                 120                 125

Asn Ser Thr His Glu Asp Gly Ile Leu Arg Pro Ser Met Lys Leu Val
    130                 135                 140

Lys Phe Arg Lys Gly Asp Ser Val Gly Leu Arg Leu Ala Gly Gly Asn
145                 150                 155                 160

Asp Val Gly Ile Phe Val Ala Gly Val Leu Glu Asp Ser Pro Ala Ala
                165                 170                 175

Lys Glu Gly Leu Glu Glu Gly Asp Gln Ile Leu Arg Val Asn Asn Val
            180                 185                 190

Asp Phe Thr Asn Ile Ile Arg Glu Glu Ala Val Leu Phe Leu Leu Asp
        195                 200                 205

Leu Pro Lys Gly Glu Glu Val Thr Ile Leu Ala Gln Lys Lys Lys Asp
    210                 215                 220

Val Tyr Arg Arg Ile Val Glu Ser Asp Val Gly Asp Ser Phe Tyr Ile
225                 230                 235                 240

Arg Thr His Phe Glu Tyr Glu Lys Glu Ser Pro Tyr Gly Leu Ser Phe
                245                 250                 255

Asn Lys Gly Glu Val Phe Arg Val Val Asp Thr Leu Tyr Asn Gly Lys
            260                 265                 270

Leu Gly Ser Trp Leu Ala Ile Arg Ile Gly Lys Asn His Lys Glu Val
        275                 280                 285

Glu Arg Gly Ile Ile Pro
    290

<210> 86
<211> 13
<212> PRT
<213> Artificial Sequence

<220>
<223> NVL-fragment(aa591-603)

<400> 86

Glu Glu Leu Thr Met Ala Ile Leu Ala Pro Val Arg Asn
1               5                   10

<210> 87
<211> 11
<212> PRT
<213> Artificial Sequence

<220>
<223> NVL-fragment(aa607-617)

<400> 87

Phe Lys Ala Leu Gly Leu Val Thr Pro Ala Gly
1               5                   10

<210> 88
<211> 11

<212>  PRT
<213>  Artificial Sequence

<220>
<223>  NVL-fragment(aa685-695)

<400>  88

Ala Leu Cys Pro Arg Arg Ser Asp Arg Glu Thr
1               5                   10


<210>  89
<211>  12
<212>  PRT
<213>  Artificial Sequence

<220>
<223>  NVL-fragment(aa742-753)

<400>  89

Lys Thr Leu Phe Val Gly Leu Pro Pro Pro Ala Asp
1               5                   10


<210>  90
<211>  12
<212>  PRT
<213>  Artificial Sequence

<220>
<223>  NVL-fragment(aa761-772)

<400>  90

Ile Thr Lys Asn Gly Thr Lys Pro Pro Leu Asp Ala
1               5                   10


<210>  91
<211>  12
<212>  PRT
<213>  Artificial Sequence

<220>
<223>  NVL-fragment(aa778-789)

<400>  91

Ala Ile Ala Gly Asp Leu Arg Cys Asp Cys Tyr Thr
1               5                   10


<210>  92
<211>  18
<212>  PRT
<213>  Artificial Sequence

<220>
<223>  NVL-fragment(aa809-826)

<400>  92

Met Ala Arg Gln Lys Ser Gly Asn Glu Lys Gly Glu Leu Lys Val Ser
1               5                   10                  15

His Lys

<210>  93
<211>  13
<212>  PRT
<213>  Artificial Sequence

<220>
<223>  NVL-fragment(aa844-856)

<400>  93

Gln Ile Met Tyr Glu Arg Leu Gln Glu Ser Leu Ser Arg
1               5                   10

## Description

The present invention relates to a method for detecting in a sample an autoantibody binding to a polypeptide having the sequence SEQ ID NO1, SEQ ID NO11 or SEQ ID NO24, a carrier comprising said polypeptide, an autoantibody binding said polypeptide, a use of the autoantibody for the diagnosis of a disease, a method for isolating an autoantibody binding to said polypeptide, a kit comprising said polypeptide, a use of said polypeptide, said carrier or said autoantibody for the manufacture of a kit or medical device, a method comprising the step contacting a medical or diagnostic device comprising said polypeptide with a buffered solution comprising an antibody binding specifically to said polypeptide, a use of a sample comprising said autoantibody as a positive control and a diluted sample comprising said autoantibody.

Autoimmune diseases are understood to mean diseases in which the immune system is directed against intact structures endogenous to the body. In many autoimmune diseases, the body produces detectable autoantibodies which can bind against proteins endogenous to the body and thus cause disorders. In other cases, the presence of autoantibodies does not appear to be causally responsible for the outbreak of a disease.

ANAs (antinuclear autoantibodies) are a group of autoantibodies that targets the nuclear components of mammalian cells. They are mainly associated with systemic autoimmune rheumatic diseases (SARD) and some of the ANAs are biomarkers for certain SARDs. For example, anti-double-strand DNA (dsDNA), anti-Sm and anti-ribosomal P are tightly associated with systemic lupus erythematosus (SLE), while anti-Scl70, anti-centromere and anti-RNA polymerase III are biomarkers for systemic sclerosis (SSc). Meanwhile, increasing evidence shows that elevated level of ANAs in cancer patients is significant in confronting tumor cells. For example, multiple studies revealed that anti-dsDNA autoantibodies can induce the apoptosis of cancer cells and inhibit the growth of tumors. Some well-established ANAs for SARD are found to be associated with different cancers, for example anti-dsDNA anti-Ro/SS-A and anti-Scl70 are presented with gastrointestinal cancer, hematological malignancies and lung cancer, respectively. Additionally, ANAs could be detected in approximately 5.9-30.8% of healthy individuals, more frequent in women than in men. Elder people show a higher rate of being ANA positive. This increases the complexity of application of ANAs in diagnostic and thus urges the identification of individual ANA and the assessment of their clinical values.

The frequency (prevalence) of anti-nuclear antibodies in inflammatory rheumatic diseases lies between 20 and 100 %. Therefore, differential ANA diagnostics is indispensable for the diagnosis of individual rheumatic diseases and their differentiation from other autoimmune diseases.

The immunoassay for the detection of ANA is dated back to 1948, Hargraves et al. discovered and applied lupus erythematosus (LE) cells in order to aid the diagnosis of SLE patients, though it was found later that the LE cell test was neither sensitive nor specific enough (R. B. Conn, "Practice Parameter - The Lupus Erythematosus Cell Test: An Obsolete Test Now Superseded by Definitive Immunologic Tests," American Journal of Clinical Pathology, pp. 65-66, 1 January 1994). In the late 1950s, the indirect immunofluorescence assay (IFA) based on animal tissue substrates, such as kidney from rats, was established. The principle of the test is that the specific ANAs bound to the antigens coated on object slides are detected by fluorescence labeled anti-human antibodies, which can be visualized under a fluorescence microscope. For the better evaluation of the result, the observed positive fluorescence signals are described as different patterns, like homogenous and granular, which is useful in subclassification of the positive results (Kavanaugh et al., "Guidelines for Clinical Use of the Antinuclear Antibody Test and Tests for Specific Autoantibodies to Nuclear Antigens," Archives of Pathology & Laboratory Medicine, vol. 124, no. 1, pp. 71-81, August 2000). Another important parameter in the evaluation of the results is the highest serum dilution, with which the patterns are still visible; this is defined as titer. Two decades later, HeLa-derived human epithelial type 2 (HEp-2) cells, which show better sensitivity and specificity, were applied in the IFA. When compared to cryosection of animal tissues, HEp-2 cells have the advantage of a large nucleus and a better visibility of other organelles, high division rate and an expression profile with more than 100 autoantigens, which contributes to easier observation and interpretation of the results. According to the guideline for ANA tests, a negative IFA result indicates a non-SARD condition while a positive result should be further differentiated with available monospecific tests.

Monospecific ANA tests were developed as the corresponding nuclear autoantigens were identified since 1960s (Tan et al., "Characteristics of a Soluble Nuclear Antigen Precipitating with Sera of Patients with Systemic Lupus Erythematosus," J Immunol, pp. 96(3):464-471, 1 March 1966). The first International Consensus Statement on standardised nomenclature of HEp-2 cell patterns in indirect immunofluorescence (ICAP, www.anapatterns.org) defined fifteen nuclear patterns and nine cytoplasmic patterns which are relevant for the diagnosis of various autoimmune diseases. Currently, different kinds of solid phase-based assays, including enzyme-linked immunosorbent assay (ELISA), lineblot, Western blot and chemiluminescence immunoassay (CLIA), are available with native or recombinant antigens. These tests have a similar principle as IFA, where the secondary anti-human antibodies are labelled with enzymes or chemical compounds that can generate color-change reactions or light emission reactions (Ludwig et al., "Mechanisms of autoantibody-induced pathology," Frontiers in Immunology, p. 8:603, 31 May 2017).

The occurrence of high titers of ANAs may be years before the development of clinical syndromes. Arbuckle and colleagues have reported that ANAs could be detected in 88% of SLE patients in a mean of 3.3 years before onset of the disease (Arbuckle et al., "Development of Autoantibodies before the Clinical Onset of Systemic Lupus Erythematosus," N Engl J Med, pp. 349:1526-33, 2003). This demonstrates the significance of ANAs in early diagnosis of the disease and a possible specific treatment. However, the utility of ANA diagnostics differs from one to another. Some ANAs showed high disease specificity and are included in the diagnostic criteria and/or reflecting disease activity, while some others show weak association with SARD or even negative relations.

Accordingly, ANAs are important diagnostic and prognostic markers. However, regardless of more than half a century of study and experience, there are still some gaps remaining in ANA diagnostics that urge to be resolved. Nowadays, more physicians, rather than just rheumatologists, are ordering ANA tests for preventive medicine and precision health, which leads to increasing numbers of positive result of ANA tests, especially positive IFA with HEp-2 cells, with unclear clinical value. However, it is not only important to report the ANA patterns and titers on HEp-2 cells but also to further distinguish ANAs for the correct diagnosis. Some ANAs, like anti-Scl70, are highly specific but with a limited prevalence in the patients with SARD; on the other hand, certain ANAs like anti-DFS70 are negatively related with SARD. Therefore, additional effort is required to identify the unknown ANAs and to study the corresponding clinical association.

Systemic scleroderma, or systemic sclerosis, is an autoimmune rheumatic disease characterised by excessive production and accumulation of collagen, called fibrosis, in the skin and internal organs and by injuries to small arteries. There are two major subgroups of systemic sclerosis based on the extent of skin involvement: limited and diffuse. The limited form affects areas below, but not above, the elbows and knees with or without involvement of the face. The diffuse form also affects the skin above the elbows and knees and can also spread to the torso. Visceral organs, including the kidneys, heart, lungs, and gastrointestinal tract can also be affected by the fibrotic process. Prognosis is determined by the form of the disease and the extent of visceral involvement. Patients with limited systemic sclerosis have a better prognosis than those with the diffuse form. Death is most often caused by lung, heart, and kidney involvement. There is also a slight increase in the risk of cancer.

About 60-95% of those with systemic scleroderma/systemic sclerosis have a positive ANA. In people who may have this condition, ANA subset tests can help to distinguish the two forms of the disease, the limited form versus the diffuse form.

Nuclear valosin-containing protein-like (synonyms: nuclear VCP-like, NVLp, Nuclear VCP-like protein, NVL) shows a high level of amino acid similarity to valosin-containing protein and locates exclusively in the nucleus. NVL belongs to the ATPase associated with various cellular activities protein family (AAA protein family). The major isoform of NVL contains two tandem AAA domains, a nuclear localization signal and a nucleolar localization signal (Fujiwara et al., "Structure and Function of the N-terminal Nucleolin Binding Domain of Nuclear Valosin-containing Protein-like 2 (NVL2) Harboring a Nucleolar Localization Signal," JOURNAL OF BIOLOGICAL CHEMISTRY, pp. pp21732-21741, 17 June 2011). It is ubiquitously expressed and mainly localized in the nucleolus and involved in biogenesis of the 60 S ribosomal subunit. It was reported that mutations in NVL may play a role in mental illness like major depressive disorder and schizophrenia (Wang et al., "The NVL gene confers risk for both major depressive disorder and schizophrenia in the Han Chinese population," Progress in Neuro-Psychopharmacology and Biological Psychiatry, pp. 7-13, 1 October 2015 and Ripke et al., "A mega-analysis of genome-wide association studies for major depressive disorder," Molecular Psychiatry, pp. 497-511, April 2013.

CD2 antigen cytoplasmic tail-binding protein 2 (CD2BP2) is ubiquitously expressed in cells. Dependent on the cell type, it has important functions in the nucleus and the cytoplasm. In CD2 receptor expressing cells, like T cells, thymocytes and natural killer cells, the GYF-domain in CD2BP2 can interact with CD2 through the proline-rich motifs in cytoplasmic tail of CD2, which signals production of interleukin-2 (Nielsen et al., "Structural Basis for the Bifunctionality of the U5 snRNP 52K Protein (CD2BP2)", Journal of Molecular Biology, pp. 369(4):902-908, 04 April 2007 and Heinze at el., "Investigating the functional role of CD2BP2 in T cells", International Immunology, vol. 19, no. 11, pp. 1313-1318, November 2007). Besides the function in immune response, CD2BP2 plays a role in pre-mRNA processing. CD2BP2 is also known as U5 snRNP 52K protein (U5-52K), because it is a component of U5 small nuclear ribonucleoprotein (snRNP), which together with other snRNPs forms the spliceosome (Nielsen et al., "Structural Basis for the Bifunctionality of the U5 snRNP 52K Protein (CD2BP2)", Journal of Molecular Biology, pp. 369(4):902-908, 04 April 2007). It was also reported that CD2BP2 is crucial for embryogenesis (Albert et al., "The GYF domain protein CD2BP2 is critical for embryogenesis and podocyte function," Journal of Molecular Cell Biology, pp. 7(5), 402-414, 16 June 2015). It has not been reported in the prior art that CD2BP2/U5-52K is associated with autoimmune diseases.

Zonula occludens-1 (ZO-1), also known as Tight junction protein-1 (TJP1) is a 220-kD peripheral membrane protein that is encoded by the TJP1 gene in humans. It belongs to the family of zona occludens proteins (ZO-1, ZO-2, and ZO-3), which are tight junction-associated proteins and of which ZO-1 is the first to be cloned. It was first isolated in 1986 by Stevenson and Goodenough using a monoclonal antibody raised in rodent liver to recognize a 225-kD polypeptide in whole liver homogenates and in tight junction-enriched membrane fractions. TJP1 is specifically enriched at the tight junctions of epithelial and endothelial cells. It belongs to the membrane associated guanylate kinase (MAGUK) protein family and shuffles between the nucleus and tight junctions (Hou, "Paracellular Channel Formation," in The Paracellular Channel, Academic Press, 2019, pp. 9-27).

The problem underlying the present invention is to provide methods, related assays and related reagents of unknown ANAs and/or to provide or define new ANAs for diagnosis and corresponding diagnostic methods, assays and related reagents that can be used for diagnosis, especially for the diagnosis of systemic autoimmune rheumatic diseases.

Another problem underlying the present invention is to increase the sensitivity of diagnostics for the diagnosis of systemic autoimmune rheumatic diseases, preferably for distinguishing autoimmune types of systemic autoimmune rheumatic diseases.

In a 1^{st} aspect, the problem underlying the present invention is solved by a method for detecting in a sample, preferably from a patient, an autoantibody binding to a polypeptide having the sequence SEQ ID NO1, SEQ ID NO11 or SEQ ID NO24 or a variant thereof.

In a preferred embodiment, the polypeptide consists of or comprises a sequence according to SEQ ID NO1 or a variant thereof. In an also preferred embodiment, the polypeptide consists of or comprises at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93. In a more preferred embodiment, the polypeptide consists of or comprises at least one of the sequences according to SEQ ID NO86, SEQ ID NO87, SEQ ID NO88, SEQ ID NO89, SEQ ID NO90, SEQ ID NO91, SEQ ID NO92 and SEQ ID NO93. In a further preferred embodiment, the polypeptide consists of or comprises two, three, four, five, six, seven or all of the sequences according to SEQ ID NO86, SEQ ID NO87, SEQ ID NO88, SEQ ID NO89, SEQ ID NO90, SEQ ID NO91, SEQ ID NO92 and SEQ ID NO93.

In a preferred embodiment, the autoantibody is an IgG class antibody.

In a preferred embodiment, the sample is a bodily fluid comprising antibodies, preferably selected from the group comprising whole blood, plasma, serum, cerebrospinal fluid and saliva. Preferably, the sample is from a mammalian, more preferably human patient.

In a preferred embodiment, the patient has one or more, preferably two or more symptoms selected from the group comprising muscle aches, fatigue, joint stiffness, swelling and redness over the joints, mild fever, trouble concentrating, rashes, including a "butterfly" rash across the cheeks, hair loss, numbness and tingling in the hands and feet, development of nodules under the skin, malaise, sun sensitivity.

In a preferred embodiment, the patient has or is supposed to have a systemic autoimmune rheumatic disease, preferably SSc.

In a preferred embodiment, the antibody, preferably autoantibody, is detected using a method from the group comprising immunodiffusion, immunoelectrophoresis, light scattering immunoassays, agglutination, labeled immunoassays such as those from the group comprising radiolabeled immunoassays, enzyme immunoassays such as colorimetric assays, chemiluminescence immunoassays and immunofluorescence, preferably immunofluorescence, more preferably indirect immunofluorescence. In a preferred embodiment, the antibody, preferably autoantibody, is detected using a recombinant indirect immunofluorescence technique with biochips.

In a 2^{nd} aspect, the problem underlying the present invention is solved by a polypeptide having the sequence SEQ ID NO1, SEQ ID NO11 or SEQ ID NO24 or a variant thereof, wherein the polypeptide is preferably immobilized, more preferably on a solid carrier, and/or wherein the polypeptide is a recombinant protein, preferably a purified recombinant protein.

In a preferred embodiment, the polypeptide according to the present invention consists of or comprises a sequence according to SEQ ID NO1 or a variant thereof. In an also preferred embodiment, the polypeptide consists of or comprises at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93. In a more preferred embodiment, the polypeptide consists of or comprises at least one of the sequences according to SEQ ID NO86, SEQ ID NO87, SEQ ID NO88, SEQ ID NO89, SEQ ID NO90, SEQ ID NO91, SEQ ID NO92 and SEQ ID NO93. In a further preferred embodiment, the polypeptide consists of or comprises two, three, four, five, six, seven or all of the sequences according to SEQ ID NO86, SEQ ID NO87, SEQ ID NO88, SEQ ID NO89, SEQ ID NO90, SEQ ID NO91, SEQ ID NO92 and SEQ ID NO93.

In a preferred embodiment, the polypeptide according to the present invention is isolated, purified and/or immobilized.

In a preferred embodiment, the polypeptide according to the present invention is a recombinant protein, preferably a purified recombinant protein.

In a preferred embodiment, the polypeptide according to the present invention is for use in a treatment of a disease, wherein the disease is preferably SARD, more preferably SSc.

In a preferred embodiment, the polypeptide according to the present invention is used in a treatment of a disease, wherein the disease is preferably SARD, more preferably SSc.

In a 3^{rd} aspect, the problem underlying the present invention is solved by a carrier, preferably a solid carrier, comprising a polypeptide having the sequence SEQ ID NO1, SEQ ID NO11 or SEQ ID NO24 or a variant thereof, wherein preferably the polypeptide is immobilized on the carrier, and/or wherein the polypeptide is preferably a recombinant protein, more preferably a purified recombinant protein.

In a preferred embodiment, the carrier comprises a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93. In a more preferred embodiment, the carrier comprises a polypeptide that consists of or comprises at least one of the sequences according to SEQ ID NO86, SEQ ID NO87, SEQ ID NO88, SEQ ID NO89, SEQ ID NO90, SEQ ID NO91, SEQ ID NO92 and SEQ ID NO93. In a further preferred embodiment, the carrier comprises a polypeptide that consists of or comprises two, three, four, five, six, seven or all of the sequences according to SEQ ID NO86, SEQ ID NO87, SEQ ID NO88, SEQ ID NO89, SEQ ID NO90, SEQ ID NO91, SEQ ID NO92 and SEQ ID NO93.

In a preferred embodiment, the carrier according to the present invention is a diagnostically useful carrier.

In a preferred embodiment, the carrier according to the present invention is a solid carrier, wherein the polypeptide is immobilized on the carrier, and wherein the polypeptide is preferably a recombinant protein, more preferably a purified recombinant protein.

In a preferred embodiment, the carrier according to the present invention is selected from the group comprising a bead, preferably a paramagnetic bead, a test strip, a microtiter plate, a membrane, preferably from the group comprising western blot, line blot and dot blot, a lateral flow device, a glass surface, a slide for microscopy, a microarray and a biochip and is preferably a slide for microscopy. In a further preferred embodiment, the carrier is a line blot or a biochip.

In a preferred embodiment, any immobilized polypeptide is expressed by a cell immobilized on the carrier according to the present invention, preferably a fixed cell, or is a recombinant or isolated polypeptide immobilized on the carrier according to the present invention.

In a preferred embodiment, any immobilized polypeptide is expressed by a cell immobilized on the carrier according to the present invention and the carrier further comprises a mock-transfected cell.

In a preferred embodiment, an autoantibody binding specifically to a polypeptide having the sequence SEQ ID NO1, SEQ ID NO11 or SEQ ID NO24 or a variant thereof, is bound to the polypeptide having the sequence SEQ ID NO1, SEQ ID NO11 or SEQ ID NO24 or a variant thereof and optionally a secondary antibody comprising a label.

In a preferred embodiment, an autoantibody binding specifically to a polypeptide having the sequence SEQ ID NO1 or a variant thereof, is bound to a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93 and optionally a secondary antibody comprising a label.

In a preferred embodiment, the carrier according to the present invention is a test strip comprising spatially separate reagents which includes a polypeptide having the sequence SEQ ID NO1, SEQ ID NO11 or SEQ ID NO24 or a variant thereof, and one or more from the group comprising one or more calibrators and a reagent indicating the presence of a secondary antibody, preferably a secondary antibody recognizing IgG class antibodies.

In a 4^{th} aspect, the problem underlying the present invention is solved by an autoantibody, preferably an isolated autoantibody, binding to a polypeptide having the sequence SEQ ID NO1, SEQ ID NO11 or SEQ ID NO24 or a variant thereof, wherein the autoantibody, preferably the isolated autoantibody, is preferably in complex with a polypeptide having the sequence SEQ ID NO1, SEQ ID NO11 or SEQ ID NO24 or a variant thereof.

In a preferred embodiment the autoantibody is isolated.

In a preferred embodiment, the autoantibody, preferably the isolated autoantibody, is diluted in a buffer solution or is included in a diluted sample, preferably patient sample and/or the autoantibody, preferably the isolated autoantibody, is in solution with one or more, preferably all reagents from the group comprising stabilizers, preferably a set of stabilizers, washing buffer, antidegradants.

In a preferred embodiment, the autoantibody or complex is detected using a method selected from the group comprising immunodiffusion techniques, immunoelectrophoretic techniques, light scattering immunoassays, agglutination techniques, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

In a 5^{th} aspect, the problem underlying the present invention is solved by a use of an autoantibody according to the present invention for the diagnosis of a disease, preferably of a systemic autoimmune rheumatic disease, more preferably of systemic sclerosis.

In a preferred embodiment, the disease is a systemic autoimmune rheumatic disease or a connective tissue disease, more preferably selected from the group comprising systemic sclerosis, anti-synthethase syndrome (ASS), rheumatoid arthritis (RA), systemic lupus erythematosis, primary Sjogren syndrome (pSS), inclusion body myositis (IBM), myositis, mixed connective tissue disease (MCTD) or undifferentiated connective tissue disease (UCTD).

In a preferred embodiment, the disease is associated with one or more, preferably two or more symptoms selected from the group comprising skin thickening and tightness, puffy finger, sclerodactyly, fingertip lesions, hyper - or hypopigmentation of the skin, swelling, general pain, chest pain, calcinosis, Raynaud's phenomenon, telangiectasias, abnormal nailfold capillaries, shortness of breath, pulmonary arterial hypertension, pulmonary fibrosis, muscle aches, fatigue, joint stiffness, swelling and redness over the joints, mild fever, trouble concentrating, rashes, including a "butterfly" rash across the cheeks, hair loss, numbness and tingling in the hands and feet, development of nodules under the skin, malaise, sun sensitivity.

In a preferred embodiment, the use comprises the step detecting in a sample an autoantibody binding to a polypeptide having the sequence SEQ ID NO1, SEQ ID NO11 or SEQ ID NO24 or a variant thereof. In a further preferred embodiment, the use comprises the step detecting in a sample an autoantibody binding to a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93.

In a 6^{th} aspect, the problem underlying the present invention is solved by a method for isolating an autoantibody binding to a polypeptide having the sequence SEQ ID NO1, SEQ ID NO11 or SEQ ID NO24 or a variant thereof, comprising the steps
a) contacting a sample comprising the autoantibody with a polypeptide having the sequence SEQ ID NO1, SEQ ID NO11 or SEQ ID NO24 or a variant thereof, under conditions compatible with formation of a complex, wherein said autoantibody binds to said polypeptide,
b) isolating the complex formed in step a),
c) dissociating the complex isolated in step b) and
d) separating the autoantibody from the polypeptide.

In a 7^{th} aspect, the problem underlying the present invention is solved by a kit comprising a polypeptide having the sequence SEQ ID NO1, SEQ ID NO11 or SEQ ID NO24 or a variant thereof.

In a preferred embodiment, the kit according to the present invention comprises a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93.

In a preferred embodiment, the kit comprises in addition a means for detecting a complex comprising the polypeptide having the sequence SEQ ID NO1, SEQ ID NO11 or SEQ ID NO24 or a variant thereof, and an antibody binding to said polypeptide.

In an 8^{th} aspect, the problem underlying the present invention is solved by a kit comprising
a) the carrier according to the present invention and a means for detecting an autoantibody bound to the carrier, which means is preferably a secondary antibody, or a polypeptide having the sequence SEQ ID NO1, SEQ ID NO11 or SEQ ID NO24 or a variant thereof, preferably a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93,
   or
b) a diagnostically useful carrier comprising a means for capturing an autoantibody according to the present invention in a liquid solution, preferably a non-labeled secondary antibody, and a means for detecting an autoantibody bound to the carrier, preferably a polypeptide having the sequence SEQ ID NO1, SEQ ID NO11 or SEQ ID NO24 or a variant thereof, more preferably a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93, even more preferably with a detectable label,
wherein the kit preferably comprises in addition to a) or b) one or more, preferably all reagents from the group comprising calibrators, preferably a set of calibrators, washing buffer, a positive control and a negative control.

In a preferred embodiment, the kit according to the present invention is a kit for the diagnosis of a disease, preferably SARD, more preferably SSc, which kit comprises a polypeptide according to the present invention, wherein the kit preferably comprises in addition a means for detecting a complex comprising the polypeptide according to the present invention and an antibody binding to said polypeptide according to the present invention.

In a preferred embodiment, the kit according to the present invention comprises in addition a means for detecting an autoantibody to another SARD-related autoantigen, preferably selected from the group comprising dsDNA, nucleosomes, histones, SS-A, Ro-52, SSB, RNP/Sm, Sm, Mi-2α, Mi-2β, Ku, CENP A, CENP B, Sp100, PML, Scl-70, PM100, PM75, RP11, RP155, gp210, PCNA, Fibrillarin, NOR90, Th/To, PDGFR and DSF70. Preferably, the kit can comprise one, two or more additional means for detecting an autoantibody to one, two or more further SARD-related autoantigens, respectively, wherein the one, two or more further SARD-related autoantigens are preferably selected from the group comprising dsDNA, nucleosomes, histones, SS-A, Ro-52, SSB, RNP/Sm, Sm, Mi-2α, Mi-2β, Ku, CENP A, CENP B, Sp100, PML, Scl-70, PM100, PM75, RP11, RP155, gp210, PCNA, Fibrillarin, NOR90, Th/To, PDGFR and DSF70.

In a 9^{th} aspect, the problem underlying the present invention is solved by a use of a polypeptide, a carrier or an autoantibody according to the present invention or an antibody to a polypeptide having the sequence SEQ ID NO1, SEQ ID NO11 or SEQ ID NO24 or a variant thereof, for the manufacture of a kit, medical device, preferably diagnostic device, preferably for the diagnosis of a disease, more preferably for the diagnosis of SARD, even more preferably for the diagnosis of SSc.

In a 10^{th} aspect, the problem underlying the present invention is solved by a pharmaceutical composition or medical device, preferably diagnostic device, comprising a polypeptide having the sequence SEQ ID NO1, SEQ ID NO11 or SEQ ID NO24 or a variant thereof.

In a preferred embodiment, the pharmaceutical composition or medical device comprises a polypeptide that consists of or comprises a sequence according to SEQ ID NO1 or a variant thereof. In an also preferred embodiment, this polypeptide consists of or comprises at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93. In a more preferred embodiment, this polypeptide consists of or comprises at least one of the sequences according to SEQ ID NO86, SEQ ID NO87, SEQ ID NO88, SEQ ID NO89, SEQ ID NO90, SEQ ID NO91, SEQ ID NO92 and SEQ ID NO93. In a further preferred embodiment, this polypeptide consists of or comprises two, three, four, five, six, seven or all of the sequences according to SEQ ID NO86, SEQ ID NO87, SEQ ID NO88, SEQ ID NO89, SEQ ID NO90, SEQ ID NO91, SEQ ID NO92 and SEQ ID NO93.

In a preferred embodiment, the pharmaceutical composition or the medical device further comprises one or more adjuvants.

In a preferred embodiment, the medical device is selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

In an 11^{th} aspect, the problem underlying the present invention is solved by a method comprising the step contacting a medical or diagnostic device comprising a polypeptide having the sequence SEQ ID NO1, SEQ ID NO11 or SEQ ID NO24 or a variant thereof with a buffered solution comprising an antibody binding specifically to said polypeptide having the sequence SEQ ID NO1, SEQ ID NO11 or SEQ ID NO24 or a variant thereof, which solution is not a sample from a patient in need of a diagnosis comprising an unknown concentration of said antibody, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgG class antibodies,
followed by detection of a signal which indicates whether or not the antibody has bound to the polypeptide, optionally a signal relating to the concentration of the antibody in the solution or solutions.

In a 12^{th} aspect, the problem underlying the present invention is solved by a use of a sample, preferably a patient sample, which is preferably diluted, comprising an autoantibody according to the present invention as a positive control.

In a 13^{th} aspect, the problem underlying the present invention is solved by a diluted sample comprising an autoantibody according to the present invention, wherein the sample is preferably diluted in an aqueous buffer solution and/or wherein the diluted sample comprises in addition one or more, preferably all reagents from the group comprising calibrators, preferably a set of calibrators, a stabilizer, washing buffer, a positive control and a negative control.

In a 14^{th} aspect, the problem underlying the present invention is solved by an aqueous solution comprising an autoantibody according to the present invention.

In a preferred embodiment, the aqueous solution comprises a sample from a human patient.

In a preferred embodiment, the solution comprises a non-physiological buffer or a physiological buffer at concentrations above physiological levels and more preferably has a pH between 5 and 9, preferably 6 to 8.

In a 15^{th} aspect, the problem underlying the present invention is solved by a device for removing an autoantibody according to the present invention, from blood, preferably serum of a patient, wherein the device comprises a carrier according to the present invention.

In a 16^{th} aspect, the problem underlying the present invention is solved by an *ex vivo* method for removing an autoantibody according to the present invention, from blood, preferably serum of a patient.

According to the present invention one or more epitopes recognized by anti-NVL autoantibodies is or are located on one, two, three, four, five, six, seven or all C-terminal fragments according to SEQ ID NO86, SEQ ID NO87, SEQ ID NO88, SEQ ID NO89, SEQ ID NO90, SEQ ID NO91, SEQ ID NO92 and SEQ ID NO93.

The present invention is based on the surprising finding of the inventors that autoantibodies to NVL, CD2BP2 and TJP1 are present and detectable in samples. They can be detected using immunoassays and can be used to set up serological immunoassays for the diagnosis of diseases, especially SARD, specifically SSc. A carrier comprising immobilized polypeptides of NVL, CD2BP2 or TJP1 or variants thereof may be used for detecting such autoantibodies.

According to the present invention, the term "a polypeptide having the sequence SEQ ID NO1, SEQ ID NO11 or SEQ ID NO24 or a variant thereof' as used herein, refers to a polypeptide having the sequence SEQ ID NO1 or a variant thereof, a polypeptide having the sequence SEQ ID NO11 or a variant thereof or a polypeptide having the sequence SEQ ID NO24 or a variant thereof.

According to the present invention, the term "a polypeptide having the sequence" as used herein, refers to a polypeptide consisting of or comprising said sequence.

According to the present invention, the term "a polypeptide having the sequence SEQ ID NO1 or a variant thereof' as used herein, refers to a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93.

According to the present invention, a polypeptide having the sequence SEQ ID NO1, SEQ ID NO11 or SEQ ID NO24 or a variant thereof is used to detect an autoantibody to SEQ ID NO1, SEQ ID NO11 or SEQ ID NO24 or a variant thereof, for example by coating the diagnostically useful carrier according to the present invention with a polypeptide having the sequence SEQ ID NO1, SEQ ID NO11 or SEQ ID NO24 or a variant thereof or by using a soluble polypeptide having the sequence SEQ ID NO1, SEQ ID NO11 or SEQ ID NO24 or a variant thereof, which may be detected if bound to the autoantibody. In a preferred embodiment, any sequence referred to is understood to be presented as part of a polypeptide or a similar form which may be used to detect the autoantibody of interest.

Preferably a polypeptide having the sequence SEQ ID NO1, SEQ ID NO11 or SEQ ID NO24 or a variant thereof is immobilized on a solid phase of the carrier. It may be directly immobilized on the solid phase when contacted with the sample, but a competitive assay, a capture bridge assay, an immunometric assay, a class-specific second antibody on the solid phase, a class capture assay, direct or indirect may also be used. The principle of each of these formats is detailed in The Immunoassay Handbook, 3rd edition, edited by David Wild, Elsevier, 2005. More preferably, the solid phase is a test strip or a well of a microtiter plate for ELISA, preferably a well of a microtiter plate for ELISA.

The teachings of the present invention may not only be carried out using the polypeptides, in particular a polypeptide comprising the native sequence of a polypeptide referred to such as human NVL-isoform1 (SEQ ID NO1), human CD2BP2 (SEQ ID NO11) or human TJP1-isoform2 (SEQ ID NO24) or polypeptides having the exact sequences referred to in this application explicitly, for example by function, name, sequence or accession number, or implicitly, but also using variants of such polypeptides.

In a preferred embodiment, the teachings of the present invention are carried out using a polypeptide having the sequence SEQ ID NO1 or a variant thereof, having the sequence SEQ ID NO11 or a variant thereof or having the sequence SEQ ID NO24 or a variant thereof.

In a preferred embodiment, the teachings of the present invention are carried out using a polypeptide having the sequence SEQ ID NO10, i.e. a polypeptide referred to such as NVL[human]-IF1 fused to a hexa Histidin-tag (H6), or a variant thereof.

In a preferred embodiment, the teachings of the present invention are carried out using a polypeptide having the sequence of a polypeptide referred to such as H8-NVL[human](aa1-117) (SEQ ID NO49), H8-NVL[human](aa106-292) (SEQ ID NO50), H8-NVL[human](aa281-560) (SEQ ID NO51), H8-NVL[human](aa549-856) (SEQ ID NO52), or a variant thereof.

In a preferred embodiment, the teachings of the present invention are carried out using a polypeptide consisting of or comprising at least one of the sequences according to SEQ ID NO1, SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93.

In a preferred embodiment, the teachings of the present invention are carried out using a polypeptide consisting of or comprising at least one of the sequences according to SEQ ID NO86, SEQ ID NO87, SEQ ID NO88, SEQ ID NO89, SEQ ID NO90, SEQ ID NO91, SEQ ID NO92 and SEQ ID NO93.

In a preferred embodiment, the teachings of the present invention are carried out using a polypeptide having the sequence of a polypeptide referred to such as CD2BP2[human] fused to a N-terminal octa Histidin-tag (H8) (SEQ ID NO23) or a variant thereof.

In a preferred embodiment, the teachings of the present invention are carried out using a polypeptide having the sequence of a polypeptide referred to such as H8-CD2BP2[human](aa117-260) (SEQ ID NO66), H8-CD2BP2[human](aa249-341) (SEQ ID NO67) or a variant thereof.

In a preferred embodiment, the teachings of the present invention are carried out using a polypeptide having the sequence of a polypeptide referred to such as pET24d-N-TJP1[human](aa1-575) fused to a N-terminal octa Histidin-tag (H8) (SEQ ID NO82) or a variant thereof.

In a preferred embodiment, the teachings of the present invention are carried out using a polypeptide having the sequence of a polypeptide referred to such as TJP1[human]-isoform2 fused to a C-terminal octa Histidin-tag (H8) (SEQ ID NO32), H8-TJP1[human](aa1-156) (SEQ ID NO83), H8-TJP1[human](aa144-306) (SEQ ID NO84), H8-TJP1[human](aa294-575) (SEQ ID NO85) or a variant thereof.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 6, 7, 8, 10, 12, 15, 20, 25, 50, 75, 100, 150, 200, 300, 400, 500, 600, 700, 800, 900, 1000, 1100, 1200, 1300, 1400, 1500, 1600 or 1650 successive amino acids of the original sequence or a variant thereof. The total length of the variant may be at least 6, 7, 8, 9, 10, 11, 12, 20, 25, 30, 40, 50, 60, 70, 80, 90, 100, 200, 300, 400, 500, 600, 700, 800, 900, 1000, 1100, 1200, 1300, 1400, 1500, 1600, 1650 or 1668 or more amino acids.

In a preferred embodiment, the term "variant", as used herein, may refer to the full length sequence or at least one fragment of the full length sequence referred to with an additional tag selected from the group of tags comprising His, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag.

The term "variant" relates not only to at least one fragment, but also to a polypeptide or a fragment thereof comprising amino acid sequences that are at least 40, 50, 60, 70, 75, 80, 85, 90, 92, 94, 95, 96, 97, 98, 99, 99,3, 99,4, 99,5, 99,6, 99,7, 99,8 or 99,9, preferably at least 99,3 % identical to the reference amino acid sequence referred to or the fragment thereof, wherein amino acids other than those essential for the biological activity, for example the ability of an antigen to bind to an (auto)antibody, or the fold or structure of the polypeptide are deleted or substituted and/or one or more such essential amino acids are replaced in a conservative manner and/or amino acids are added such that the biological activity of the polypeptide is preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default setting.

In a preferred embodiment, the polypeptide and variants thereof may, in addition, comprise chemical modifications, for example isotopic labels or covalent modifications such as glycosylation, phosphorylation, acetylation, decarboxylation, citrullination, methylation, hydroxylation and the like. The person skilled in the art is familiar with methods to modify polypeptides. Any modification is designed such that it does not abolish the biological activity of the variant.

Moreover, variants may also be generated by N- or/and C-terminal fusion of polypeptides, fragments or variants thereof with other known polypeptides or variants thereof and comprise active portions or domains, preferably having a sequence identity of at least 70, 75, 80, 85, 90, 92, 94, 95, 96, 97, 98 or 99 % when aligned with the active portion of the reference sequence, wherein the term "active portion", as used herein, refers to an amino acid sequence, which is less than the full length amino acid sequence or, in the case of a nucleic acid sequence, codes for less than the full length amino acid sequence, respectively, and/or is a variant of the natural sequence, but retains at least some of the biological activity. Preferably the active portion is an active portion having sequence SEQ ID NO10 or a variant thereof, having sequence SEQ ID NO23 or a variant thereof or having sequence SEQ ID NO82 or a variant thereof. Preferably the active portion is an active portion having sequence SEQ ID NO86, SEQ ID NO87, SEQ ID NO88, SEQ ID NO89, SEQ ID NO90, SEQ ID NO91, SEQ ID NO92 or SEQ ID NO93 or a variant thereof.

In a preferred embodiment of the present invention, a variant of SEQ ID NO1 refers to a sequence according to SEQ ID NO35, SEQ ID NO38, SEQ ID NO41, SEQ ID NO44, SEQ ID NO45, SEQ ID NO46, SEQ ID NO47, SEQ ID NO48, SEQ ID NO49, SEQ ID NO50, SEQ ID NO51, SEQ ID NO52, SEQ ID NO86, SEQ ID NO87, SEQ ID NO88, SEQ ID NO89, SEQ ID NO90, SEQ ID NO91, SEQ ID NO92 and SEQ ID NO93.

The variant of the polypeptide has biological activity. In a preferred embodiment, such biological activity is the ability to bind specifically to an autoantibody binding specifically to the autoantigen of interest, preferably the autoantigen having the sequence of SEQ ID NO1, SEQ ID NO11, SEQ ID NO24, SEQ ID NO10, SEQ ID NO23 or SEQ ID NO82 or a variant thereof as found preferably in a patient suffering from an autoimmune disease associated with such autoantibody, preferably SARD, more preferably SSc. For example, whether or not a variant of the polypeptide has such biological activity may be checked by determining whether or not it binds specifically to an autoantibody from a sample of a SARD, preferably of a SSc patient comprising an autoantibody binding specifically to wild type autoantigen, preferably as determined by indirect immunofluorescence.

According to the present invention, a polypeptide having the sequence SEQ ID NO1, SEQ ID NO11 or SEQ ID NO24 or a variant thereof may be a recombinant protein. In a preferred embodiment, the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, a polypeptide provided or used according to the present invention such as a polypeptide having the sequence SEQ ID NO1, SEQ ID NO11 or SEQ ID NO24 or a variant thereof or an autoantibody according to the present invention is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

According to the present invention, a medical or diagnostic device such as the diagnostically useful carrier may be prepared by expressing a recombinant variant of a polypeptide having the sequence SEQ ID NO1, SEQ ID NO11 or SEQ ID NO24 or a variant thereof, comprising an affinity tag, optionally with an artificial linker which may include a protease cleavage site, in a cell such as a eukaryotic or prokaryotic cell, contacting the expressed variant with a ligand binding specifically to the affinity tag, which ligand is immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site, before the immobilization. The affinity tag may be selected from the group of tags comprising His, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV and Xpress Tag. Useful proteases include, but are not limited to TEV, Thrombin, factor Xa or Enteropeptidase. Suitable linkers are part of vectors, for example pET vector series (Novagen).

According to the present invention, a cell is provided which overexpresses a polypeptide having the sequence SEQ ID NO1, SEQ ID NO11 or SEQ ID NO24 or a variant thereof, preferably in combination with another cell which overexpresses the sequence of another SARD-related autoantigen in addition or a variant thereof. Another SARD-related autoantigen herein is preferably selected from the group comprising dsDNA, nucleosomes, histones, SS-A, Ro-52, SSB, RNP/Sm, Sm, Mi-2α, Mi-2β, Ku, CENP A, CENP B, Sp100, PML, Scl-70, PM100, PM75, RP11, RP155, gp210, PCNA, Fibrillarin, NOR90, Th/To, PDGFR and DSF70. In a preferred embodiment, the term "overexpressing", as used herein, means that the cell has been transfected with a nucleic acid, either transiently or stably in the sense that the nucleic acid has been incorporated in the genome of the cell, that comprises a nucleic acid sequence encoding a polypeptide having the sequence SEQ ID NO1, SEQ ID NO11 or SEQ ID NO24 or a variant thereof or another SARD-related autoantigen or a variant thereof under the control of a promotor. The cell overexpressing a polypeptide having the sequence SEQ ID NO1, SEQ ID NO11 or SEQ ID NO24 or a variant thereof may additionally overexpress a polypeptide to another SARD-related autoantigen or a variant thereof. Consequently, the transfected cell expresses more polypeptide recognized by the autoantibody to be detected than the same type of cell normally would, probably at least 10, 20, 30, 50, 100, 200 or 500 % more as judged by quantitative Western Blot. The promotor may be an inducible promotor, which allows for the induction of expression by addition of an inducer. The person skilled in the art is familiar with protocols and vectors for transiently overexpressing a polypeptide in a eukaryotic cell, for example the pTriEx system from Novagen and with protocols and vectors for stably transfecting a eukaryotic cell, for example the pcDNA^{™}4/TO vector system from Invitrogen.

In a preferred embodiment, a fixed mammalian cell may be used. In a preferred embodiment, the term "fixed" cell, as used herein, refers to a cell that has been treated with a reactive chemical compound to the effect that the cell is no longer metabolically active, but still presents its epitopes for immunostaining with antibodies and their subsequent detection, for example by fluorescence. More preferably, the reactive chemical compound is selected from the group comprising acetone, formalin, methanol and ethanol or mixtures thereof, preferably all of them. The person skilled in the art is familiar with protocols that may be used to prepare fixed cells. Essentially, the cell which is attached to a solid support is washed by using washing buffer, followed by contacting with the reactive compound, for example immersion. Pure acetone or formalin or aqueous dilutions of the reactive chemical compound may be used.

According to the present invention, the cell is on a carrier for microscopic immunofluorescence analysis. Such a carrier may be a glass slide. The cell on the glass slide may be covered with a mounting buffer. A mounting medium is a liquid which helps maintain a near physiological pH to maintain the molecular structure of any diagnostically relevant molecular and their epitopes, is compatible with the emission of a fluorescence signal and prevents a premature loss of fluorescence due to bleaching of the fluorophore. At the same time its optical properties are matched with other buffers used, in particular its refractive index which allows for an efficient microscopic fluorescence analysis. The mounting medium comprises a base component, preferably selected from the group comprising water, glycerol, natural oil or plastic or a mixture thereof, preferably water and glycerol. It may further comprise an antifade constituent which may reduce bleaching, preferably selected from the group comprising NPG (N-propyl gallate), DABCO (1,4-diazabicyclo[2.2.2]octane), 4POBN ((4-Pirydyl-1-oxide)-N-tert-butyl nitrone) and PPD (P-phenylanediamine). Various compositions and methods are described in the state of the art, for example in "Mountants and Antifades", published by Wright Cell Imaging Facility, Toronto Western Research Institute University Health Network, (https://de.scribd.com/document/47879592/Mountants-Antifades), Krenek et al. (1989) Comparison of antifading agents used in immunofluorescence, J. Immunol. Meth 117, 91-97 and Nairn et al. (1969) Microphotometry in Immunofluorescence, Clin. Exp. Immunol. 4, 697-705.

A cover glass may be placed on top of the composition comprising the sample and the mounting medium. Slides with cover glasses (FB 112d-1005-1 or ZZ 3000-0112) are available from EUROIMMUN Medizinische Labordiagnostika AG, Lübeck, Germany. However, any carrier compatible with microscopic analysis of the fluorescence pattern may be used. The carrier may comprise a mock-transfected cell, which has been transfected with the same vector as the cell overexpressing a polypeptide having the sequence SEQ ID NO1, SEQ ID NO11 or SEQ ID NO24 or a variant thereof, but without the nucleic acid encoding for the latter. Such mock-transfected cell may serve as a negative control. The carrier is configured for analysis using an immunofluorescence microscope.

In a preferred embodiment, the carrier may comprise a field comprising the cell according to the invention. In addition the carrier may comprise additional fields. The fields are preferably surrounded by a hydrophobic surface. Each of these fields may comprise a cell overexpressing another SARD-related autoantigen or a variant thereof. A field may comprise a section of mammalian kidney or liver tissue, preferably primate.

Preferably a polypeptide having the sequence SEQ ID NO1, SEQ ID NO11 or SEQ ID NO24 or a variant thereof is immobilized on a solid phase of the carrier. It may be directly immobilized on the solid phase when contacted with the sample, but a competitive assay, a capture bridge assay, an immunometric assay, a class-specific second antibody on the solid phase, a class capture assay, direct or indirect may also be used. The principle of each of these formats is detailed in The Immunoassay Handbook, 3rd edition, edited by David Wild, Elsevier, 2005. More preferably, the solid phase is a test strip or a well of a microtiter plate for ELISA, preferably a well of a microtiter plate for ELISA.

In a preferred embodiment, a secondary antibody is an antibody binding specifically to all antibodies from an antibody class, preferably a mammalian antibody class, more preferably human antibody class such as IgG. Secondary antibodies typically recognize the constant domain of said class, but may also recognize other epitopes shared by antibodies from the class of interest, for example a conformational epitope across the 3D structure. A wide range of them is commercially available, for example from Thermo Fisher. It may be a monoclonal or a polyclonal antibody. In a preferred embodiment, the term "recognized", as used herein, means that the secondary antibody binds specifically to the antibody or antibodies to be detected. A secondary antibody may bind specifically to all isotypes from the antibody class. For example a secondary antibody to IgG class antibodies may bind to IgG1, IgG2, IgG3 and IgG4 isotypes. This may be achieved by using as a secondary antibody to the class, preferably to IgG class antibodies, a mixture comprising an antibody binding specifically to each IgG isotype or a single antibody which reacts with all isotypes of interest. The use of secondary antibodies is explained in Kruger, N. J., Detection of Polypeptides on Blots Using Secondary Antibodies, in The Protein Protocols Handbook (ed. J. M. Walker), page 967, volume 1996, Springer. Briefly, such secondary antibodies may be generated by immunizing a laboratory animal with the antibody to be recognized or a mixture of the antibodies to be recognized.

The autoantibody to be detected or a secondary antibody used binds preferably specifically to the autoantigen or antibody to be detected, respectively. In a preferred embodiment, the term "binding specifically", as used herein, preferably means that the binding reaction is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

In a preferred embodiment, the cell is bound to an autoantibody to a polypeptide having the sequence SEQ ID NO1, SEQ ID NO11 or SEQ ID NO24 or a variant thereof, and a secondary antibody is bound to the autoantibody. In a more preferred embodiment, the secondary antibody recognizes IgG class antibodies. For immunofluorescence analysis, the secondary antibody may comprise a detectable fluorescent label, more preferably FITC (fluorescein isothiocyanate).

In a preferred embodiment, the method according to the present invention comprises the step providing the carrier according to the present invention. The carrier may then be contacted with the sample suspected of comprising the autoantibody under conditions allowing for binding of any autoantibodies to the cell and the polypeptide having the sequence SEQ ID NO1, SEQ ID NO11 or SEQ ID NO24 or a variant thereof expressed by the cell. The sample may then be removed and the carrier with the cell may be washed to remove any remaining sample. A secondary autoantibody or similar reagent or means binding to the autoantibody and carrying a detectable label such as a fluorescent dye may then be contacted with the carrier under conditions allowing formation of a complex between any bound autoantibody and the secondary antibody. The carrier may be washed then to remove non-bound secondary antibody. Finally, the presence of the autoantibody is detected by checking whether the secondary antibody may be detected, preferably by immunofluorescence, more preferably emitted by fluorescein or a derivative thereof, most preferably FITC.

In a preferred embodiment, the term "diagnosis", as used herein, is to be used in its broadest possible sense and may to any kind of procedure aiming to obtain information instrumental in the assessment whether a patient, known or an anonymous subject from a cohort, suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient or patients in general with regard to a certain treatment, for example the administration of immunosuppressive drugs, or to find out whether a sample is from such a patient. Such information may be used for a clinical diagnosis, but may also be obtained by an experimental and/or research laboratory for the purpose of general research, for example to determine the proportion of subjects suffering from the disease in a patient cohort or in a population. In other words, the term "diagnosis" comprises not only diagnosing, but also prognosticating and/or monitoring the course of a disease or disorder, including monitoring the response of one or more patients to the administration of a drug or candidate drug, for example to determine its efficacy. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to a sample from an anonymized patient. In another preferred embodiment, the detection of an autoantibody to a polypeptide having the sequence SEQ ID NO1, SEQ ID NO11 or SEQ ID NO24 or a variant thereof is considered to imply a definitive diagnosis of SARD, preferably SSc, because of the presence of the autoantibody.

In a preferred embodiment, the methods and products according to the present invention may be used for interaction studies, including determining whether a drug candidate or other compound may interfere with the binding of an autoantibody to a polypeptide having the sequence SEQ ID NO1, SEQ ID NO11 or SEQ ID NO24 or a variant thereof or may affect any downstream process or the strength of its binding to its target. In a preferred embodiment, they may be used for monitoring the immune response, more preferably the emergence and/or titer of antibodies to a polypeptide having the sequence SEQ ID NO1, SEQ ID NO11 or SEQ ID NO24 or a variant thereof, following the administration of an immunogenic composition comprising a polypeptide having the sequence SEQ ID NO1, SEQ ID NO11 or SEQ ID NO24 or a variant thereof or an immunogenic variant thereof, for example to a mammal, which may be a mammal other than a human such as a laboratory animal.

In another preferred embodiment, the methods and products according to the present invention may be used for determining the concentration of an antibody to a polypeptide having the sequence SEQ ID NO1, SEQ ID NO11 or SEQ ID NO24 or a variant thereof. In a more preferred embodiment, said antibody is an autoantibody from a SARD patient, preferably from a SSc patient. In another preferred embodiment, said antibody is a recombinant antibody which binds to a polypeptide having the sequence SEQ ID NO1, SEQ ID NO11 or SEQ ID NO24 or a variant thereof, but is recognized by a secondary antibody binding specifically to human IgG class antibodies, preferably IgG1, IgG2, IgG3 and IgG4 isotypes. In a more preferred embodiment, such a concentration needs to be determined for the purposes of research, for the preparation or for monitoring the quality of reagents, animal models or devices that may or may not be used for the diagnosis of SARD, preferably SSc.

In many cases the mere detection of the autoantibody, in other words determining whether or not detectable levels of the antibody are present in the sample, is sufficient for the diagnosis. If the autoantibody can be detected, this will be information instrumental for the clinician's diagnosis and indicates an increased likelihood that the patient suffers from a disease.

The person skilled in the art will appreciate that a clinician does usually not conclude whether or not the patient suffers or is likely to suffer from a disease, condition or disorders solely on the basis of a single diagnostic parameter, but needs to take into account other aspects, for example the presence of other autoantibodies, markers, blood parameters, clinical assessment of the patient's symptoms or the results of medical imaging or other noninvasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also be the possibility to rule out one disease, thus allowing for the indirect diagnosis of another. In a preferred embodiment, the meaning of any symptoms or diseases referred to throughout this application is in line with the person skilled in the art's understanding as of the filing date or, preferably, earliest priority date of this application as evidenced by text books and scientific publications. It should be mentioned that the inventive methods or uses or products, taken alone, cannot be used to arrive at a definite, final diagnosis.

In a preferred embodiment, the term "diagnosis" may also refer to a method or agent used to choose the most promising treatment regime for a patient. In other words, the method or agent may relate to selecting a treatment regimen for a subject. For example, the detection of autoantibodies may indicate that an immunosuppressive therapy is to be selected, which may include administrating to the patient one or more immunosuppressive drugs.

In a preferred embodiment, any information or data demonstrating the presence of absence of the autoantibody may be communicated to the patient or a medical doctor treating the patient, preferably by telephone, by fax, in a written form or via the internet, for example as an email or text message.

In a preferred embodiment, the term "autoantibody", as used herein, refers to an antibody binding specifically to an endogenous molecule of the animal, preferably mammal, more preferably human, which produces said autoantibody, wherein the level of such antibody is more preferably elevated compared to the average healthy subject. The autoantibody may have the sequence of an antibody's constant regions from the animal, preferably human, making it, but the variable region is able to bind specifically to the endogenous molecule of the animal, more specifically to a polypeptide having the sequence SEQ ID NO1, SEQ ID NO11 or SEQ ID NO24 or a variant thereof or a SARD-related, preferably SSc-related autoantigen. In a preferred embodiment, the autoantibody is isolated and/or purified from a sample, preferably tissue, serum, plasma, blood or CSF from the animal, preferably human. The autoantibody is a polyclonal, native antibody from the animal rather than a synthetic or recombinant antibody.

The method according to the present invention is preferably an *in vitro* method.

According to the present invention, a kit is provided, comprising the cell or the carrier and further comprising one or more, preferably all reagents from the group comprising a secondary antibody, preferably labeled with a detectable label, a washing solution, a positive control, a negative control, a detergent, a cover glass, a mounting medium and a physiological salt solution, preferably PBS, or salt required to prepare it. In a preferred embodiment, the positive control is a diluted sample, preferably serum or CSF, from a patient suffering from SARD, preferably SSc or a monoclonal antibody to a polypeptide having the sequence SEQ ID NO1, SEQ ID NO11 or SEQ ID NO24 or a variant thereof. The negative control may be a diluted sample from a healthy subject, for example a blood donor. The kit may comprise instructions how to carry out the assay. Preferably, the secondary antibody is a secondary antibody to IgG class antibodies, preferably human IgG class antibodies.

In a preferred embodiment, the present invention provides a use of the cell, the polypeptide, the carrier for the manufacture of kit a composition for the diagnosis of a disease.

In a preferred embodiment, any method or use according to the present invention may be intended for a non-diagnostic use, i.e. determining the presence of an autoantibody to binding to a polypeptide having the sequence SEQ ID NO1, SEQ ID NO11 or SEQ ID NO24 or a variant thereof, for a use other than diagnosing a patient. For example, the method or use may be for testing in vitro the efficiency of a medical device designed to remove an autoantibody from a patient's blood, wherein the testing is performed on a liquid other than patient's blood. After the use of the medical device with a patient, its capacity to remove autoantibody may be checked by running a solution comprising antibody to a polypeptide having the sequence SEQ ID NO1, SEQ ID NO11 or SEQ ID NO24 or a variant thereof through the device, followed by use of the method according to the present invention to confirm that less or no antibody is in the solution that has been passed through the device, i.e. showing that the device has still the capacity to remove antibody from the solution.

In another preferred embodiment, the method may be for confirming the reliability of a diagnostic assay and may involve detecting an antibody to a polypeptide having the sequence SEQ ID NO1, SEQ ID NO11 or SEQ ID NO24 or a variant thereof in a solution, which is not a sample from a patient who requires a diagnosis, but is known to comprise an antibody to a polypeptide having the sequence SEQ ID NO1, SEQ ID NO11 or SEQ ID NO24 or a variant thereof, preferably at a known concentration. For example, it may be a recombinant antibody or a sample diluted in a dilution buffer such as PBS from an anonymous patient whose identity cannot be traced back. Alternatively, the solution may be a negative control not comprising the antibody to check the background. Such method may be run in parallel with, after or before a diagnostic method. In a preferred embodiment, any method or use according to the present invention may be intended for generating an autoantibody profile, preferably for detecting a disease in a mammal, preferably a human.

In a preferred embodiment, any method or use according to the present invention may be for identifying a subject at risk of suffering from or developing a disease, preferably SARD, more preferably SSc, and/or a tumor.

In a preferred embodiment, the method may be for detecting an antibody, preferably autoantibody in a solution which is not a sample from a mammal to be diagnosed or for the purpose of providing a diagnosis, in particular not a diagnosis of SARD.

In a preferred embodiment, the present invention provides an apparatus for analyzing a sample from a patient to detect an autoantibody against a polypeptide having the sequence SEQ ID NO1, SEQ ID NO11 or SEQ ID NO24 or a variant thereof, indicating an increased likelihood of a disease or of developing a disease, comprising:
a. a carrier, which contains a means for capturing the autoantibody from the sample when the sample is contacted with the carrier, wherein the means is the cell and the carrier is the carrier according to the present invention,
b. a detectable means capable of binding to the antibody captured by the carrier when the detectable means is contacted with the carrier, wherein the detectable means is preferably a labeled secondary antibody capable of binding to the autoantibody captured on the carrier,
c. optionally a means for removing any sample from the carrier and the detectable means, preferably by washing;
d. a detecting device for detecting the presence of the detectable means and converting the results into an electrical signal, for example a fluorescence reader or a fluorescence microscope connected with a software capable of recognizing a pattern characteristic of a stained cell overexpressing a polypeptide having the sequence SEQ ID NO1, SEQ ID NO11 or SEQ ID NO24 or a variant thereof in an image of the cell taken by the fluorescence reader or camera, and
optionally a means for receiving the electronical signal from the detecting device and determining if the level of the signal is indicative of an increased likelihood of having or developing a disease, by comparing with the patterns characteristic of wild type or nonstained cells, preferably by a mock-transfected cell or cells not positively stained by an autoantibody binding specifically to a polypeptide having the sequence SEQ ID NO1, SEQ ID NO11 or SEQ ID NO24 or a variant thereof on the same carrier, or an input reference value obtained with samples from healthy subjects or by comparing the level of signal obtained with one sample with the level of signal obtained with a second sample obtained at a later time point, preferably at least one month later.

According to the present invention, a device for removing an autoantibody to a polypeptide having the sequence SEQ ID NO1, SEQ ID NO11 or SEQ ID NO24 or a variant thereof, from blood, preferably serum of a SARD patient, preferably of an SSc patient, wherein the device comprises a carrier coated with a polypeptide having the sequence SEQ ID NO1, SEQ ID NO11 or SEQ ID NO24 or a variant thereof is provided as an *ex vivo* method for removing an autoantibody to a polypeptide having the sequence SEQ ID NO1, SEQ ID NO11 or SEQ ID NO24 or a variant thereof, from blood, preferably serum of a SARD patient, preferably of a SSc patient. A device coated with a polypeptide having the sequence SEQ ID NO1, SEQ ID NO11 or SEQ ID NO24 or a variant thereof may be used or a device coated with a secondary antibody or protein capturing all IgG class antibodies, among them IgG class autoantibodies to a polypeptide having the sequence SEQ ID NO1, SEQ ID NO11 or SEQ ID NO24 or a variant thereof. Suitable methods are described in Eisei Noiri and Noria Hanafusa, The Concise Manual of Apharesis Therapy, Springer Tokyo, 2014. Hamilton, P., Kanigicherla, D., Hanumapura, P., Walz, L., Kramer, D., Fischer, M., Brenchley, P., and Mitra, S. (2018) J. Clin. Aph. 33(3), 283-290. Another method is disclosed in EP3477300.

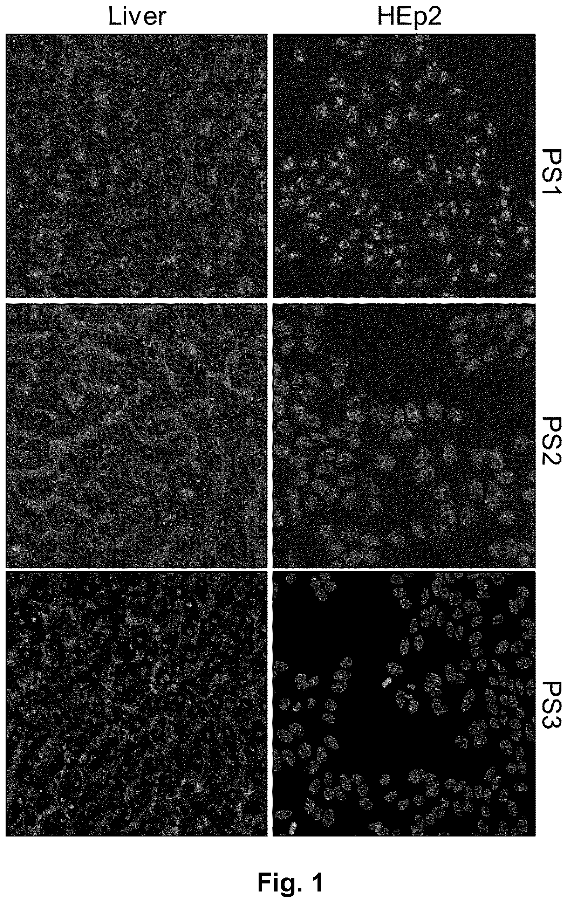

Fig. 1 shows IFA with HEp-2 cells or monkey liver with 1:100 diluted patient sera (PS). Anti-Human-IgG-Alexa488 (1:500) was used as secondary antibody. PS1 showed a nucleolar pattern on both substrates. PS2 and PS3 showed a pseudo-dense fine speckled pattern (magnification 200x).

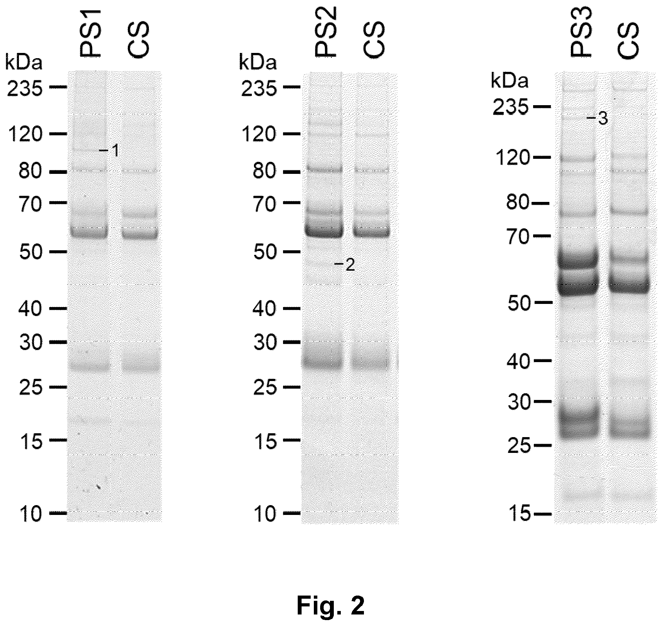

Fig. 2 shows immunoprecipitation of patient (PS1-3) or control sera (CS) with HEp2 extract. Immunoprecipitates were separated by SDS-PAGE, followed by coomassie staining. Selected protein bands were identified as NVL (band 1), CD2BP2 (band 2), or TJP1 (band 3) by MALDI-TOF-MS.

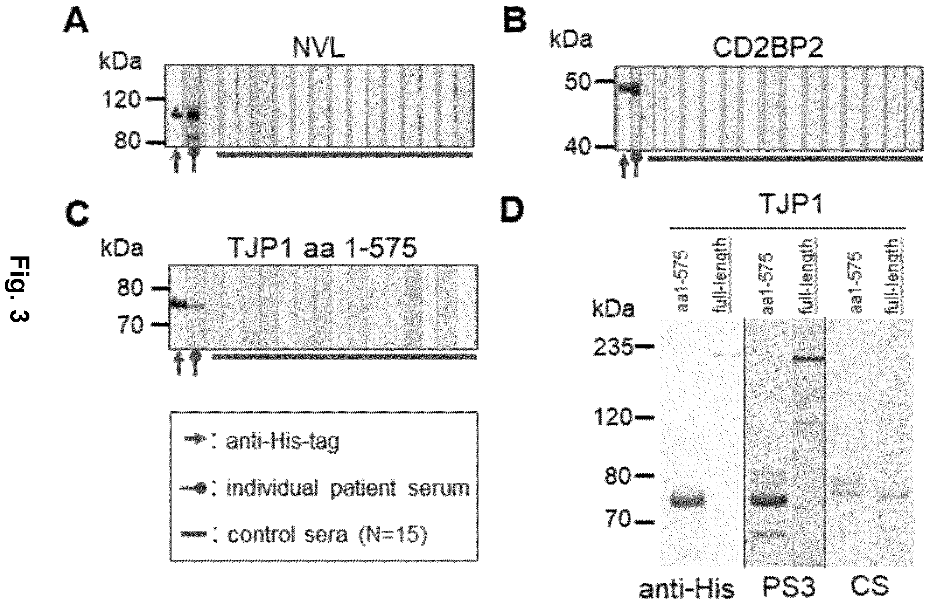

Fig. 3 shows immunoblots with anti-His-tag (1:2000), patient sera (1:200) and healthy control sera (1:200) with recombinant antigens: A) purified NVL-His expressed in E.coli and PS1, B) purified His-CD2BP2 expressed in E.coli and PS2, C) purified His-TJP1 aa1-575 expressed in E.coli and PS3 D) full-length TJP1-His (HEK293 cell lysate) or His-TJP1 aa1-575 (E.coli lysate). Anti-mouse-IgG-AP (1:2000) or anti-human-IgG-B/E-AP (1:10) were used in secondary incubation.

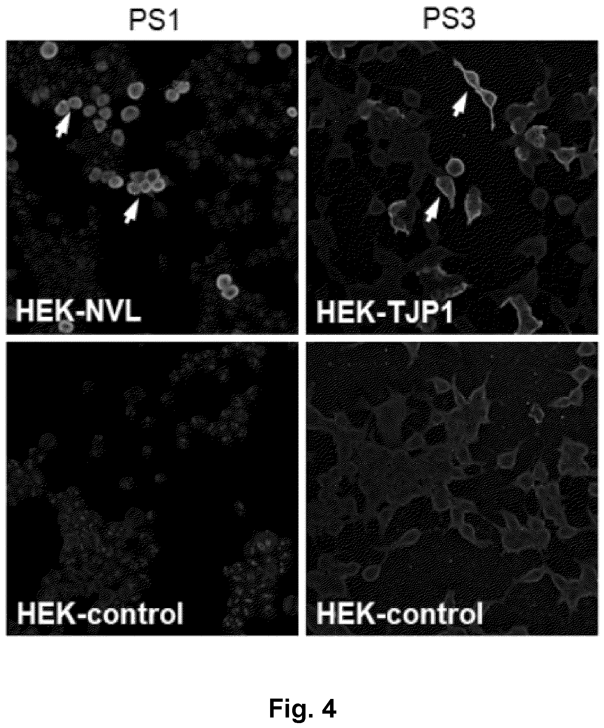

**Fig. 4** shows IFA with NVL-His, TJP1-His or control transfected HEK293 cells and PS1 (1:100) or PS3, respectively (1:10). Anti-human-IgG-Alexa488 (1:500) was applied as secondary antibody. Arrows indicate the specific fluorescence signal (magnification 200x).

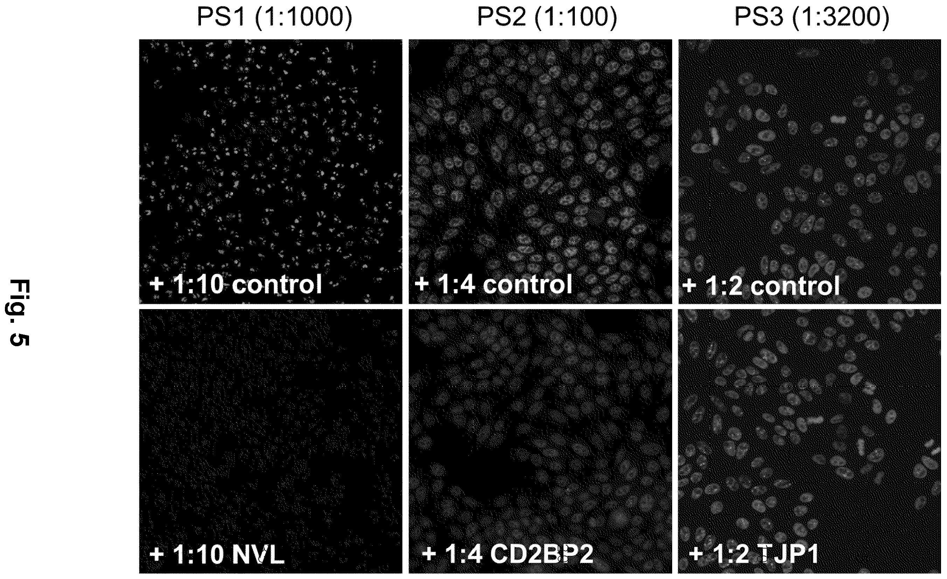

**Fig. 5** shows IFA neutralization assay with patient sera and HEp-2 cells. Patient sera were incubated with the diluted purified recombinant antigens or control buffer prior to application in IFA with HEp-2 cells. Individual serum dilutions are indicated. Anti-Human-IgG-Alexa488 (1:500) was used as secondary antibody (magnification 200x).

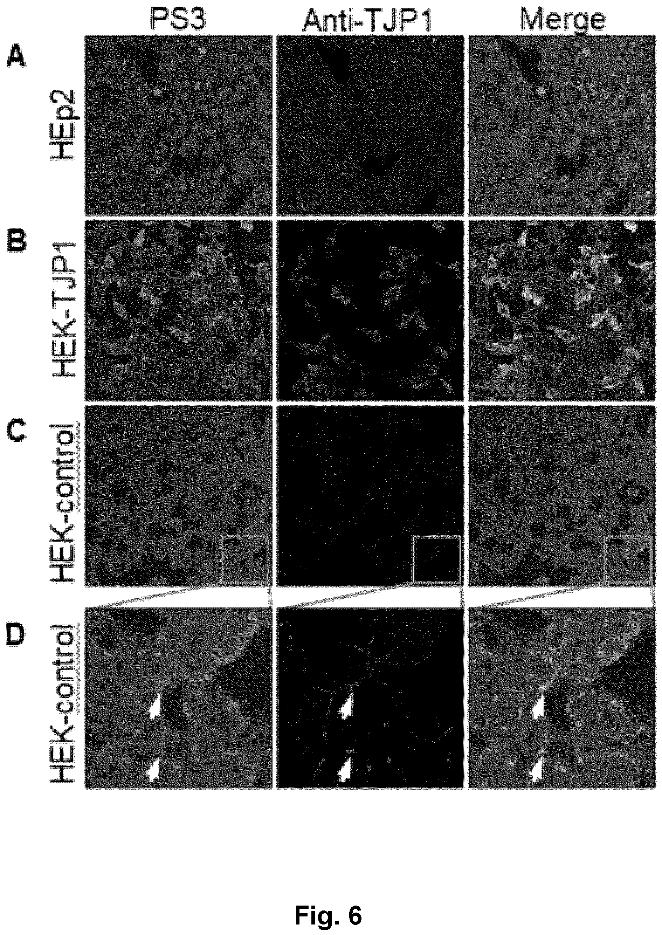

**Fig. 6** shows colocalization analysis with anti-TJP1 antibody (mouse, monoclonal) and PS3 in HEp-2, HEK-TJP1 and HEK-control cells. A mixture of PS3 (1:10) and anti-TJP1 (1:50) was applied in IFA. Anti-human-IgG-alexa488 (1:500) and anti-mouse-IgG-Cy3 (1:200) were employed as secondary antibodies. **A)** HEp-2 cells. **B)** transfected HEK293 cells overexpressing TJP1-His. **C,D)** control HEK293 cells transfected with empty vector (A-C magnification 200x, D magnification 3200x).

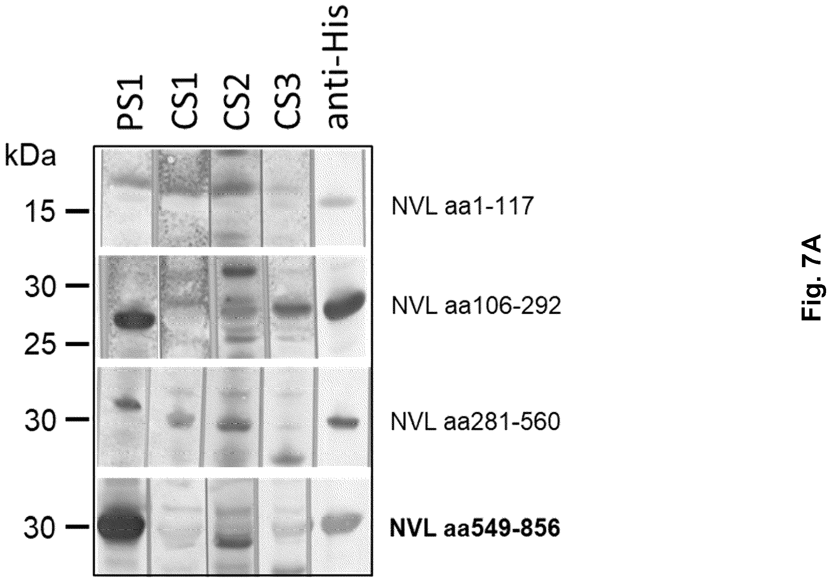

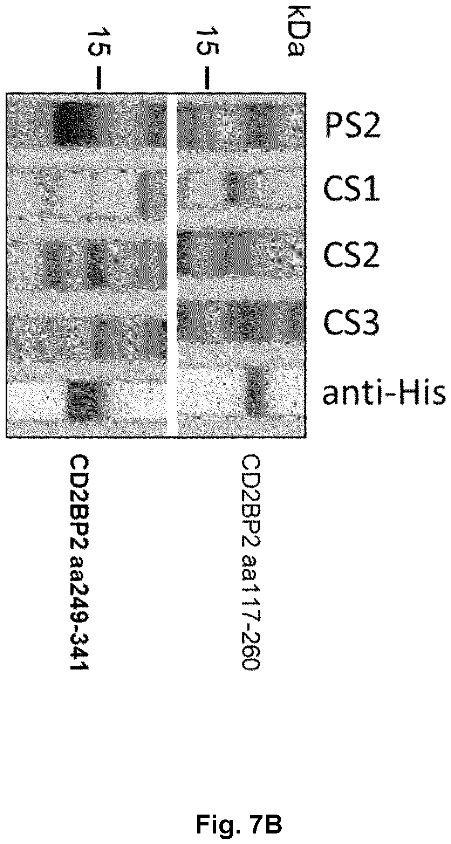

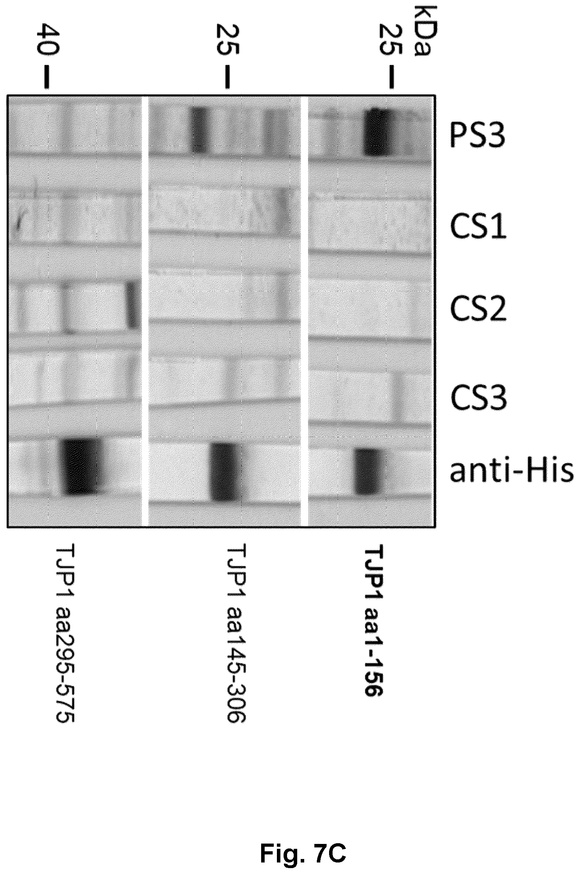

**Fig. 7A****,** **Fig. 7B** **and** **Fig. 7C** show immunoblots with E.coli lysates containing **Fig. 7A****)** NVL fragments (aa1-117, aa106-292, aa281-560, aa549-856) **Fig. 7B****)** CD2BP2 fragments (aa117-260, aa249-341) or **Fig. 7C****)** TJP1 fragments (aa1-156, aa145-306, aa295-575) and respective patient sera (PS1-3, 1:200), control sera (CS1-3, 1:200), or anti-His-tag antibody (1:2000). Anti-mouse-IgG-AP (1:2000) or anti-human-IgG-B/E-AP (1:10) were used in secondary incubation. Fragments with main reactions are indicated by bold letters.

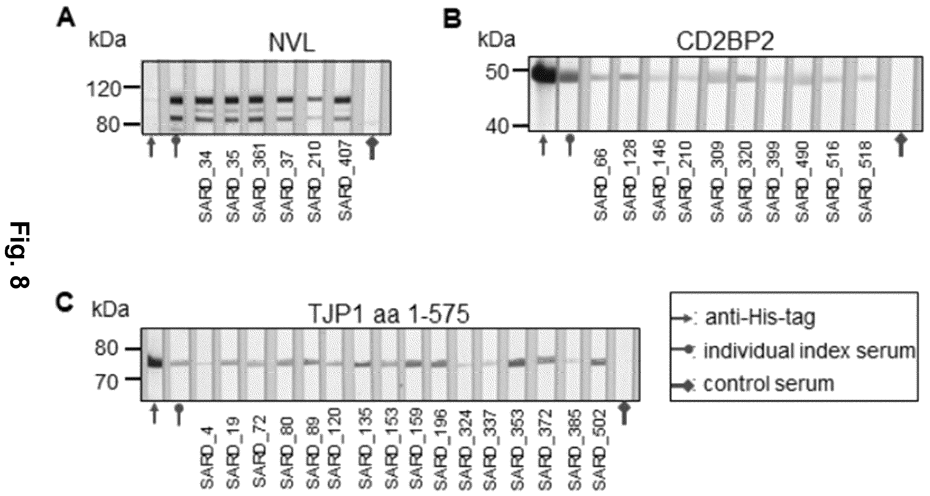

**Fig. 8** shows immunoblots with purified NVL-His (**A**), His-CD2BP2 (**B**) or His-TJP1 aa1-575 (**C**) and anti-NVL, anti-CD2BP2 or anti-TJP1 lineblot positive sera (1:200) from the systemic rheumatic disease (SARD) cohort. As positive and negative controls anti-His-tag-AP (1:8000), PS1-3 (1:200) and control sera (1:200) were incubated in parallel. Anti-human-IgG-B/E-AP (1:10) was used in secondary incubation.

### Sequences:

The present invention comprises a range of polypeptides. The exact sequences are provided with the sequence listing if not disclosed in the following:
**SEQ ID NO1 [NVL-isoform1, Uniprot O15381-1]** 
MKPRPAGFVDNKLKQRVIQYLTSNKCGKYVDIGVLASDLQRVYSIDYGRRKRNAFRIQVEKV
FSIISSEKELKNLTELEDEHLAKRARQGEEDNEYTESYSDDDSSMEDYPDPQSANHMNSSL
LSLYRKGNPDSVSNTPEMEQRETTSSTPRISSKTGSIPLKTPAKDSEGGWFIDKTPSVKKDS
FFLDLSCEKSNPKKPITEIQDSKDSSLLESDMKRKGKLKNKGSKRKKEDLQEVDGEIEAVLQ
KKAKARGLEFQISNVKFEDVGGNDMTLKEVCKMLIHMRHPEVYHHLGVVPPRGVLLHGPPG
CGKTLLAHAIAGELDLPILKVAAPEIVSGVSGESEQKLRELFEQAVSNAPCIIFIDEIDAITPKRE
VASKDMERRIVAQLLTCMDDLNNVAATARVLVIGATNRPDSLDPALRRAGRFDREICLGIPD
EASRERILQTLCRKLRLPQAFDFCHLAHLTPGFVGADLMALCREAAMCAVNRVLMKLQEQQ
KKNPEMEDLPSKGVQEERLGTEPTSETQDELQRLLGLLRDQDPLSEEQMQGLCIELNDFIV
ALSSVQPSAKREGFVTVPNVTWADIGALEDIREELTMAILAPVRNPDQFKALGLVTPAGVLLA
GPPGCGKTLLAKAVANESGLNFISVKGPELLNMYVGESERAVRQVFQRAKNSAPCVIFFDE
VDALCPRRSDRETGASVRVVNQLLTEMDGLEARQQVFIMAATNRPDIIDPAILRPGRLDKTL
FVGLPPPADRLAILKTITKNGTKPPLDADVNLEAIAGDLRCDCYTGADLSALVREASICALRQ
EMARQKSGNEKGELKVSHKHFEEAFKKVRSSISKKDQIMYERLQESLSR
**SEQ ID NO2 [NVL-isoform3]**
**SEQ ID NO3 [NVL gene synthesis]**
**SEQ ID NO4 [sense NVL-IF1]**
**SEQ ID NO5 [asense NVL-IF1]**
**SEQ ID NO6 [NVL-PCR-fragment]**
**SEQ ID NO7 [pTriEx-1-NVL[human]-IF1-H8]**
**SEQ ID NO8 [NVL[human]-IF1-H8]**
**SEQ ID NO9 [pET24d-NVL[human]-IF1-H6]**
**SEQ ID NO10 [NVL[human]-IF1-H6]** 
MKPRPAGFVDNKLKQRVIQYLTSNKCGKYVDIGVLASDLQRVYSIDYGRRKRNAFRIQVEKV
FSIISSEKELKNLTELEDEHLAKRARQGEEDNEYTESYSDDDSSMEDYPDPQSANHMNSSL
LSLYRKGNPDSVSNTPEMEQRETTSSTPRISSKTGSIPLKTPAKDSEGGWFIDKTPSVKKDS
FFLDLSCEKSNPKKPITEIQDSKDSSLLESDMKRKGKLKNKGSKRKKEDLQEVDGEIEAVLQ
KKAKARGLEFQISNVKFEDVGGNDMTLKEVCKMLIHMRHPEVYHHLGVVPPRGVLLHGPPG
CGKTLLAHAIAGELDLPILKVAAPEIVSGVSGESEQKLRELFEQAVSNAPCIIFIDEIDAITPKRE
 
VASKDMERRIVAQLLTCMDDLNNVAATARVLVIGATNRPDSLDPALRRAGRFDREICLGIPD
EASRERILQTLCRKLRLPQAFDFCHLAHLTPGFVGADLMALCREAAMCAVNRVLMKLQEQQ
KKNPEMEDLPSKGVQEERLGTEPTSETQDELQRLLGLLRDQDPLSEEQMQGLCIELNDFIV
ALSSVQPSAKREGFVTVPNVTWADIGALEDIREELTMAILAPVRNPDQFKALGLVTPAGVLLA
GPPGCGKTLLAKAVANESGLNFISVKGPELLNMYVGESERAVRQVFQRAKNSAPCVIFFDE
VDALCPRRSDRETGASVRVVNQLLTEMDGLEARQQVFIMAATNRPDIIDPAILRPGRLDKTL
FVGLPPPADRLAILKTITKNGTKPPLDADVNLEAIAGDLRCDCYTGADLSALVREASICALRQ
EMARQKSGNEKGELKVSHKHFEEAFKKVRSSISKKDQIMYERLQESLSRVEHHHHHH
**SEQ ID NO11 [CD2BP2, Uniprot O95400]** 
MPKRKVTFQGVGDEEDEDEIIVPKKKLVDPVAGSGGPGSRFKGKHSLDSDEEEDDDDGGS
SKYDILASEDVEGQEAATLPSEGGVRITPFNLQEEMEEGHFDADGNYFLNRDAQIRDSWLD
NIDWVKIRERPPGQRQASDSEEEDSLGQTSMSAQALLEGLLELLLPRETVAGALRRLGARG
GGKGRKGPGQPSSPQRLDRLSGLADQMVARGNLGVYQETRERLAMRLKGLGCQTLGPHN
PTPPPSLDMFAEELAEEELETPTPTQRGEAESRGDGLVDVMWEYKWENTGDAELYGPFTS
AQMQTWVSEGYFPDGVYCRKLDPPGGQFYNSKRIDFDLYT
**SEQ ID NO12 [CD2BP2, Acc. BC001947]**
**SEQ ID NO13 [CD2BP2, gene synthesis fragment 1, aa1-173]**
**SEQ ID NO14 [CD2BP2, gene synthesis fragment 2, aa174-341]**
**SEQ ID NO15 [CD2BP2, gene synthesis fragment 1, Bbsl]**
**SEQ ID NO16 [CD2BP2, gene synthesis fragment 2, Bbsl]**
**SEQ ID NO17 [sense CD2BP2-fragment-2]**
**SEQ ID NO18 [asense CD2BP2-fragment-2]**
**SEQ ID NO19 [CD2BP2-PCR-fragment]**
**SEQ ID NO20 [pTriEx-1-CD2BP2[human]]**
**SEQ ID NO21 [CD2BP2[human]-H8]**
**SEQ ID NO22 [pET24d-N-CD2BP2[human]]**
**SEQ ID NO23 [H8-CD2BP2[human]]** 
MSHHHHHHHHSMPKRKVTFQGVGDEEDEDEIIVPKKKLVDPVAGSGGPGSRFKGKHSLDS
DEEEDDDDGGSSKYDILASEDVEGQEAATLPSEGGVRITPFNLQEEMEEGHFDADGNYFLN
RDAQIRDSWLDNIDWVKIRERPPGQRQASDSEEEDSLGQTSMSAQALLEGLLELLLPRETV
AGALRRLGARGGGKGRKGPGQPSSPQRLDRLSGLADQMVARGNLGVYQETRERLAMRLK
GLGCQTLGPHNPTPPPSLDMFAEELAEEELETPTPTQRGEAESRGDGLVDVMWEYKWENT
GDAELYGPFTSAQMQTWVSEGYFPDGVYCRKLDPPGGQFYNSKRIDFDLYT
**SEQ ID NO24 [TJP1-isoform2, Uniprot Q07157-2]** 
MSARAAAAKSTAMEETAIWEQHTVTLHRAPGFGFGIAISGGRDNPHFQSGETSIVISDVLKG
GPAEGQLQENDRVAMVNGVSMDNVEHAFAVQQLRKSGKNAKITIRRKKKVQIPVSRPDPE
PVSDNEEDSYDEEIHDPRSGRSGVVNRRSEKIWPRDRSASRERSLSPRSDRRSVASSQPA
KPTKVTLVKSRKNEEYGLRLASHIFVKEISQDSLAARDGNIQEGDVVLKINGTVTENMSLTDA
KTLIERSKGKLKMVVQRDERATLLNVPDLSDSIHSANASERDDISEIQSLASDHSGRSHDRP
PRRSRSRSPDQRSEPSDHSRHSPQQPSNGSLRSRDEERISKPGAVSTPVKHADDHTPKTV
EEVTVERNEKQTPSLPEPKPVYAQVGQPDVDLPVSPSDGVLPNSTHEDGILRPSMKLVKFR
KGDSVGLRLAGGNDVGIFVAGVLEDSPAAKEGLEEGDQILRVNNVDFTNIIREEAVLFLLDLP
KGEEVTILAQKKKDVYRRIVESDVGDSFYIRTHFEYEKESPYGLSFNKGEVFRVVDTLYNGK
LGSWLAIRIGKNHKEVERGIIPNKNRAEQLASVQYTLPKTAGGDRADFWRFRGLRSSKRNLR
KSREDLSAQPVQTKFPAYERVVLREAGFLRPVTIFGPIADVAREKLAREEPDIYQIAKSEPRD
AGTDQRSSGIIRLHTIKQIIDQDKHALLDVTPNAVDRLNYAQWYPIVVFLNPDSKQGVKTMRM
RLCPESRKSARKLYERSHKLRKNNHHLFTTTINLNSMNDGWYGALKEAIQQQQNQLVWVS
EGKADGATSDDLDLHDDRLSYLSAPGSEYSMYSTDSRHTSDYEDTDTEGGAYTDQELDET
LNDEVGTPPESAITRSSEPVREDSSGMHHENQTYPPYSPQAQPQPIHRIDSPGFKPASQQV
YRKDPYPEEMMRQNHVLKQPAVSHPGHRPDKEPNLTYEPQLPYVEKQASRDLEQPTYRYE
SSSYTDQFSRNYEHRLRYEDRVPMYEEQWSYYDDKQPYPSRPPFDNQHSQDLDSRQHPE
ESSERGYFPRFEEPAPLSYDSRPRYEQAPRASALRHEEQPAPGYDTHGRLRPEAQPHPSA
GPKPAESKQYFEQYSRSYEQVPPQGFTSRAGHFEPLHGAAAVPPLIPSSQHKPEALPSNTK
PLPPPPTQTEEEEDPAMKPQSVLTRVKMFENKRSASLETKKDVNDTGSFKPPEVASKPSGA
PIIGPKPTSQNQFSEHDKTLYRIPEPQKPQLKPPEDIVRSNHYDPEEDEEYYRKQLSYFDRR
SFENKPPAHIAASHLSEPAKPAHSQNQSNFSSYSSKGKPPEADGVDRSFGEKRYEPIQATP
PPPPLPSQYAQPSQPVTSASLHIHSKGAHGEGNSVSLDFQNSLVSKPDPPPSQNKPATFRP
PNREDTAQAAFYPQKSFPDKAPVNGTEQTQKTVTPAYNRFTPKPYTSSARPFERKFESPKF
NHNLLPSETAHKPDLSSKTPTSPKTLVKSHSLAQPPEFDSGVETFSIHAEKPKYQINNISTVP
KAIPVSPSAVEEDEDEDGHTVVATARGIFNSNGGVLSSIETGVSIIIPQGAIPEGVEQEIYFKV
CRDNSILPPLDKEKGETLLSPLVMCGPHGLKFLKPVELRLPHCDPKTWQNKCLPGDPNYLV
GANCVSVLIDHF
**SEQ ID NO25 [sense TJP1-IF2-ZF1]**
**SEQ ID NO26 [asense TJP1-IF2-ZF1]**
**SEQ ID NO27 [sense TJP1-IF2-ZF2]**
**SEQ ID NO28 [asense TJP1-IF2-ZF2]**
**SEQ ID NO29 [TJP1-PCR-fragment-1]**
**SEQ ID NO30 [TJP1-PCR-fragment-2]**
**SEQ ID NO31 [pTriEx-1-TJP1[human]-IF2]**
**SEQ ID NO32 [TJP1[human]-IF2-H8]**
**SEQ ID NO33 [sense NVL-F1]**
**SEQ ID NO34 [asense NVL-F1]**
**SEQ ID NO35 [NVL-PCR-fragment 1-117]**
**SEQ ID NO36 [sense NVL-F2]**
**SEQ ID NO37 [asense NVL-F2]**
**SEQ ID NO38 [NVL-PCR-fragment 106-292]**
**SEQ ID NO39 [sense NVL-F3]**
**SEQ ID NO40 [asense NVL-F3]**
**SEQ ID NO41 [NVL-PCR-fragment 281-560]**
**SEQ ID NO42 [sense NVL-F4]**
**SEQ ID NO43 [asense NVL-F4]**
**SEQ ID NO44 [NVL-PCR-fragment 549-856]**
**SEQ ID NO45 [pET24d-N-NVL[human](aa1-117)]**
**SEQ ID NO46 [pET24d-N-NVL[human](aa106-296)]**
**SEQ ID NO47 [pET24d-N-NVL[human](aa281-560)]**
**SEQ ID NO48 [pET24d-N-NVL[human](aa549-856)]**
**SEQ ID NO49 [H8-NVL[human](aa1-117)]**
**SEQ ID NO50 [H8-NVL[human](aa106-292)]**
**SEQ ID NO51 [H8-NVL[human](aa281-560)]**
**SEQ ID NO52 [H8-NVL[human](aa549-856)]**
**SEQ ID NO53 [sense CD2BP2-F1]**
**SEQ ID NO54 [asense CD2BP2-F1]**
**SEQ ID NO55 [CD2BP2-PCR-fragment 1-128]**
**SEQ ID NO56 [sense CD2BP2-F2]**
**SEQ ID NO57 [asense CD2BP2-F2]**
**SEQ ID NO58 [CD2BP2-PCR-fragment 117-260]**
**SEQ ID NO59 [sense CD2BP2-F3]**
**SEQ ID NO60 [asense CD2BP2-F3]**
**SEQ ID NO61 [CD2BP2-PCR-fragment 249-341]**
**SEQ ID NO62 [pET24d-N-CD2BP2[human](aa1-128)]**
**SEQ ID NO63 [pET24d-N-CD2BP2[human](aa117-260)]**
**SEQ ID NO64 [pET24d-N-CD2BP2[human](aa249-341)]**
**SEQ ID NO65 [H8-CD2BP2[human](aa1-128)]**
**SEQ ID NO66 [H8-CD2BP2[human](aa117-260)]**
**SEQ ID NO67 [H8-CD2BP2[human](aa249-341)]**
**SEQ ID NO68 [sense TJP1-F1/2]**
**SEQ ID NO69 [asense TJP1-F1/4]**
**SEQ ID NO70 [TJP1-PCR-fragment 1-575]**
**SEQ ID NO71 [asense TJP1-F2]**
**SEQ ID NO72 [TJP1-PCR-fragment 1-156]**
**SEQ ID NO73 [sense TJP1-F3]**
**SEQ ID NO74 [asense TJP1-F3]**
**SEQ ID NO75 [TJP1-PCR-fragment 144-306]**
**SEQ ID NO76 [sense TJP1-F4]**
**SEQ ID NO77 [TJP1-PCR-fragment 294-575]**
**SEQ ID NO78 [pET24d-N-TJP1[human](aa1-575)]**
**SEQ ID NO79 [pET24d-N-TJP1[human](aa1-156)]**
**SEQ ID NO80 [pET24d-N-TJP1[human](aa144-306)]**
**SEQ ID NO81 [pET24d-N-TJP1[human](aa294-575)]**
**SEQ ID NO82 [H8-TJP1[human](aa1-575)]** 
MSHHHHHHHHSMSARAAAAKSTAMEETAIWEQHTVTLHRAPGFGFGIAISGGRDNPHFQS
GETSIVISDVLKGGPAEGQLQENDRVAMVNGVSMDNVEHAFAVQQLRKSGKNAKITIRRKK
KVQIPVSRPDPEPVSDNEEDSYDEEIHDPRSGRSGVVNRRSEKIWPRDRSASRERSLSPRS
DRRSVASSQPAKPTKVTLVKSRKNEEYGLRLASHIFVKEISQDSLAARDGNIQEGDVVLKIN
GTVTENMSLTDAKTLIERSKGKLKMVVQRDERATLLNVPDLSDSIHSANASERDDISEIQSLA
SDHSGRSHDRPPRRSRSRSPDQRSEPSDHSRHSPQQPSNGSLRSRDEERISKPGAVSTP
VKHADDHTPKTVEEVTVERNEKQTPSLPEPKPVYAQVGQPDVDLPVSPSDGVLPNSTHED
GILRPSMKLVKFRKGDSVGLRLAGGNDVGIFVAGVLEDSPAAKEGLEEGDQILRVNNVDFTN
IIREEAVLFLLDLPKGEEVTILAQKKKDVYRRIVESDVGDSFYIRTHFEYEKESPYGLSFNKGE
VFRVVDTLYNGKLGSWLAIRIGKNHKEVERGIIP
**SEQ ID NO83 [H8-TJP1[human](aa1-156)]**
**SEQ ID NO84 [H8-TJP1[human](aa144-306)]**
**SEQ ID NO85 [H8-TJP1[human](aa294-575)]**
**SEQ ID NO86 [NVL-fragment(aa591-603)]**
   EELTMAILAPVRN
**SEQ ID NO87 [NVL-fragment(aa607-617)]**
   FKALGLVTPAG
**SEQ ID NO88 [NVL-fragment(aa685-695)]**
   ALCPRRSDRET
**SEQ ID NO89 [NVL-fragment(aa742-753)]**
   KTLFVGLPPPAD
**SEQ ID NO90 [NVL-fragment(aa761-772)]**
   ITKNGTKPPLDA
**SEQ ID NO91 [NVL-fragment(aa778-789)]**
   AIAGDLRCDCYT
**SEQ ID NO92 [NVL-fragment(aa809-826)]**
   MARQKSGNEKGELKVSHK
**SEQ ID NO93 [NVL-fragment(aa844-856)]**
   QIMYERLQESLSR

The present invention is further illustrated by the following non-limiting examples from which further features, embodiments, aspects and advantages of the present invention may be taken.

### Example 1:

### Summary

Methods: Three patient sera (PS1-3) with pre-determined ANA against unknown nuclear autoantigens (UNA), as determined by indirect immunofluorescence assay (IFA) on HEp2 cells and lineblot with 23 known nuclear antigens were selected. Sera were analyzed in immunoprecipitation assays with HEp-2 cell lysates and mass spectrometry. The identified candidate autoantigens were recombinantly expressed in *E.coli* and/or HEK293 cells and were applied in immunoblot, lineblot, recombinant IFA and neutralization assays. In addition, the prevalence of ANAs against these antigens in a cohort of patients with systemic autoimmune rheumatic diseases (SARD cohort, n = 488) was studied.

Results: Nuclear VCP-like protein (NVL), CD2 antigen cytoplasmic tail-binding protein 2 (CD2BP2), and tight junction protein ZO-1 (TJP1) were identified as the respective target antigen of PS1, PS2 and PS3. In immunoblots with respective purified recombinant antigens NVL, CD2BP2, and TJP1 aa1-575 PS1-3 showed a positive reaction with the individual antigen, whereas healthy controls (n = 15) were nonreactive. Preincubation of PS1 with the respective NVL antigen and PS2 with the respective CD2BP2 antigen abolished the ANA reaction of the patient sera in HEp2 cell IFA. Additionally, PS1 and PS3 reacted with the individual antigen in recombinant HEK293 cell IFA. Screening a SARD cohort in a lineblot based on the respective purified recombinant antigens NVL, CD2BP2, and TJP1 aa1-575 identified four patients with anti-NVL (4x SSc), ten patients with anti-CD2BP2 (8x SSc, 1x MCTD, 1x Myositis) and 16 patients with anti-TJP1 autoantibodies (15x SSc, 1x UCTD), whereas all control sera (n = 50) were negative.

ANAs against NVL, CD2BP2 and TJP1 present novel markers for SARD, especially SSc, or other rheumatic diseases.

### Reagents

Reagents were obtained from Merck, Darmstadt, Germany or Sigma-Aldrich, Heidelberg, Germany if not specified otherwise.

### Methods

### Patients

Control collectives included 50 healthy donor sera. For assessment of the clinical association a cohort of patients with systemic autoimmune rheumatic disease (SARD) was used which consisted of 529 serum samples from 488 patients, of which 378 patients had systemic sclerosis (SSc) and 110 patients had other diseases (anti-synthetase syndrome (ASS) n = 10; inclusion body myositis (IBM) n = 4; mixed connective tissue disease (MCTD) n = 6; myositis n = 15; primary Sjogren syndrome (pSS) n = 11; rheumatoid arthritis (RA) n = 24; systemic lupus erythematosis (SLE) n = 30; undifferentiated connective tissue disease (UCTD) n = 10). The repeated sera were obtained from the same patients at different time points.

### Indirect immunofluorescence assay (IFA)

IFA was conducted using slides with a biochip array of acetone fixed HEp-2 cells combined with liver tissue cryosections. Each biochip mosaic was incubated with 35 µL of 1:100 PBS-diluted sample at room temperature for 30 min, washed with PBS-Tween and immersed in PBS-Tween for 5 min. In the second step, anti-human-IgG-Alexa488 (1:500) (Jackson ImmunoResearch, Suffolk, UK) was applied and incubated at a room temperature of 23°C for 30 min. Slides were washed again with a flush of PBS-Tween and then immersed in PBS-Tween for 5 min. Slides were embedded in PBS-buffered, DABCO containing glycerol (approximately 10 µL per field) and examined by fluorescence microscopy.

For colocalization analysis a mixture of anti-TJP1 antibody (1:50, 33-9100, Thermo Fisher Scientific, Dreieich, Germany) and patient serum (1:10) was used in the first incubation step and anti-human-IgG-alexa488 (1:500) and anti-mouse-IgG-Cy3 (1:200) (Jackson ImmunoResearch) in the second incubation step.

In competitive inhibition experiments, recombinant antigens were mixed with the individual patient serum 1 h prior to the IFA on HEp2 cells. Positive and negative controls were included. Samples were classified as positive or negative based on fluorescence intensity of the transfected cells in direct comparison with control-transfected cells and control samples. Endpoint titers refer to the last dilution showing visible fluorescence.

Results were evaluated by two independent observers using a EUROStar II microscope (EUROIMMUN Medizinische Labordiagnostika AG, Lübeck, Germany).

### Immunoprecipitation

The immunoprecipitation was performed with 500 µl cell HEp-2 cells (10 × 10⁶ cells/ml) and 5 µl patient or control sera. HEp-2 cells were centrifuged at 16000 x g for 10 minutes at 4°C. The sediment together with patient or control sera was resuspended in 500 µl RIPA lysis buffer (PBS pH 7.4, 0.1% (v/v) Nonidet^{™} P 40, 1% (w/v) Deoxycholate, 0.1% (w/v) SDS, 1 mM Protease inhibitor cocktail [Sigma]) and rotated for 1 h at 4°C. The suspension was spun down at 16000 x g for 20 minutes at 4°C. The supernatants were then incubated with Protein G Dynabeads (ThermoFisher Scientific, Schwerte, Germany) at 4°C for 3 h to capture immunocomplexes. Beads were washed three times with PBS, and eluted with NuPage LDS sample buffer (ThermoFisher Scientific) containing 25 mmol/L dithiothreitol at 70°C for 10 min. Carbamidomethylation with 59 mM iodoacetamide (Bio-Rad, Hamburg, Germany) was performed prior to SDS-PAGE (NuPAGE, ThermoFisher Scientific). Separated proteins were visualized with Coomassie Brillant Blue (G-250), and identified by mass spectrometric analysis.

### Mass spectrometry

Visible protein bands were excised from Coomassie Brilliant Blue G-250 stained gels. After destaining and tryptic digestion peptides were extracted and spotted with α-cyano-4-hydroxycinnamic acid onto a MTP AnchorChip^{™} 384 TF target.

MALDI-TOF/TOF measurements were performed with an Autoflex III smartbeam TOF/TOF200 System using flexControl 3.4 software. MS spectra for peptide mass fingerprinting (PMF) were recorded in positive ion reflector mode with 4,000-10,000 shots and in a mass range from 600 Da to 4,000 Da. Spectra were calibrated externally with the commercially available Peptide Calibration Standard II, processed with flexAnalysis 3.4 and peak lists were analyzed with BioTools 3.2.

The Mascot search engine Mascot Server 2.3 (Matrix Science, London, UK) was used for protein identification by searching against the NCBI or SwissProt database limited to Mammalia. Search parameters were as follows: Mass tolerance was set to 80 ppm, one missed cleavage site was accepted, and carbamidomethylation of cysteine residues as well as oxidation of methionine residues were set as fixed and variable modifications, respectively. To evaluate the protein hits, a significance threshold of p<0.05 was chosen.

For further confirmation of the PMF hits two to five peptides of each identified protein were selected for MS/MS measurements using the WARP feedback mechanism of BioTools. Parent and fragment masses were recorded with 400 and 1000 shots, respectively. Spectra were processed and analyzed as described above with a fragment mass tolerance of 0.7 Da.

### Recombinant expression of full length NVL, CD2BP2 and TJP1 in HEK293 or E. coli NVL-isoform1 (Nuclear valosin-containing protein-like)

The cDNA clone IRAUp969F0866D encoding a partial sequence (amino acid 206-856) of human NVL-isoform1 (SEQ ID NO 1) was obtained from Source BioScience UK Limited. This cDNA clone is coding NVL-isoform3 (Acc. BC012105, SEQ ID NO 2). A synthetic DNA fragment was ordered coding for the missing amino acids 1-205 of NVL-isoform1 from Eurofins Genomics Germany GmbH (Ebersberg). The gene synthesis product (SEQ ID NO 3) was digested with Bsal (R3733, New England Biolabs, Frankfurt am Main, Germany) and the 614 bp fragment was isolated by Gel-purification.

The coding sequence for the amino acid 206-856 of NVL-isoform1 was amplified by PCR using cDNA clone IRAUp969F0866D and DNA oligonucleotide primers sense NVL-IF1 (SEQ ID NO 4) and asense NVL-IF1 (SEQ ID NO 5) resulting in SEQ ID NO 6.

The amplification product was digested with Bsal and together with the gene synthesis fragment ligated with Ncol and Xhol linearized pTriEx-1 vector (Merck, Darmstadt, Germany) resulting in SEQ ID NO 7, coding for NVL[human]-IF1 fused to an octa Histidin-tag (H8) (SEQ ID NO 8) or pET24d vector (Merck) resulting in SEQ ID NO 9, coding for NVL[human]-IF1 fused to a hexa Histidin-tag (H6) (SEQ ID NO 10).

### CD2BP2 (CD2 antigen cytoplasmic tail-binding protein 2)

A cDNA clone coding for full length of human CD2BP2 (SEQ ID NO 11) was not available. Therefore two synthetic gene fragments coding for human CD2BP2 (Acc. BC001947, SEQ ID NO 12) were obtained from Eurofins Genomics Germany GmbH (Ebersberg).

The first fragment coding for amino acids 1-173 of human CD2BP2 and the other one was coding for amino acids 174-341 of human CD2BP2 including the Stop-codon. Both gene synthesis products (SEQ ID NO 13 and SEQ ID NO 14) were digested with Bbsl (R3539, New England Biolabs) resulting in 517 bp fragment-1 and 510 bp fragment-2 (SEQ ID NO 15 and SEQ ID NO 16) that were both Gel-purified. By PCR a third fragment of CD2BP2 was amplified using the gene synthesis product (SEQ ID NO 14) as template and the DNA oligonucleotide primers sense CD2BP2-fragment-2 (SEQ ID NO 17) and asense CD2BP2-fragment-2 (SEQ ID NO 18), resulting in SEQ ID NO 19 without the Stop-codon. The amplification product was digested with Bbsl (R3539, New England Biolabs).

The fragments SEQ ID NO 15 and SEQ ID NO 16 were ligated with Ncol and Xhol linearized pTriEx-1 vector (Merck) resulting in SEQ ID NO 20, coding for CD2BP2human] fused to a C-terminal octa Histidin-tag (H8) (SEQ ID NO 21). The fragment SEQ ID NO 15 and the Bbsl-digested fragment SEQ ID NO 19 were ligated with Ncol and Xhol linearized pET24d vector (Merck) resulting in SEQ ID NO 22, coding for CD2BP2human] fused to a N-terminal octa Histidin-tag (H8) (SEQ ID NO 23).

### TJP1-isoform2 (Tight junction protein ZO-1)

The cDNA clone IRCMp5012C0531D encoding human TJP1-isoform1 (Acc. BC111712) was obtained from Source BioScience UK Limited. For expression of human TJP1-isoform2 (SEQ ID NO 24) two Fragments using DNA oligonucleotide primers sense TJP1-IF2-ZF1 (SEQ ID NO 25) and asense TJP1-IF2-ZF1 (SEQ ID NO 26) as well as sense TJP1-IF2-ZF2 (SEQ ID NO 27) and asense TJP1-IF2-ZF2 (SEQ ID NO 28) were amplified by PCR. One fragment (SEQ ID NO 29) was digested with Bsal (R3733, New England Biolabs) and the other fragment (SEQ ID NO 30) was digested with Esp3I (R0734, New England Biolabs). Both fragments were ligated with Ncol and Xhol linearized pTriEx-1 vector (Merck) resulting in SEQ ID NO 31, coding for human TJP1-isoform2 fused to a C-terminal octa Histidin-tag (H8) (SEQ ID NO 32).

NVL-His and TJP1-His, respectively, were expressed in the human cell line HEK293 after ExGen500-mediated transfection (ThermoFisher Scientific, Dreieich, Germany) according to the manufacturer's instructions (Biomol GmbH, Hamburg, Germany). For the production of immunofluorescence substrates, the cells were grown on cover slides and acetone fixed two days after transfection. For other purposes cells were harvested 5 d after transfection and lysed with NuPage LDS sample buffer (ThermoFisher Scientific, Schwerte, Germany).

### Recombinant expression of NVL, CD2BP2 and TJP1 fragments in E. coli

### NVL

For epitope mapping fragments of human NVL spanning amino acid residues 1 to 117, 106 to 292, 281 to 560 and 549 to 856 were amplified by PCR using SEQ ID NO 7 as template and the DNA oligonucleotide primers sense NVL-F1 (SEQ ID NO 33) and asense NVL-F1 (SEQ ID NO 34) for NVL-fragment 1-117 (SEQ ID NO 35), sense NVL-F2 (SEQ ID NO 36) and asense NVL-F2 (SEQ ID NO 37) for NVL-fragment 106-292 (SEQ ID NO 38), sense NVL-F3 (SEQ ID NO 39) and asense NVL-F3 (SEQ ID NO 40) for NVL-fragment 281-560 (SEQ ID NO 41) and sense NVL-F4 (SEQ ID NO 42) and asense NVL-F4 (SEQ ID NO 43) for NVL-fragment 549-856 (SEQ ID NO 44). The amplification products were digested with Bsal (R3733, New England Biolabs) and ligated with Ncol and Xhol linearized pET24d vector (Merck) resulting in pET24d-N-NVL[human](aa1-117) (SEQ ID NO 45), pET24d-N-NVL[human](aa106-296) (SEQ ID NO 46), pET24d-N-NVL[human](aa281-560) (SEQ ID NO 47) and pET24d-N-NVL[human](aa549-856) (SEQ ID NO 48) coding for four proteins fused to a N-terminal octa Histidin-tag (H8) (SEQ ID NO 49 to SEQ ID NO 52).

### CD2BP2

For epitope mapping fragments of human CD2BP2 spanning amino acid residues 1 to 128, 117 to 260 and 249 to 341 were amplified by PCR using SEQ ID NO 22 as template and the DNA oligonucleotide primers sense CD2BP2-F1 (SEQ ID NO 53) and asense CD2BP2-F1 (SEQ ID NO 54) for CD2BP2-fragment 1-128 (SEQ ID NO 55), sense CD2BP2-F2 (SEQ ID NO 56) and asense CD2BP2-F2 (SEQ ID NO 57) for CD2BP2-fragment 117-260 (SEQ ID NO 58) and sense CD2BP2-F3 (SEQ ID NO 59) and asense CD2BP2-F3 (SEQ ID NO 60) for CD2BP2-fragment 249-341 (SEQ ID NO 61). The amplification products were digested with Esp3I (R0734, New England Biolabs) and ligated with Ncol and Xhol linearized pET24d vector (Merck) resulting in pET24d-N-CD2BP2[human](aa1-128) (SEQ ID NO 62), pET24d-N-CD2BP2[human](aa117-260) (SEQ ID NO 63) and pET24d-N-CD2BP2[human](aa249-341) (SEQ ID NO 64) coding for three proteins fused to a N-terminal octa Histidin-tag (H8) (SEQ ID NO 65 to SEQ ID NO 67).

### TJP1

For epitope mapping fragments of human TJP1 spanning amino acid residues 1 to 575, 1 to 156, 144 to 306 and 294 to 575 were amplified by PCR using SEQ ID NO 31 as template and the DNA oligonucleotide primers sense TJP1-F1/2 (SEQ ID NO 68) and asense TJP1-F1/4 (SEQ ID NO 69) for TJP1-fragment 1-575 (SEQ ID NO 70), sense TJP1-F1/2 (SEQ ID NO 68) and asense TJP1-F2 (SEQ ID NO 71) for TJP1-fragment 1-156 (SEQ ID NO 72), sense TJP1-F3 (SEQ ID NO 73) and asense TJP1-F3 (SEQ ID NO 74) for TJP1-fragment 144-306 (SEQ ID NO 75) and sense TJP1-F4 (SEQ ID NO 76) and asense TJP1-F1/4 (SEQ ID NO 69) for TJP1-fragment 294-575 (SEQ ID NO 77). The amplification products were digested with Bsal (R3733, New England Biolabs) and ligated with Ncol and Xhol linearized pET24d vector (Merck) resulting in pET24d-N-TJP1[human](aa1-575) (SEQ ID NO 78), pET24d-N-TJP1[human](aa1-156) (SEQ ID NO 79), pET24d-N-TJP1[human](aa144-306) (SEQ ID NO 80) and pET24d-N-TJP1[human](aa294-575) (SEQ ID NO 81) coding for four proteins fused to a N-terminal octa Histidin-tag (H8) (SEQ ID NO 82 to SEQ ID NO 85).

Bacterial expression was essentially performed as described in the pET system manual (Merck) employing E. coli strain RosettaBlue(DE3)pLacl (Merck). Protein expression was induced by the addition of 2 mM isopropyl β-D-thiogalactoside (IPTG) for 3 hours at 37°C.

### Protein purification

Bacterial cells were lysed, and proteins were solubilized in 1x PBS pH 7.4, 1 M NaCl, 8 M urea buffer (NVL and TJP1) or 20 mM Tris-HCl pH 8.5, 600 mM NaCl buffer (CD2BP2) and purified by Ni²⁺-affinity chromatography and ion exchange chromatography following the protocol as in C. Probst, W. Schlumberger, W. Stocker et al., "Development of ELISA for the specific determination of autoantibodies against envoplakin and periplakin in paraneoplastic pemphigus," Clinica Chimica Acta, vol. 410, no. 1-2, pp. 13-18, 2009. The proteins were stored in aliquots at -80°C until further use.

### Immunoblot

Purified recombinant antigens (SEQ ID NO 10, 23, 82) in the respective buffer or recombinant antigen fragments (SEQ ID NO 49, 50, 51, 52, 66, 67, 83, 84, 85) expressing *E.coli* or recombinant antigens (SEQ ID NO 32) containing HEK-cell lysates were incubated with NuPage LDS sample buffer (ThermoFisher Scientific, Schwerte, Germany) containing 25 mmol/L dithiothreitol at 70°C for 10 min, followed by SDS-PAGE (NuPAGE, ThermoFisher Scientific). Separated proteins were electrotransferred onto a nitrocellulose membrane by tank blotting with transfer buffer (ThermoFisher Scientific) according to the manufacturer's instructions. The membranes were blocked with Universal Blot Buffer plus (EUROIMMUN Medizinische Labordiagnostika AG, Lübeck, Germany) for 15 min and incubated with the patient or control sera (dilution 1:200) or anti-His (1:2000, 70796-3, Merck) in Universal Blot Buffer plus for 3 h, followed by three washing steps with Universal Blot Buffer (EUROIMMUN Medizinische Labordiagnostika AG, Lübeck, Germany), a second incubation for 30 min with anti-human-IgG-AP (1:10, EUROIMMUN Medizinische Labordiagnostika AG, Lübeck, Germany) or anti-mouse-IgG-AP (1:2000, Jackson immunoresearch, Suffolk, UK), three washing steps, and staining with NBT/BCIP substrate (EUROIMMUN Medizinische Labordiagnostika AG, Lübeck, Germany). Alternatively, monoclonal anti-His-AP antibody produced in mouse (1:8000, A5588, Sigma-Aldrich), was used for a one-step incubation.

### Lineblot

With the Euroline ANA profile (IgG) 23 (DL 1590-5001-23 G, EUROIMMUN Medizinische Labordiagnostika AG, Lübeck, Germany) the 23 most common ANAs were determined (dsDNA, nucleosomes, histones, SS-A, Ro-52, SSB, RNP/Sm, Sm, Mi-2α, Mi-2β, Ku, CENP A, CENP B, Sp100, PML, Scl-70, PM100, PM75, RP11, RP155, gp210, PCNA, DSF70). Briefly, strips were incubated with 1 ml casein sample buffer (EUROIMMUN Medizinische Labordiagnostika AG, Lübeck, Germany) for 5 min and then in 1:101 diluted serum sample for 30 min. Afterwards, each strip was washed three times with 1.5 ml washing buffer for 5 min each time prior to incubation with 1 ml anti-human IgG-AP (1:10, EUROIMMUN Medizinische Labordiagnostika AG, Lübeck, Germany) for 30 min. Strips were washed again as described above and incubated with NBT/BCIP substrate solution (EUROIMMUN Medizinische Labordiagnostika AG, Lübeck, Germany) for 10 min. The reaction was stopped by washing three times with 1 ml distilled water. The strips were analyzed using the software EUROLineScan (EUROIMMUN Medizinische Labordiagnostika AG, Lübeck, Germany).

In addition, recombinant antigens (SEQ ID NO 10, 23, 82) expressed in *E.coli* and purified as described above, where diluted in the respective buffer and spotted onto precut (3 mm x 5 mm) empty Biodyne^{™} B membranes (Biodyne^{™} B Nylon Membrane, 0.45 µm, Thermo Fisher Scientific, Schwerte, Germany). Membranes were dried for 20 min at RT, before patient serum incubation was performed as described above.

### Results

### Characterization of the patients' autoantibodies

The patient sera showed a nucleolar (PS1) or pseudo-dense fine speckled pattern (PS2, PS3) in IFA with HEp2 cells and liver tissue section (**Fig. 1**). In further monospecific analysis with lineblots coated with 23 ANA autoantigens (dsDNA, nucleosomes, histones, SS-A, Ro-52, SSB, RNP/Sm, Sm, Mi-2α, Mi-2β, Ku, CENP A, CENP B, Sp100, PML, Scl-70, PM100, PM75, RP11, RP155, gp210, PCNA, DSF70) the presence of known ANAs was excluded.

### Identification of NVL, CD2BP2 and TJP1 as the target autoantigens

In order to identify the target antigens patient sera were subjected to immunoprecipitation (IP) with HEp-2 cell homogenate. Immunoprecipitated proteins were resolved by SDS-PAGE and stained with blue silver coomassie stain. In IP eluates of patient sera, bands at approximately 100 kDa (PS 1, band 1), 50 kDa (PS2, band 2) and 220 kDa (PS3, band 3) were detected (**Fig. 2**), which were absent in control sera IP eluates. The marked bands were analyzed by mass spectrometry and identified as nuclear valosin-containing protein-like (NVL, band 1), CD2 antigen cytoplasmic tail-binding protein 2 (CD2BP2, band 2), or tight junction protein ZO-1 (TJP1, band 3).

### Verification of NVL, CD2BP2 and TJP1 as the target autoantigens

Human NVL, CD2BP2 and TJP1 aa1-575 were recombinantly expressed in *E.coli.* The purified antigens were applied in immunoblots and tested with an anti-His-tag antibody, the individual patient serum and healthy control sera (n=15). As it is shown in **Fig. 3 A-C**, the anti-His-tag antibody and the patient sera showed positive reactions with the recombinant antigens, whereas the fifteen healthy control sera showed no comparable reactivity. A binding of PS3 but not control serum to full length TJP1 could also be detected (**Fig. 3** **D**). In addition, IFA with HEK293 cells expressing recombinant NVL or TJP1, confirmed the presence of autoantibodies against these proteins in the respective patient serum (**Fig. 4**). The purified antigens were further applied in a neutralization test to examine their ability to abolish the IFA ANA pattern of the patient sera. PS1-3 were incubated with the individual antigens or control buffer prior to standard IFA on HEp-2 cells. Figure 4 shows that preincubation of PS1 with NVL and PS2 with CD2BP2 led to a complete or partial reduction of the ANA pattern on HEp-2 cells. These results confirmed that the respective ANA pattern of PS1 and 2 were indeed caused by an autoantibody targeting NVL or CD2BP2. However, after incubation of PS3 with purified TJP1 aa1-575 the same fluorescence signal was observed compared to the control incubation. This indicates that anti-TJP1 autoantibodies present in PS3 do not cause the observed ANA pattern.

To further confirm this, PS3 and a commercial anti-TJP1 antibody were incubated with HEp-2 cells, recombinant HEK-TJP1 cells and HEK-control cells. As **Fig. 6** **A** illustrates, the commercial anti-TJP1 antibody showed a diffused cytoplasmic stain in HEp-2 cells, which was also observed in IFA with PS3. The ANA pattern observed in IFA with PS3 was therefore probably not caused by anti-TJP1 autoantibodies. However, the commercial anti-TJP1 antibody showed the same specific immunoreaction against transfected HEK293 cells expressing TJP1 as PS3 (**Fig. 6** **B**) and a specific reaction at junctions between HEK-control cells (**Fig. 6** **C**). The magnification of the selected area (**Fig. 6** **D**) shows that autoantibodies from PS3 also localized in the area of intercellular contacts, indicating that PS3 recognizes, besides of an unknown nuclear antigen, endogenous TJP1 at cell junctions.

### Characterization of the epitopes recognized by anti-NVL, anti-CD2BP2 and anti-TJP1 autoantibodies

In Western Blot with E.coli lysates containing NVL fragments (aa1-117, aa106-292, aa281-560, aa549-856, **Fig. 7** **A**), CD2BP2 fragments (aa117-260, aa249-341, **Fig. 7** **B**) or TJP1 fragments (aa1-156, aa145-306, aa295-575, **Fig. 7** **C**) PS1 or PS2 showed the strongest reactivity with the respective C-terminal fragment of NVL or CD2BP2, indicating that patient's autoantibodies recognize an epitope located in the last 308 aa of NVL or the last 93 aa of CD2BP2. PS3 reacted predominantly with the N-terminal fragment of TJP1, indicating that the epitope is located in the first 156 aa of TJP1.

Further results (data not shown) indicated that one or more epitopes recognized by anti-NVL is or are located on one, two, three, four, five, six, seven or all C-terminal fragments according to SEQ ID NO86, SEQ ID NO87, SEQ ID NO88, SEQ ID NO89, SEQ ID NO90, SEQ ID NO91, SEQ ID NO92 and SEQ ID NO93.

### Validation of the clinical association of anti-NVL, anti-CD2BP2 and anti-TJP1 autoantibodies

For the assessment of the clinical association of anti-NVL, anti-CD2BP2 and anti-TJP1 autoantibodies, a lineblot coated with purified recombinant NVL, CD2BP2 and TJP1 aa1-575 was developed and validated with anti-His-tag-AP, PS1-3 and healthy control sera (n=50) (**Tab. 1**), before clinical characterized patient collectives were analyzed. PS1-3 showed positive reactions against the corresponding antigens, whereas 50 healthy control sera did not. Following this, 529 sera from 488 patients with systemic rheumatic diseases (SARD cohort) were analyzed. Six sera from four SARD patients were anti-NVL, ten patients were anti-CD2BP2 and 16 patients were anti-TJP1 positive (**Tab. 1**). Western blot analysis with purified NVL, CD2BP2 or TJP1 aa1-575 confirmed all positive reactions (**Fig. 8**). All anti-NVL positive patients, as well as eight anti-CD2BP2 positive and 15 anti-TJP1 positive patients were diagnosed with SSc. One anti-CD2BP2 positive patient had MCTD and one had myositis. In addition, one anti-TJP1 positive patient suffered on UCTD.

In **Tab. 1** summarized data are shown. Lineblot with purified NVL, CD2BP2 and TJP1 aa1-575 have been incubated with anti-His-tag-AP, PS1-3, healthy control sera (n=50) and sera from patients with systemic rheumatic diseases (SARD cohort, n=529). Band intensities of all positive reactions and three representative negative control reactions (CS1-3) are shown as well as the diagnosis of anti-NVL, anti-CD2BP2 and anti-TJP1 positive patient sera.

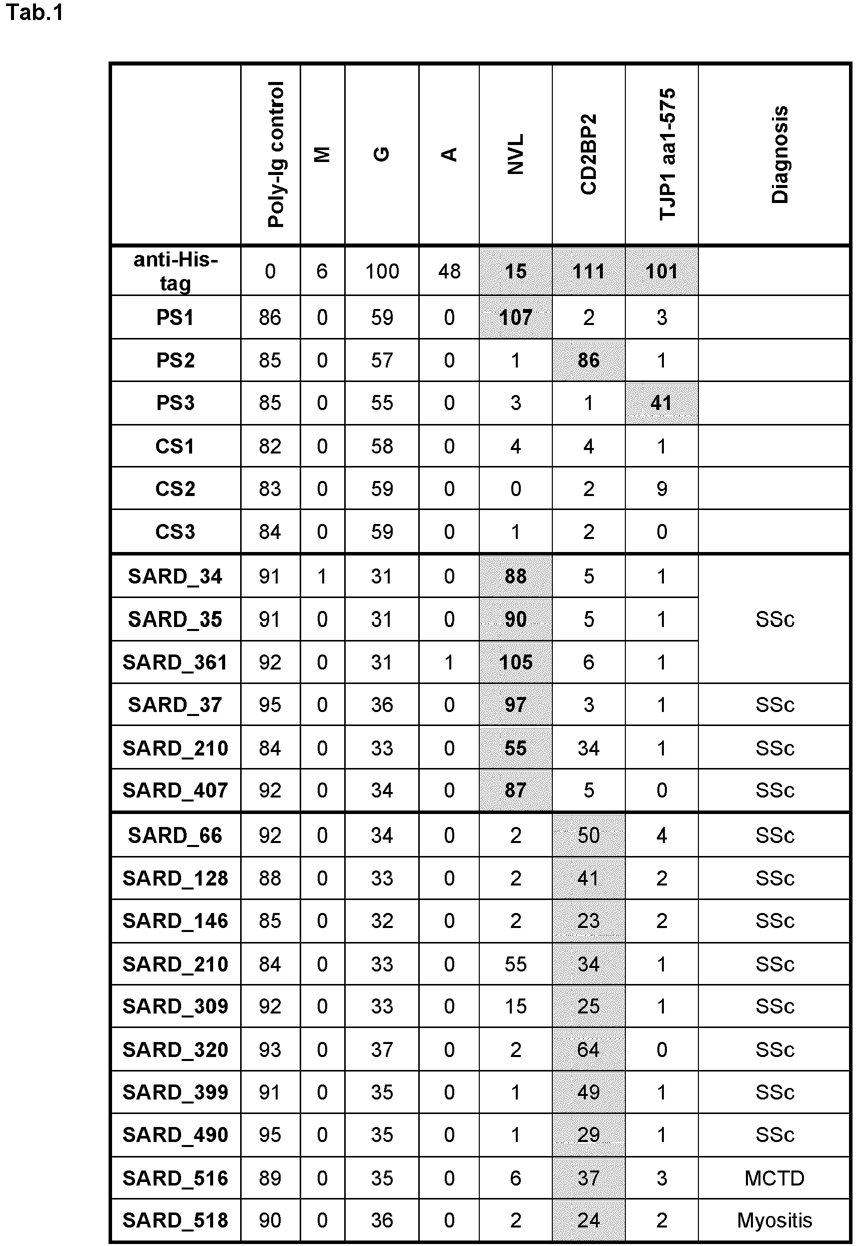

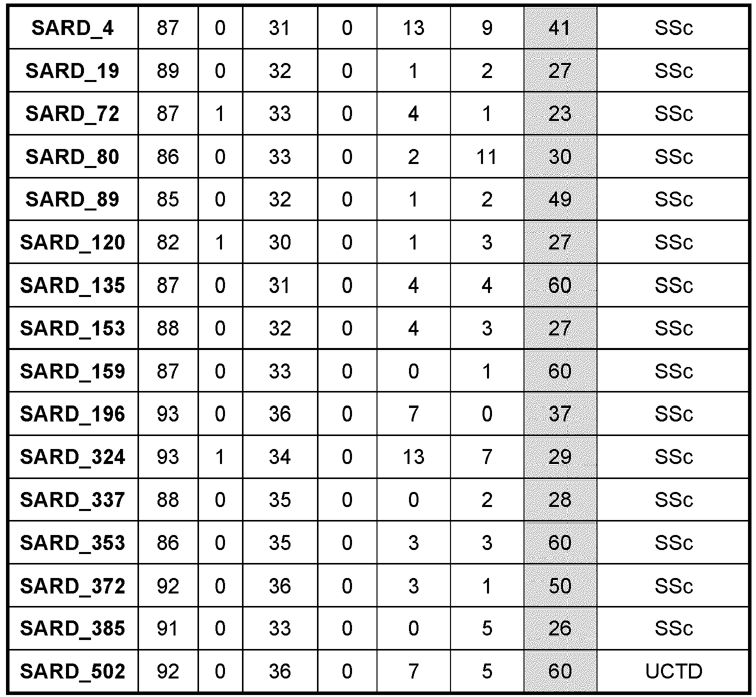

### Example 2:

### Supplement: Assessment of clinical association of anti-NVL autoantibodies with larger cohort

The clinical association of anti-NVL autoantibodies was further studied with additional control collectives including healthy donor sera (n=100) and sera from 205 diseased control patients (autoimmune hepatitis (AIH) n = 40, rheumatoid arthritis (RA) n = 30; systemic lupus erythematosis (SLE) n = 135).

For the supplementary study, the additional sera were analyzed with a lineblot coated with purified recombinant NVL as described in Example 1. None sera from the additional control collective showed a positive reaction.

Taken data from Example 1 and Example 2 together, only sera from PS1 and six sera of four SSc patients showed positive reaction against recombinant NVL, which were confirmed with Western blot analysis with purified recombinant NVL. No reaction was detected with 150 healthy control sera and 315 diseased control sera.

## Claims

1. A method for detecting in a sample, preferably from a patient, an autoantibody binding to a polypeptide having the sequence SEQ ID NO1, SEQ ID NO11 or SEQ ID NO24 or a variant thereof.

2. A polypeptide having the sequence SEQ ID NO1, SEQ ID NO11 or SEQ ID NO24 or a variant thereof, wherein the polypeptide is preferably immobilized, more preferably on a solid carrier, and/or wherein the polypeptide is a recombinant protein, preferably a purified recombinant protein.

3. A carrier, preferably a solid carrier, comprising the polypeptide according to claim 2, wherein preferably the polypeptide is immobilized on the carrier, and/or wherein the polypeptide is preferably a recombinant protein, more preferably a purified recombinant protein.

4. An autoantibody, preferably an isolated autoantibody, binding to a polypeptide having the sequence SEQ ID NO1, SEQ ID NO11 or SEQ ID NO24 or a variant thereof, wherein the autoantibody, preferably the isolated autoantibody, is preferably in complex with the polypeptide according to claim 2.

5. Autoantibody according to claim 4 wherein the autoantibody, preferably the isolated autoantibody, is diluted in a buffer solution or is included in a diluted sample, preferably patient sample and/or wherein the autoantibody, preferably the isolated autoantibody, is in solution with one or more, preferably all reagents from the group comprising stabilizers, preferably a set of stabilizers, washing buffer, antidegradants.

6. A use of the autoantibody according to claim 4 or 5 for the diagnosis of a disease preferably of a systemic autoimmune rheumatic disease, more preferably of systemic sclerosis.

7. A method for isolating an autoantibody binding to a polypeptide having the sequence SEQ ID NO1, SEQ ID NO11 or SEQ ID NO24 or a variant thereof, comprising the steps
a) contacting a sample comprising the autoantibody with a polypeptide according to claim 2 under conditions compatible with formation of a complex, wherein said autoantibody binds to said polypeptide,
b) isolating the complex formed in step a),
c) dissociating the complex isolated in step b) and
d) separating the autoantibody from the polypeptide.

8. A kit comprising the polypeptide according to claim 2,
wherein preferably the kit comprises in addition a means for detecting a complex comprising the polypeptide according to claim 2 and an antibody binding to said polypeptide.

9. A kit comprising
a) the carrier according to claim 3 and a means for detecting an autoantibody bound to the carrier, which means is preferably a secondary antibody, or a polypeptide having the sequence SEQ ID NO1, SEQ ID NO11 or SEQ ID NO24 or a variant thereof,
or
b) a diagnostically useful carrier comprising a means for capturing an autoantibody in a liquid solution, preferably a non-labeled secondary antibody, and a means for detecting an autoantibody bound to the carrier, preferably a polypeptide having the sequence SEQ ID NO1, SEQ ID NO11 or SEQ ID NO24 or a variant thereof, more preferably with a detectable label,
wherein the kit preferably comprises in addition to a) or b) one or more, preferably all reagents from the group comprising calibrators, preferably a set of calibrators, washing buffer, a positive control and a negative control.

10. A use of a polypeptide according to claim 2 or a carrier according to claim 3 or an autoantibody according to claim 4 or 5 or an antibody to a polypeptide having the sequence SEQ ID NO1, SEQ ID NO11 or SEQ ID NO24 or a variant thereof, for the manufacture of a kit, medical device, preferably diagnostic device, preferably for the diagnosis of a disease, more preferably for the diagnosis of a systemic autoimmune rheumatic disease, even more preferably for the diagnosis of systemic sclerosis.

11. The method or autoantibody according to any of claims 1, 5 and 7, wherein the sample is a bodily fluid comprising antibodies, preferably selected from the group comprising whole blood, plasma, serum, cerebrospinal fluid and saliva.

12. The method, use or kit according to any of claims 1 and 6 to 11 wherein the autoantibody or complex is detected using a method selected from the group comprising immunodiffusion techniques, immunoelectrophoretic techniques, light scattering immunoassays, agglutination techniques, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

13. A method comprising the step contacting a medical or diagnostic device comprising a polypeptide having the sequence SEQ ID NO1, SEQ ID NO11 or SEQ ID NO24 or a variant thereof with a buffered solution comprising an antibody binding specifically to said polypeptide having the sequence SEQ ID NO1, SEQ ID NO11 or SEQ ID NO24 or a variant thereof, which solution is not a sample from a patient in need of a diagnosis comprising an unknown concentration of said antibody, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgG class antibodies,
followed by detection of a signal which indicates whether or not the antibody has bound to the polypeptide, optionally a signal relating to the concentration of the antibody in the solution or solutions.

14. Use of a sample, preferably a patient sample, which is preferably diluted, comprising an autoantibody according to claim 4 or 5 as a positive control, preferably for the diagnosis of a disease, more preferably for the diagnosis of a systemic autoimmune rheumatic disease, even more preferably for the diagnosis of systemic sclerosis.

15. Diluted sample comprising an autoantibody according to claim 4, 5, 10 or 11 wherein the sample is preferably diluted in an aqueous buffer solution and/or wherein the diluted sample comprises in addition one or more, preferably all reagents from the group comprising calibrators, preferably a set of calibrators, a stabilizer, washing buffer, a positive control and a negative control.
